(19) Europäisches Patentamt
European Patent Office
Office européen des brevets

(11) EP 1 544 200 A1

(12) EUROPEAN PATENT APPLICATION
published in accordance with Art. 158(3) EPC

(43) Date of publication:
**22.06.2005 Bulletin 2005/25**

(21) Application number: **03753943.4**

(22) Date of filing: **24.09.2003**

(51) Int Cl.[7]: **C07D 487/04**, A61K 31/519, A61K 31/5377, A61K 31/55, A61K 31/553, A61P 9/00, A61P 9/10, A61P 21/00, A61P 25/00, A61P 25/14, A61P 25/16, A61P 25/20, A61P 25/24, A61P 25/28, A61P 43/00

(86) International application number:
**PCT/JP2003/012158**

(87) International publication number:
**WO 2004/029056 (08.04.2004 Gazette 2004/15)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **24.09.2002 JP 2002276896**
**19.05.2003 JP 2003139994**

(71) Applicant: **KYOWA HAKKO KOGYO CO., LTD.**
**Chiyoda-ku, Tokyo 100-8185 (JP)**

(72) Inventors:
- **IIDA, Kyoichiro, Kyowa Hakko Kogyo Co., Ltd.**
  **Sunto-gun, Shizuoka 411-8 (JP)**
- **SUGITA, Takamasa,**
  **Kyowa Hakko Kogyo Co., Ltd.**
  **Sunto-gun, Shizuoka 411-8 (JP)**
- **SHIOZAKI, Shizuo,**
  **Kyowa Hakko Kogyo Co., Ltd.**
  **Sunto-gun, Shizuoka 411-8 (JP)**
- **KANDA, Tomoyuki,**
  **Kyowa Hakko Kogyo Co., Ltd.**
  **Sunto-gun, Shizuoka 411-8 (JP)**
- **KUWANA, Yoshihisa,**
  **Kyowa Hakko Kogy Co., Ltd.**
  **Tokyo 100-8185 (JP)**
- **SHIMADA, Junichi,**
  **Kyowa Hakko Kogyo Co., Ltd.**
  **Sunto-gun, Shizuoka 411-8 (JP)**

(74) Representative: **VOSSIUS & PARTNER**
**Siebertstrasse 4**
**81675 München (DE)**

(54) **1,2,4 -TRIAZOLO 1,5-c PYRIMIDINE DERIVATIVE**

(57) The present invention provides [1,2,4]triazolo [1,5-c]pyrimidine derivatives or pharmaceutically acceptable salts thereof which have adenosine $A_{2A}$ receptor antagonism and are useful for treating and/or preventing a disease induced by hyperactivity of an adenosine $A_{2A}$ receptor, the derivatives being represented by formula (I):

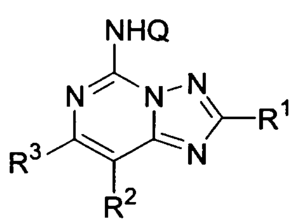

(I)

(wherein $R^1$ represents substituted or unsubstituted aryl or a substituted or unsubstituted aromatic heterocyclic group; $R^2$ represents a hydrogen atom, halogen, lower alkyl, lower alkanoyl, aroyl, substituted or unsubstituted aryl, or a substituted or unsubstituted aromatic heterocyclic group; $R^3$ represents lower alkyl, lower cycloalkyl, substituted or unsubstituted lower alkanoyl, substituted or unsubstituted aryl, or a substituted or unsubstituted aromatic heterocyclic group; and Q represents a hydrogen atom or 3,4-dimethoxybenzyl).

EP 1 544 200 A1

## Description

### Technical Field

The present invention relates to [1,2,4]triazolo[1,5-c]pyrimidine derivatives or pharmaceutically acceptable salts thereof which have adenosine $A_{2A}$ receptor antagonism and are useful for treating and/or preventing a disease induced by hyperactivity of an adenosine $A_{2A}$ receptor (for example, Parkinson's disease, dementia including senile dementia, depression or the like).

### Background Art

It is known that adenosine shows an inhibitory action against the liberation of a neurotransmitter via an $A_{2A}$ receptor (European Journal of Pharmacology (Netherlands), vol. 168, pp. 285-290 (1989)). Therefore, adenosine $A_{2A}$ receptor antagonists are expected as a therapeutic agent or a preventive agent for a disease induced by hyperactivity of adenosine $A_{2A}$ receptors, such as a remedy for Parkinson's disease, an anti-dementia drug or a remedy for depression. Furthermore, adenosine $A_{2A}$ receptor antagonists are expected to show a therapeutic effect, a symptom improving effect or the like, for example, on Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease, intermittent claudications or the like.

On the other hand, [1,2,4]triazolo[1,5-c]pyrimidine derivatives are disclosed as compounds having a diuretic action (Japanese Published Unexamined Patent Application No. 13792/85), compounds having an antiasthmatic action (Japanese Published Unexamined Patent Application No. 56983/85), compounds having a bronchodilative action (Japanese Published Unexamined Patent Application No. 167592/84), compounds having a Syk tyrosine kinase inhibitory action (WO01/17999), and compounds having adenosine $A_{2A}$ receptor antagonism (WO00/17201, WO98/42711, WO03/022283, WO03/048163 and WO03/048164). And also, 5-aminotriazolopyridine derivatives are disclosed as compounds having adenosine receptor antagonism (Japanese Published Unexamined Patent Application No. 302667/2001).

### Disclosure of the Invention

An object of the present invention is to provide [1,2,4]triazolo[1,5-c]pyrimidine derivatives which have adenosine $A_{2A}$ receptor antagonism and are useful for treating and/or preventing a disease (for example, Parkinson's disease, dementia including senile dementia, depression or the like) induced by hyperactivity of adenosine $A_{2A}$ receptors.

The present invention relates to the following (1) to (37):

(1) A [1,2,4]triazolo[1,5-c]pyrimidine derivative represented by formula (I):

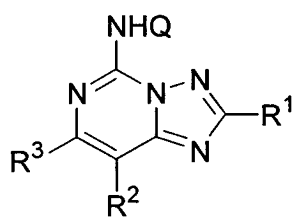

(I)

{wherein
$R^1$ represents substituted or unsubstituted aryl, or a substituted or unsubstituted aromatic heterocyclic group;
$R^2$ represents a hydrogen atom, halogen, lower alkyl, lower alkanoyl, aroyl, substituted or unsubstituted aryl, or a substituted or unsubstituted aromatic heterocyclic group;
$R^3$ represents the following:

1) lower alkyl or hydroxy-substituted lower alkyl;
2) lower cycloalkyl;
3) formyl;
4) substituted or unsubstituted lower alkanoyl;
5) substituted or unsubstituted aroyl;
6) formula ($A^3$)

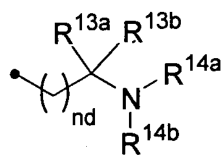

**($A^3$)**

[wherein

nd represents an integer of 0 to 3;

$R^{13a}$ and $R^{13b}$ may be the same or different and each represent a hydrogen atom, halogen, lower alkyl, lower cycloalkyl, substituted or unsubstituted lower alkanoyl, substituted or unsubstituted aralkyl, substituted or unsubstituted aryl, a substituted or unsubstituted aromatic heterocyclic group, substituted or unsubstituted aroyl, lower alkoxycarbonyl, or lower alkoxy-substituted lower alkyl; $R^{13a}$ and $R^{13b}$ form a lower cycloalkane ring together with the adjacent carbon atom; or $R^{13a}$ and $R^{13b}$ are combined together to represent an oxygen atom or a sulfur atom; and

$R^{14a}$ and $R^{14b}$ may be the same or different and each represent a hydrogen atom, substituted or unsubstituted lower alkyl, lower cycloalkyl, substituted or unsubstituted lower alkanoyl, substituted or unsubstituted aryl, a substituted or unsubstituted aromatic heterocyclic group, substituted or unsubstituted aroyl, lower alkoxycarbonyl, formyl, or formula ($B^1$)

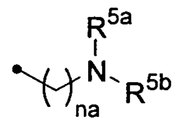

**($B^1$)**

(wherein

na represents an integer of 2 to 5; and

$R^{5a}$ and $R^{5b}$ may be the same or different and each represent a hydrogen atom, lower alkyl, lower cycloalkyl, substituted or unsubstituted lower alkanoyl, substituted or unsubstituted aralkyl, substituted or unsubstituted aryl, a substituted or unsubstituted aromatic heterocyclic group, substituted or unsubstituted aroyl, lower alkoxycarbonyl, lower alkoxy-substituted lower alkyl, or formyl; or $R^{5a}$ and $R^{5b}$ form a substituted or unsubstituted heterocyclic group together with the adjacent nitrogen atom); or

$R^{14a}$ and $R^{14b}$ form a substituted or unsubstituted heterocyclic group together with the adjacent nitrogen atom];

7) formula ($C^3$)

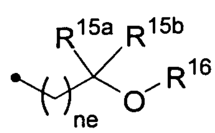

**($C^3$)**

(wherein

ne, $R^{15a}$ and $R^{15b}$ have the same meanings as the above-described nd, $R^{13a}$ and $R^{13b}$, respectively; and

$R^{16}$ represents a hydrogen atom, lower alkyl, lower cycloalkyl, substituted or unsubstituted lower alkanoyl, substituted or unsubstituted aralkyl, substituted or unsubstituted aryl, a substituted or unsubstituted aromatic heterocyclic group, substituted or unsubstituted aroyl, or lower alkoxy-substituted lower alkyl);

8) formula ($E^1$)

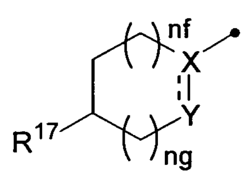

**($E^1$)**

[wherein

nf represents an integer of 0 to 3;

ng represents an integer of 1 to 4;

X---Y represents $CR^{18}$-$CH_2$ (wherein $R^{18}$ represents a hydrogen atom, hydroxy, halogen, nitro, cyano, trifluoromethyl, lower alkyl, lower alkoxy, lower alkanoyl, or lower alkoxycarbonyl), or C=CH; and

$R^{17}$ represents a hydrogen atom, substituted or unsubstituted lower alkyl, substituted or unsubstituted lower alkanoyl, substituted or unsubstituted lower cycloalkyl, substituted or unsubstituted aryl, a substituted or unsubstituted aromatic heterocyclic group, substituted or unsubstituted aroyl, lower alkoxycarbonyl, or formyl];

9) formula ($F^1$)

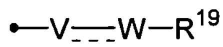

**($F^1$)**

[wherein

V---W represents $CR^{20}$=$CR^{21}$ (wherein $R^{20}$ and $R^{21}$ may be the same or different and each represent a hydrogen atom, substituted or unsubstituted lower alkyl, substituted or unsubstituted lower alkanoyl, substituted or unsubstituted lower cycloalkyl, substituted or unsubstituted aryl, a substituted or unsubstituted aromatic heterocyclic group, substituted or unsubstituted aroyl, or lower alkoxycarbonyl) or $C\equiv C$; and

$R^{19}$ represents a hydrogen atom, substituted or unsubstituted lower alkyl, substituted or unsubstituted lower alkanoyl, substituted or unsubstituted lower cycloalkyl, substituted or unsubstituted aryl, a substituted or unsubstituted aromatic heterocyclic group, substituted or unsubstituted aroyl, lower alkoxycarbonyl, or formula ($A^4$)

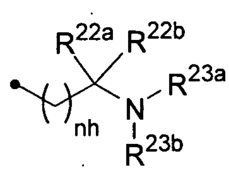

**($A^4$)**

(wherein

nh, $R^{22a}$, $R^{22b}$, $R^{23a}$ and $R^{23b}$ have the same meanings as the above-described nd, $R^{13a}$, $R^{13b}$, $R^{14a}$ and $R^{14b}$, respectively), provided that $R^{19}$ is not substituted or unsubstituted aryl when V---W is CH=CH];

10) aryl substituted with a substituent selected from the

group consisting of

-$CH_2NHR^{4a}$ [wherein $R^{4a}$ represents substituted or unsubstituted lower alkyl, lower cycloalkyl, substituted or unsubstituted lower alkanoyl, substituted or unsubstituted aryl, a substituted or unsubstituted aromatic heterocyclic group, substituted or unsubstituted aroyl, lower alkoxycarbonyl, formyl, or formula ($B^1$)

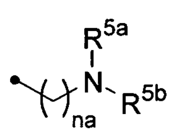

**($B^1$)**

(wherein na, $R^{5a}$ and $R^{5b}$ have the same meanings as defined above, respectively)],

-$(CH_2)_{nb}$-C($R^{6a}$)($R^{6b}$)($OR^7$) (wherein nb, $R^{6a}$, $R^{6b}$ and $R^7$ have the same meanings as the above-described nd, $R^{13a}$, $R^{13b}$ and $R^{16}$, respectively), and

-$NR^{8a}R^{8b}$ [wherein $R^{8b}$ and $R^{8b}$ may be the same or different and each represent a hydrogen atom, substituted or unsubstituted lower alkyl, lower cycloalkyl, substituted or unsubstituted lower alkanoyl, substituted or unsubstituted aryl, a substituted or unsubstituted aromatic heterocyclic group, substituted or unsubstituted aroyl, lower alkoxycarbonyl, formyl, or formula ($B^1$)

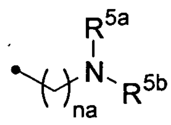

(B^1^)

(wherein na, $R^{5a}$ and $R^{5b}$ have the same meanings as defined above, respectively)]; or

11) an aromatic heterocyclic group substituted with a substituent selected from the group consisting of

$-CH_2NR^{4b}R^{4c}$ (wherein $R^{4b}$ and $R^{4c}$ have the same meanings as the above-described $R^{14a}$ and $R^{14b}$, respectively),

$-(CH_2)_{nb}-C(R^{6a})(R^{6b})(OR^7)$ (wherein nb, $R^{6a}$, $R^{6b}$ and $R^7$ have the same meanings as defined above, respectively), and

$-NR^{8a}R^{8b}$ (wherein $R^{8a}$ and $R^{8b}$ have the same meanings as defined above, respectively); and

Q represents a hydrogen atom or 3,4-dimethoxybenzyl}, or a pharmaceutically acceptable salt thereof.

(2) The [1,2,4]triazolo[1,5-c]pyrimidine derivative according to the above (1), wherein $R^3$ is the following:

1) lower alkyl or hydroxy-substituted lower alkyl;
2) lower cycloalkyl;
3) formyl;
4) substituted or unsubstituted lower alkanoyl;
5) substituted or unsubstituted aroyl;
6) formula ($A^3$)

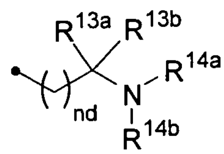

($A^3$)

(wherein nd, $R^{13a}$, $R^{13b}$, $R^{14a}$ and $R^{14b}$ have the same meanings as defined above, respectively);

7) formula ($C^3$)

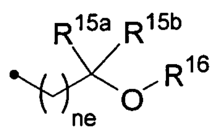

($C^3$)

(wherein ne, $R^{15a}$, $R^{15b}$ and $R^{16}$ have the same meanings as defined above, respectively);

8) formula ($E^1$)

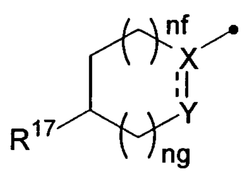

($E^1$)

(wherein nf, ng, X---Y and $R^{17}$ have the same meanings as defined above, respectively); or

9) formula ($F^1$)

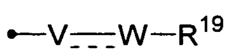

($F^1$)

(wherein V---W and $R^{19}$ have the same meanings as defined above, respectively),

or a pharmaceutically acceptable salt thereof.

(3) The [1,2,4]triazolo[1,5-c]pyrimidine derivative according to the above (1), wherein $R^3$ is aryl substituted with a substituent selected from the group consisting of $-CH_2NHR^{4a}$ (wherein $R^{4a}$ has the same meaning as defined above), $-(CH_2)_{nb}-C(R^{6a})(R^{6b})(OR^7)$ (wherein nb, $R^{6a}$, $R^{6b}$ and $R^7$ have the same meanings as defined above, respectively), and $-NR^{8a}R^{8b}$ (wherein $R^{8b}$ and $R^{8b}$ have the same meanings as defined above, respectively), or a pharmaceutically acceptable salt thereof.

(4) The [1,2,4]triazolo[1,5-c]pyrimidine derivative according to the above (1), wherein $R^3$ is aryl substituted with $-CH_2NHR^{4a}$ (wherein $R^{4a}$ has the same meaning as defined above), or a pharmaceutically acceptable salt thereof.

(5) The [1,2,4]triazolo[1,5-c]pyrimidine derivative according to the above (3) or (4), wherein the aryl is phenyl, or a pharmaceutically acceptable salt thereof.

(6) The [1,2,4]triazolo[1,5-c]pyrimidine derivative according to the above (1), wherein $R^3$ is an aromatic heterocyclic group substituted with a substituent selected from the group consisting of $-CH_2NR^{4b}R^{4c}$ (wherein $R^{4b}$ and $R^{4c}$ have the same meanings as defined above, respectively), $-(CH_2)_{nb}-C(R^{6a})(R^{6b})(OR^7)$ (wherein nb, $R^{6a}$, $R^{6b}$ and $R^7$ have the same meanings as defined above, respectively), and $-NR^{8a}R^{8b}$ (wherein $R^{8b}$ and $R^{8b}$ have the same meanings as defined above, respectively), or a pharmaceutically acceptable salt thereof.

(7) The [1,2,4]triazolo[1,5-c]pyrimidine derivative according to the above (1), wherein $R^3$ is an aromatic heterocyclic group substituted with $-(CH_2)_{nb}-C(R^{6a})(R^{6b})(OR^7)$ (wherein nb, $R^{6a}$, $R^{6b}$ and $R^7$ have the same meanings as defined above, respectively), or a pharmaceutically acceptable salt thereof.

(8) The [1,2,4]triazolo[1,5-c]pyrimidine derivative according to the above (1), wherein $R^3$ is an aromatic heterocyclic group substituted with $-NR^{8a}R^{8b}$ (wherein $R^{8b}$ and $R^{8b}$ have the same meanings as defined above, respectively), or a pharmaceutically acceptable salt thereof.

(9) The [1,2,4]triazolo[1,5-c]pyrimidine derivative according to any one of the above (6) to (8), wherein the aromatic heterocyclic group is pyridyl or thiazolyl, or a pharmaceutically acceptable salt thereof.

(10) The [1,2,4]triazolo[1,5-c]pyrimidine derivative according to the above (1), wherein $R^3$ is formula ($C^3$)

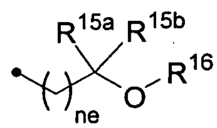

**($C^3$)**

(wherein ne, $R^{15a}$, $R^{15b}$ and $R^{16}$ have the same meanings as defined above, respectively), or a pharmaceutically acceptable salt thereof.

(11) The [1,2,4]triazolo[1,5-c]pyrimidine derivative according to the above (1), wherein $R^3$ is $-CH_2OR^{16}$ (wherein $R^{16}$ has the same meaning as defined above), or a pharmaceutically acceptable salt thereof.

(12) The [1,2,4]triazolo[1,5-c]pyrimidine derivative according to the above (1), wherein $R^3$ is formula ($E^1$)

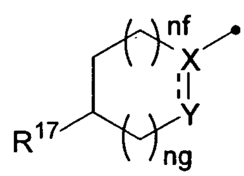

**($E^1$)**

(wherein nf, ng, X---- Y and $R^{17}$ have the same meanings as defined above, respectively), or a pharmaceutically acceptable salt thereof.

(13) The [1,2,4]triazolo[1,5-c]pyrimidine derivative according to the above (12), wherein nf is 1, ng is 1, and X---- Y is C=CH, or a pharmaceutically acceptable salt thereof.

(14) The [1,2,4]triazolo[1,5-c]pyrimidine derivative according to the above (12) or (13), wherein $R^{17}$ is substituted or unsubstituted lower alkyl, or a pharmaceutically acceptable salt thereof.

(15) The [1,2,4]triazolo[1,5-c]pyrimidine derivative according to the above (1), wherein $R^3$ is formula ($F^1$)

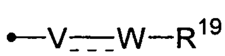

**($F^1$)**

(wherein V---- W and $R^{19}$ have the same meanings as defined above, respectively), or a pharmaceutically acceptable salt thereof.

(16) The [1,2,4]triazolo[1,5-c]pyrimidine derivative according to the above (1), wherein $R^3$ is formula ($A^3$)

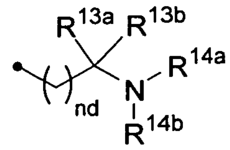

($A^3$)

(wherein nd, $R^{13a}$, $R^{13b}$, $R^{14a}$ and $R^{14b}$ have the same meanings as defined above, respectively), or a pharmaceutically acceptable salt thereof.

(17) The [1,2,4]triazolo[1,5-c]pyrimidine derivative according to the above (16), wherein nd is 0, and $R^{13a}$ and $R^{13b}$ are combined together to represent an oxygen atom, or a pharmaceutically acceptable salt thereof.

(18) The [1,2,4]triazolo[1,5-c]pyrimidine derivative according to the above (16), wherein nd is 0, and $R^{13a}$ and $R^{13b}$ are each a hydrogen atom, or a pharmaceutically acceptable salt thereof.

(19) The [1,2,4]triazolo[1,5-c]pyrimidine derivative according to any one of the above (16) to (18), wherein $R^{14a}$ and $R^{14b}$ may be the same or different and are each a hydrogen atom or substituted or unsubstituted lower alkyl, or a pharmaceutically acceptable salt thereof.

(20) The [1,2,4]triazolo[1,5-c]pyrimidine derivative according to any one of the above (16) to (18), wherein $R^{14a}$ and $R^{14b}$ form a substituted or unsubstituted heterocyclic group together with the adjacent nitrogen atom, or a pharmaceutically acceptable salt thereof.

(21) The [1,2,4]triazolo[1,5-c]pyrimidine derivative according to the above (1), wherein $R^3$ is formyl, substituted or unsubstituted lower alkanoyl, or substituted or unsubstituted aroyl, or a pharmaceutically acceptable salt thereof.

(22) The [1,2,4]triazolo[1,5-c]pyrimidine derivative according to any one of the above (1) to (21), wherein Q is a hydrogen atom, or a pharmaceutically acceptable salt thereof.

(23) The [1,2,4]triazolo[1,5-c]pyrimidine derivative according to any one of the above (1) to (22), wherein $R^1$ is furyl, or a pharmaceutically acceptable salt thereof.

(24) The [1,2,4]triazolo[1,5-c]pyrimidine derivative according to any one of the above (1) to (23), wherein $R^2$ is a hydrogen atom, or a pharmaceutically acceptable salt thereof.

(25) A pharmaceutical composition comprising the [1,2,4]triazolo[1,5-c]pyrimidine derivative according to any one of the above (1) to (24) or a pharmaceutically acceptable salt thereof as an active ingredient.

(26) A therapeutic agent for Parkinson's disease comprising the [1,2,4]triazolo[1,5-c]pyrimidine derivative according to any one of the above (1) to (24) or a pharmaceutically acceptable salt thereof as an active ingredient.

(27) A therapeutic agent for depression comprising the [1,2,4]triazolo[1,5-c]pyrimidine derivative according to any one of the above (1) to (24) or a pharmaceutically acceptable salt thereof as an active ingredient.

(28) A therapeutic and/or preventive agent for a disease induced by hyperactivity of an adenosine $A_{2A}$ receptor, comprising the [1,2,4]triazolo[1,5-c]pyrimidine derivative according to any one of the above (1) to (24) or a pharmaceutically acceptable salt thereof as an active ingredient.

(29) Use of the [1,2,4]triazolo[1,5-c]pyrimidine derivative according to any one of the above (1) to (24) or a pharmaceutically acceptable salt thereof for the manufacture of a therapeutic agent for Parkinson's disease.

(30) Use of the [1,2,4]triazolo[1,5-c]pyrimidine derivative according to any one of the above (1) to (24) or a pharmaceutically acceptable salt thereof for the manufacture of a therapeutic agent for depression.

(31) Use of the [1,2,4]triazolo[1,5-c]pyrimidine derivative according to any one of the above (1) to (24) or a pharmaceutically acceptable salt thereof for the manufacture of a therapeutic and/or preventive agent for a disease induced by hyperactivity of an adenosine $A_{2A}$ receptor.

(32) A therapeutic agent for a disease selected frpm the group consisting of Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease and intermittent claudications, comprising the [1,2,4]triazolo[1,5-c]pyrimidine derivative according to any one of the above (1) to (24) or a pharmaceutically acceptable salt thereof as an active ingredient.

(33) Use of the [1,2,4]triazolo[1,5-c]pyrimidine derivative according to any one of the above (1) to (24) or a pharmaceutically acceptable salt thereof for the manufacture of a therapeutic agent for a disease selected from the group consisting of Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease and intermittent claudications.

(34) A method for treating Parkinson's disease, comprising administering an effective amount of the [1,2,4]triazolo [1,5-c]pyrimidine derivative according to any one of the above (1) to (24) or a pharmaceutically acceptable salt

thereof.

(35) A method for treating depression, comprising administering an effective amount of the [1,2,4]triazolo[1,5-c] pyrimidine derivative according to any one of the above (1) to (24) or a pharmaceutically acceptable salt thereof.

(36) A method for treating and/or preventing a disease induced by hyperactivity of an adenosine $A_{2A}$ receptor, comprising administering an effective amount of the [1,2,4]triazolo[1,5-c]pyrimidine derivative according to any one of the above (1) to (24) or a pharmaceutically acceptable salt thereof.

(37) A method for treating a disease selected from the group consisting of Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease and intermittent claudications, comprising administering an effective amount of the [1,2,4]triazolo[1,5-c] pyrimidine derivative according to any one of the above (1) to (24) or a pharmaceutically acceptable salt thereof.

In the definition of each group in formula (I):

Examples of the lower alkyl include straight-chain or branched alkyl having 1 to 6 carbon atoms. Specific examples thereof include methyl, ethyl, propyl, isopropyl, butyl, isobutyl, sec-butyl, tert-butyl, pentyl, neopentyl and hexyl.

The lower alkyl moiety of the lower alkoxy, the lower alkanoyl and the lower alkoxycarbonyl has the same meaning as the above-described lower alkyl.

The lower alkylene moiety of the hydroxy-substituted lower alkyl has the same meaning as the group produced by removing one hydrogen atom from the above-described lower alkyl.

The lower alkoxy moiety and the lower alkylene moiety of the lower alkoxy-substituted lower alkyl have the same meanings as the above-described lower alkoxy and the group produced by removing one hydrogen atom from the above-described lower alkyl, respectively.

Examples of the lower cycloalkyl include cycloalkyl having 3 to 8 carbon atoms. Specific examples thereof include cyclopropyl, cyclopentyl, cyclohexyl, cycloheptyl and cyclooctyl.

The halogen includes fluorine, chlorine, bromine and iodine atoms.

Examples of the lower cycloalkane ring formed together with the adjacent carbon atom include a cycloalkane ring having 3 to 8 carbon atoms. Specific examples thereof include a cyclopropane ring, a cyclopentane ring, a cyclohexane ring, a cycloheptane ring and a cyclooctane ring.

Examples of the aryl include phenyl, naphthyl, indenyl and anthryl.

The aryl moiety of the aroyl has the same meaning as the above-described aryl.

Examples of the aromatic heterocyclic group include furyl, thienyl, pyrrolyl, pyridyl, isoxazolyl, oxazolyl, thiazolyl, imidazolyl, pyrazolyl, pyrimidinyl, triazinyl, indolyl, quinolyl, purinyl, benzoxazolyl, benzothiazolyl, thiadiazolyl, benzimidazolyl and pyridonyl.

Examples of the aralkyl include aralkyl having 7 to 15 carbon atoms. Specific examples thereof include phenyl-substituted lower alkyl such as benzyl, 1-phenylethyl, 2-phenylethyl, 3-phenylpropyl, 2-phenylpropyl and diphenylmethyl; naphthyl-substituted lower alkyl such as 1-naphthylmethyl and 2-naphthylmethyl; indenyl-substituted lower alkyl; and anthryl-substituted lower alkyl. The lower alkylene moiety of the phenyl-substituted lower alkyl, the naphthyl-substituted lower alkyl, the indenyl-substituted lower alkyl and the anthryl-substituted lower alkyl has the same meaning as the group produced by removing one hydrogen atom from the above-described lower alkyl.

Examples of the heterocyclic group formed together with the adjacent nitrogen atom include pyrrolidinyl, piperidino, pyrrolidinonyl, piperidinonyl, piperazinyl, morpholino, thiomorpholino, homopiperidino, homopiperazinyl, tetrahydropyridinyl, tetrahydroquinolyl, tetrahydroisoquinolyl, perhydroazepinyl, perhydro-1,4-oxazepinyl, decahydroquinolyl, decahydroisoquinolyl and 1,4-dioxa-8-azaspiro[4.5]decanyl.

The substituted lower alkyl, the substituted lower alkanoyl and the substituted cycloalkyl each have, for example, 1 to 3 substituents which may be the same or different. Specific examples of the substituents include hydroxy, carboxy, lower cycloalkyl, lower alkoxy, lower alkoxycarbonyl, substituted or unsubstituted aryl, aryloxy, aralkyloxy, a substituted or unsubstituted aromatic heterocyclic group, hydroxy-substituted lower alkoxy, lower alkoxy-substituted lower alkoxy, lower alkanoyl, aryl-substituted lower alkanoyl, aroyl, formyl, halogen, trifluoromethyl, vinyl, styryl, phenylethynyl and cyano.

In the definition of the substituents in the substituted lower alkyl, the substituted lower alkanoyl and the substituted cycloalkyl:

The lower cycloalkyl, the aryl, the aroyl, the aromatic heterocyclic group, the halogen, the lower alkoxy, the lower alkanoyl and the lower alkoxycarbonyl have the same meanings as defined above, respectively. The aryl moiety of the aryloxy and the aralkyl moiety of the aralkyloxy have the same meanings as the above-described aryl and aralkyl, respectively. The lower alkylene moiety of the hydroxy-substituted lower alkoxy has the same meaning as the group produced by removing one hydrogen atom from the above-described lower alkyl. The lower alkoxy moiety and the

lower alkylene moiety of the lower alkoxy-substituted lower alkoxy have the same meanings as the above-described lower alkoxy and the group produced by removing one hydrogen atom from the above-described lower alkyl, respectively. The aryl moiety and the lower alkylene moiety of the aryl-substituted lower alkanoyl have the same meanings as the above-described aryl and the group produced by removing one hydrogen atom from the above-described lower alkyl, respectively. Examples of the substituents in the substituted aryl and the substituted aromatic heterocyclic group include examples of the substituents in the substituted aryl described below.

The substituted aryl, the substituted aroyl, the substituted aromatic heterocyclic group and the substituted aralkyl each have, for example, 1 to 3 substituents which may be the same or different. Specific examples of the substituents include lower alkyl, hydroxy, hydroxy-substituted lower alkyl, halogeno lower alkyl, lower alkoxy, lower alkoxycarbonyl, aryl, aryloxy, aralkyl, aralkyloxy, an aromatic heterocyclic group, halogenoaryloxy, halogenoaralkyloxy, carboxy, carbamoyl, formyl, lower alkanoyl, aroyl, halogen, nitro, cyano, trifluoromethyl, trifluoromethoxy, methylenedioxy, ethylenedioxy, and formyl and equivalents thereof (the equivalents include 1,3-dioxolan-2-yl).

In 5) substituted or unsubstituted aroyl in the definition of $R^3$:

Examples of the substituents in the substituted aroyl include $-CH_2NR^{4b}R^{4c}$ (wherein $R^{4b}$ and $R^{4c}$ have the same meanings as defined above, respectively), $-(CH_2)_{nb}-C(R^{6a})(R^{6b})(OR^7)$ (wherein nb, $R^{6a}$, $R^{6b}$ and $R^7$ have the same meanings as defined above, respectively) and $-NR^{8a}R^{8b}$ (wherein $R^{8b}$ and $R^{8b}$ have the same meanings as defined above, respectively), in addition to the above-described examples of the substituents in the substituted aryl.

In the definition of the substituents in the substituted aryl, the substituted aroyl, the substituted aromatic heterocyclic group and the substituted aralkyl:

The lower alkyl, the hydroxy-substituted lower alkyl, the lower alkoxy, the lower alkoxycarbonyl, the lower alkanoyl, the aryl, the aryloxy, the aroyl, the aralkyl, the aralkyloxy, the aromatic heterocyclic group and the halogen have the same meanings as defined above, respectively. The halogen moiety and the lower alkylene moiety of the halogeno-lower alkyl have the same meanings as the above-described halogen and the group produced by removing one hydrogen atom from the above-described lower alkyl, respectively. The halogen moiety and the arylene moiety of the halogenoaryloxy have the same meanings as the above-described halogen and the group produced by removing one hydrogen atom from the above-described aryl, respectively. The halogen moiety, the arylene moiety and the lower alkylene moiety of the halogenoaralkyloxy have the same meanings as the above-described halogen, the group produced by removing one hydrogen atom from the above-described aryl and the group produced by removing one hydrogen atom from the above-described lower alkyl, respectively.

The substituted heterocyclic group formed together with the adjacent nitrogen atom has, for example, 1 to 3 substituents which may be the same or different. Specific examples of the substituents include lower alkyl, lower cycloalkyl, substituted or unsubstituted aryl, substituted or unsubstituted aralkyl, lower alkoxy, lower alkanoyl, lower alkoxycarbonyl, carboxy, carbamoyl, cyano, hydroxy, an aromatic heterocyclic group, an aliphatic heterocyclic group, hydroxy-substituted lower alkyl, lower alkoxy-substituted lower alkyl and lower cycloalkyl-substituted lower alkyl. In these examples, the lower alkyl, the lower cycloalkyl, the aryl, the aralkyl, the lower alkoxy, the lower alkanoyl, the lower alkoxycarbonyl, the aromatic heterocyclic group, the hydroxy-substituted lower alkyl and the lower alkoxy-substituted lower alkyl have the same meanings as defined above, respectively. The substituents in the substituted aryl and the substituted aralkyl have the same meanings as defined above, respectively. The lower cycloalkyl moiety and the lower alkylene moiety of the lower cycloalkyl-substituted lower alkyl have the same meanings as the above-described lower cycloalkyl and the group produced by removing one hydrogen atom from the above-described lower alkyl, respectively. Examples of the aliphatic heterocyclic group include pyrrolidinyl, 2,5-dioxopyrrolidinyl, thiazolidinyl, oxazolidinyl, piperidyl, piperidino, piperazinyl, homopiperazinyl, homopiperidyl, homopiperidino, morpholinyl, morpholino, thiomorpholinyl, thiomorpholino, tetrahydropyranyl, tetrahydrofuranyl, tetrahydroquinolyl, tetrahydroisoquinolyl, octahydroquinolyl, indolinyl and 1,4-benzodioxanyl.

Hereinafter, compounds represented by formula (I) is referred to as Compound (I). This applies to compounds represented by the other formula Nos. Among Compound (I), compounds wherein Q is 3,4-dimethoxybenzyl has adenosine $A_{2A}$ receptor antagonism and is also useful as a. synthetic intermediate for Compound (I) wherein Q is a hydrogen atom. Such compounds are referred to as Compound (IQ) hereinafter. Compounds represented by formula (I) wherein Q is a hydrogen atom is referred to as Compound (IH), if necessary.

Preferred examples of compounds of the present invention include Compound (IH) wherein Q in formula (I) is a hydrogen atom. Preferred examples of Compound (IH) include the following:

Compounds wherein $R^1$ is furyl, and $R^2$ is a hydrogen atom are preferable. In addition, compounds wherein $R^3$ is aryl substituted with a substituent selected from the group consisting of $-CH_2NHR^{4a}$ (wherein $R^{4a}$ has the same meaning as defined above), $-(CH_2)_{nb}-C(R^{6a})(R^{6b})(OR^7)$ (wherein nb, $R^{6a}$, $R^{6b}$ and $R^7$ have the same meanings as defined above, respectively) and $-NR^{8a}R^{8b}$ (wherein $R^{8b}$ and $R^{8b}$ have the same meanings as defined above, respectively);

compounds wherein $R^3$ is an aromatic heterocyclic group substituted with a substituent selected from the group consisting of $-CH_2NR^{4b}R^{4c}$ (wherein $R^{4b}$ and $R^{4c}$ have the same meanings as defined above, respectively), $-(CH_2)_{nb}-C$

$(R^{6a})(R^{6b})(OR^7)$ (wherein nb, $R^{6a}$, $R^{6b}$ and $R^7$ have the same meanings as defined above, respectively) and $-NR^{8a}R^{8b}$ (wherein $R^{8b}$ and $R^{8b}$ the same meanings as defined above, respectively);

compounds wherein $R^3$ is aryl substituted with $-CH_2NHR^{4a}$ (wherein $R^{4a}$ has the same meaning as defined above);

compounds wherein $R^3$ is aryl substituted with $-(CH_2)_{nb}-C(R^{6a})(R^{6b})(OR^7)$ (wherein nb, $R^{6a}$, $R^{6b}$ and $R^7$ have the same meanings as defined above, respectively);

compounds wherein $R^3$ is an aromatic heterocyclic group substituted with $-NR^{8a}R^{8b}$ (wherein $R^{8b}$ and $R^{8b}$ have the same meanings as defined above, respectively);

compounds wherein $R^3$ is formula ($C^3$);

compounds wherein $R^3$ is formula ($F^1$); or

compounds wherein $R^3$ is formula ($A^3$) are preferable.

And also, among the compounds wherein $R^3$ is formula ($A^3$), specially, compounds wherein nd is 0, and $R^{13a}$ and $R^{13b}$ are combined together to represent an oxygen atom; or compounds wherein nd is 0, and $R^{13a}$ and $R^{13b}$ are a hydrogen atom are preferable.

And also, compounds wherein $R^1$ is furyl, $R^2$ is a hydrogen atom, and $R^3$ is formula (E1) are also preferable. Among them, compounds wherein nf is 1, ng is 1 and X$\overline{---}$Y is C=CH is more preferable.

Pharmaceutically acceptable salts of Compound (I) include pharmaceutically acceptable metal salts, ammonium salts, organic amine addition salts, amino acid addition salts and acid addition salts. Examples of pharmaceutically acceptable metal salts of Compound (I) include alkali metal salts such as a sodium salt and a potassium salt; alkali earth metal salts such as a magnesium salt and a calcium salt; an aluminum salt; and a zinc salt. Examples of pharmaceutically acceptable ammonium salts include ammonium and tetramethylammonium. Examples of pharmaceutically acceptable organic amine addition salts include addition salts with morpholine and piperidine. Examples of pharmaceutically acceptable amino acid addition salts include addition salts with lysine, glycine and phenylalanine. Examples of pharmaceutically acceptable acid addition salts include inorganic acid salts such as a hydrochloride, a sulfate and a phosphate; and organic acid salts such as an acetate, a maleate, a fumarate, a tartrate and a citrate.

Next, the production processes of Compound (I) are described below.

In description of the production processes, for example, Compound (IQ-a) and Compound (IH-a) are not necessarily included in the range of Compound (I).

Production Process 1

Compound (IH-a), wherein $R^3$ is substituted or unsubstituted aryl or a substituted or unsubstituted aromatic heterocyclic group, can be manufactured by the following steps.

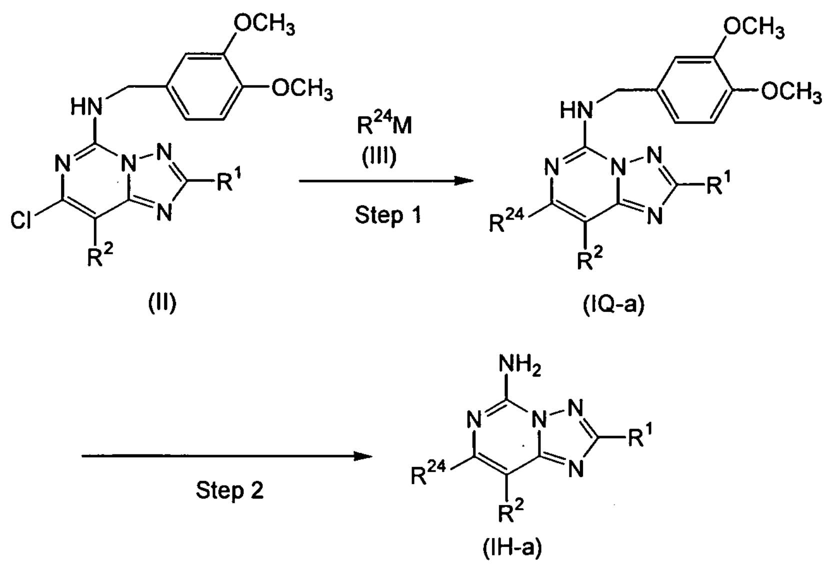


(II)

(IQ-a)

(IH-a)

[wherein $R^1$ and $R^2$ have the same meanings as defined, $R^{24}$ represents substituted or unsubstituted aryl or a substituted or unsubstituted aromatic heterocyclic group, M represents tributylstannyl, trimethylstannyl, triphenylstannyl, di (lower alkyl)boranyl, lower cycloalkylboranyl or borane dihydroxyboranyl.]

In the above definition, the substituted or unsubstituted aryl, and the substituted or unsubstituted aromatic

heterocyclic group have the same meanings as defined above. The two lower alkyl moieties of the di(lower alkyl)boranyl may be the same or different and each have the same meanings as the above-described lower alkyl. The lower cycloalkyl moiety of the lower cycloalkylboranyl has the same meaning as the above-described lower cycloalkyl.

Step 1

Compound (IQ-a) can be obtained by reacting Compound (II), which is produced by the method described in WO98/42711, with 1 to 10 equivalents of Compound (III) in the presence of a catalytic amount of a palladium compound in an inert solvent, ordinarily, at a temperature between room temperature and 140°C for 10 minutes to 48 hours.

The reaction can also be carried out by adding 0.2 to 5 equivalents thereto, preferably 1 equivalent, of an inorganic salt or a base. Examples of the inorganic salt include lithium chloride, potassium chloride, silver oxide, copper oxide, silver nitrate and silver acetate. Examples of the base include triethylamine, sodium ethoxide, sodium carbonate and sodium hydroxide. Among them, sodium carbonate is preferable.

Examples of the inert solvent include diethyl ether, tetrahydrofuran (THF), 1,4-dioxane, N,N-dimethylformamide (DMF), N,N-dimethylacetamide (DMA), dimethylsulfoxide (DMSO), benzene, toluene, xylene, dichloromethane, chloroform, carbon tetrachloride, 1,2-dichloroethane, acetonitrile, ethyl acetate, methyl acetate, methyl ethyl ketone, methanol, ethanol, propanol, isopropyl alcohol, butanol, hexane and water. THF, DMF and water are preferable. These solvents can be used alone or as a mixture thereof.

Examples of the palladium compound include bis(triphenylphosphine)palladium (II) dichloride, tetrakis(triphenylphosphine)palladium (0), [1,2-bis(diphenylphosphino)ethane]palladium (II) dichloride, and [1,1'-bis(diphenylphosphino)ferrocene]palladium (II) chloride. Among these compounds, bis(triphenylphosphine)palladium (II) dichloride and tetrakis(triphenylphosphine)palladium (0) are preferable.

Step 2-1

Compound (IH-a) can be obtained by treating Compound (IQ-a) with an acid such as hydrochloric acid, acetic acid, dichloroacetic acid, trifluoroacetic acid and trifluoromethanesulfonic acid, preferably trifluoroacetic acid or a mixture of trifluoroacetic acid and trifluoromethanesulfonic acid, ordinarily, at a temperature between 10°C and 100°C for 10 minutes to 24 hours. Alternatively, Compound (IH-a) can be obtained by treating with 1 to 50 equivalents, preferably 3 to 5 equivalents of trifluoromethanesulfonic acid or sulfuric acid in the presence of 1 to 10 equivalents, preferably 4 equivalents of anisole, dimethoxybenzene or trimethoxybenzene, preferably anisole, in an acid such as hydrochloric acid, acetic acid, dichloroacetic acid and trifluoroacetic acid, preferably trifluoroacetic acid, ordinarily, at a temperature between -0°C to 80°C, preferably a temperature between 10°C to 40°C, for 10 minutes to 18 hours.

Step 2-2

Compound (IH-a) can also be obtained from Compound (IQ-a) by the following method.

Compound (IH-a) can be obtained by treating Compound (IQ-a) with 1 to 10 equivalents, preferably 3 to 5 equivalents of an appropriate oxidizing agent such as cerium diammonium nitrate (CAN) or dichlorodicyanobenzoquinone (DDQ), preferably DDQ, in an inert solvent, ordinarily, at a temperature between 0°C and the boiling point of the inert solvent used, preferably room temperature, for 1 to 48 hours, preferably 3 to 5 hours.

Examples of the inert solvent include diethyl ether, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methyl ethyl ketone, methanol, ethanol, propanol, isopropyl alcohol, butanol, hexane and water. These solvents can be used alone or as a mixture thereof. Among them, a two-layered solvent containing chloroform and water is preferable.

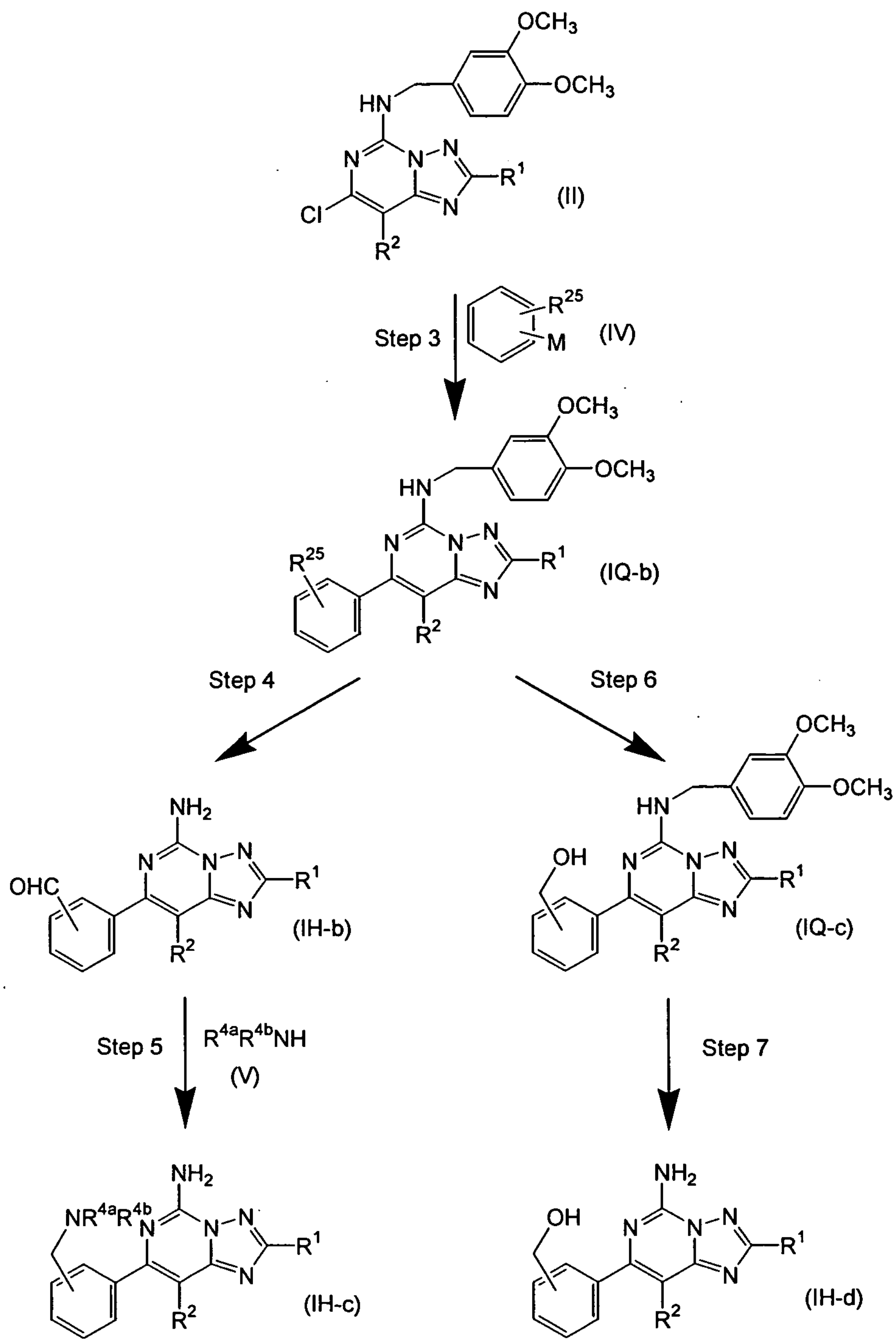


(wherein $R^1$, $R^2$, $R^{4a}$, $R^{4b}$ and M have the same meanings as defined above; and $R^{25}$ represents formyl or a formyl equivalent, examples of the formyl equivalent include acetal such as 1,3-dioxolan-2-yl).

Production Process 2

Compound (IH-b) can be manufactured by the following steps.

Step 3

Compound (IQ-b) can be obtained by using Compound (II) and 1 to 10 equivalents of Compound (IV) in a reaction similar to that in Step 1 of Production Process 1.

Step 4

Compound (IH-b) can be obtained by using Compound (IQ-b) in a reaction similar to that in Step 2-1 or 2-2 of Production Process 1.

When the reaction similar to that in Step 2-2 of Production Process 1 is carried out using Compound (IQ-b) having a formyl equivalent as $R^{25}$, Compound (IH-b) can be derived by further treating the compound obtained in the reaction with 1 to 50 equivalents, preferably 3 to 5 equivalents of an acid such as hydrochloric acid, acetic acid, dichloroacetic acid and trifluoroacetic acid, in an inert solvent, at a temperature between 0°C and the boiling point of the inert solvent used, preferably room temperature, for 1 to 48 hours, preferably 3 to 10 hours.

Examples of the inert solvent include diethyl ether, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methyl ethyl ketone, methanol, ethanol, propanol, isopropyl alcohol, butanol, hexane and water. These solvents can be used alone or as a mixture thereof. Among them, a mixed solvent containing THF and water is preferable.

Production Process 3

Compound (IH-c) can be manufactured by the following step.

Step 5

Compound (IH-c) can be obtained by reacting Compound (IH-b) with 1 equivalent to a large excess, preferably 1 to 10 equivalents of Compound (V) in the presence of 1 equivalent to a large excess, preferably 1 to 3 equivalents of a reducing agent in an inert solvent, ordinarily, at a temperature between -78°C and 100°C, preferably a temperature between 0°C and 50°C, for 10 minutes to 24 hours.

Examples of the inert solvent include dichloromethane, chloroform, carbon tetrachloride, dichloroethane, benzene, toluene, xylene, diethyl ether, THF, 1,4-dioxane, DMF, DMA, acetonitrile and hexane. These solvents can be used alone or as a mixture thereof. Among them, THF, dichloromethane or a mixed solvent containing THF and dichloromethane is preferable.

Examples of the reducing agent include sodium borohydride, sodium triacetoxyborohydride and sodium cyanoborohydride. Among them, sodium triacetoxyborohydride is preferable.

The reaction may be performed by adding a catalytic amount to a large excess, preferably 0.5 to 5 equivalents of an acid thereto, if necessary. Examples of the acid include formic acid, acetic acid, trifluoroacetic acid, propionic acid and hydrochloric acid. Among them, acetic acid is preferable.

Production Process 4

Compound (IH-d) can be manufactured by the following steps.

Step 6

Compound (IQ-c) can be obtained by treating Compound (IQ-b), which is obtained in Step 3 of Production Process 2, in the presence of 2 to 4 equivalents of a reducing agent in an inert solvent, ordinarily, at a temperature between -78°C and 40°C for 10 minutes to 24 hours, preferably 1 to 3 hours.

Examples of the inert solvent include dichloromethane, chloroform, carbon tetrachloride, dichloroethane, benzene, toluene, xylene, diethyl ether, THF, 1,4-dioxane, DMF, DMA, acetonitrile, methanol, ethanol and propanol. These solvents can be used alone or as a mixture thereof. Among them, methanol or THF is preferable.

Examples of the reducing agent include lithium aluminum hydride, sodium borohydride and diisopropyl lithium aluminum hydride. Among them, sodium borohydride or diisopropyl lithium aluminum hydride is preferable.

Step 7

Compound (IH-d) can be obtained by using Compound (IQ-c) in a reaction similar to that in Step 2-1 or Step 2-2 of Production Process 1.

Production Process 5

Compound (IH-e) can be manufactured by the following steps.

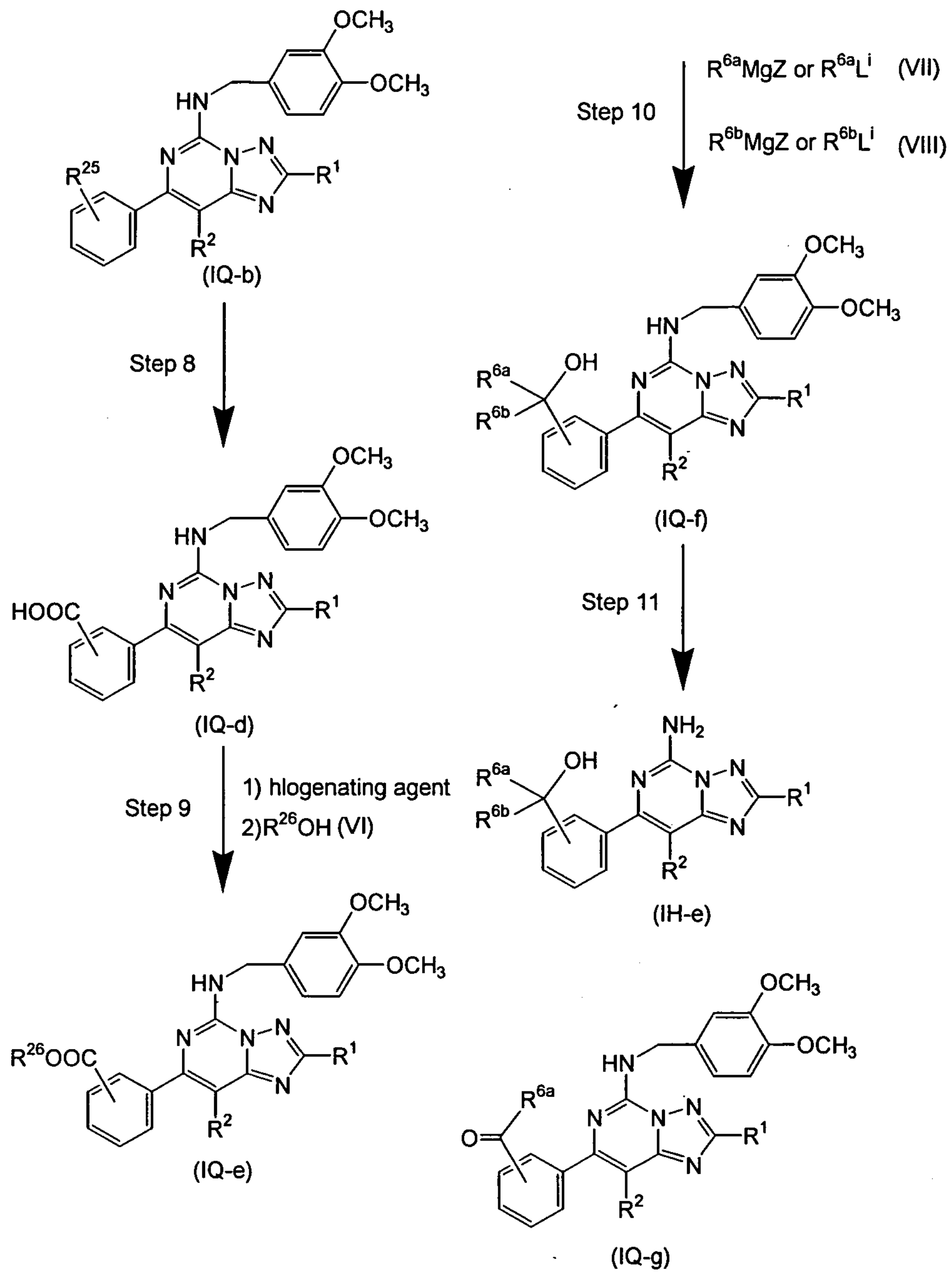


(wherein $R^1$, $R^2$, $R^{6a}$, $R^{6b}$ and $R^{25}$ have the same meanings as defined above; $R^{26}$ represents lower alkyl; and Z represents halogen; the lower alkyl and the halogen have the same meanings as defined above, respectively).

Step 8

Compound (IQ-d) can be obtained by treating Compound (IQ-b), which is obtained in Step 3 of Production Process 2, with 2 to 4 equivalents of an oxidizing agent in an inert solvent, ordinarily, at a temperature between 0°C and 80°C for 10 minutes to 24 hours, preferably 1 to 3 hours.

The reaction may be carried out by adding 0.1 to 4 equivalents of an additive thereto, if necessary. Examples of the additive include organic substances such as acetic acid, and inorganic substances such as sulfuric acid, sulfamic acid and ruthenium oxide.

Examples of the inert solvent include diethyl ether, THF, 1,4-dioxane, DMF, DMA, DMSO, benzene, toluene, xylene, dichloromethane, chloroform, 1,2-dichloroethane, acetonitrile, ethyl acetate, methyl acetate, methyl ethyl ketone, hydrochloric acid, acetic acid, acetic anhydride, sulfuric acid and water. These solvents can be used alone or as

a mixture thereof. Among them, acetic acid, water or a mixed solvent containing acetic acid and water is preferable.

Examples of the oxidizing agent include silver nitrate, silver (I) oxide, silver (II) oxide, chromic acid, pyridinium chlorochromate, pyridinium dichlorochromate, potassium permanganate, sodium periodate, sodium perchlorate, and hydrogen peroxide. Among them, silver nitrate or sodium perchlorate is preferable.

Step 9

Compound (IQ-e) can be obtained by reacting Compound (IQ-d) with 1 to 20 equivalents of a halogenating agent in an inert solvent, ordinarily, at a temperature between 0°C and 80°C, preferably room temperature, for 10 minutes to 24 hours, and then reacting the resulting acid halide of Compound (IQ-d) with 1 equivalent to a large excess of Compound (VI).

Examples of the halogenating agent include thionyl chloride, oxalyl chloride and phosphorus oxychloride. Among them, oxalyl chloride is preferable.

Examples of the inert solvent include dichloromethane, chloroform, THF, 1,4-dioxane, DMF, DMA, acetonitrile; benzene, toluene, xylene, pyridine, triethylamine, diisopropylethylamine, 1,8-diazabicyclo[5.4.0]-7-undecene (DBU) and N-methylmorpholine. These solvents can be used alone or as a mixture thereof. Among them, dichloromethane is preferable.

Step 10-1: Production of Compound (IQ-f) wherein $R^{6a}$ and $R^{6b}$ are the same

Compound (IQ-f), wherein $R^{6a}$ and $R^{6b}$ are the same, can be obtained by reacting Compound (IQ-e) with 1 to 20 equivalents, preferably 2 to 5 equivalents of an organometallic agent (VII) in an inert solvent, at a temperature between -100°C and 50°C, preferably a temperature between -78°C and 25°C, for 1 minute to 48 hours.

Examples of the inert solvent include benzene, toluene, xylene, THF, diethyl ether, diisopropyl ether, dimethoxyethane and dichloromethane. These solvents can be used alone or as a mixture thereof. Among them, THF or diethyl ether is preferable.

Step 10-2: Production of Compound (IQ-f) wherein $R^{6a}$ and $R^{6b}$ are different

Compound (IQ-g) can be obtained by using Compound (IQ-e) in a mannner similar to that in Step 10-1 except that 1 to 1.2 equivalents of an organometallic agent (VII) is/are used.

Compound (IQ-f), wherein $R^{6a}$ and $R^{6b}$ are different, can be obtained by using the resulting Compound (IQ-g) in a mannner similar to that in Step 10-1 except that 1 to 20 equivalents of an organometallic agent (VIII) is/are used.

Step 11

Compound (IH-e) can be obtained using Compound (IQ-f) in a reaction similar to that in Step 2-1 or Step 2-2 of Production Process 1.

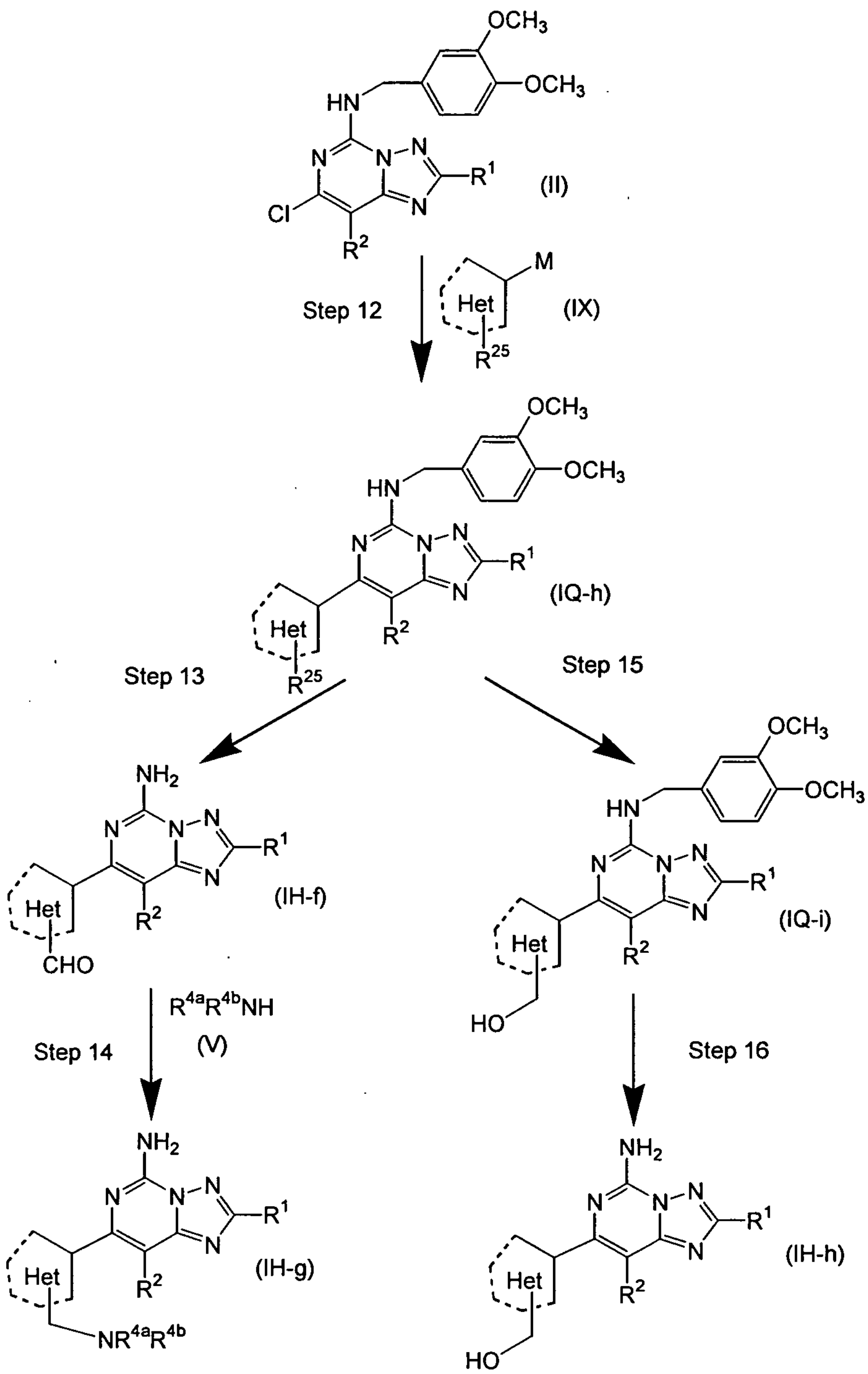


[wherein $R^1$, $R^2$, $R^{4a}$, $R^{4b}$, $R^{25}$ and M have the same meanings as defined above, respectively; and the moiety represented by the following:

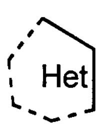

represents an aromatic heterocyclic ring, the aromatic heterocyclic ring has the same meaning as the ring produced by adding one hydrogen atom to the above-descried aromatic heterocyclic group].

Production Process 6

Compound (IH-f) can be manufactured by the following steps.

Step 12

Compound (IQ-h) can be obtained by using Compound (II) and 1 to 10 equivalents of Compound (IX) in a reaction similar to that in Step 3 of Production Process 2.

Step 13

Compound (IH-f) can be obtained by using Compound (IQ-h) in a reaction similar to that in Step 4 of Production Process 2.

Production Process 7

Compound (IH-g) can be manufactured by the following step.

Step 14

Compound (IH-g) can be obtained by using Compound (IH-f), which is obtained in Step 13 of Production Process 6, i in a reaction similar to that in Step 5 of Production Process 3.

Production Process 8

Compound (IH-h) can be manufactured by the following steps.

Step 15

Compound (IQ-i) can be obtained by using Compound (IQ-h), which is obtained in Step 12 of Production Process 6, in a reaction similar to that in Step 6 of Production Process 4. Step 16
Compound (IH-h) can be obtained by using Compound (IQ-i) in a reaction similar to that in Step 2-1 or Step 2-2 of Production Process 1.

Production Process 9

Compound (IH-i) can be manufactured by the following steps.

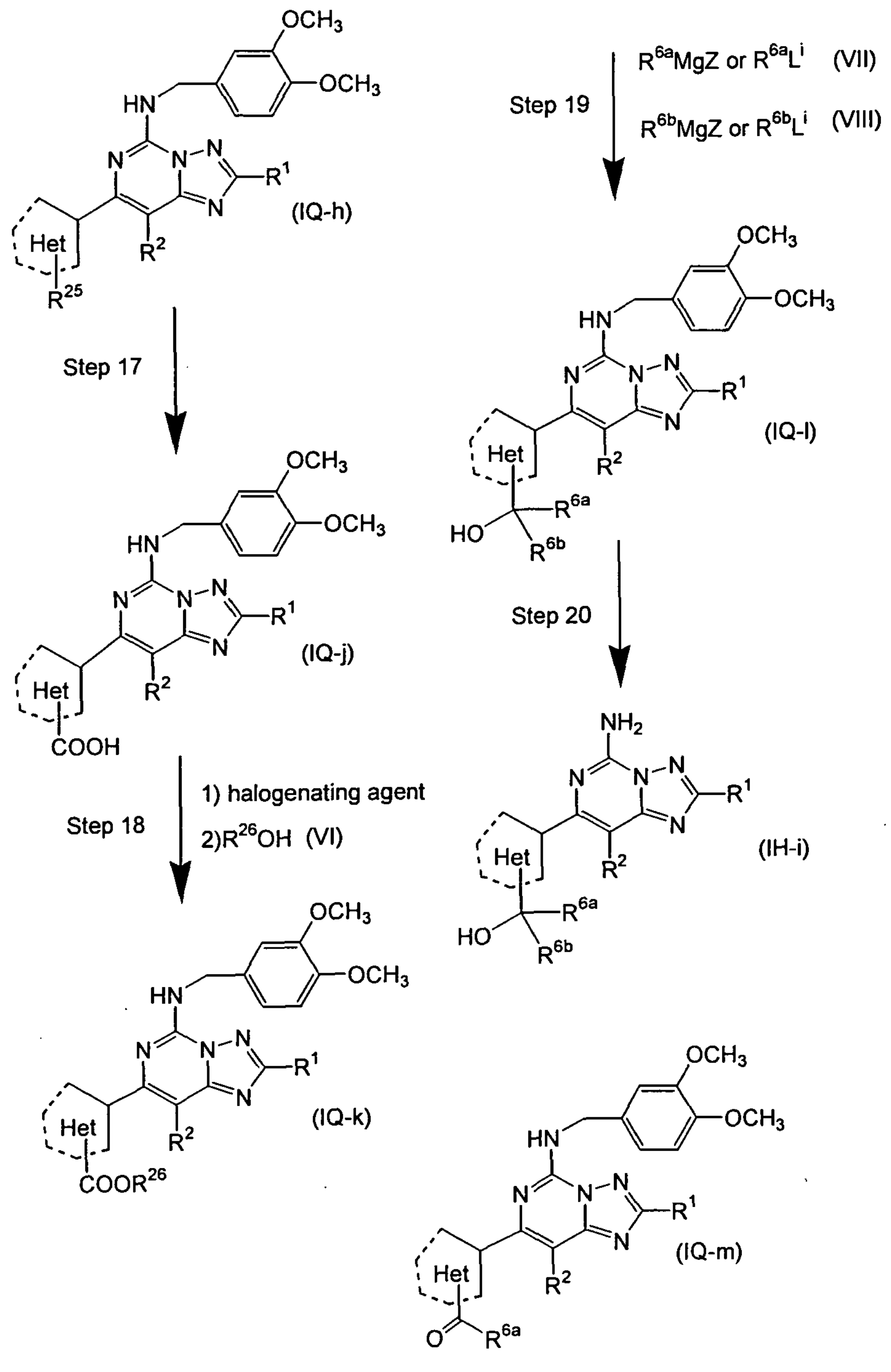


(IQ-h) (IQ-l) (IQ-j) (IH-i) (IQ-k) (IQ-m)

(wherein $R^1$, $R^2$, $R^{6a}$, $R^{6b}$, $R^{25}$, $R^{26}$, Z and

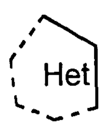

have the same meanings as defined above, respectively).

Step 17

Compound (IQ-j) can be obtained by using Compound (IQ-h), which is produced in Step 12 of Production Process 6, in a reaction similar to that in Step 8 of Production Process 5.

Step 18

Compound (IQ-k) can be obtained by using Compound (IQ-j) in a reaction similar to that in Step 9 of Production Process 5.

Step 19

Compound (IQ-1), wherein $R^{6a}$ and $R^{6b}$ are the same, can be obtained by using Compound (IQ-k) in a reaction similar to that in Step 10-1 of Production Process 5. Compound (IQ-1), wherein $R^{6a}$ and $R^{6b}$ are different, can be obtained by using Compound (IQ-k) in a reaction similar to that in Step 10-2 of Production Process 5 through Compound (IQ-m).

Step 20

Compound (IH-i) can be obtained by using Compound (IQ-1) in a reaction similar to that in Step 11 of Production Process 5.

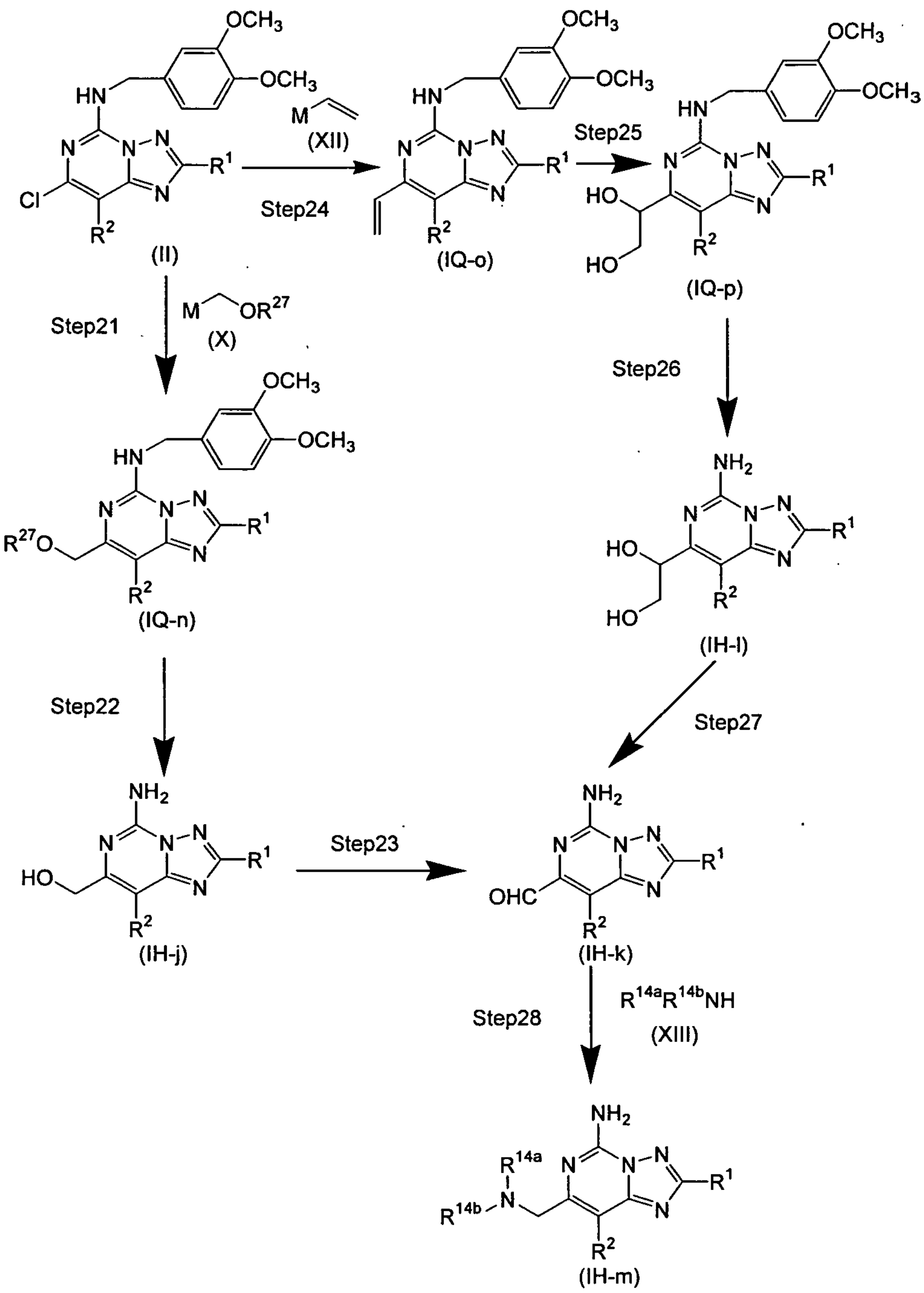


(wherein $R^1$, $R^2$, M, $R^{14a}$ and $R^{14b}$ have the same meanings as defined above, respectively; and $R^{27}$ represents a hydrogen atom, trimethylsilyl, triethylsilyl or tert-butyldimethylsilyl)

Production Process 10

Compound (IH-j) can be manufactured by the following steps.

Step 21

Compound (IQ-n) can be obtained by using Compound (II) and Compound (X) in a reaction similar to that in Step 1 of Production Process 1.

Step 22

Compound (IH-j) can be obtained by using Compound (IQ-n) in a reaction similar to that in Step 2-1 of Production Process 1.

Production Process 11

Compound (IH-k) can be manufactured by the following steps.

Step 23

Compound (IH-k) can be obtained by treating Compound (IH-j), which is obtained in Step 22 of Production Process 10, with 1 equivalent to a large excess of an oxidizing agent in an inert solvent, ordinarily, at a temperature between 0°C and 100°C, preferably room temperature, for 10 minutes to 24 hours.

Examples of the inert solvent include diethyl ether, THF, 1,4-dioxane, DMF, DMA, DMSO, dichloromethane, chloroform, carbon tetrachloride, dichloroethane, benzene, toluene, xylene, ethyl acetate, acetic acid, propionic acid, butylic acid, trifluoroacetic acid, water and pyridine. These solvents can be used alone or as a mixture thereof. Among them, DMF is preferable.

Examples of the oxidizing agent include manganese dioxide, chromic acid, pyridinium chlorochromate, pyridinium dichromate, potassium permanganate, sulfur trioxide-pyridine and Oxone (registered trademark). Among them, manganese dioxide is preferable.

Compound (IH-k) can be also obtained by the following steps.

Step 24

Compound (IQ-o) can be obtained by using Compound (II) and Compound (XII) in a reaction similar to that in Step 1 of Production Process 1.

Step 25

Compound (IQ-p) can be obtained by treating Compound (IQ-o) in the presence of a catalytic amount to 2 equivalents, preferably 0.01 equivalent of osmium tetroxide and 1 to 10 equivalents, preferably 2 equivalents of an appropriate oxidizing agent in an inert solvent, at a temperature between 0°C and 100°C, preferably room temperature, for 1 to 24 hours, preferably 2 to 3 hours. The reaction may be carried out by further adding 1 to 10 equivalents, preferably 2 equivalents of a tertiary amine, thereto.

Examples of the inert solvent include acetone, diethyl ether, THF, acetonitrile, water, toluene, benzene and dichloromethane. These solvents can be used alone or as a mixture thereof. Among them, DMF or acetone is preferable.

Examples of the oxidizing agent include 4-methylmorpholine-N-oxide, trimethylamine-N-oxide and potassium ferricyanate. Among them, 4-methylmorpholine-N-oxide is preferable.

Examples of the tertiary amine include 4-methylmorpholine, triethylamine and pyridine. Among them, 4-methylmorpholine is preferable.

Step 26

Compound (IH-1) can be obtained by using Compound (IQ-p) in a reaction similar to that in Step 2-1 of Production Process 1.

Step 27

Compound (IH-k) can be obtained by treating Compound (IH-1) with 1 to 10 equivalents, preferably 1.5 equivalents of sodium metaperiodate in an inert solvent.

Examples of the inert solvent include THF, water, toluene, benzene, dichloromethane and chloroform. These solvents can be used alone or as a mixture thereof. Among them, dichloromethane or water is preferable.

Production Process 12

Compound (IH-m) can be manufactured by the following steps.

Step 28

Compound (IH-m) can be obtained by using Compound (IH-k), which is obtained in Step 23 or Step 27 of Production Process 11, and Compound (XIII) in a reaction similar to that in Step 5 of Production Process 3.

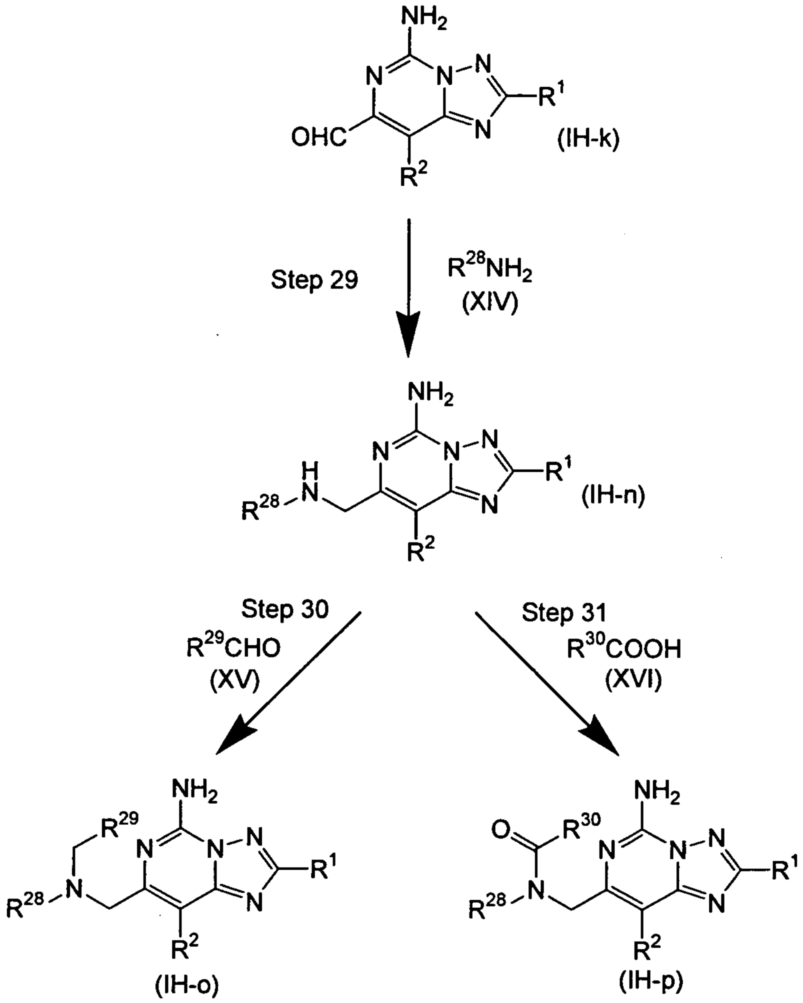


(wherein $R^1$ and $R^2$ have the same meanings as defined above, respectively; $R^{28}$ has the same meaning as the above-described $R^{14a}$; $R^{29}$ represents the group produced by removing the terminal methylene chain from the above-described lower alkyl $R^{14a}$; and $R^{30}$ represents substituted or unsubstituted lower alkyl, the lower alkyl and the substituents of the substituted lower alkyl have the same meanings as defined above, respectively).

Production Process 13

Compound (IH-n) can be manufactured by the following step.

Step 29

Compound (IH-n) can be obtained by using Compound (IH-k), which is obtained in Step 23 or Step 27 of Production Process 11, and Compound (XIV) in a reaction similar to that in Step 5 of Production Process 3.

Production Process 14

Compound (IH-o) can be obtained by the following step.

Step 30

Compound (IH-o) can be obtained by using Compound (IH-n), which is obtained in Step 29 of Production Process 13, and Compound (XV) in a reaction similar to that in Step 5 of Production Process 3.

Production Process 15

Compound (IH-p) can be obtained by the following step.

Step 31

Compound (IH-p) can be obtained by reacting Compound (IH-n), which is obtained in Step 29 of Production Process 13, with an acid halide of Compound (XVI), which is prepared from 1 to 20 equivalents of a halogenating agent and 1 to 10 equivalents of Compound (XVI), in a basic solvent, ordinarily, at a temperature between -10°C and 100°C, preferably room temperature, for 10 minutes to 24 hours.

As the basic solvent, for example, pyridine, triethylamine, diisopropylethylamine, N-methylmorpholine or the like can be singly used, or a mixture thereof. Alternatively, a mixture containing, for example, a solvent such as dichloromethane, chloroform, ethyl acetate, THF, 1,4-dioxane, DMF, DMA, acetonitrile, benzene, toluene and xylene; and pyridine, triethylamine, diisopropylethylamine, N-methylmorpholine and the like, can be used. Among them, pyridine is preferable.

Examples of the halogenating agent include thionyl chloride, oxalyl chloride and phosphorus oxychloride. Among them, thionyl chloride is preferable.

Compound (IH-p) can be also obtained by a method generally used in the peptide chemistry.

Namely, Compound (IH-p) can be obtained by reacting Compound (IH-n) with 1 to 10 equivalents of Compound (XVI) in the presence of 0.5 to 10 equivalents of a condensing agent in an inert solvent, ordinarily, at a temperature between 0°C and 50°C, for 10 minutes to 70 hours.

Examples of the inert solvent include diethyl ether, THF, 1,4-dioxane, DMF, DMA, DMSO, benzene, toluene, xylene, acetonitrile, ethyl acetate, pyridine, dichloromethane, chloroform and carbon tetrachloride. These solvents can be used alone or as a mixture thereof. Among them, DMF or THF is preferable.

Examples of the condensing agent include 1,3-dicyclohexyl carbodiimide, 1-ethyl-3-(3-dimethylaminopropyl) carbodiimide hydrochloride and 1-ethyl-3-(3-dimethylaminopropyl) carbodiimide-bonded polystyrene resin (EDC resin). Also, an additive such as N-hydroxysuccinimide, 3,4-dihyro-3-hydroxy4-oxo-1,2,3-benzotriazine or 1-hydroxybenzotriazole, preferably 1-hydroxybenzotriazole can be used together with the condensing agent.

The EDC resin can be obtained by the method described in Tetrahedron Letters, vol. 34, No. 48, p. 7685 (1993):

Production Process 16

Compound (IH-q) can be manufactured by the following steps.

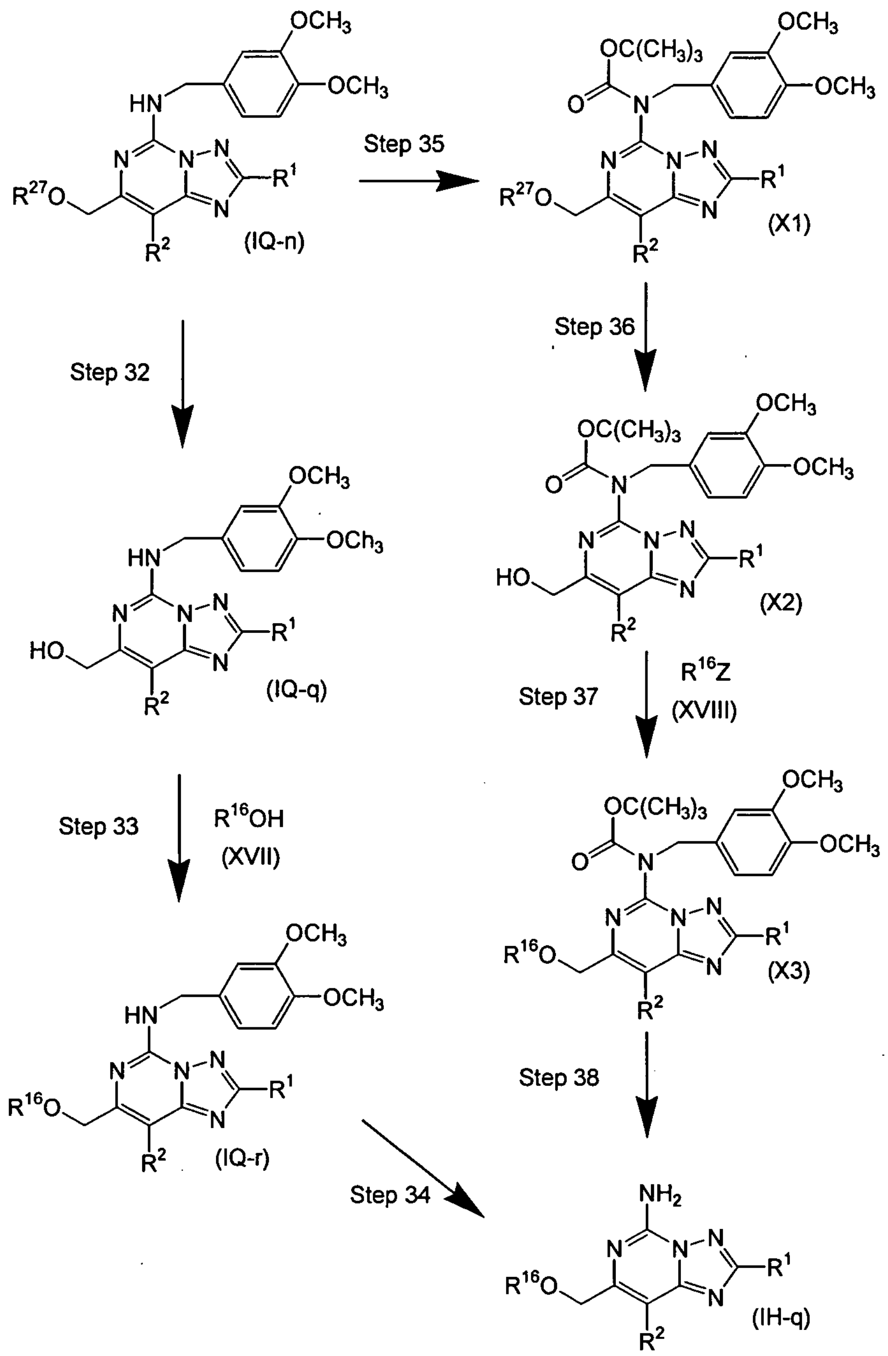


(wherein $R^1$, $R^2$, $R^{16}$ and $R^{27}$ have the same meanings as defined above, respectively; and Z represents halogen, the halogen has the same meaning as defined above).

Step 32

Compound (IQ-q) can be obtained by treating Compound (IQ-n), which is obtained in Step 21 of Production Process 10, with 1 to 10 equivalents, preferably 1 to 3 equivalents of a fluorinating reagent in an inert solvent.

Examples of the inert solvent include benzene, toluene, xylene, THF, diethyl ether, diisopropyl ether, dimethoxyethane, dichloromethane and water. These solvents can be used alone or as a mixture thereof. Among them, THF is preferable.

Examples of the fluorinating reagent include tetrabutylammonium fluoride, hydrogen fluoride, hydrogen fluoride-pyridine salt, cesium fluoride, potassium fluoride and boron trifluoride ether complex. Among them, tetrabutylammonium fluoride is preferable.

Step 33

Compound (IQ-r) can be obtained by reacting Compound (IQ-q) with 1 to 10 equivalents, preferably 1 to 3 equivalents of Compound (XVII) in the presence of 1 to 10 equivalents, preferably 1 to 3 equivalents of an oxygen-acceptor and a two-hydrogen-acceptor in an inert solvent.
Examples of the inert solvent include benzene, toluene, xylene, THF, diethyl ether, diisopropyl ether, dimethoxyethane and dichloromethane. These solvents can be used alone or as a mixture thereof. Among them, THF is preferable.
Examples of the oxygen-acceptor include triphenylphosphine and tributylphosphine. Examples of the two-hydrogen-acceptor include diethyl azodicarboxylate, N,N,N',N'-tetramethyl azadicarboxamide, 1,1'-(azodicarbonyl)dipiperazine, N,N,N',N'-tetraisopropyl azadicarboxamide, cyanomethyl tributylphospholane and [(cyanomethylene)tributylphospholane]. These acceptors can be used alone or as a mixture thereof. Among them, a combination of triphenylphosphine and diethyl azodicarboxylate is preferable. The reaction can be carried out by adding cyanomethyl tributylphospholane alotwo-hydrogen-acceptorne instead of the oxygen-acceptor and the two-hydrogen-acceptor thereto.

Step 34

Compound (IH-q) can be obtained by using Compound (IQ-r) in a reaction similar to that in Step 2-1 or 2-2 of Production Process 1.

Step 35

Compound (XI) can be obtained by reacting Compound (IQ-n), which is obtained in Step 21 of Production Process 10, with 1 to 10 equivalents, preferably 1 to 1.5 equivalents of, for example, di-tert-butyl dicarbonate, N-tert-butoxycarbonyloxyimino-2-phenylacetonitrile, S-tert-butoxycarbonyl-4,6-dimethyl-2-mercaptopyrimidine or tert-butyl-2,4,5-trichlorophenyl carbonate, preferably di-tert-butyl dicarbonate, in the presence of 0.1 to a large excess of a base in an inert solvent at a temperature between 0°C and 100°C, preferably between 0°C and room temperature.
Examples of the inert solvent include diethyl ether, THF, 1,4-dioxane, DMF, DMA, DMSO, benzene, toluene, xylene, acetonitrile, ethyl acetate, pyridine, dichloromethane, chloroform and carbon tetrachloride. These solvents can be used alone or as a mixture thereof. Among them, dichloromethane is preferable.
Examples of the base include triethylamine, pyridine, diisopropylethylamine, N-methylmorpholine and 4-dimethylaminopyridine. These bases can be used alone or as a mixture thereof. Among them, a mixture containing 2 to 4 equivalents of triethylamine and 0.1 to 0.3 equivalent of 4-dimethylaminopyridine is preferable.

Step 36

Compound (X2) can be obtained by using Compound (XI) in a reaction similar to that in Step 32.

Step 37

Compound (X3) can be obtained by reacting Compound (X2) with 1 equivalent to a large excess of Compound (XVIII) in the presence of 1 to 3 equivalents of a base in an inert solvent or without a solvent, ordinarily, at a temperature between 10°C and 200°C for 10 minutes to 48 hours.
Examples of the inert solvent include dichloromethane, chloroform, THF, 1,4-dioxane, dimethoxyethane, DMF, DMA, benzene, toluene, xylene, acetonitrile, ethyl acetate, pyridine and tetralin. These solvents can be used alone or as a mixture thereof.
Examples of the base include triethylamine, diisopropylethylamine, pyridine, N-methylmorpholine, potassium carbonate, sodium hydride and calcium hydride.
Compound (X3) can also be obtained by reacting Compound (X2) with 1 equivalent to a large excess of Compound (XVIII) in the presence of preferably 1 to 3 equivalents of silver oxide in an inert solvent or without a solvent.
Examples of the inert solvent include dichloromethane, chloroform, THF, 1,4-dioxane, dimethoxyethane, DMF, DMA, benzene, toluene, xylene, acetonitrile and ethyl acetate. These solvents can be used alone or as a mixture thereof.

Step 38

Compound (IH-q) can be obtained by using Compound (X3) in a reaction similar to that in Step 2-1 or Step 2-2 of Production Process 1.

Production Process 17

Compound (IH-r) can be manufactured by the following steps:

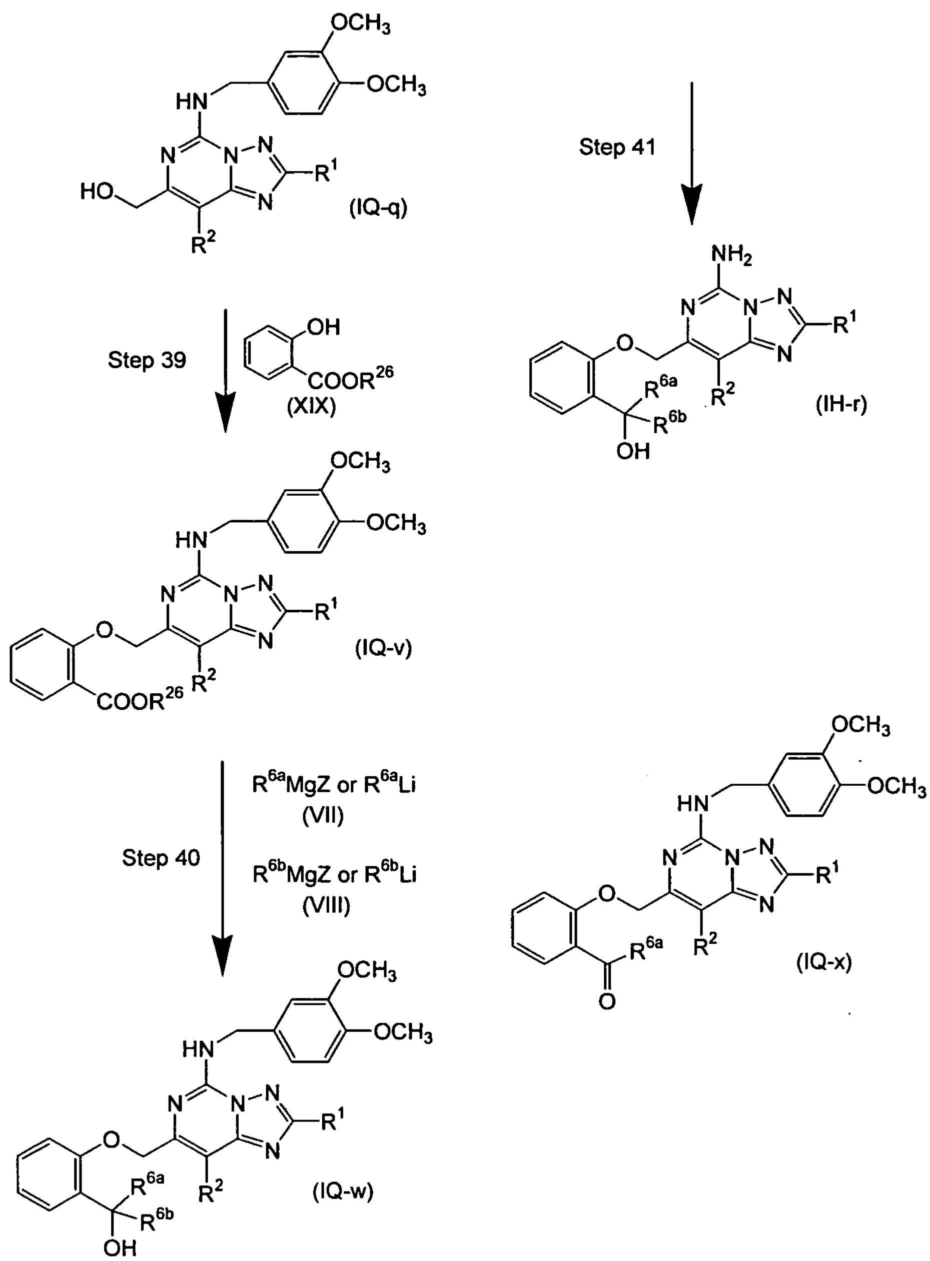


(wherein $R^1$, $R^2$, $R^{6a}$, $R^{6b}$, $R^{26}$ and Z have the same meanings as defined above, respectively).

Step 39

Compound (IQ-v) can be obtained by using Compound (IQ-q), which is obtained in Step 32 of Production Process 16, and Compound (XIX) in a reaction similar to that in Step 33 of Production Process 16.

Step 40

Compound (IQ-w), wherein $R^{6a}$ and $R^{6b}$ are the same, can be obtained by using Compound (IQ-v) in a reaction similar to that in Step 10-1 of Production Process 5. On the other hand, Compound (IQ-w), wherein $R^{6a}$ and $R^{6b}$ are different, can be obtained through Compound (IQ-x) by using Compound (IQ-v) in a reaction similar to that in Step 10-2 of Production Process 5.

Step 41

Compound (IH-r) can be obtained by using Compound (IQ-w) in a reaction similar to that in Step 2-1 or Step 2-2 of Production Process 1.

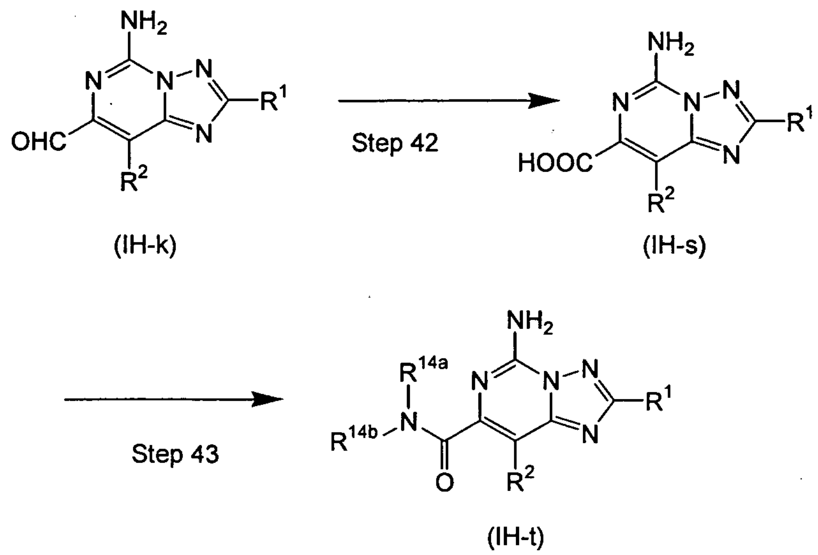


(IH-k) (IH-s) (IH-t)

(wherein $R^1$, $R^2$, $R^{14a}$ and $R^{14b}$ have the same meanings as defined above, respectively).

Production Process 18

Compound (IH-s) can be manufactured by the following step.

Step 42

Compound (IH-s) can be obtained by using Compound (IH-k), which is obtained in Step 23 or Step 27 of Production Process 11, in a reaction similar to that in Step 8 of Production Process 5.

Production Process 19

Compound (IH-t) can be manufactured by the following step.

Step 43

Compound (IH-t) can be obtained by using Compound (IH-s), which is obtained in Step 42 of Production Process 18, and $R^{14a}R^{14b}NH$ in a reaction similar to that in Step 31 of Production Process 15.

Production Process 20

Compound (IH-u) can be manufactured by the following steps.

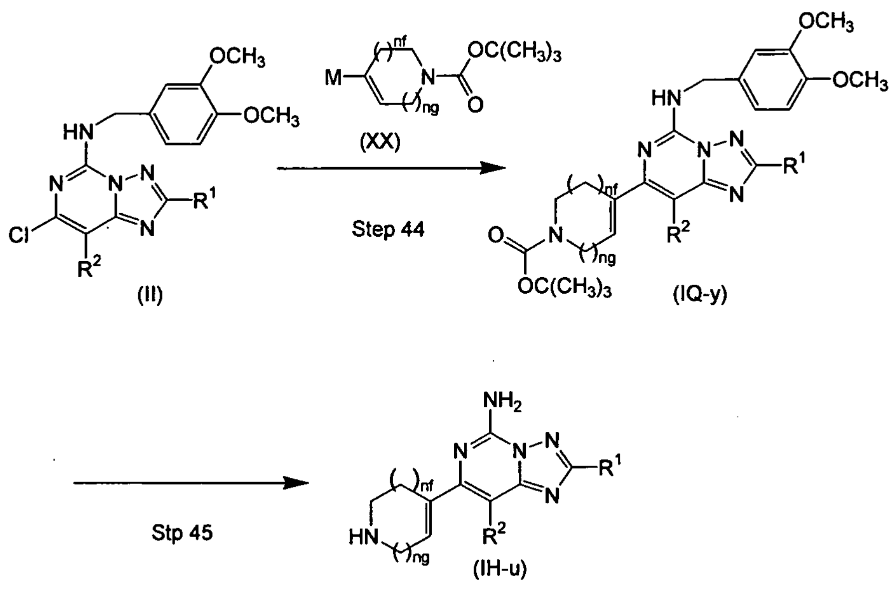


(wherein $R^1$, $R^2$, M, nf and ng have the same meanings as defined above, respectively).

Step 44

Compound (IQ-y) can be obtained by using Compound (II) and Compound (XX) in a reaction similar to that in Step 1 of Production Process 1.

Step 45

Compound (IH-u) can be obtained by using Compound (IQ-y) in a reaction similar to that in Step 2-1 of Production Process 1.

Production Process 21

Compound (IH-v) can be manufactured by the following step.

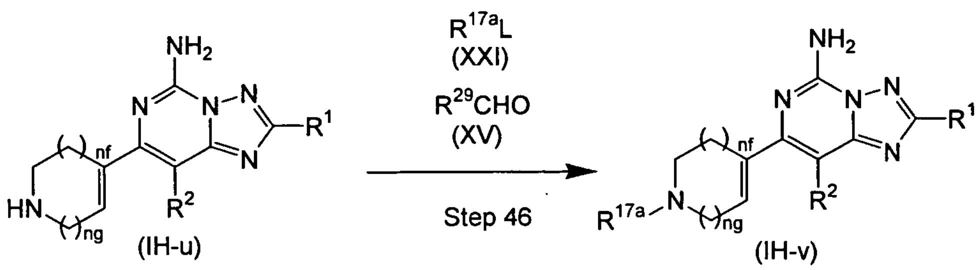


(wherein $R^1$, $R^2$, $R^{29}$, nf and ng have the same meanings as defined above, respectively; L represents halogen, methanesulfonyl, p-toluenesulfonyl or trifluoromethanesulfonyl; $R^{17a}$ represents the group produced by removing one hydrogen atom from the above-described $R^{17}$; the halogen has the same meaning as defined above).

Step 46

Compound (IH-v) can be obtained by reacting Compound (IH-u), which is obtained in Step 45 of Production Process 20, with 1 equivalent to a large excess of Compound (XXI) in the presence of 1 to 3 equivalents of a base in an inert solvent or without a solvent, ordinarily, at a temperature between 10°C and 200°C for 10 minutes to 48 hours.

Examples of the inert solvent include dichloromethane, chloroform, THF, 1,4-dioxane, dimethoxyethane, DMF, DMA, benzene, toluene, xylene, acetonitrile, ethyl acetate, pyridine and tetralin. These solvents can be used alone or

as a mixture thereof.

Examples of the base include triethylamine, diisopropylethylamine, pyridine, N-methylmorpholine, potassium carbonate and DBU.

Compound (IH-v) can also be obtained by using Compound (IH-u) and Compound (XV) in the same method as in Step 5 of Production Process 3.

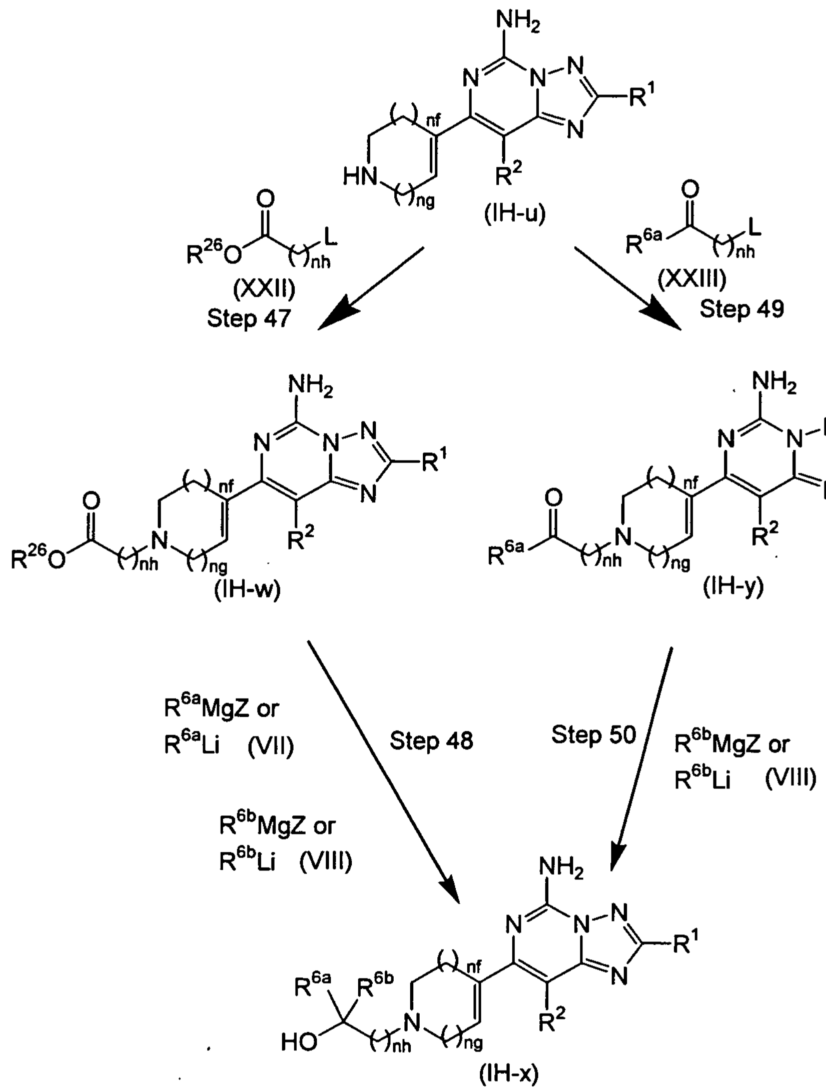


(wherein $R^1$, $R^2$, $R^{6a}$, $R^{6b}$, $R^{26}$, L, nf and ng have the same meanings as defined above, respectively; and nh represents an integer of 0 to 3).

Production Process 22

Compound (IH-w) can be manufactured by the following step.

Step 47

Compound (IH-w) can be obtained by using Compound (IH-u), which is obtained in Step 45 of Production Process 20, and Compound (XXII) in a reaction similar to that in Step 46 of Production Process 21.

Production Process 23

Compound (IH-x) can be manufactured by the following step.

Step 48

Compound (IH-x), wherein $R^{6a}$ and $R^{6b}$ are the same, can be obtained by using Compound (IH-w), which is obtained in Step 47 of Production Process 22, in a reaction similar to that in Step 10-1 of Production Process 5. On the other hand, Compound (IH-x), wherein $R^{6a}$ and $R^{6b}$ are different, can be obtained through Compound (IH-y) by using Compound (IH-w) in a reaction similar to that in Step 10-2.

Production Process 24

Compound (IH-x) can also be manufactured by the following steps.

Step 49

Compound (IH-y) can be obtained by using Compound (IH-u), which is obtained in Step 45 of Production Process 20, and Compound (XXIII) in a reaction similar to that in Step 46 of Production Process 21.

Step 50

Compound (IH-x) can be obtained by using Compound (IH-y) in a reaction similar to that in Step 10-1 of Production Process 5.

Production Process 25

Compound (IH-z) can be manufactured by the following step.

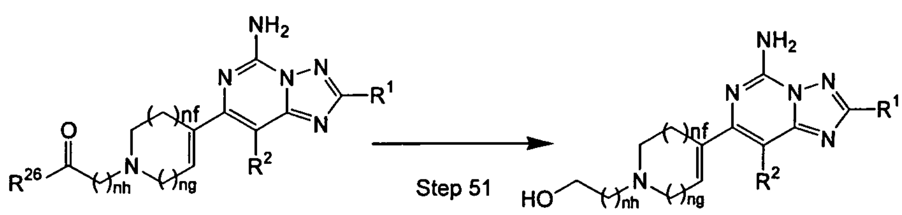


(IH-w) (IH-z)

(wherein $R^1$, $R^2$, $R^{26}$, nf, ng and nh have the same meanings as defined above, respectively).

Step 51

Compound (IH-z) can be obtained by using Compound (IH-w), which is obtained in Step 47 of Production Process 23, in a reaction similar to that in Step 6 of Production Process 4.

Production Process 26

Compound (IH-aa) can be manufactured by the following step.

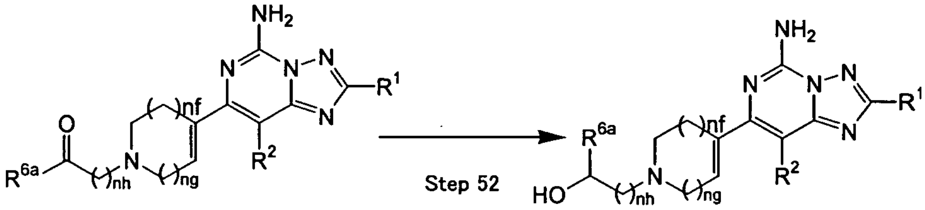


(IH-y) (IH-aa)

(wherein $R^1$, $R^2$, $R^{6a}$, nf, ng and nh have the same meanings as defined above, respectively).

Step 52

Compound (IH-aa) can be obtained by using Compound (IH-y), which is obtained in Step 49 of Production Process 24, in a reaction similar to that in Step 6 of Production Process 4.

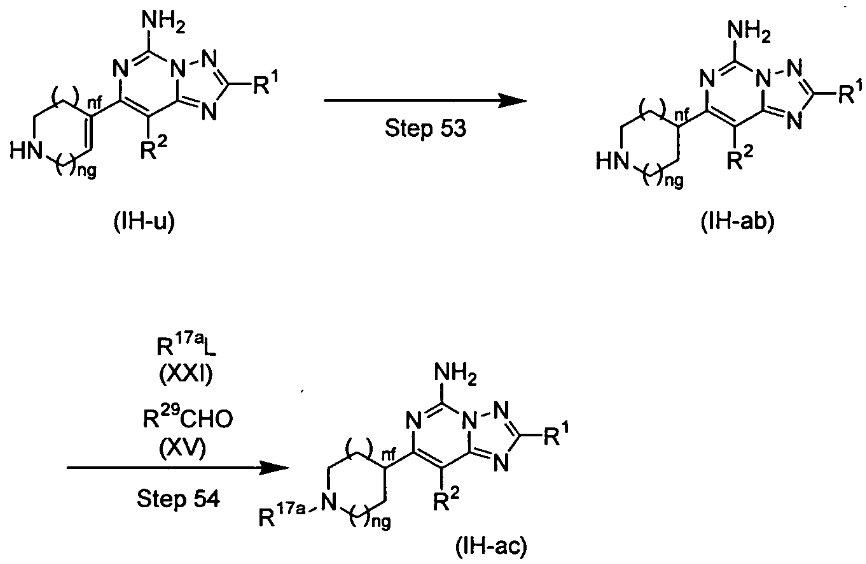


(IH-u) (IH-ab)

(IH-ac)

(wherein $R^1$, $R^2$, $R^{17a}$, L, nf and ng have the same meanings as defined above, respectively).

Production Process 27

Compound (IH-ab) can be manufactured by the following step.

Step 53

Compound (IH-ab) can be obtained by treating Compound (IH-u), which is obtained in Step 45 of Production Process 20, in the presence of 0.01 to 1 equivalent, preferably 0.01 equivalent of, for example, 10% palladium (0)/ carbon powders, in an inert solvent in an atmosphere of hydrogen, at a temperature between 0°C and 100°C, preferably a temperature between 40°C and 80°C, for 10 minutes to 5 hours, preferably 1 to 2 hours.

Examples of the inert solvent include THF, 1,4-dioxane, DMF, DMA, DMSO, benzene, toluene, xylene, dichloromethane, chloroform, carbon tetrachloride, methanol, ethanol, propanol, isopropyl alcohol, butanol and hexane. These solvents can be used alone or as a mixture thereof. Among thesm, methanol or ethanol is preferable.

Production Process 28

Compound (IH-ac) can be manufactured by the following step.

Step 54

Compound (IH-ac) can be obtained by using Compound (IH-ab), which is obtained in Step 53 of Production Process 27, and Compound (XXI) or Compound (XV) in a reaction similar to that in Step 46 of Production Process 21.

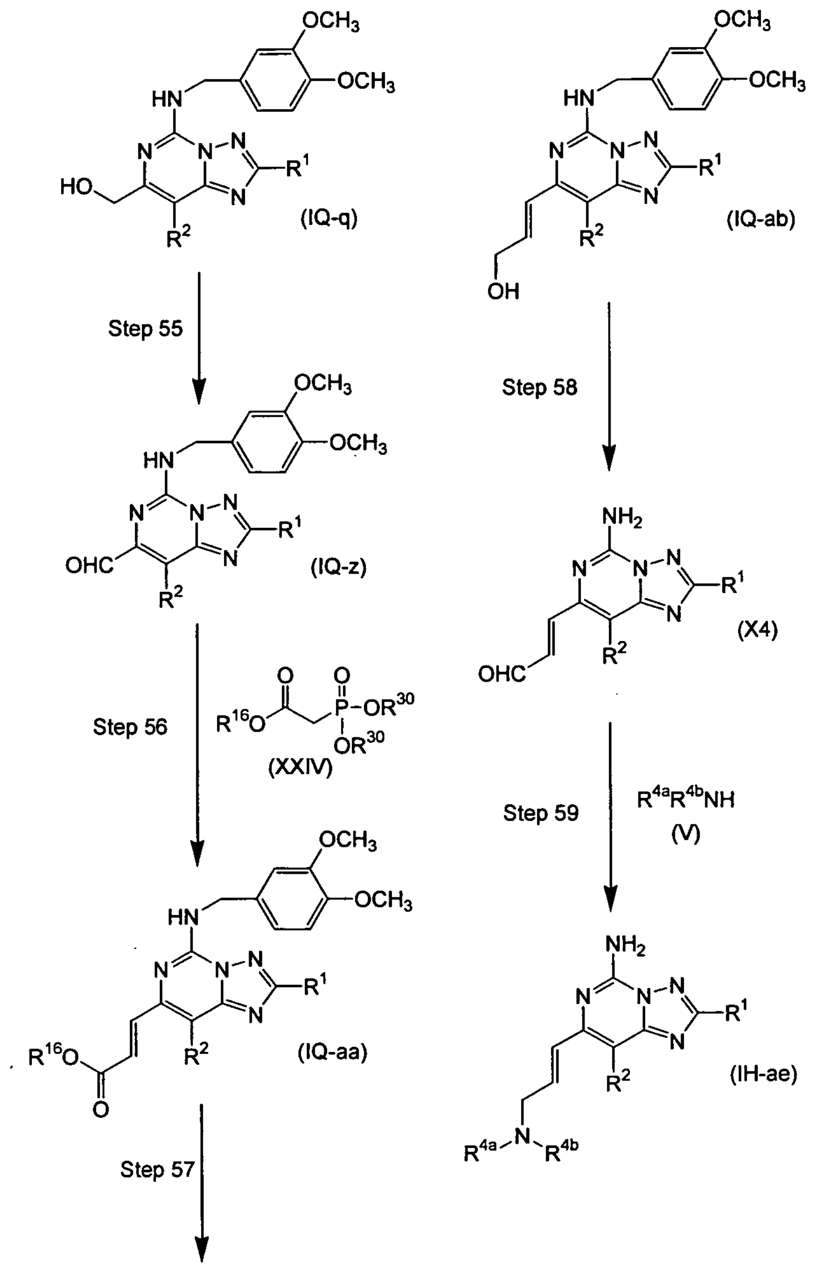


(wherein $R^1$, $R^2$, $R^{4a}$, $R^{4b}$ and $R^{16}$ r have the same meanings as defined above, respectively; and $R^{30}$ represents lower alkyl, the lower alkyl has the same meaning as defined above).

<u>Production Process 29</u>

Compound (X4) can be manufactured by the following steps.

Step 55

Compound (IQ-z) can be obtained by using Compound (IQ-q), which is obtained in Step 32 of Production Process 16, in a reaction similar to that in Step 23 of Production Process 11.

Step 56

Compound (IQ-aa) can be obtained by reacting Compound (IQ-z) with 1 to 5 equivalents of Compound (XXIV)

in the presence of 1 to 5 equivalents of a base in an inert solvent, ordinarily, at a temperature between 0°C and 200°C for 10 minutes to 48 hours.

Examples of the inert solvent include dichloromethane, chloroform, THF, 1,4-dioxane, dimethoxyethane, DMF, DMA, benzene, toluene, xylene, acetonitrile, ethyl acetate, pyridine and tetralin. These solvents can be used alone or as a mixture thereof. Among them, THF is preferable.

Examples of the base include triethylamine, diisopropylethylamine, pyridine, N-methylmorpholine, potassium carbonate, sodium hydride, potassium hydride and calcium hydride. Among them, sodium hydride or potassium hydride is preferable.

Step 57

Compound (IQ-ab) can be obtained by using Compound (IQ-aa) in a reaction similar to that in Step 6 of Production Process 4.

Step 58

Compound (X4) can be obtained by using Compound (IQ-ab) in a reaction similar to that in Step 2-2 of Production Process 1.

Production Process 30

Compound (IH-ae) can be manufactured by the following step.

Step 59

Compound (IH-ae) can be obtained by using Compound (X4) and Compound (V) in a reaction similar to that in Step 5 of Production Process 3.

Production Process 31

Compound (IH-af) can be manufactured by the following steps.

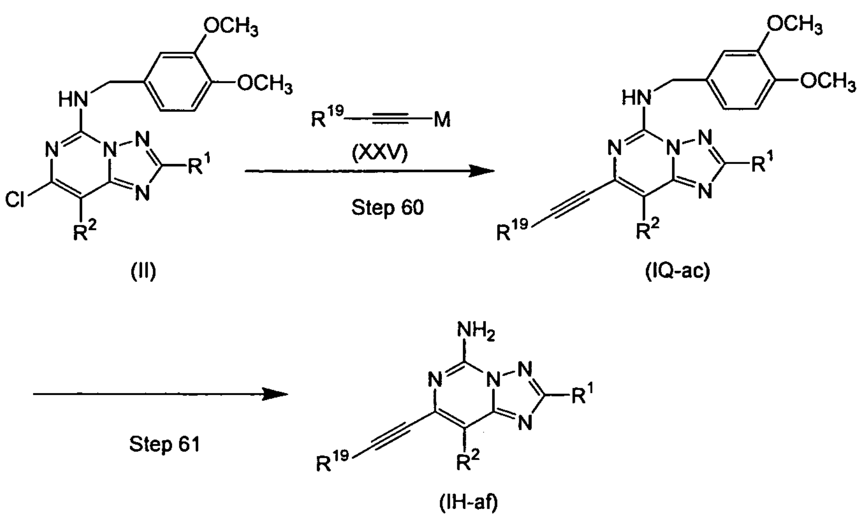


(II) (IQ-ac) (IH-af)

(wherein $R^1$, $R^2$, $R^{19}$ and M have the same meanings as defined above, respectively).

Step 60

Compound (IQ-ac) can be obtained by using Compound (II) and Compound (XXV) in a reaction similar to that in Step 1 of Production Process 1.

Step 61

Compound (IH-af) can be obtained by using Compound (IQ-ac) in a reaction similar to that in Step 2-1 or Step 2-2 of Production Process 1.

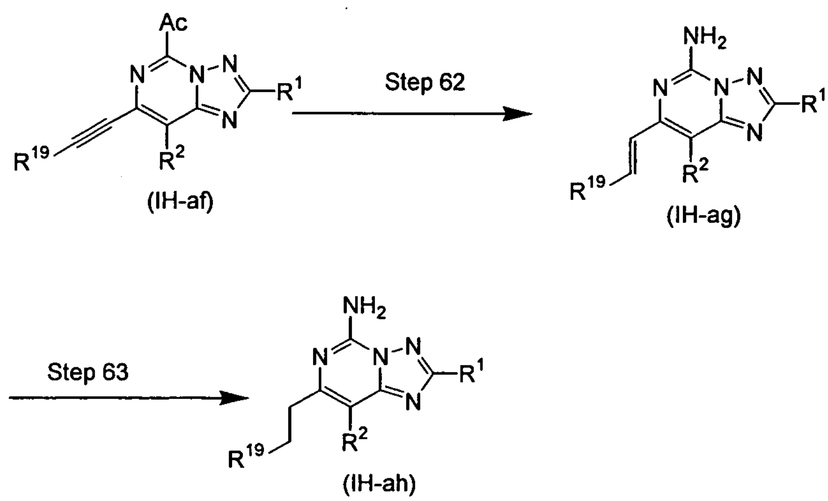


(IH-af) (IH-ag) (IH-ah)

(wherein $R^1$, $R^2$ and $R^{19}$ have the same meanings as defined above, respectively).

Production Process 32

Compound (IH-ag) can be manufactured by the following step.

Step 62

Compound (IH-ag) can be obtained by using Compound (IH-af), which is obtained in Step 61 of Production Process 31, in a reaction similar to that in Step 53 of Production Process 27.

Production Process 33

Compound (IH-ah) can be manufactured by the following step.

Step 63

Compound (IH-ah) can be obtained by using Compound (IH-ag), which is obtained in Step 62 of Production Process 32, in a reaction similar to that in Step 53 of Production Process 27.

Production Process 34

Compound (IH-ai) can be manufactured by the following steps.

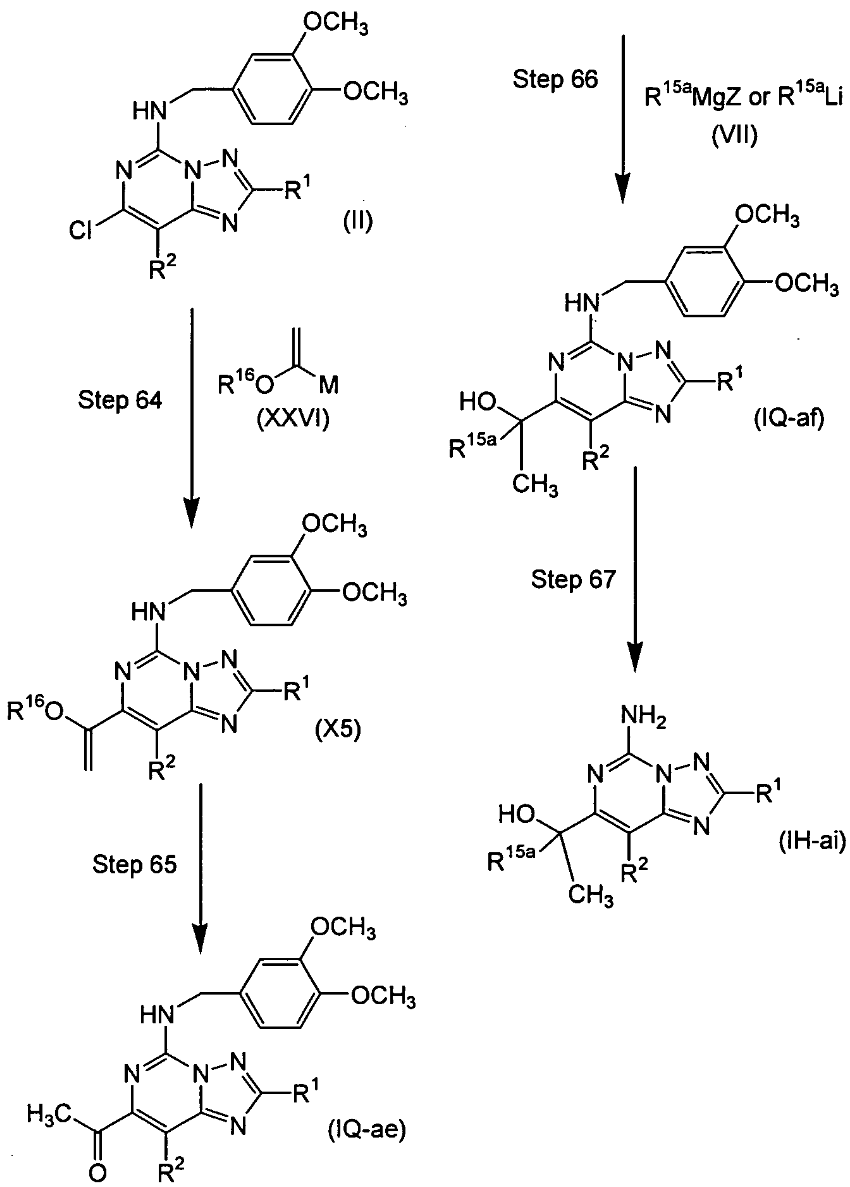


(wherein $R^1$, $R^2$, $R^{15a}$, $R^{16}$, M and Z represent the same as the above).

Step 64

Compound (X5) can be obtained by using Compound (II) and Compound (XXVI) in a reaction similar to that in Step 1 of Production Process 1.

Step 65

Compound (IQ-ae) can be obtained by treating Compound (X5) with an acid in an inert solvent, at a temperature between 0°C and the boiling point of the inert solvent used, preferably room temperature, for 1 to 48 hours, preferably 3 to 5 hours.

Examples of the inert solvent include diethyl ether, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methyl ether ketone, methanol, ethanol, propanol, isopropyl alcohol, butanol, hexane and water. These solvents can be used alone or as a mixture thereof. Among them, a mixed solvent containing THF and water is preferable.

Examples of the acid include hydrochloric acid, acetic acid, sulfuric acid, trifluoroacetic acid and trifluoromethanesulfonic acid. Among them, sulfuric acid is preferable.

Step 66

Compound (IQ-af) can be obtained by using Compound (IQ-ae) in a reaction similar to that in Step 10-1 of Production Process 5.

Step 67

Compound (IH-ai) can be obtained by using Compound (IQ-af) in a reaction similar to that in Step 2-1 or Step 2-2 of Production Process 1.

Production Process 35

Compound (IH-aj) can be manufactured by the following step.

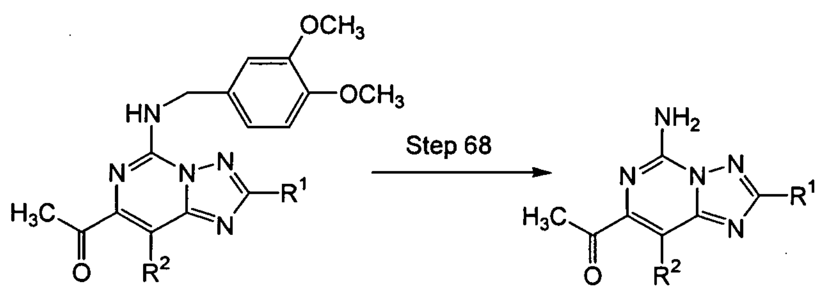


(IQ-ae) (IH-aj)

(wherein $R^1$ and $R^2$ have the same meanings as defined above, respectively).

Step 68

Compound (IH-aj) can be obtained by using Compound (IQ-ae) produced in Step 65 of Production Process 34 in a reaction similar to that in Step 2-1 or Step 2-2 of Production Process 1.

Production Process 36

Compound (IH-ak) can be manufactured by the following steps. Also, Compound (IH-aj) can be manufactured by the following steps.

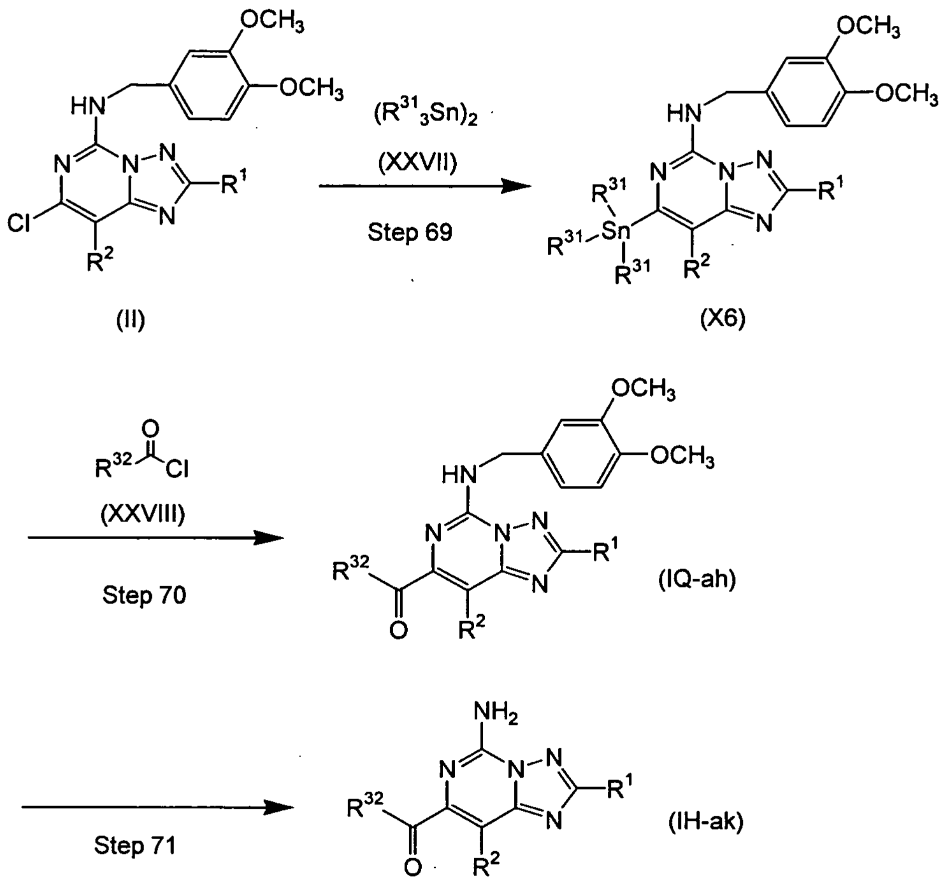


(wherein $R^1$ and $R^2$ have the same meanings as defined above, respectively; $R^{31}$ represents methyl or butyl; $R^{32}$ represents lower alkyl, aralkyl, substituted or unsubstituted aryl, or a substituted or unsubstituted aromatic heterocyclic group; the lower alkyl, the aralkyl, the aryl, the aromatic heterocyclic group, the substituents in the substituted aryl and the substituents in the substituted aromatic heterocyclic group have the same meanings as defined above, respectively).

Step 69

Compound (X6) can be obtained by using Compound (II) and 1 to 10 equivalents, preferably 1.5 equivalents of Compound (XXVII) in a reaction similar to that in Step 1 of Production Process 1.

Step 70

Compound (IQ-ah) can be obtained by using Compound (X6) and 1 to 10 equivalents, preferably 2 equivalents of Compound (XXVIII) in a reaction similar to that in Step 1 of Production Process 1.

Step 71

Compound (IH-ak) can be obtained by using Compound (IQ-ah) in a reaction similar to that in Step 2-1 or Step 2-2 of Production Process 1. Compound (IH-ak) having a methyl group as $R^{32}$ corresponds to Compound (IH-aj).

Production Process 37

Compound (IH-al) can be manufactured by the following steps.

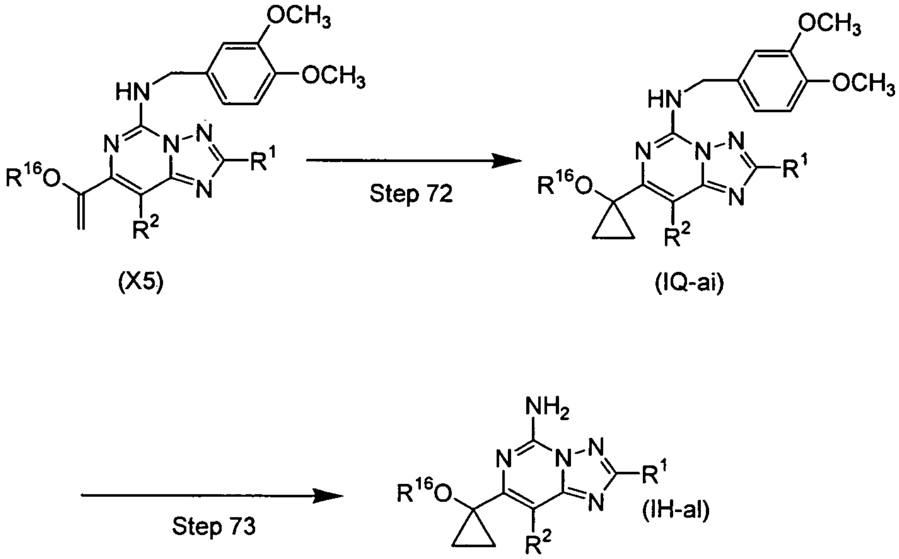


(X5) (IQ-ai) (IH-al)

(wherein $R^1$, $R^2$ and $R^{16}$ have the same meanings as defined above, respectively).

Step 72

Compound (IQ-ai) can be obtained by treating Compound (X5), which is obtained in Step 64 of Production Process 34, with 1 equivalent to a large excess of, for example, diiodomethane, dibromomethane, diazomethane or the like, preferably 5 to 10 equivalents of diiodomethane, and 1 equivalent to a large excess, preferably 5 to 10 equivalents of, for example, diethyl zinc, ethylzinc iodide, zinc, copper (I) chloride, copper (I) bromide, copper, silver or the like, which are used alone or as a mixture thereof, generally diethyl zinc, zinc and copper, or zinc and silver, preferably diethyl zinc, in an inert solvent, at a temperature between -78°C and 100°C, preferably between 0°C and room temperature.

Examples of the inert solvent include diethyl ether, THF, 1,4-dioxane, DMF, DMA, DMSO, benzene, toluene and xylene. These solvents can be used alone or as a mixture thereof. Among them, toluene is preferable.

Step 73

Compound (IH-al) can be obtained by using Compound (IQ-ai) in a reaction similar to that in Step 2-1 or Step 2-2 of Production Process 1.

Production Process 38

Compound (IH-am) can be manufactured by the following steps:

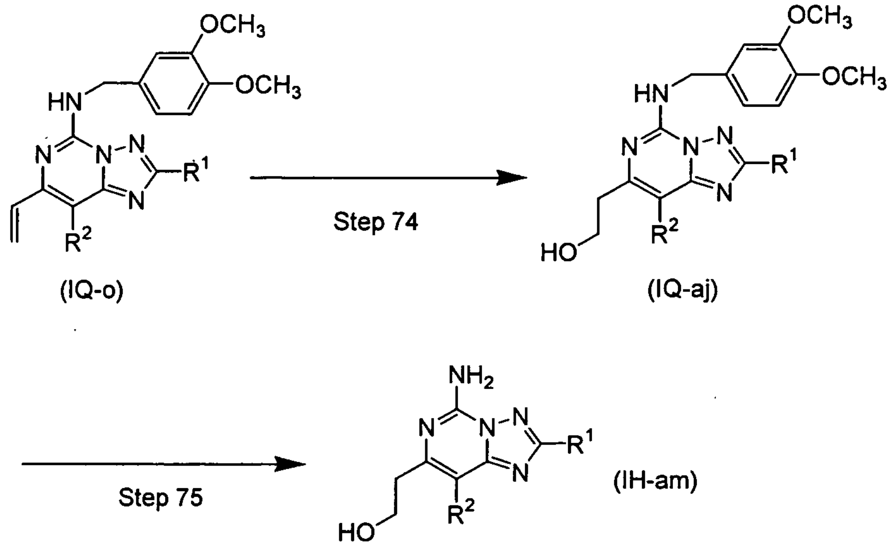


(IQ-o) (IQ-aj) (IH-am)

(wherein $R^1$ and $R^2$ have the same meanings as defined above, respectively).

Step 74

Compound (IQ-aj) can be obtained by treating Compound (IQ-o), which is obtained in Step 24 of Production Process 11, with 1 to 5 equivalents, preferably 2.5 equivalents of a hydroboration reagent in an inert solvent at a temperature between 0°C and 100°C, and further treated with 1 equivalent to a large excess of aqueous hydrogen peroxide in the presence of a base at a temperature between -30°C and room temperature.
Examples of the inert solvent include diethyl ether, THF, 1,4-dioxane, DMF, DMA, DMSO, benzene, toluene, xylene, dichloromethane and chloroform. These solvents can be used alone or as a mixture thereof. Among them, THF is preferable.
Examples of the hydroboration reagent include a borane-THF complex, methylborane, a borane-dimethyl sulfide complex, 9-borabicyclo[3.3.1]nonane and catecholborane. Among them, 9-borabicyclo[3.3.1]nonane is preferable.
Examples of the base include sodium hydroxide, potassium hydroxide, calcium hydroxide and potassium carbonate. Among them, sodium hydroxide is preferable.

Step 75

Compound (IH-am) can be obtained by using Compound (IQ-aj) in a reaction similar to that in Step 2-1 or Step 2-2 of Production Process 1.

Production Process 39

Compound (IH-an) can be manufactured by the following steps.

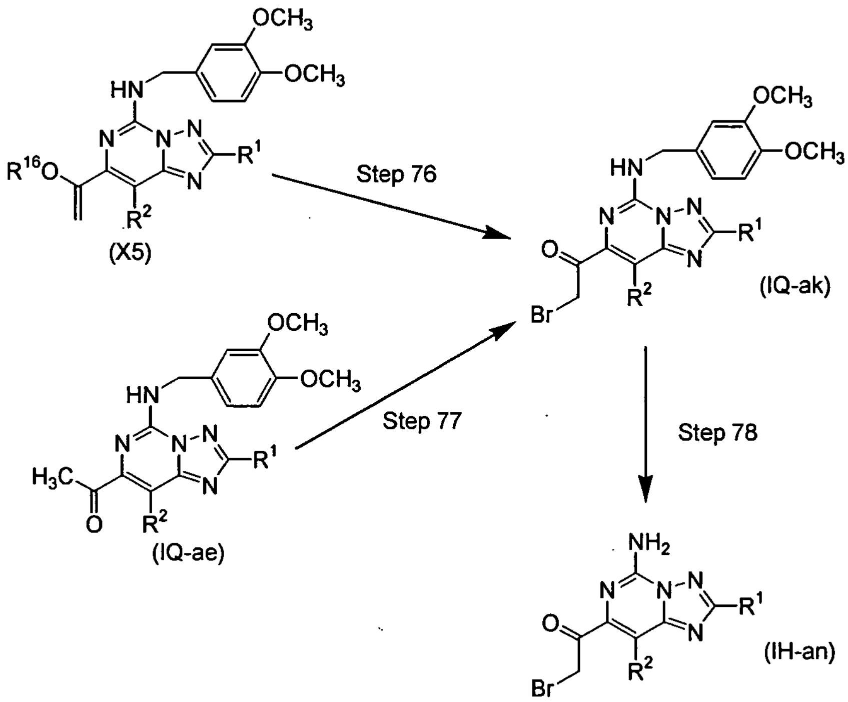


(X5) (IQ-ae) (IQ-ak) (IH-an)

(wherein $R^1$, $R^2$ and $R^{16}$ have the same meanings as defined above, respectively).

Step 76

Compound (IQ-ak) can be obtained by reacting Compound (X5), which is obtained in Step 64 of Production Process 34, with 1 to 3 equivalents, preferably 1 equivalent of a brominating agent in an inert solvent at a temperature between 0°C and room temperature.

Examples of the brominating agent include bromine, N-bromosuccinimide, 5,5-dibromo-2,2-dimethyl-4,6-dioxo-1,3-dioxane and tetrabutylammonium bromide. Among them, N-bromosuccinimide is preferable.

Examples of the inert solvent include diethyl ether, THF, 1,4-dioxane, DMF, DMA, DMSO, benzene, toluene, xylene, dichloromethane, chloroform, carbon tetrachloride, 1,2-dichloroethane, methanol, ethanol, propanol, isopropyl alcohol, butanol, hexane, acetic acid and hydrobromic acid. These solvents can be used alone or as a mixture thereof. Among them, THF is preferable.

Step 77

Compound (IQ-ak) can be obtained by using Compound (IQ-ae), which is obtained in Step 65 of Production Process 34, in a reaction similar to that in Step 76.

Step 78

Compound (IH-an) can be obtained by using Compound (IQ-k) in a reaction similar to that in Step 2-2 of Production Process 1.

Production Process 40

Compound (IH-ao) can be manufactured by the following step.

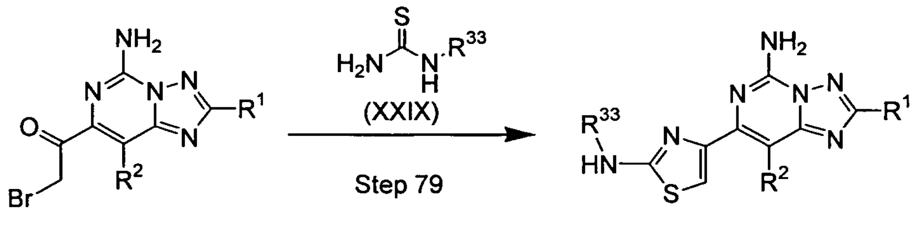


(IH-an) (IH-ao)

(wherein $R^1$ and $R^2$ have the same meanings as defined above, respectively, and $R^{33}$ has the same meaning as the above-described $R^{8a}$).

Step 79

Compound (IH-ao) can be obtained by reacting Compound (IH-an), which is obtained in Step 78 of Production Process 39, with 1 to 3 equivalents, preferably 2 equivalents of Compound (XXIX) in an inert solvent at a temperature between room temperature and 150°C.

Examples of the inert solvent include diethyl ether, THF, 1,4-dioxane, DMF, DMA, DMSO, benzene, toluene, xylene, dichloromethane, chloroform, carbon tetrachloride, 1,2-dichloroethane, methanol, ethanol, propanol, isopropyl alcohol, butanol and hexane. These solvents can be used alone or as a mixture thereof. Among them, ethanol is preferable.

Production Process 41

Compound (IH-ap) can be manufactured by the following step.

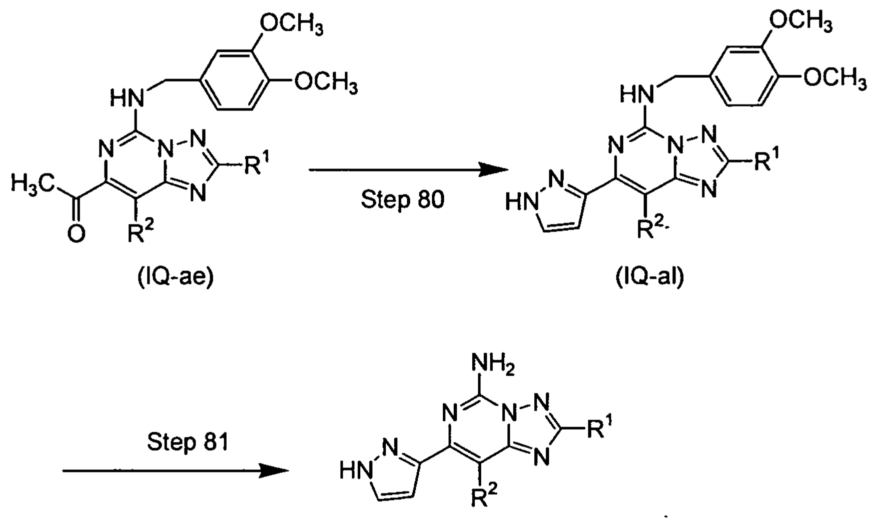


(IQ-ae) (IQ-al)

(IH-ap)

(wherein $R^1$ and $R^2$ have the same meanings as defined above, respectively).

Step 80

Compound (IQ-al) can be obtained by reacting Compound (IQ-ae), which is obtained in Step 65 of Production Process 34, with N,N-dimethylformamide dimethylacetal, which is added thereto as both a reagent and a solvent, at a temperature between room temperature and 200°C, preferably 140°C, for 1 to 48 hours, preferably 3 to 5 hours, and then reacting the resulting product with 1 to 3 equivalents, preferably 2 equivalents of hydrazine in an inert solvent.

Examples of the inert solvent include diethyl ether, THF, 1,4-dioxane, DMF, DMA, DMSO, benzene, toluene, xylene, dichloromethane, chloroform, carbon tetrachloride, 1,2-dichloroethane, methanol, ethanol, propanol, isopropyl alcohol, butanol and hexane. These solvents can be used alone or as a mixture thereof. Among them, ethanol is

preferable.

Step 81

Compound (IH-ak) can be obtained by using Compound (IQ-al) in a reaction similar to that in Step 2-2 of Production Process 1.

The intermediates and the desired compounds in the above-described production processes can be isolated and purified by subjecting them to purification methods usually used in synthetic organic chemistry, for example, filtration, extraction, washing, drying, concentration, recrystallization, various kinds of chromatography, such as high performance liquid chromatography, thin layer chromatography and silica gel chromatography. The intermediates can also be subjected to the subsequent reactions without purification. In separation and purification, a scavenger resin, which are usually used in combinatorial chemistry, such as acid chloride resin, aldehyde resin, basic ion-exchange resin or acid ion-exchange resin, can be used. The acid chloride resin can be obtained by the method described in a document [Tetrahedron Letters, vol. 37, No. 40, p. 7193 (1996), or the like] and used.

When it is desired to obtain a salt of Compound (I), in the case where Compound (I) is produced in the form of the salt, it can be purified as it is, but where it is produced in its free form, it can be converted into a salt, after being dissolved or suspended in an appropriate solvent, by adding an appropriate acid or base and then isolated and purified. Furthermore, Compound (I) or pharmaceutically acceptable salts thereof may exist in the form of adducts with water or various solvents, and these adducts are also included among the present invention.

Examples of Compound (I) obtained in the present invention are shown in Tables 1 to 8. In the tables, Me, Et and $^{i}Pr$ represent methyl, ethyl and isopropyl, respectively.

Table 1

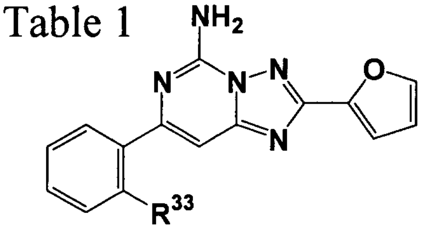

| Compound No. | $R^{33}$ |
| --- | --- |
| 1 | H |
| 2 | CHO |
| 3 | 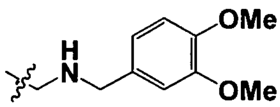 |
| 4 | 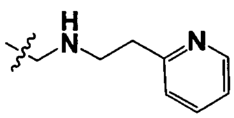 |
| 5 | 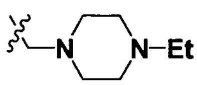 |
| 6 | 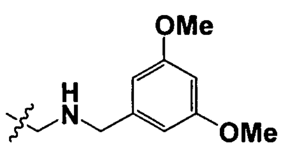 |
| 7 | 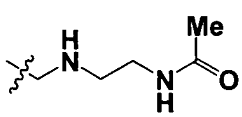 |
| 8 | 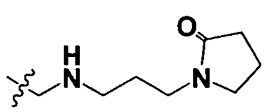 |
| 9 | 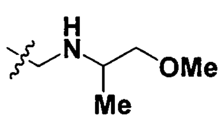 |

Table 2

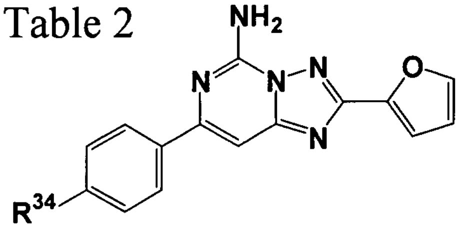

| Compound No. | $R^{34}$ |
| --- | --- |
| 10 | CHO |
| 11 | 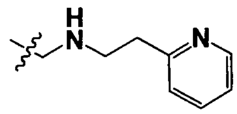 |
| 12 | 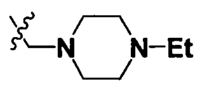 |
| 13 | 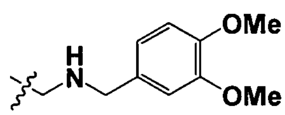 |
| 14 | 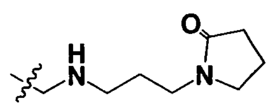 |
| 15 | 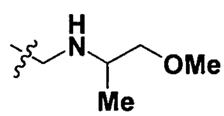 |
| 16 | 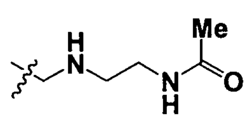 |
| 17 | 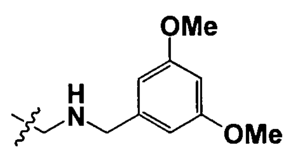 |

Table 3

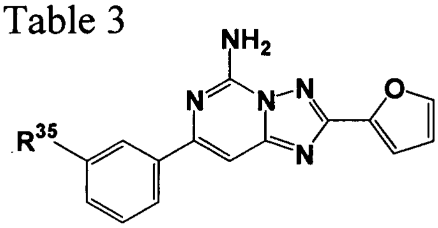

| Compound No. | $R^{35}$ |
|---|---|
| 18 | CHO |
| 19 | 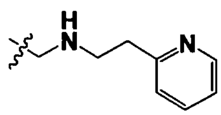 |
| 20 | 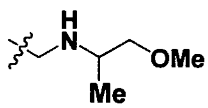 |
| 21 | 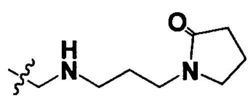 |

Table 4

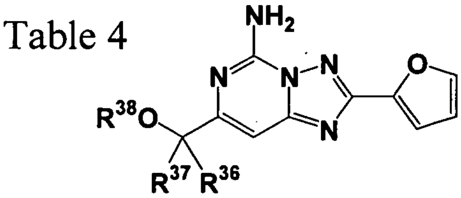

| Compound No. | $R^{36}$ | $R^{37}$ | $R^{38}$ |
|---|---|---|---|
| 22 | H | H | H |
| 23 | H | H | Me |
| 24 | H | H | Et |
| 25 | Me | Me | H |
| 26 | cyclopropyl | | Et |
| 27 | H | H | 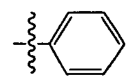 |
| 28 | H | H | 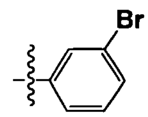 |
| 29 | H | H | 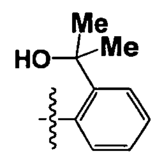 |
| 30 | H | H | 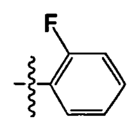 |
| 31 | H | H | 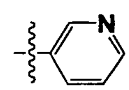 |
| 32 | H | H | 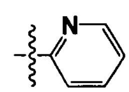 |
| 33 | H | H | 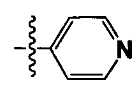 |

Table 5

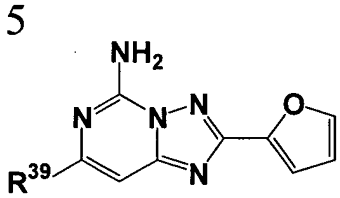

| Compound No. | $R^{39}$ | Compound No. | $R^{39}$ |
|---|---|---|---|
| 34 | 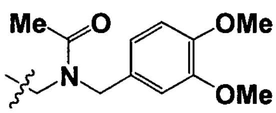 | 44 | 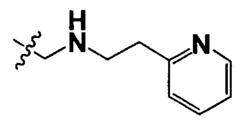 |
| 35 | 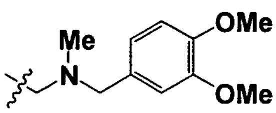 | 45 | 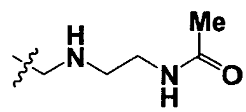 |
| 36 | 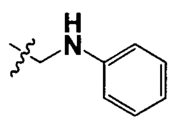 | 46 | 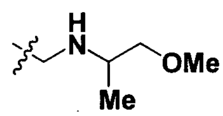 |
| 37 | 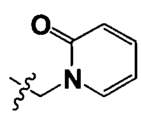 | 47 | 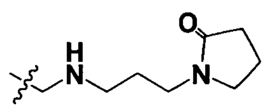 |
| 38 | COMe | 48 | 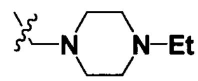 |
| 39 | 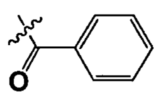 | 49 | 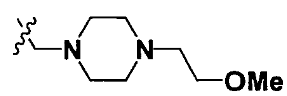 |
| 40 | 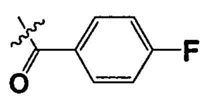 | 50 | 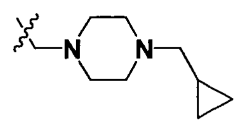 |
| 41 | CHO | 51 | 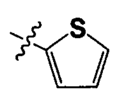 |
| 42 | 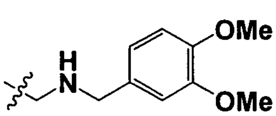 | 52 | 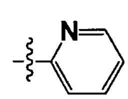 |
| 43 | 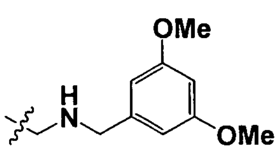 | 53 | 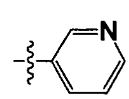 |

Table 5 (continued)

| Compound No. | $R^{39}$ |
|---|---|
| 54 | 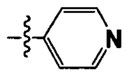 |
| 55 | 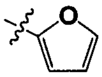 |
| 56 | 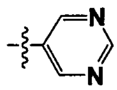 |
| 57 | 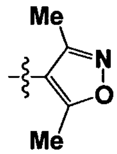 |
| 58 | 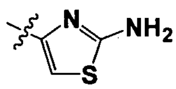 |
| 59 | 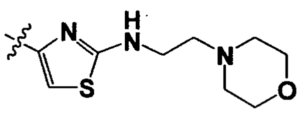 |
| 60 | 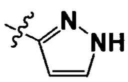 |
| 61 | 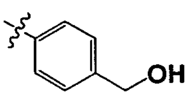 |
| 62 | 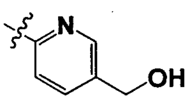 |
| 63 | 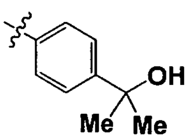 |

Table 6 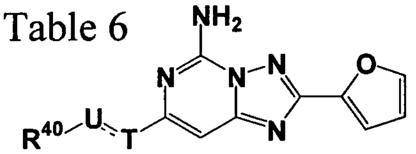

| Compound No. | T---U | $R^{40}$ |
|---|---|---|
| 64 | $H_2C—CH_2$ | Et |
| 65 | C≡C | 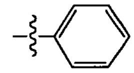 |
| 66 | $H_2C—CH_2$ | 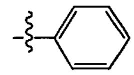 |
| 67 | $H_2C—CH_2$ | OH |
| 68 | *(E)*- HC═CH | 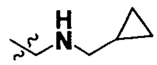 |
| 69 | C≡C | 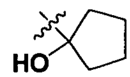 |

Table 7

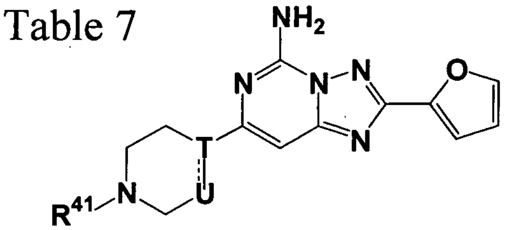

| Compound No. | T–U | $R^{41}$ |
|---|---|---|
| 70 | C═CH | 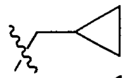 |
| 71 | HC—CH$_2$ | 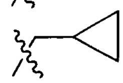 |
| 72 | C═CH | 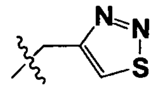 |
| 73 | C═CH | $CH_2CH_2OMe$ |
| 74 | C═CH | 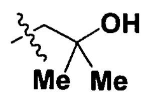 |
| 75 | C═CH | 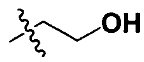 |
| 76 | C═CH | 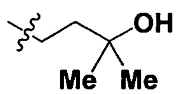 |
| 97 | C═CH | $CH_2CF_3$ |

Table 8

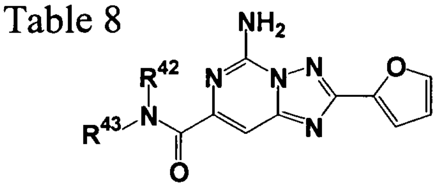

| Compound No. | $R^{42}$ | $R^{43}$ | Compound No. | $R^{42}$ | $R^{43}$ |
|---|---|---|---|---|---|
| 77 | H | 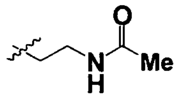 | 89 | H | 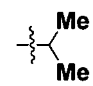 |
| 78 | Me | 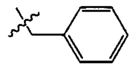 | 90 | H | 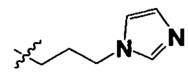 |
| 79 | 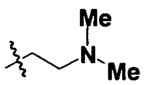 | 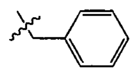 | 91 | H | 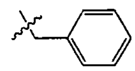 |
| 80 | H | 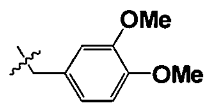 | 92 | H | 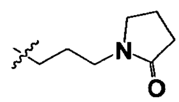 |
| 81 | Me | 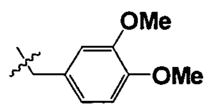 | 93 | H | 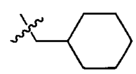 |
| 82 | H | 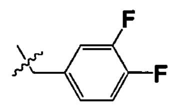 | 94 | 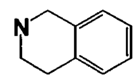 | |
| 83 | H | 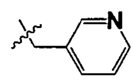 | 95 | 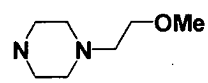 | |
| 84 | H | 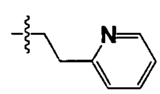 | 96 | 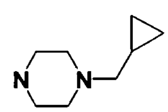 | |
| 85 | Me | 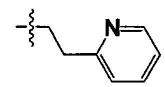 | 98 | H | Et |
| 86 | H | 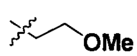 | | | |
| 87 | Me | 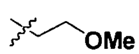 | | | |
| 88 | 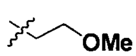 | 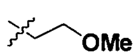 | | | |

Table 8 (continued)

| Compound No. | $R^{42}$ | $R^{43}$ |
|---|---|---|
| 99 | Et | 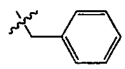 |
| 100 | $^{i}$Pr | 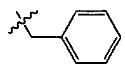 |
| 101 | Me | 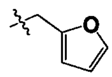 |
| 102 | Et | 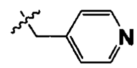 |
| 103 | 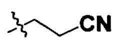 | 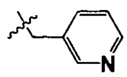 |
| 104 | Me | 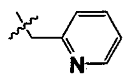 |
| 105 | Me | 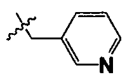 |
| 106 | Me | 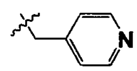 |
| 107 | Me | 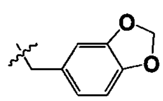 |
| 108 | Me | 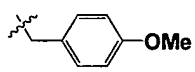 |
| 109 | Me | 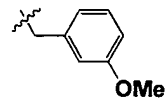 |
| 110 | Me | 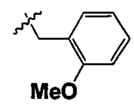 |
| 111 | Me | 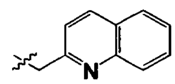 |
| 112 | Me | 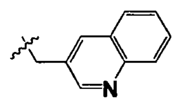 |
| 113 | Me | 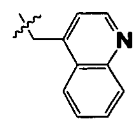 |
| 114 | $^{i}$Pr | 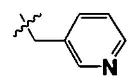 |
| 115 | Me | 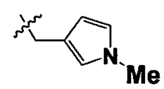 |
| 116 | $^{i}$Pr | 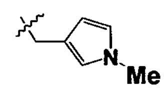 |
| 117 | Me | 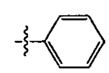 |
| 118 | Me | 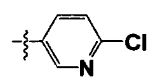 |

Table 8 (continued)

| Compound No. | $R^{42}$ | $R^{43}$ | Compound No. | $R^{42}$ | $R^{43}$ |
|---|---|---|---|---|---|
| 119 | Me | 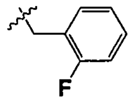 | 129 | Et | 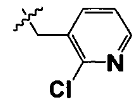 |
| 120 | Me | 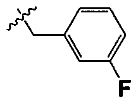 | 130 | Me | 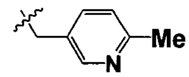 |
| 121 | Me | 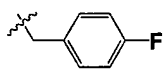 | 131 | Me | 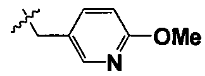 |
| 122 | Me | 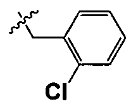 | 132 | Et | 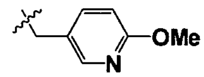 |
| 123 | Me | 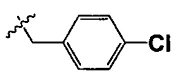 | 133 | 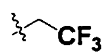 | 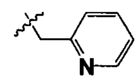 |
| 124 | Me | 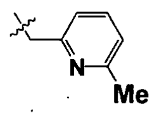 | 134 | Me | 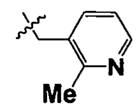 |
| 125 | $^{i}$Pr | 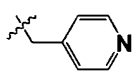 | 135 | Me | 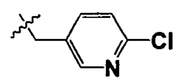 |
| 126 | 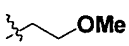 | 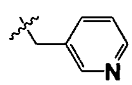 | 136 | Et | 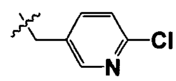 |
| 127 | Et | 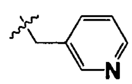 | 137 | Me | 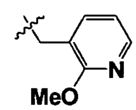 |
| 128 | Me | 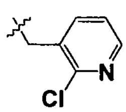 | 138 | Me | 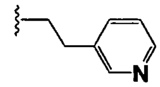 |
| | | | 139 | Me | 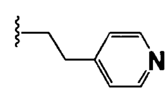 |

Table 8 (continued)

| Compound No. | $-NR^{42}R^{43}$ | Compound No. | $-NR^{42}R^{43}$ |
|---|---|---|---|
| 140 | 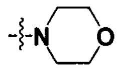 | 150 | 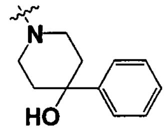 |
| 141 | 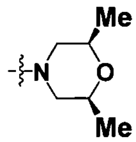 | 151 | 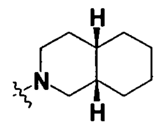 |
| 142 | 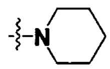 | 152 | 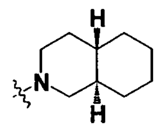 |
| 143 | 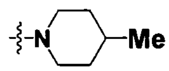 | 153 | 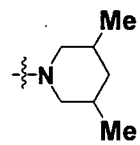 |
| 144 | 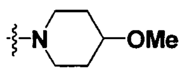 | 154 | 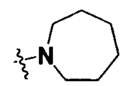 |
| 145 | 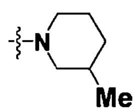 | 155 | 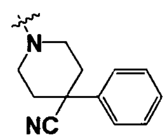 |
| 146 | 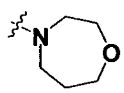 | 156 | 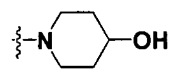 |
| 147 | 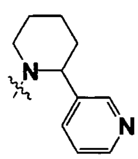 | 157 | 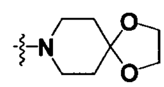 |
| 148 | 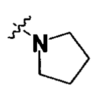 | 158 | 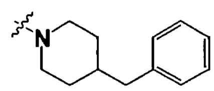 |
| 149 | 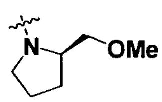 | 159 | 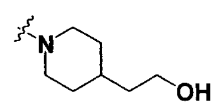 |

Table 8 (continued)

| Compound No. | $-NR^{42}R^{43}$ |
| --- | --- |
| 160 | 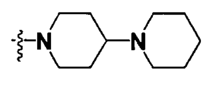 |
| 161 | 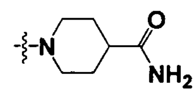 |
| 162 | 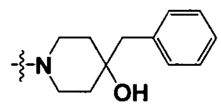 |

The pharmacological activity of Compound (I) will be described below with reference to test examples.

Test Example 1 Adenosine Receptor Binding Activity (Adenosine $A_{2A}$ Receptor Binding Test)

The test was carried out according to the method of Bruns et al. [Molecular Pharmacology, vol. 29, p. 331z (1986)] with slight modification.

Rat corpus striatum was suspended in an ice-cold 50 mmol/L tris(hydroxymethyl)aminomethane hydrochloride (referred to as "Tris HCl" hereinafter) buffer (pH 7.7) using a Polytron homogenizer (produced by Kinematica Co.). The suspension was centrifuged (50,000 xg, 10 minutes), and the resulting precipitate was again suspended in the same amount of a 50 mmol/L Tris HCl buffer, followed by centrifugation under the same conditions. The resulting finalprecipitate was again suspended in a 50 mmol/L Tris HCl buffer [containing 10 mmol/L of magnesium chloride and 0.02 unit/mg tissue of adenosine deaminase (produced by Sigma Co.)] so that the tissue concentration was 5 mg (wet weight)/mL.

To 1 ml of the thus-prepared tissue suspension were added 50 μL of tritium-labeled CGS 21680 [$^3$H-2-[p-(2-carboxyethyl)phenethylamino]-5'-(N-ethylcarboxamido) adenosine: 40 Ci/mmol; produced by New England Nuclear Co. (The Journal of Pharmacology and Experimental Therapeutics, vol. 251, p. 888 (1989)] (final concentration: 4.0 nmol/L) and 50 μL of a test compound. The resulting mixture was allowed to stand at 25°C for 120 minutes and then rapidly filtered by suction through a glass fiber filter (GF/C produced by Whatman Co.). The filter was immediately washed three times with 5 μL each of an ice-cold 50 mmol/L Tris HCl buffer. Then, the glass fiber filer was transferred to a vial, and a scintillator (EX-H produced by Wako Pure Chemical Industries, Ltd.) was added thereto. The radioactivity on the filter was determined with a liquid scintillation counter (produced by Packard Instrument Co.).

The inhibition rate of each test compound against $A_{2A}$ receptor binding ($^3$H-CGS 21680 binding) was calculated by the following equation:

$$\text{Inhibition rate (\%)} = \{1 - (X - Y)/(Z - Y)\} \times 100$$

In the equation, X represents Amount of binding in the presence of a medicament, Y represents Amount of nonspecific binding, and Z represents Amount of total binding.

(Note) "Amount of total binding (Z)" represents the radioactivity of $^3$H-CGS 21680 bound in the absence of the test compound. "Amount of nonspecific binding (Y)" represents the radioactivity of $^3$H-CGS 21680 bound in the presence of 100 μmol/L cyclopentyladenosine (CPA; produced by Sigma Co.). "Amount of binding (X) in the presence of a medicine" represents the radioactivity of $^3$H-CGS 21680 bound in the presence of the test compound at a varied concentrations.

The results are shown in Table 9.

Table 9

| Compound No. | $A_{2A}$ Receptor inhibition rate (%) at $10^{-7}$ mol/L | Compound No. | $A_{2A}$ Receptor inhibition rate (%) at $10^{-7}$ mol/L |
|---|---|---|---|
| 1 | 65 | 99 | 69 |
| 7 | 64 | 101 | 83 |
| 11 | 53 | 103 | 54 |
| 16 | 36 | 107 | 71 |
| 17 | 70 | 108 | 65 |
| 25 | 47 | 113 | 66 |
| 38 | 84 | 116 | 62 |
| 39 | 66 | 120 | 61 |
| 40 | 58 | 122 | 74 |
| 41 | 54 | 128 | 59 |
| 42 | 62 | 131 | 51 |
| 43 | 60 | 134 | 60 |
| 44 | 56 | 142 | 71 |
| 46 | 73 | 143 | 70 |
| 49 | 89 | 145 | 60 |
| 50 | 65 | 148 | 75 |
| 51 | 68 | 151 | 71 |
| 52 | 92 | 154 | 56 |
| 58 | 70 | | |
| 64 | 81 | | |
| 65 | 96 | | |
| 66 | 82 | | |
| 67 | 91 | | |
| 68 | 96 | | |
| 69 | 78 | | |
| 71 | 87 | | |
| 73 | 98 | | |
| 76 | 72 | | |
| 85 | 70 | | |
| 89 | 89 | | |
| 94 | 95 | | |

Compound (I) or pharmaceutically acceptable salt thereof show strong adenosine $A_{2A}$ receptor antagonism. Therefore, it was suggested that a medicament containing Compound (I) as an active ingredient is effective for diseases (for example, Parkinson's disease, dementia including senile dementia, depression, and the like) induced by hyperactivity of an adenosine $A_{2A}$ receptor.

Test Example 2 Activity on CGS 21680-Induced Catalepsy

Parkinson's disease is a motor disorder based on degeneration and cell death of the nigrostriatal dopaminergic neurons. When CGS 21680 (adenosine $A_{2A}$ receptor agonist) is administered into the intracerebral ventricle, the inhibitory synaptic transmission of GABA in the medium sized spiny neurons in the corpus striatum is inhibited directly via the adenosine $A_{2A}$ receptor [Journal of Neuroscience, vol. 16, p. 605 (1996)]. Accordingly, it is concidered that the adenosine $A_{2A}$ receptor agonists positively regulate the output of the GABAergic neurons from the corpus striatum to the globus pallidus lateralis, and, as a result, catalepsy is induced by the administration of CGS 21680.

The test was carried out using ten 5-week age male ddY mice (22 - 25 g in body weight, Japan SLC) per group. CGS 21680 (produced by RBI Co) was dissolved in physiological saline (produced by Otsuka Pharmaceutical Co., Ltd.), and 10 µg/20 µL of the solution was injected into mouse intracerebral ventricle. Each test compound was used by suspending it in distilled water containing 0.3% of polyoxylene (20) sorbitan monooleate (referred to as "Tween 80" hereinafter) (produced by Otsuka Pharmaceutical Co., Ltd.). The suspension containing each test compound or a solution containing no test Compound (distilled water containing 0.3% of Tween 80; used as a control) was orally administered (0.1 mL per 10 g of mouse body weight) 30 minutes before the injection of CGS 21680 into the intracerebral ventricle. One hour after the administration of the each test compound, only forelimbs or only hindlimbs of each mouse were laid on a vertically arranged stand made of acryl and having a size of 4.5 cm in height and 1.0 cm in width to measure catalepsy symptoms. All of the test compounds were administered orally in a dose of 10 mg/kg.

The criteria of the catalepsy score are shown below.

Table 10

| Criteria of Catalepsy Score | |
|---|---|
| Score | Duration of catalepsy |
| 0 | The postures of both forelimbs and hindlimbs laid on the stand lasted for less than 5 seconds. |
| 1 | The posture of forelimbs laid on the stand lasted for 5 seconds or more and less than 10 seconds, and that of hindlimbs lasted for less than 5 seconds. |
| 2 | The posture of forelimbs laid on the stand lasted for 10 second or more, and that of hindlimbs lasted for less than 5 seconds. |
| 3 | (1) The postures of both forelimbs and hindlimbs laid on the stand lasted for 5 seconds or more and less than 10 seconds, or (2) The posture of forelimbs laid on the stand lasted for less than 5 seconds, and that of hindlimbs lasted for 5 seconds or more. |
| 4 | (1) The posture of forelimbs laid on the stand lasted for 10 seconds or more, and that of hindlimbs lasted for 5 seconds or more and less than 10 seconds, or (2) The posture of forelimbs laid on the stand lasted for 5 seconds or more and less than 10 seconds, and that of hindlimbs lasted for 10 seconds or more. |
| 5 | The postures of both forelimbs and hindlimbs laid on the stand lasted for 10 seconds or more. |

The effect was decided by a total catalepsy score of 10 mice in one group (the maximum score: 50 points). When the total score was 40 points or less, the activity of the compounds was decided positive. The number of the animals showing remission of catalepsy was expressed by the number of cases showing a catalepsy score of 4 points or less in 10 cases. The catalepsy remission ratio was expressed as the percentage of the total score in the test compound-administered group to the total score in the control group.

The results are shown in Table 11.

Table 11

| Compound No. | Number of animals used | Total score | Number of animals showing remission | Remission ratio (%) |
|---|---|---|---|---|
| 22 | 10 | 8 | 10 | 84 |
| 26 | 10 | 9 | 10 | 82 |
| 29 | 10 | 6 | 10 | 88 |
| 35 | 10 | 5 | 9 | 90 |
| 40 | 10 | 3 | 10 | 94 |
| 49 | 10 | 4 | 10 | 92 |
| 52 | 10 | 8 | 9 | 84 |
| 53 | 10 | 8 | 10 | 84 |

Table 11 (continued)

| Compound No. | Number of animals used | Total score | Number of animals showing remission | Remission ratio (%) |
|---|---|---|---|---|
| 58 | 10 | 10 | 10 | 80 |
| 60 | 10 | 1 | 10 | 98 |
| 62 | 10 | 10 | 9 | 80 |
| 63 | 10 | 1 | 10 | 98 |
| 69 | 10 | 2 | 10 | 96 |
| 72 | 10 | 0 | 10 | 100 |
| 73 | 10 | 6 | 9 | 88 |
| 75 | 10 | 2 | 10 | 96 |
| 76 | 10 | 5 | 10 | 80 |
| 80 | 10 | 6 | 9 | 88 |
| 81 | 10 | 2 | 10 | 96 |
| 83 | 10 | 2 | 10 | 96 |
| 84 | 10 | 4 | 10 | 92 |
| 85 | 10 | 3 | 10 | 94 |
| 86 | 10 | 0 | 10 | 100 |
| 87 | 10 | 9 | 9 | 82 |
| 89 | 10 | 8 | 9 | 84 |
| 91 | 10 | 3 | 10 | 94 |
| 92 | 10 | 9 | 9 | 82 |
| 94 | 10 | 5 | 10 | 90 |
| 100 | 10 | 15 | 8 | 64 |
| 102 | 10 | 4 | 10 | 92 |
| 105 | 10 | 9 | 9 | 82 |
| 107 | 10 | 5 | 10 | 90 |
| 108 | 10 | 2 | 10 | 96 |
| 113 | 10 | 12 | 8 | 76 |
| 116 | 10 | 9 | 9 | 82 |
| 118 | 10 | 3 | 10 | 94 |
| 121 | 10 | 14 | 9 | 72 |
| 122 | 10 | 10 | 10 | 80 |
| 129 | 10 | 5 | 9 | 90 |
| 130 | 10 | 15 | 8 | 70 |
| 131 | 10 | 6 | 10 | 88 |
| 138 | 10 | 11 | 9 | 78 |
| 143 | 10 | 5 | 10 | 90 |
| 144 | 10 | 7 | 9 | 84 |
| 149 | 10 | 15 | 7 | 70 |

Table 11 (continued)

| Compound No. | Number of animals used | Total score | Number of animals showing remission | Remission ratio (%) |
|---|---|---|---|---|
| 150 | 10 | 11 | 8 | 76 |
| 151 | 10 | 12 | 8 | 74 |
| 154 | 10 | 16 | 7 | 66 |
| 158 | 10 | 7 | 9 | 86 |
| 162 | 10 | 13 | 8 | 72 |

Test Example 3 Activity in Parkinson's Disease Model [common marmoset treated with 1-methyl-4-phenyl-1,2,3,6-tetrahydropyridine (MPTP)]

Parkinson's disease is a disease based on the degeneration and cell death of the nigrostriatal dopaminergic neurons. In the primates, treatment with a dopamine neurotoxin, 1-methyl-4-phenyl-1,2,3,6-tetrahydropyridine (abbreviated to "MPTP" hereinafter), causes selective degeneration and drop out of the nigrostriatal dopaminergic neurons and shows skinesia and muscular rigidity, or the like. These MPTP-treated primates are known as a model of Parkinson's disease [Proceedings of the National Academy of Science USA, vol. 80, p. 4546 (1983)]. Common marmoset belongs to Anthropoidae, and it is known that it shows symptoms of Parkinson's disease caused by MPTP as in the case of other animals of Anthropoidae [Neuroscience Letter, vol. 57, p. 37 (1985)].

The test was carried out using 4 female and male common marmosets of 2 to 3 year age (300 to 375 g in body weight, Japan CLEA) per group. MPTP (produced by RBI Co.) was dissolved in physiological saline for injection (produced by Otsuka Pharmaceutical Co., Ltd.), and hypodermically administered to the common marmosets once a day for 5 days in a dose of 2.0 mg/kg. Six weeks or more after the administration, animals showing chronic symptoms of Parkinson's disease were used in the test. Each test compound was used by suspending it in an aqueous solution containing 0.3% Tween 80 and 10% sucrose. Also, a solution containing no test compound was used as a control. One hour before the administration of the test compound, the animals to be tested were put into an observation cage (provided with a spontaneous locomotor activity measuring apparatus) to adapt them to the environment. The symptoms of Parkinson's disease were observed from a one-way see through window at intervals of 30 minutes for 8 hours to score the motor disability. The spontaneous locomotor activity was measured at intervals of 30 minutes for 12 hours by a computer-controlled automatic measuring apparatus. The symptoms of Parkinson's disease were decided on the basis of the criteria of each observation item shown below, and the total of the points was used as the score of each animal.

Relationship between observation items and scores is shown in Table 12.

Table 12

| Decision criteria for symptoms of Parkinson's disease | | | | | | |
|---|---|---|---|---|---|---|
| Observation items | Score | 0 | 1 | 2 | 3 | 4 |
| Attention | | Normal | Decrease | Sleeping tendency | | |
| Observation | | | Yes | Decrease | No | |
| Blinking | | | Normal | Abnormal | | |
| Posture | | Normal | Abnormality in trunk, tail or limbs (1 point) | | | All abnormal |
| Balance | | Normal | Asymmetry | Cannot stand | Drop out | |
| Reaction | | Normal | Decrease | Slow | No | |
| Utterance | | Normal | Decrease | No | | |
| Total | 0-17 points | | | | | |

The effect was decided by comparing average scores of the symptoms of Parkinson's disease in 4 animals per group between the test compound-administered group and the solvent-administered group (significance test: Sign-Wilcoxon test). The spontaneous locomotor activity was also decided by comparison between the test compound-administered group and the solvent-administered group.

It was found that compounds 71, 76, and 85 are effective in the common marmoset MPTP-treated Parkinson's disease model.

Test Example 4 Forced Swimming Method (Measurement of Immobility Time)

Ten male ddY mice (21 to 26 g in body weight, Japan SLC) per group were used as test animals. During the preliminary feeding period, the animals were bred to be allowed to freely have feed (CRF-1; Oriental Yeast Co., Ltd, Tokyo) and water (tap water) in an animal room at a room temperature for 23±1°C and a humidity of 55±5%.

Animals showing abnormal reactions in terms of spontaneous activity, myotonia, eyesight and the like were previously excluded. Each test compound was suspended in a 0.3% Tween 80 solution and orally administered one hour before the test. In the negative control group, 10 mL/kg of a 0.3% Tween 80 solution alone was orally administered. The immobility time was measured according to the Porsolt method [Arch. Int. Pharmacodyn., vol. 229, pp. 327-336 (1977)]. Namely, a cylindrical transparent acrylic water tank (10 cm in diameter and 25 cm in height) was filled with water at a temperature between 23±1°C to a height of 9 cm, and the mice were forced to swim for 6 minutes. When the mice are put into water, they immediately start to swim trying to escape from the tank, but the motion gradually decreases 1 to 2 minutes thereafter. The immobility time was measured by leaving the mice for 2 minutes and then measuring the period of time during which they did not show the escaping action (immobility time: behavioral despair) at intervals of one second for 4 minutes (240 seconds). In order to reduce the effects of daily rhythm, the test was carried out using 5 of the 10 animals per group in the morning, and the remaining 5 animals in the afternoon. The immobility time was measured by observing 2 animals at the same time without telling the observers distinctions about the control group administered with the solvent alone and doses of the test compounds. Statistical analysis of the results was carried out by a multiple comparison Steel-test between the solvent-administered group and the test compound-administered group.

Compounds 71, 83, 89, and 94 showed a significant effect of decreasing the immobility time when being orally administered in a dose of 10 mg/kg. All of these compounds showing this effect have strong antagonism against the $A_{2A}$ receptor (inhibition ratio of 50% or more at $10^{-7}$ mol/L). Therefore, general correlation was observed between $A_{2A}$ antagonism and anti-depression activity.

Test Example 5 Activity in Learned Helplessness (LH) Model 1) Animals used

As test animals, 10 to 15 male SD rats (220 to 320 g in body weight, 7 week age, Charles River Japan, Inc., Atsugi) per group were used. During the preliminary feeding period, the animals were bred to be allowed to freely have feed (CRF-1; Oriental Yeast Co., Ltd, Tokyo) and water (tap water) in an animal room at a room temperature of 22 to 24°C and a humidity of 50 to 60%. Each test compound was orally administered in a dose of 2 mg/kg 1 hour before RF1 (refer to the contents described below) on the second day of the test.

2) Preparation of learned helplessness model

A shuttle box apparatus (TK-401S; produced by UNICOM, Chiba) was used as a learning experimental apparatus. On the first day, the shuttle box was divided into two rooms by setting a partition at its center, and one rat was put in each of the rooms. Each of the two rooms (22 x 20 x 26 cm) had a stainless steel floor grid so that electric shock (1.3 mA, scramble stimulus) can be applied. Although each rat was put in the shuttle box for 50 minutes, the charging time of inescapable shock (IES) was set to 25 minutes by computer control using a random duration time (10 to 90 seconds) and random on-off and off-on (10 to 90 seconds).

On the second day, a shuttle box test was carried out by the method of Maier [J. Comp. Physiol. Psychol., vol. 85, pp. 581-592 (1973)] and the method of Geoffroy and Christensen [Drug Dev. Res., vol. 29, pp. 48-55 (1993)] which were slightly modified. The partition at the center of the shuttle box was removed, and the box was divided into two rooms by a hurdle of 2 cm in height instead of the partition. In the shuttle box test, FRI (an electric shock of 0.6 mA was applied to the rat for 5 seconds unless the rat moved to the other room within 5 seconds after the start of buzzer sounds, and when the rat moved to the other room within 5 seconds after the start of buzzer sounds, the floor shock could be avoided, this operation being carried as one trial and repeated 15 times with an interval of 10 seconds between trials.) was carried out, continuously followed by FR2 (an electric shock of 0.6 mA was applied to the rat for 10 seconds, and then an electric shock of 0.6 mA was applied at intervals of 0.5 second for 10 seconds, this operation being carried out as one trial and repeated 15 times with an interval of 15 seconds between trials). Escape reaction was determined by the escape response calculated by the equation below on the assumption that cases showing two escape latency times of less than 10 seconds in FR2 were regarded as successful escape.

$$\text{Escape response (\%)} = (\text{Number of trials of successful escape}/15) \times 100$$

Furthermore, migration (intertrial response %) in the box other than the escape reaction observed during a period between trials (resting time) was used as an index of psychomotor stimulant action, the intertrial response being calculated by the following equation.

$$\text{Intertrial response (\%)} = (\text{Total number of migrations in the box}/15) \times 100$$

3) Statistical Treatment

The differences between the normal control group and the IES-loaded control group were treated with Student-t, and a multiple comparison test was carried out for differences of the escape responses and intertrial responses between the IES-loaded control group and the test compound-administered group by the Steel method. The results were decided as significant when the level of significance was less than 5%. In this case, an SAS statistical analysis software was used in the statistical analysis.
As a result, it was shown that compounds 71 and 83 can significantly recover a reduced escape response induced by the IES loading, and thus have an anti-depressant activity. It was also suggested that the psychomotor stimulant activity is weak because of no difference in the intertrial responses between the test compound-administered group and the electric shock loaded control group.
Compound (I) or its pharmaceutically acceptable salt can be singly administered as it is, but it is generally preferred to provide it as various types of pharmaceutical preparations. Such pharmaceutical preparations are used for animals and humans.
The pharmaceutical preparations of the present invention can contain Compound (I) or its pharmaceutically acceptable salt alone as an active ingredient or a mixture with other optional active ingredients for treating different diseases. Furthermore, these pharmaceutical preparations are produced by mixing the active ingredient with one or more pharmaceutically acceptable carriers according to any desired method well known in the technical field of pharmaceutics.
It is preferable to select a route of administration which is most effective in treatment. Examples of the administration route include oral administration and parenteral administrations, such as intravenous administration.
Examples of dosage forms include tablets and injections.
Preparations suitable for oral administration, for example, tablets and the like, can be obtained by using an excipient such as lactose and mannitol; a disintegrator such as starch; a lubricant such as magnesium stearate; a binder such as polyvinyl alcoholo and hydroxypropyl cellulose; a surfactant such as a fatty acid ester; and a plasticizer such as glycerin.
Preparations suitable for parenteral administration are preferably sterile aqueous preparations containing an active compound which becomes isotonic in the blood of an acceptor. For example, in the case of injections, a solution for injection is prepared using a carrier comprising a salt solution, a glucose solution or a mixture of salt water with a glucose solution. In this case, the injections are prepared in the form of a solution, suspension or dispersion by a usual method using a suitable auxiliary.
These parenteral preparations can also be mixed with one or more auxiliary components selected from the above-described examples of the excipient, disintegrator, lubricant, binder, surfactant, plasticizer, and diluent for the oral preparations.
The effective amount of Compound (I) or its pharmaceutical acceptable salt and the number of times of administration thereof depend on the administration mode, the age and body weight of a patient, and properties or seriousness of symptoms to be treated. However, it is generally preferred to divide a dose of 1 to 50 mg/kg into 3 to 4 doses per day.
However, the dose may vary depending on the above-described various conditions.

## Best Mode for Carrying Out the Invention

Examples and preparation examples are described below. In $^1$H NMR measurement data, a symbol "br" prefixed to a symbol expressing multiplicity means that a broad signal was observed. For example "brs" means a broad

singlet.

Example 1

5-Amino-2-(2-furyl)-7-phenyl[1,2,4]triazolo[1,5-c]pyrimidine (Compound 1)

In THF (5 mL), 7-chloro-5-(3,4-dimethoxybenzylamino)-2-(2-furyl)[1,2,4]triazolo[1,5-c]pyrimidine (Compound G, 500 mg, 1.17 mmol) was dissolved, and phenylboric acid (213 mg, 1.75 mmol), bistriphenylphosphine palladium dichloride (25 mg, 0.035 mmol), and a 2 mol/L aqueous solution of sodium carbonate (2.5 mL) were added to the resulting solution, followed by reflux for 9 hours. The reaction solution was cooled to room temperature, and brine was added to the solution, followed by extraction with chloroform. The organic layer was dried over anhydrous magnesium sulfate, filtered, and then concentrated under reduced pressure. The residue was purified by silica gel column chromatography (elution solvent: a mixed solvent of hexane and ethyl acetate) to obtain 5-(3,4-dimethoxybenzylamino)-2-(2-furyl)-7-phenyl[1,2,4]triazolo[1,5-c]pyrimidine (424 mg, 0.99 mmol) as a white amorphous substance in a yield of 85%.

The resulting 5-(3,4-dimethoxybenzylamino)-2-(2-furyl)-7-phenyl[1,2,4]triazolo[1,5-c]pyrimidine was dissolved in trifluoroacetic acid (3.0 mL), and anisole (541 μL, 4.95 mmol) and trifluoromethanesulfonic acid (230 μL, 1.98 mmol) were added to the solution, followed by stirring at room temperature for 4 hours. Then, a 1 mol/L aqueous solution of sodium hydroxide was added to the reaction solution, and extraction with chloroform was performed. The organic layer was dried over anhydrous magnesium sulfate, filtered, and then concentrated under reduced pressure. The residue was triturated with methanol to obtain Compound 1 (168 mg, 0.606 mmol) as a white solid in a yield of 61%. Furthermore, the white solid of Compound 1 was recrystallized from ethanol to obtain Compound 1 as white crystals.

$^1$H NMR (δ ppm, DMSO-$d_6$): 8.15 (d, J=7.9 Hz, 2H), 8.04 (brs, 2H), 7.95 (d, J=2.0 Hz, 1H), 7.55 (s, 1H), 7.4-7.6 (m, 3H), 7.22 (d, J=3.3 Hz, 1H), 6.74 (dd, J=3.3 Hz, 2.0 Hz, 1H)

Mass (m/z): 277 ($M^+$)

IR (KBr, $cm^{-1}$): 1652, 1646, 1618, 1600, 1552, 1508, 1419

Melting point: 218.5 - 219.0°C

Example 2

5-Amino-7-(2-formylphenyl)-2-(2-furyl)[1,2,4]triazolo[1,5-c] pyrimidine (Compound 2)

Compound G (5.00 g, 13.0 mmol) was dissolved in THF (100 mL), and 2-formylphenylboric acid (2.50 g, 16.7 mmol), bistriphenylphosphine palladium dichloride (680 mg, 0.970 mmol, and a 2 mol/L aqueous solution of sodium carbonate (25 mL) were added to the resulting solution, followed by reflux for 9 hours. The reaction solution was cooled to room temperature, and brine was added to the solution, followed by extraction with chloroform. The organic layer was dried over anhydrous magnesium sulfate, filtered, and then concentrated under reduced pressure. The residue was purified by silica gel column chromatography (elution solvent: a mixed solvent of hexane and ethyl acetate) to obtain 5-(3,4-dimethoxybenzylamino)-7-(2-formylphenyl)-2-(2-furyl)[1,2,4]triazolo[1,5-c]pyrimidine (4.95 g, 10.8 mmol) as a white amorphous substance in a yield of 84%.

The resulting 5-(3,4-dimethoxybenzylamino)-7-(2-formylphenyl)-2-(2-furyl)[1,2,4]triazolo[1,5-c]pyrimidine was dissolved in trifluoroacetic acid (32 mL), and trifluoromethanesulfonic acid (2.4 mL, 26.8 mmol) was added to the solution, followed by stirring at room temperature for 12 hours. Then, the reaction solution was diluted with ice-cold chloroform and washed with a 1 mol/L aqueous solution of sodium hydroxide. The organic layer was dried over anhydrous magnesium sulfate, filtered, and then concentrated under reduced pressure. The residue was triturated with chloroform to obtain Compound 2 (1.74 g, 5.70 mmol) as a white solid. Furthermore, the filtrate was concentrated under reduced pressure, and the residue was purified by silica gel column chromatography (elution solvent: a mixed solvent of chloroform and 2 mol/L ammonia-methanol) to obtain Compound 2 (334 mg, 1.10 mmol) in a total yield of 63%. The resulting Compound 2 was recrystallized from ethanol to obtain Compound 2 as white crystals.

$^1$H NMR (δ ppm, DMSO-$d_6$): 10.20 (brs, 1H), 8.12 (brs, 2H), 7.95 (d, J=1.7 Hz, 1H), 7.60-7.90 (m, 4H), 7.31 (s, 1H), 7.24 (d, J=3.3 Hz, 1H), 6.74 (dd, J=3.3 Hz, 1.7 Hz, 1H)

Mass (m/z): 305 ($M^+$), 207

IR (KBr, $cm^{-1}$): 1683, 1678, 1659, 1632, 1616, 1541, 760

| Elemental Analysis: as $C_{16}H_{11}N_5O_2 \cdot 0.1\ H_2O$ | |
|---|---|
| Observed | C 62.47%, H 3.68%, N 22.53% |

(continued)

| Elemental Analysis: as $C_{16}H_{11}N_5O_2 \cdot 0.1\ H_2O$ | | | |
|---|---|---|---|
| Calculated | C 62.57%, | H 3.67%, | N 22.80% |

Melting point: 224.0 - 224.5°C

Example 3

5-Amino-7-[2-(3,4-dimethoxybenzylaminomethyl)phenyl]-2-(2-furyl)[1,2,4]triazolo[1,5-c]pyrimidine (Compound 3)

Compound 2 (250 mg, 0.82 mmol) was dissolved in THF (8 mL), and veratrylamine (164 mg, 0.98 mmol) was added to the resulting solution, followed by stirring at room temperature for 2 hours. Then, sodium triacetoxyborohydride (521 mg, 2.46 mmol) was added to the solution, followed by stirring at room temperature for 5 hours. Furthermore, Bio-Rad AG1X-8 resin (produced by Bio-Rad Laboratories Inc., 2.0 g) was added to the reaction solution, followed by stirring at room temperature overnight. Then, the solution was filtered, and the filtrate was concentrated under reduced pressure. Then, chloroform and aldehyde resin (produced by Sigma-Aldrich Co., 500 mg) were added to the residue, followed by stirring at room temperature overnight. After filtration, the filtrate was concentrated under reduced pressure, and the residue was recrystallized from a mixed solvent containing acetonitrile (3 mL) and diisopropyl ether (10 mL) to obtain Compound 3 (80 mg, 0.18 mmol) as white crystals in a yield of 21%.
$^1$H NMR (δ ppm, $CDCl_3$): 7.64 (d, J=2.0 Hz, 1H), 7.3-7.5 (m, 4H), 7.25 (d, J=3.3 Hz, 1H), 7.20 (s, 1H), 6.88 (d, J=1.6 Hz, 1H), 6.77 (dd, J=7.9 Hz, 1.6 Hz, 1H), 6.72 (d, J=7.9 Hz, 1H), 6.60 (dd, J=3.3 Hz, 2.0 Hz, 1H), 3.86 (s, 2H), 3.84 (s, 3H), 3.82 (s, 3H), 3.73 (s, 2H)
IR (KBr, $cm^{-1}$): 1660, 1610, 1512, 1417, 1240, 759

| Elemental Analysis: as $C_{25}H_{24}N_6O_3$ | | | |
|---|---|---|---|
| Observed | C 65.50%, | H 5.47%, | N 18.41% |
| Calculated | C 65.78%, | H 5.30%, | N 18.41% |

Melting point: 168.0 - 168.7°C

Example 4

5-Amino-2-(2-furyl)-7-{2-[2-(2-pyridyl)ethylaminomethyl]phenyl}[1,2,4]triazolo[1,5-c]pyrimidine (Compound 4)

The reaction was carried out in a manner similar to that in Example 3 except that veratrylamine was replaced by N-methyl-2-(2-pyridyl)ethylamine. The resulting crude product was recrystallized from a mixed solvent containing isopropyl alcohol (1 mL) and diisopropyl ether (4 mL) to obtain Compound 4 (50 mg, 0.12 mmol) as white crystals in a yield of 15%.
$^1$H NMR (δ ppm, DMSO-$d_6$): 8.42 (dd, J=5.0 Hz, 1.6 Hz, 1H), 8.18 (brs, 2H), 7.96 (d, J=1.6 Hz, 1H), 7.4-7.7 (m, 5H), 7.10-7.30 (m, 4H), 6.74 (dd, J=3.3 Hz, 1.6 Hz, 1H), 4.00 (s, 2H), 3.00-3.10 (m, 4H)
Mass (m/z): 289
IR (KBr, $cm^{-1}$): 1663, 1606, 1558, 1417, 1326, 773

| Elemental Analysis: as $C_{23}H_{21}N_7O \cdot 1.1\ H_2O$% | | | |
|---|---|---|---|
| Observed | C 64.09%, | H 5.28%, | N 22.50% |
| Calculated | C 64.05%, | H 5.42%, | N 22.73% |

Melting point: 179.2 - 180.0°C

Example 5

5-Amino-7-[2-(4-ethylpiperazin-1-ylmethyl)phenyl]-2-(2-furyl)[1,2,4]triazolo[1,5-c]pyrimidine dihydrochloride (Compound 5)

Compound 2 (250 mg, 0.82 mmol) was dissolved in THF (8 mL), and 1-ethylpiperazine (112 mg, 0.98 mmol) and acetic acid (200 μL) were added to the resulting solution, followed by stirring at room temperature for 1 hour. Then, sodium triacetoxyborohydride (521 mg, 2.46 mmol) was added to the reaction solution, followed by stirring at room temperature for 16 hours. Furthermore, Bio-Rad AG1X-8 resin (produced by Bio-Rad Laboratories Inc., 2.0 g) was added to the reaction solution, followed by stirring at room temperature overnight. Then, the solution was filtered, and the filtrate was concentrated under reduced pressure. The residue was dissolved in chloroform, and acid chloride resin (850 mg) and polyvinylpyridine resin (produced by Sigma-Aldrich Co., 850 mg) were added to the resulting solution, followed by stirring at room temperature overnight. After the resins were filtered off, the filtrate was concentrated under reduced pressure, and an ethyl acetate solution of hydrogen chloride was added to the residue. The resulting solid was recrystallized from a mixed solvent containing ethanol (10 mL) and methanol (10 mL) to obtain Compound 5 (217 mg, 0.45 mmol) as white crystals in a yield of 55%.
$^1$H NMR (δ ppm, DMSO-$d_6$): 8.34 (brs, 2H), 7.97 (d, J=2.0 Hz, 1H), 7.83 (m, 1H), 7.50-7.70 (m, 3H), 7.24 (d, J=3.6 Hz, 1H), 7.18 (s, 1H), 6.75 (dd, J=3.6 Hz, 2.0 Hz, 1H), 4.53 (brs, 2H), 3.00-3.80 (m, 8H), 3.21 (m, 2H), 1.27 (t, J=7.0 Hz, 3H)
Mass (m/z): 403 ($M^+$), 289
IR (KBr, $cm^{-1}$): 1651, 1639, 1632, 1612, 1558, 1512

| Elemental Analysis: as $C_{22}H_{25}N_7O \cdot 2.0\ HCl \cdot 0.2\ H_2O$ | | | |
|---|---|---|---|
| Observed | C 55.13%, | H 5.94%, | N 20.18% |
| Calculated | C 55.05%, | H 5.75%, | N 20.42% |

Melting point: 253.0 - 253.4°C

Example 6

5-Amino-7-[2-(3,5-dimethoxybenzylaminomethyl)phenyl]-2-(2-furyl)[1,2,4]triazolo[1,5-c]pyrimidine monohydrochloride (Compound 6)

The reaction was carried out in a manner similar to that in Example 3 except that veratrylamine was replaced by 3,5-dimethoxybenzylamine. Then, an ethyl acetate solution of hydrogen chloride was added to the resulting crude product, and the resulting mixture was concentrated under reduced pressure. The residue was recrystallized from a mixed solvent containing THF (3 mL) and diisopropyl ether (10 mL) to obtain Compound 6 (152 mg, 0.30 mmol) as white crystals in a yield of 36%.
$^1$H NMR (δ ppm, DMSO-$d_6$): 9.58 (m, 2H), 8.41 (brs, 2H), 7.97 (d, J=1.7 Hz, 1H), 7.50-7.70 (m, 4H), 7.24 (d, J=3.3 Hz, 1H), 7.18 (s, 1H), 6.75 (d, J=2.2 Hz, 2H), 6.74 (dd, J=3.3 Hz, 1.7 Hz, 1H), 6.45 (t, J=2.2 Hz, 1H), 4.22 (brs, 2H), 4.09 (brs, 2H), 3.72 (s, 6H)
Mass (m/z): 456 ($M^+$), 288
IR (KBr, $cm^{-1}$): 1660, 1639, 1622, 1583, 1458, 1419, 766

| Elemental Analysis: as $C_{25}H_{24}N_6O_3 \cdot 1.0HCl \cdot 0.1\ [(CH_3)_2CH]_2O \cdot 0.6\ H_2O$ | | | |
|---|---|---|---|
| Observed | C 59.87%, | H 5.30%, | N 16.33% |
| Calculated | C 59.82%, | H 5.41%, | N 16.35% |

Melting point: 213.5 - 214.3°C

Example 7

5-Amino-7-{2-[2-(N-acetylamino)ethylaminomethyl]phenyl}-2-(2-furyl)[1,2,4]triazolo[1,5-c]pyrimidine monohydrochloride (Compound 7)

The reaction was carried out in a manner similar to that in Example 3 except that veratrylamine was replaced by N-acetylethylenediamine. Then, an ethyl acetate solution of hydrogen chloride was added to the resulting crude

product, and the resulting mixture was concentrated under reduced pressure. The residue was recrystallized from isopropyl alcohol (7 mL) to obtain Compound 7 (187 mg, 0.41 mmol) as white crystals in a yield of 50%.
$^1$H NMR (δ ppm, DMSO-$d_6$): 9.24 (m, 2H), 8.30-8.40 (m, 3H), 7.97 (d, J=1. 6 Hz, 1H), 7.60-7.70 (m, 2H), 7.50-7.60 (m, 2H), 7.25 (d, J=3.3 Hz, 1H), 7.22 (s, 1H), 6.75 (dd, J=3.3 Hz, 1.6 Hz, 1H), 4.25 (brs, 2H), 3.42 (m, 2H), 3.10 (m, 2H), 1.85 (s, 3H)
Mass (m/z): 391 ($M^+$), 289
IR (KBr, $cm^{-1}$): 1637, 1597, 1508, 1417

| Elemental Analysis: as $C_{20}H_{21}N_7O_2$·1.0HCl·0.3 $(CH_3)_2CHOH$·0.7 $H_2O$ | | | |
|---|---|---|---|
| Observed | C 54.61%, | H 5.45%, | N 21.37% |
| Calculated | C 54.75%, | H 5.67%, | N 21.38% |

Melting point: 230.8 - 231.3°C

Example 8

5-Amino-2-(2-furyl)-7-{2-[3-(2-oxopyrrolidin-1-yl)propylaminomethyl]phenyl}[1,2,4]triazolo[1,5-c]pyrimidine monohydrochloride (Compound 8)

The reaction was carried out in a manner similar to that in Example 3 except that veratrylamine was replaced by 1-(3-aminopropyl)-2-pyrrolidone. Then, an ethyl acetate solution of hydrogen chloride was added to the resulting crude product, and the resulting mixture was concentrated under reduced pressure. The residue was recrystallized from ethanol (5 mL) to obtain Compound 8 (174 mg, 0.34 mmol) as white crystals in a yield of 41%.
$^1$H NMR (δ ppm, DMSO-$d_6$): 9.21 (m, 2H), 8.40 (brs, 2H), 7.97 (d, J=1.6 Hz, 1H), 7.70-7.80 (m, 2H), 7.50-7.60 (m, 2H), 7.24 (d, J=3.3 Hz, 1H), 7. 22 (s, 1H), 6.74 (dd, J=3.3 Hz, 1.6 Hz, 1H), 4.23 (brs, 2H), 3.34 (t, J=7.2 Hz, 2H), 3.24 (t, J=6.4 Hz, 2H), 3.02 (m, 2H), 2.20 (t, J=7.9 Hz, 2H), 1.80-2.00 (m, 4H)
Mass (m/z): 431 ($M^+$), 289
IR (KBr, $cm^{-1}$): 1684, 1670, 1662, 1655, 1647

| Elemental Analysis: as $C_{23}H_{25}N_7O_2$·1.0HCl·0.4 $C_2H_5OH$·1.4 $H_2O$ | | | |
|---|---|---|---|
| Observed | C 55.77%, | H 5.88%, | N 19.09% |
| Calculated | C 55.88%, | H 6.15%, | N 19.16% |

Melting point: 153.5 - 154.2°C

Example 9

(±)-5-Amino-2-(2-furyl)-7-{2-[(2-methoxy-1-methylethyl) aminomethyl]phenyl}[1,2,4]triazolo[1,5-c]pyrimidine monohydrochloride (Compound 9)

The reaction was carried out in a manner similar to that in Example 3 except that veratrylamine was replaced by (±)-2-aminomethoxypropane. Then, an ethyl acetate solution of hydrogen chloride was added to the resulting crude product, and the resulting mixture was concentrated under reduced pressure. The residue was recrystallized from isopropyl alcohol (7 mL) to obtain Compound 9 (66 mg, 0.15 mmol) as white crystals in a yield of 18%.
$^1$H NMR (δ ppm, DMSO-$d_6$): 9.43 (m, 1H), 9.01 (brs, 1H), 8.19 (brs, 2H), 7.98 (d, J=1.6 Hz, 1H), 7.60-7.70 (m, 2H), 7.50-7.60 (m, 2H), 7.26 (s, 1H), 7.24 (d, J=3.3 Hz, 1H), 6.75 (dd, J=3.3 Hz, 1.6 Hz, 1H), 4.24 (brs, 2H), 3.80 (m, 1H), 3.50-3.60 (m, 2H), 3.27 (s, 3H), 1.30 (d, J=5.2 Hz, 3H)
Mass (m/z): 289
IR (KBr, $cm^{-1}$): 1668, 1654, 1641, 1616, 1558, 1508

| Elemental Analysis: as $C_{20}H_{22}N_6O_2$·1.0HCl·1.6 $H_2O$ | | | |
|---|---|---|---|
| Observed | C 54.03%, | H 5.72%, | N 19.00% |
| Calculated | C 54.14%, | H 5.95%, | N 18.94% |

Melting point: 178.9 - 179.7°C

Example 10

5-Amino-7-(4-formylphenyl)-2-(2-furyl)[1,2,4]triazolo[1,5-c] pyrimidine (Compound 10)

The reaction was carried out in a manner similar to that in Example 2 except that 2-formylphenylboric acid was replaced by 4-formylphenylboric acid to obtain 5-(3,4-dimethoxybenzylamino)-7-(4-formylphenyl)-2-(2-furyl)[1,2,4] triazolo[1,5-c]pyrimidine (4.27 g, 9.34 mmol) as a white amorphous substance in a yield of 75%.

The resulting 5-(3,4-dimethoxybenzylamino)-7-(4-formylphenyl)-2-(2-furyl)[1,2,4]triazolo[1,5-c]pyrimidine was dissolved in ethyl acetate (200mL), and DDQ (4.24 g, 18.7 mmol) was added to the resulting solution, followed by reflux for 2 hours. Then, the reaction solution was diluted with ice-cold chloroform, and washed with a 1 mol/L aqueous sodium hydroxide solution. The organic layer was dried over anhydrous magnesium sulfate, filtered, and then concentrated under reduced pressure. The residue was triturated with methanol to obtain Compound 10 (2.59 g, 8.48 mmol) as a white solid in a yield of 91%. Furthermore, Compound 10 was recrystallized from ethanol to obtain white crystals.

$^1$H NMR (δ ppm, DMSO-$d_6$): 10.09 (s, 1H), 8.36 (d, J=8.3 Hz, 2H), 8.13 (brs, 2H), 8.04 (d, J=8.3 Hz, 2H), 7.96 (d, J=1.6 Hz, 1H), 7.72 (s, 1H), 7. 23 (d, J=3.3 Hz, 1H), 6.74 (dd, J=3.3 Hz, 1.6 Hz, 1H)

Mass (m/z): 305 ($M^+$)

IR (KBr, $cm^{-1}$): 1655, 1614, 1604, 1575, 1427, 1209

| Elemental Analysis: as $C_{16}H_{11}N_5O_2 \cdot 0.6\ H_2O$ | | | |
|---|---|---|---|
| Observed | C 60.79%, | H 3.62%, | N 22.04% |
| Calculated | C 60.79%, | H 3.89%, | N 22.15% |

Melting point: 273.5 - 274.0°C

Example 11

5-Amino-2-(2-furyl)-7-{4-[2-(2-pyridyl)ethylaminomethyl]phenyl}[1,2,4]triazolo[1,5-c]pyrimidine (Compound 11)

The reaction was carried out in a manner similar to that in Example 4 except that Compound 2 was replaced by Compound 10. The resulting crude product was recrystallized from a mixed solvent containing acetonitrile (3 mL) and diisopropyl ether (10 mL) to obtain Compound 11 (90 mg, 0.22 mmol) as white crystals in a yield of 27%.

$^1$H NMR (δ ppm, DMSO-$d_6$): 8.48 (dd, J=5.4 Hz, 1.0 Hz, 1H), 8.18 (d, J=8. 5 Hz, 2H), 8.00 (brs, 2H), 7.96 (d, J=1.6 Hz, 1H), 7.65 (ddd, J=8.4 Hz, 8.2 Hz, 1.0 Hz, 1H), 7.57 (s, 1H), 7.43 (d, J=8.5 Hz, 2H), 7.28 (d, J=8. 4 Hz, 1H), 7.10-7.20 (m, 2H), 6.74 (dd, J=3.3 Hz, 1.6 Hz, 1H), 3.79 (s, 2H), 2.80-2.90 (m, 4H)

Mass (m/z): 411 ($M^+$), 290

IR (KBr, $cm^{-1}$): 1664, 1602, 1560, 1421, 1178, 765, 744

| Elemental Analysis: as $C_{23}H_{21}N_7O \cdot 1.5\ H_2O$ | | | |
|---|---|---|---|
| Observed | C 63.00%, | H 5.15%, | N 21.96% |
| Calculated | C 63.00%, | H 5.51%, | N 22.36% |

Melting point: 159.8 - 160.5°C

Example 12

5-Amino-7-[4-(4-ethylpiperazin-1-ylmethyl)phenyl]-2-(2-furyl)[1,2,4]triazolo[1,5-c]pyrimidine dihydrochloride (Compound 12)

The reaction was carried out in a manner similar to that in Example 5 except that Compound 2 was replaced by Compound 10. The resulting crude product was recrystallized from ethanol (20 mL) to obtain Compound 12 (133 mg, 0.30 mmol) as white crystals in a yield of 37%.

$^1$H NMR (δ ppm, DMSO-$d_6$): 11.68 (m, 1H), 8.21 (d, J=8.0 Hz, 2H), 8.08 (brs, 2H), 7.95 (d, J=1.6 Hz, 1H), 7.76 (d, J=8.0 Hz, 2H), 7.62 (s, 1H), 7.22 (d, J=3.3 Hz, 1H), 6.74 (dd, J=3.3 Hz, 1.6 Hz, 1H), 4.37 (brs, 2H), 3.30-3.70 (m, 9H), 3.16 (m, 2H), 1.25 (t, J=7.3 Hz, 3H)

Mass (m/z): 403 ($M^+$), 289

IR (KBr, $cm^{-1}$): 2360, 1655, 1614, 1597, 1558

| Elemental Analysis: as $C_{22}H_{25}N_7O \cdot 2.0$ HCl | | | |
|---|---|---|---|
| Observed | C 55.43%, | H 5.76%, | N 20.53% |
| Calculated | C 55.46%, | H 5.71%, | N 20.58% |

Melting point: 265.0°C (decomposed)

Example 13

5-Amino-7-[4-(3,4-dimethoxybenzylaminomethyl)phenyl]-2-(2-furyl)[1,2,4]triazolo[1,5-c]pyrimidine monohydrochloride (Compound 13)

The reaction was carried out in a manner similar to that in Example 3 except that Compound 2 was replaced by Compound 10. Then, an ethyl acetate solution of hydrogen chloride was added to the resulting crude product, and the resulting mixture was concentrated under reduced pressure. The residue was recrystallized from ethanol (10 mL) to obtain Compound 13 (212 mg, 0.43 mmol) as white crystals in a yield of 53%.
$^1$H NMR (δ ppm, DMSO-$d_6$): 9.63 (m, 2H), 8.19 (d, J=8.3 Hz, 2H), 8.07 (brs, 2H), 7.95 (d, J=1.7 Hz, 1H), 7.67 (d, J=8.3Hz, 2H), 7.62 (s, 1H), 7.24 (d, J=1.9 Hz, 1H), 7.21 (d, J=3.3 Hz,1H), 7.05 (dd, J=8.3 Hz, 1.9 Hz, 1H), 6.99 (d, J=8.3 Hz, 1H), 6.74 (dd, J=3.3 Hz, 1.7 Hz, 1H), 4.18 (brs, 2H), 4.11 (brs, 2H), 3.78 (s, 3H), 3.77 (s, 3H)
Mass (m/z): 456 ($M^+$), 305
IR (KBr, $cm^{-1}$): 1643, 1598, 1520, 1425, 1026

| Elemental Analysis: as $C_{25}H_{24}N_6O_3 \cdot 1.0HCl \cdot 1.9\ H_2O \cdot 0.6\ C_2H_5OH$ | | | |
|---|---|---|---|
| Observed | C 56.44%, | H 5.66%, | N 15.15% |
| Calculated | C 56.72%, | H 5.89%, | N 15.15% |

Melting point: 256.2 - 256.8°C

Example 14

5-Amino-2-(2-furyl)-7-{4-[3-(2-oxopyrrolidin-1-yl)propylaminomethyl]phenyl}[1,2,4]triazolo[1,5-c]pyrimidine monohydrochloride (Compound 14)

The reaction was carried out in a manner similar to that in Example 8 except that Compound 2 was replaced by Compound 10. Then, the crude product was recrystallized from ethanol (5 mL) to obtain Compound 14 (141 mg, 0.30 mmol) as white crystals in a yield of 37%.
$^1$H NMR (δ ppm, DMSO-$d_6$): 9.28 (m, 2H), 8.19 (d, J=8.3 Hz, 2H), 8.06 (brs, 2H), 7.95 (d, J=2.0 Hz, 1H), 7.67 (d, J=8.3 Hz, 2H), 7.62 (s, 1H), 7. 21 (d, J=3.3 Hz, 1H), 6.74 (dd, J=3.3 Hz, 2.0 Hz, 1H), 4.20 (brs, 2H), 3.34 (t, J=7.2 Hz, 2H), 3.26 (t, J=6.6 Hz, 2H), 2.88 (m, 2H), 2.23 (t, J=8.0 Hz, 2H), 1.80-2.00 (m, 4H)
Mass (m/z): 431 ($M^+$), 305, 291
IR (KBr, $cm^{-1}$): 1672, 1666, 1633, 1606, 1599, 1512, 1423

| Elemental Analysis: as $C_{23}H_{25}N_7O_2 \cdot 1.0HCl \cdot 0.9\ H_2O$ | | | |
|---|---|---|---|
| Observed | C 57.08%, | H 5.73%, | N 20.17% |
| Calculated | C 57.06%, | H 5.79%, | N 20.25% |

Melting point: 269.2 - 269.8°C

Example 15

(±)-5-Amino-2-(2-furyl)-7-{4-[(2-methoxy-1-methylethyl)aminomethyl] phenyl}[1,2,4]triazolo[1,5-c]pyrimidine monohydrochloride (Compound 15)

The reaction was carried out in a manner similar to that in Example 9 except that Compound 2 was replaced

by Compound 10. Then, the crude product was recrystallized from isopropyl alcohol (3 mL) to obtain Compound 15 (97 mg, 0.24 mmol) as white crystals in a yield of 29%.
$^1$H NMR (δ ppm, DMSO-$d_6$): 9.46 (m, 1H), 9.27 (m, 1H), 8.18 (d, J=7.9 Hz, 2H), 8.09 (brs, 2H), 7.95 (d, J=1.7 Hz, 1H), 7.72 (d, J=7.9 Hz, 2H), 7. 62 (s, 1H), 7.22 (d, J=3.3 Hz, 1H), 6.74 (dd, J=3.3 Hz, 1.7 Hz, 1H), 4.23 (brs, 2H), 3.50-3.60 (m, 2H), 3.35 (m, 1H), 3.32 (s, 3H), 1.30 (d, J=6.6 Hz, 3H)
Mass (m/z): 378 ($M^+$), 333, 290
IR (KBr, $cm^{-1}$): 1701, 1635, 1616, 1597, 1510
Melting point: 225.7 - 226.0°C

Example 16

7-{4-[2-(N-acetylamino)ethylaminomethyl]phenyl}-5-amino-2-(2-furyl)[1,2,4]triazolo[1,5-c]pyrimidine monohydrochloride (Compound 16)

The reaction was carried out in a manner similar to that in Example 7 except that Compound 2 was replaced by Compound 10. Then, the crude product was recrystallized from a mixed solvent containing ethanol (5 mL) and isopropyl alcohol (5 mL) to obtain Compound 16 (147 mg, 0.33 mmol) as white crystals in a yield of 40%.
$^1$H NMR (δ ppm, DMSO-$d_6$): 9.33 (m, 2H), 8.22 (t, J=5.3 Hz, 1H), 8.19 (d, J=8.3 Hz, 2H), 8.06 (brs, 2H), 7.95 (d, J=1.6 Hz, 1H), 7.68 (d, J=8.3 Hz, 2H), 7.62 (s, 1H), 7.22 (d, J=3.3 Hz, 1H), 6.74 (dd, J=3.3 Hz, 1.6 Hz, 1H), 4.23 (brs, 2H), 3.39 (m, 2H), 2.99 (m, 2H), 1.85 (s, 3H)
Mass (m/z): 391 ($M^+$), 290
IR (KBr, $cm^{-1}$): 1658, 1643, 1637, 1616, 1603, 1560, 1510, 1421

| Elemental Analysis: as $C_{20}H_{21}N_7O_2 \cdot 1.0HCl \cdot 0.3\ C_2H_5OH \cdot 0.5\ H_2O$ | | | |
|---|---|---|---|
| Observed | C 54.84%, | H 5.64%, | N 21.67% |
| Calculated | C 54.89%, | H 5.55%, | N 21.75% |

Melting point: 238.5 - 240.0°C

Example 17

5-Amino-7-[4-(3,5-dimethoxybenzylaminomethyl)phenyl]-2-(2-furyl)[1,2,4]triazolo[1,5-c]pyrimidine monohydrochloride (Compound 17)

The reaction was carried out in a manner similar to that in Example 6 except that Compound 2 was replaced by Compound 10. Then, the crude product was recrystallized from ethanol (5 mL) to obtain Compound 17 (80 mg, 0.16 mmol) as white crystals in a yield of 20%.
$^1$H NMR (δ ppm, DMSO-$d_6$): 9.73 (m, 2H), 8.19 (d, J=8.3 Hz, 2H), 8.06 (brs, 2H), 7.95 (d, J=1.7 Hz, 1H), 7.68 (d, J=8.3 Hz, 2H), 7.62 (s, 1H), 7. 21 (d, J=3.0 Hz, 1H), 6.78 (d, J=2.0 Hz, 2H), 6. 73 (dd, J=3. 0 Hz, 1. 7 Hz, 1H), 6. 53 (t, J=2. 0 Hz, 1H), 4.19 (brs, 2H), 4.11 (brs, 2H), 3.77 (s, 6H)
Mass (m/z): 456 ($M^+$), 305
IR (KBr, $cm^{-1}$): 1647, 1601, 1558, 1510, 1473, 1458, 1421, 1209, 1155

| Elemental Analysis: as $C_{25}H_{24}N_6O_3 \cdot 1.0HCl \cdot 1.3\ H_2O$ | | | |
|---|---|---|---|
| Observed | C 58.13%, | H 5.32%, | N 15.98% |
| Calculated | C 58.15%, | H 5.39%, | N 16.27% |

Melting point: 224.6 - 225.2°C

Example 18

5-Amino-7-(3-formylphenyl)-2-(2-furyl)[1,2,4]triazolo[1,5-c] pyrimidine (Compound 18)

Compound G (3.22 g, 8.35 mmol) was dissolved in toluene (51 mL), and 2-(3-tributylstannylphenyl)-1,3-dioxalane (10.5 g, 24.0 mmol) and bistriphenylphosphine palladium dichloride (590 mg, 0.840 mmol) were added to the resulting solution, followed by reflux for 9 hours. The reaction solution was cooled to room temperature, and then

chloroform and a 2 mol/L aqueous solution of ammonium fluoride were added to the solution, followed by stirring. Then, the reaction mixture was filtered with Celite, and the filtrate was subjected to separation. The organic layer was dried over anhydrous magnesium sulfate, filtered, and then concentrated under reduced pressure. The residue was purified by silica gel column chromatography (elution solvent: mixed solvent of hexane and ethyl acetate) to obtain 5-(3,4-dimethoxybenzylamino)-7-[3-(1,3-dioxolan-2-yl)phenyl]-2-(2-furyl)[1,2,4]triazolo[1,5-c]pyrimidine (4.95 g, 10.8 mmol) as a white amorphous substance in a yield of 89%.

The resulting 5-(3,4-dimethoxybenzylamino)-7-[3-(1,3-dioxolan-2-yl)phenyl]-2-(2-furyl)[1,2,4]triazolo[1,5-c]pyrimidine (3.09 g, 7.21 mmol) was dissolved in a mixed solvent containing THF (90 mL) and 2 mol/L hydrochloric acid (60 mL), followed by stirring at room temperature for 5 hours. Then, a 2 mol/L aqueous solution of sodium hydroxide was added to the reaction solution, and the resulting mixture was subjected to extraction with ethyl acetate after being made basic. The organic layer was dried over anhydrous magnesium sulfate, filtered, and then concentrated under reduced pressure to obtain 5-(3,4-dimethoxybenzylamino)-7-(3-formylphenyl)-2-(2-furyl)[1,2,4]triazolo[1,5-c]pyrimidine (2.59 g, 5.70 mmol) as a crude product in a yield of 79%.

The resulting crude product was dissolved in dichloromethane (63 mL), and distilled water (3.5 mL) and DDQ (2.59 g, 11.4 mmol) were added to the resulting solution, followed by stirring at room temperature for 2 hours. Then, the reaction solution was poured into a 1 mol/L aqueous solution of sodium hydroxide, followed by extraction with chloroform. The organic layer was dried over anhydrous magnesium sulfate, filtered, and then concentrated under reduced pressure. The residue was triturated with chloroform to obtain Compound 18 (1.11 g, 3.64 mmol) as a white solid in a yield of 64%. Furthermore, Compound 18 was recrystallized from ethanol to obtain white crystals.
$^1$H NMR (δ ppm, DMSO-$d_6$): 10.12 (s, 1H), 8.69 (s, 1H), 8.45 (d, J=8.4 Hz, 1H), 8.13 (brs, 2H), 7.97 (d, J=8.9 Hz, 1H), 7.95 (d, J=1.7 Hz, 1H), 7. 75 (dd, J=8.9 Hz, 8.4 Hz, 1H), 7.68 (s, 1H), 7.23 (d, J=3.3 Hz, 1H), 6.74 (dd, J=3.3 Hz, 1.7 Hz, 1H)
Mass (m/z): 305 ($M^+$)
IR (KBr, $cm^{-1}$): 1676, 1641, 1606, 1560, 1514, 1411, 1221

| Elemental Analysis: as $C_{16}H_{11}N_5O_2 \cdot 0.3\ C_2H_5OH$ | | | |
|---|---|---|---|
| Observed | C 62.19%, | H 4.01%, | N 21.88% |
| Calculated | C 62.48%, | H 4.04%, | N 21.95% |

Melting point: 212.8 - 213.2°C

Example 19

5-Amino-2-(2-furyl)-7-{3-[2-(2-pyridyl)ethylaminomethyl]phenyl}[1,2,4]triazolo[1,5-c]pyrimidine (Compound 19)

The reaction was carried out in a manner similar to that in Example 4 except that Compound 2 was replaced by Compound 18. Then, the crude product was recrystallized from ethanol (10 mL) to obtain Compound 19 (165 mg, 0.37 mmol) as white crystals in a yield of 45%.
$^1$H NMR (δ ppm, DMSO-$d_6$): 8.47 (d, J=4.5 Hz, 1H), 8.00-8.10 (m, 4H), 7.94 (d, J=1.6 Hz, 1H), 7.66 (dd, J=8.0 Hz, 7.6 Hz, 1H), 7.51 (s, 1H), 7.40-7.50 (m, 2H), 7.27 (dd, J=8.0 Hz, 1.0 Hz, 1H), 7.21 (d, J=3.3 Hz, 1H), 7.18 (ddd, J=7.6 Hz, 4.5 Hz, 1.0 Hz, 1H), 6.74 (dd, J=3.3 Hz, 1.6 Hz, 1H), 3.89 (s, 2H), 2.80-2.90 (m, 4H)
Mass (m/z): 411 ($M^+$), 291
IR (KBr, $cm^{-1}$): 1666, 1655, 1602, 1560, 1415, 1331, 1234, 756

| Elemental Analysis: as $C_{23}H_{21}N_7O \cdot 0.7\ H_2O$ | | | |
|---|---|---|---|
| Observed | C 65.06%, | H 5.14%, | N 23.38% |
| Calculated | C 65.14%, | H 5.32%, | N 23.12% |

Melting point: 159.2 - 159.4°C

Example 20

(±)-5-Amino-2-(2-furyl)-7-{3-[(2-methoxy-1-methylethyl)aminomethyl]phenyl}[1,2,4]triazolo[1,5-c]pyrimidine monohydrochloride (Compound 20)

The reaction was carried out in a manner similar to that in Example 9 except that Compound 2 was replaced

by Compound 18. Then, the crude product was recrystallized from isopropyl alcohol (10 mL) to obtain Compound 20 (126 mg, 0.30 mmol) as white crystals in a yield of 37%.

$^1H$ NMR (δ ppm, DMSO-$d_6$): 9.55 (m, 1H), 9.38 (m, 1H), 8.40 (s, 1H), 8.16 (d, J=8.0 Hz, 1H), 8.10 (brs, 2H), 7.96 (d, J=1.7 Hz, 1H), 7.69 (d, J=7.6 Hz, 1H), 7.61 (s, 1H), 7.56 (dd, J=8.0 Hz, 7.6 Hz, 1H), 7.23 (d, J=3. 3 Hz, 1H), 6.74 (dd, J=3.3 Hz, 1.7 Hz, 1H), 4.25 (brs, 2H), 3.55-3.65 (m, 2H), 3.40 (m, 1H), 3.34 (s, 3H), 1.32 (d, J=6.6 Hz, 3H)

Mass (m/z): 378 ($M^+$), 333, 290

IR (KBr, $cm^{-1}$): 1705, 1683, 1670, 1620, 1614, 1606, 1560, 1458

| Elemental Analysis: as $C_{20}H_{22}N_6O_2 \cdot 1.0HCl \cdot 2.0\ H_2O$ | | | |
|---|---|---|---|
| Observed | C 53.27%, | H 5.66%, | N 18.26% |
| Calculated | C 53.27%, | H 6.03%, | N 18.64% |

Melting point: 244.9 - 245.5°C

Example 21

5-Amino-2-(2-furyl)-7-{3-[3-(2-oxopyrrolidin-1-yl)propylaminomethyl]phenyl}[1,2,4]triazolo[1,5-c]pyrimidine monohydrochloride (Compound 21)

The reaction was carried out in a manner similar to that in Example 8 except that Compound 2 was replaced by Compound 18. Then, the crude product was recrystallized from ethanol (8 mL) to obtain Compound 21 (80 mg, 0.17 mmol) as white crystals in a yield of 21%.

$^1H$ NMR (δ ppm, DMSO-$d_6$): 9.40 (m, 2H), 8.34 (s, 1H), 8.16 (d, J=7.6 Hz, 1H), 8.07 (brs, 2H), 7.96 (d, J=1.6 Hz, 1H), 7.63 (m, 1H), 7.60 (s, 1H), 7.57 (m, 1H), 7.23 (d, J=3.3 Hz, 1H), 6.74 (dd, J=3.3 Hz, 1.6 Hz, 1H), 4.22 (brs, 2H), 3.43 (t, J=5.7 Hz, 2H), 3.34 (t, J=6.6 Hz, 2H), 2.90 (m, 2H), 2.22 (t, J=7.5 Hz, 2H), 1.80-2.00 (m, 4H)

Mass (m/z): 431 ($M^+$), 305, 291

IR (KBr, $cm^{-1}$): 1686, 1674, 1637, 1602, 1597, 1508, 1414, 1329, 1217

| Elemental Analysis: as $C_{23}H_{25}N_7O_2 \cdot 1.0HCl \cdot 0.7\ H_2O$ | | | |
|---|---|---|---|
| Observed | C 57.45%, | H 5.45%, | N 20.13% |
| Calculated | C 57.48%, | H 5.75%, | N 20.40% |

Melting point: 252.2 - 252.9°C

Example 22

5-Amino-2-(2-furyl)-7-hydroxymethyl[1,2,4]triazolo[1,5-c]pyrimidine (Compound 22)

Compound G (10.0 g, 26.0 mmol) was dissolved in toluene (150 mL), and tert-butyldimethylsilyloxymethyltributyltin (14.7 g, 33.7 mmol) and bistriphenylphosphine palladium dichloride (1.00 g, 1.42 mmol) were added to the resulting solution, followed by reflux for 8 hours. The reaction solution was cooled to room temperature, and brine was added to the solution, followed by extraction with ethyl acetate. The organic layer was dried over anhydrous magnesium sulfate, filtered, and then concentrated under reduced pressure. The residue was purified by silica gel column chromatography (elution solvent: a mixed solvent of hexane and ethyl acetate) to obtain 7-(tert-butyldimethylsilyloxymethyl)-5-(3,4-dimethoxybenzylamino)-2-(2-furyl)[1,2,4]triazolo[1,5-c]pyrimidine (3.80 g, 7. 67 mmol) as a white solid in a yield of 30%, and also obtain 7-butyl-5-(3,4-dimethoxybenzylamino)-2-(2-furyl)[1,2,4]triazolo[1,5-c]pyrimidine (1.85 g, 4.54 mmol) as a light yellow oily substance in a yield of 18%.

The resulting 7-(tert-butyldimethylsilyloxymethyl)-5-(3,4-dimethoxybenzylamino)-2-(2-furyl)[1,2,4]triazolo[1,5-c] pyrimidine (3.80 g, 7.67 mmol) was dissolved in trifluoroacetic acid (80 mL), and anisole (4.20 mL, 38.4 mmol) and trifluoromethanesulfonic acid (3.40 mL, 38.4 mmol) were added to the resulting solution, followed by stirring at room temperature for 7 hours. Then, a 4 mol/L aqueous solution of sodium hydroxide was added to the mixture, and the precipitated white solid was filtered off and washed with diethyl ether to obtain Compound 22 (1.69 g, 7.32 mmol) as a white solid in a yield of 95%. Furthermore, Compound 22 was recrystallized from DMF to obtain white crystals.

$^1H$ NMR (δ ppm, DMSO-$d_6$): 7.93 (d, J=1.6 Hz, 1H), 7.92 (brs, 2H), 7.19 (d, J=3.3 Hz, 1H), 6.91 (s, 1H), 6.72 (dd, J=3.3 Hz, 1.6 Hz, 1H), 5.52 (t, J=5.7 Hz, 1H), 4.42 (d, J=5.7 Hz, 2H)

Mass (m/z): 232 ($M^+$+1)

IR (KBr, $cm^{-1}$): 1662, 1614, 1608, 1568, 1452, 1333, 1222, 982, 777
Melting point: 298.0 - 300.0°C

Example 23

5-Amino-2-(2-furyl)-7-methoxymethyl[1,2,4]triazolo[1,5-c] pyrimidine (Compound 23)

The 7-(tert-butyldimethylsilyloxymethyl)-5-(3,4-dimethoxybenzylamino)-2-(2-furyl)[1,2,4]triazolo[1,5-c] pyrimidine (2.70 g, 5.45 mmol) produced in Example 22 was dissolved in dichloromethane (84 mL), and di(tert-butyl) dicarbonate (1.78 g, 8.17 mmol), triethylamine (2.28 mL, 16.4 mmol), and 4-dimethylaminopyridine (133 mg, 0.109 mmol) were added to the resulting solution, followed by stirring at room temperature for 2 hours. Then, distilled water was added to the reaction solution, and extraction with ethyl acetate was performed. The organic layer was dried over anhydrous magnesium sulfate, filtered, and then concentrated under reduced pressure. The residue was purified by silica gel column chromatography (elution solvent: a mixed solvent of hexane and ethyl acetate) to obtain 5-[N-(tert-butoxycarbonyl)-3,4-dimethoxybenzylamino]-7-(tert-butyl dimethylsilyloxymethyl)-2-(2-furyl)[1,2,4]triazolo[1,5-c]pyrimidine (3.11 g, 5.23 mmol) as a white solid in a yield of 96%.

The resulting 5-[N-(tert-butoxycarbonyl)-3,4-dimethoxybenzylamino]-7-(tert-butyldimethylsilyloxymethyl)-2-(2-furyl) [1,2,4]triazolo[1,5-c]pyrimidine was dissolved in THF (60 mL), and a THF solution (60 mL) of 1 mol/L tetrabutylammonium fluoride was added to the resulting solution, followed by stirring at room temperature for 1 hour. The reaction solution was concentrated, and the residue was purified by silica gel column chromatography (elution solvent: a mixed solvent of hexane and ethyl acetate) to obtain 5-[N-(tert-butoxycarbonyl)-3,4-dimethoxybenzylamino]-2-(2-furyl)-7-hydroxymethyl[1,2,4]triazolo[1,5-c]pyrimidine (2.00 g, 4.16 mmol) as a white solid in a yield of 80%.

The resulting 5-[N-(tert-butoxycarbonyl)-3,4-dimethoxybenzylamino]-2-(2-furyl)-7-hydroxymethyl[1,2,4]triazolo[1,5-c]pyrimidine (800 mg, 1.66 mmol) was dissolved in iodomethane (8 mL), and silver oxide (1.15 g, 4.97 mmol) was added to the resulting solution, followed by stirring at room temperature for 12 hours. Then, the reaction solution was concentrated under reduced pressure to obtain 5-[N-(tert-butoxycarbonyl)-3,4-dimethoxybenzylamino]-2-(2-furyl)-7-methoxymethyl[1,2,4]triazolo[1,5-c]pyrimidine as a crude product.

The resulting crude product was dissolved in trifluoroacetic acid (6 mL), and anisole (0.544 mL, 4.98 mmol) and trifluoromethanesulfonic acid (0.440 mL, 4.98 mmol) were added to the resulting solution, followed by stirring at room temperature for 12 hours. Then, a 4 mol/L aqueous solution of sodium hydroxide was added to the mixture, and extraction with ethyl acetate was performed. The organic layer was dried over anhydrous magnesium sulfate, filtered, and then concentrated under reduced pressure. The residue was purified by silica gel column chromatography (elution solvent: a mixed solvent of chloroform and methanol), and then recrystallized from ethanol (10 mL) to obtain Compound 23 (174 mg, 0.709 mmol) as white crystals in a yield of 43%.
$^1$H NMR (δ ppm, DMSO-$d_6$): 7.98 (brs, 2H), 7.93 (d, J=1.6 Hz, 1H), 7.19 (d, J=3.3 Hz, 1H), 6.87 (s, 1H), 6.73 (dd, J=3.3 Hz, 1.6 Hz, 1H), 4.35 (s, 2H), 3.40 (s, 3H)
Mass (m/z): 245 ($M^+$), 215
IR (KBr, $cm^{-1}$): 1675, 1610, 1427, 1122, 746

| Elemental Analysis: as $C_{11}H_{11}N_5O_2$ | | | |
|---|---|---|---|
| Observed | C 53.93%, | H 4.65%, | N 28.51% |
| Calculated | C 53.87%, | H 4.52%, | N 28.56% |

Melting point: 167.2 - 167.6°C

Example 24

5-Amino-7-ethoxymethyl-2-(2-furyl)[1,2,4]triazolo[1,5-c]pyrimidine (Compound 24)

The reaction was carried out in a manner similar to that in Example 23 except that iodomethane was replaced by iodoethane. Then, the crude product was recrystallized from ethanol (10 mL) to obtain Compound 24 (118 mg, 0.363 mmol) as white crystals in a yield of 43%.
$^1$H NMR (δ ppm, DMSO-$d_6$): 7.97 (brs, 2H), 7.93 (d, J=1.7 Hz, 1H), 7.18 (d, J=3.3 Hz, 1H), 6.88 (s, 1H), 6.72 (dd, J=3.3 Hz, 1.7 Hz, 1H), 4.39 (s, 2H), 3.59 (q, J=7.2 Hz, 2H), 1.21 (t, J=7.2 Hz, 3H)
Mass (m/z): 259 ($M^+$), 215
IR (KBr, $cm^{-1}$): 1679, 1668, 1618, 1612, 1603, 1558, 725

| Elemental Analysis: as $C_{12}H_{13}N_5O_2$ | | | |
|---|---|---|---|
| Observed | C 55.47%, | H 5.19%, | N 26.88% |
| Calculated | C 55.59%, | H 5.05%, | N 27.01% |

Melting point: 153.8 - 154.6°C

Example 25

5-Amino-2-(2-furyl)-7-(1-methyl-1-methoxyethyl)[1,2,4]triazolo[1,5-c]pyrimidine (Compound 25)

THF (15 mL) was added to a THF solution (10.7 mL, 10.2 mmol) of 0.95 mol/L methylmagnesium bromide, followed by stirring at -30°C in an argon atmosphere. A THF (52 mL) solution of 7-acetyl-5-(3,4-dimethoxybenzylamino)-2-(2-furyl)[1,2,4]triazolo[1,5-c]pyrimidine (1.00 g, 2.55 mmol) produced in Example 38 was added dropwise to the reaction mixture at the same temperature, followed by further stirring at the same temperature for 1 hour. After the reaction solution was cooled to -78°C, 0.1 mol/L hydrochloric acid and distilled water were successively added to the solution, and extraction with ethyl acetate was performed. The organic layer was dried over anhydrous magnesium sulfate, filtered, and then concentrated under reduced pressure. The residue was dissolved in chloroform (300 mL), and distilled water (17 mL) and DDQ (1.74 g, 7.65 mmol) were added to the resulting solution, followed by stirring at room temperature for 3 hours. The reaction solution was poured into a mixture of chloroform and aqueous sodium bicarbonate, and then subjected to separation. The organic layer was dried over anhydrous magnesium sulfate, filtered, and then concentrated under reduced pressure. The residue was purified by silica gel column chromatography (elution solvent: a mixed solvent of chloroform and methanol), and then recrystallized from ethanol (6 mL) to obtain Compound 25 (110 mg, 0.424mmol) as white crystals in a yield of 17%.
$^1$H NMR (δ ppm, DMSO-$d_6$): 7.93 (d, J=1.6 Hz, 1H), 7.85 (brs, 2H), 7.18 (d, J=3.3 Hz, 1H), 7.08 (s, 1H), 6.71 (dd, J=3.3 Hz, 1.6 Hz, 1H), 5.26 (s, 1H), 1.44 (s, 6H)
Mass (m/z): 259 ($M^+$), 244
IR (KBr, $cm^{-1}$): 1666, 1657, 1612, 1558, 1508, 763

| Elemental Analysis: as $C_{12}H_{13}N_5O_2$ | | | |
|---|---|---|---|
| Observed | C 55.63%, | H 5.23%, | N 27.03% |
| Calculated | C 55.59%, | H 5.05%, | N 27.01% |

Melting point: 208.2 - 208.8°C

Example 26

5-Amino-7-(1-ethoxycyclopropyl)-2-(2-furyl)[1,2,4]triazolo[1,5-c]pyrimidine (Compound 26)

First, 5-(3,4-dimethoxybenzylamino)-7-(1-ethoxyvinyl)-2-(2-furyl)[1,2,4]triazolo[1,5-c]pyrimidine (4.77 g, 11.1 mmol) produced in Example 38 was dissolved in (250 mL), and a hexane solution (66.7 mL, 66.7 mmol) of 1.0 mol/L diethylzinc was added to the resulting solution at 0°C under stirring at the same temperature. Furthermore, a solution of diiodomethane (6.30 mL, 77.8 mmol) in toluene (10 mL) was added dropwise to the resulting mixture, followed by stirring at room temperature for 25 hours. The reaction solution was poured into a mixture containing chloroform and an aqueous saturated ammonium acetate solution, and filtered with Celite. Then, the filtrate was subjected to extraction, and the organic layer was dried over anhydrous magnesium sulfate, filtered, and then concentrated under reduced pressure. The residue was purified by silica gel column chromatography (elution solvent: a mixed solvent of hexane and ethyl acetate) to obtain 5-(3,4-dimethoxybenzylamino)-7-(1-ethoxycyclopropyl)-2-(2-furyl)[1,2,4]triazolo[1,5-c]pyrimidine (500 mg, 1.15 mmol) as a white amorphous substance in a yield of 10%.

The resulting 5-(3,4-dimethoxybenzylamino)-7-(1-ethoxycyclopropyl)-2-(2-furyl)[1,2,4]triazolo[1,5-c]pyrimidine (178 mg, 0.409 mmol) was dissolved in chloroform (4.5 mL), and distilled water (0.25 mL) and DDQ (278 mg, 1.23 mmol) were added to the resulting solution, followed by stirring at room temperature for 2 hours. The reaction solution was poured into a 1 mol/L aqueous sodium hydroxide solution, and extraction with chloroform was performed. The organic layer was dried over anhydrous magnesium sulfate, filtered, and then concentrated under reduced pressure. The residue was purified by silica gel column chromatography (elution solvent: a mixed solvent of hexane and ethyl acetate), and then the resulting white solid was recrystallized from ethanol (5 mL) to obtain Compound 26 (150 mg,

0.526 mmol) as white crystals in a yield of 34%.
$^1$H NMR (δ ppm, DMSO-$d_6$): 7.92 (d, J=1.7 Hz, 1H), 7.86 (brs, 2H), 7.17 (d, J=3.3 Hz, 1H), 7.00 (s, 1H), 6.72 (dd, J=3.3 Hz, 1.7 Hz, 1H), 3.56 (q, J=6.9 Hz, 2H), 1.08-1.16 (m, 7H)
Mass (m/z): 286 ($M^+$+1)
IR (KBr, $cm^{-1}$): 1653, 1645, 1622, 1606, 1546, 1259, 1062

| Elemental Analysis: as $C_{14}H_{15}N_5O_2$ | | | |
|---|---|---|---|
| Observed | C 58.99%, | H 5.29%, | N 24.31% |
| Calculated | C 58.94%, | H 5.30%, | N 24.55% |

Melting point: 187.2 - 187.6°C

Example 27

5-Amino-2-(2-furyl)-7-phenoxymethyl[1,2,4]triazolo[1,5-c]pyrimidine (Compound 27)

Compound 22 (200 mg, 0.866 mmol) was dissolved in THF (12 mL), and triphenylphosphine (682 mg, 2.60 mmol), phenol (245 mg, 2.60 mmol), and diethyl azodicarboxylate (0.409 mL, 2.60 mmol) were added to the resulting solution, followed by stirring at room temperature for 12 hours. Then, 2 mol/L hydrochloric acid (12 mL) was added to the reaction solution, followed boy reflux for 1 hour. Then, a 4 mol/L aqueous solution of sodium hydroxide was added to the resulting mixture, and the reaction solution was subjected to extraction with chloroform after neutralization. The organic layer was dried over anhydrous magnesium sulfate, filtered, and then concentrated under reduced pressure. The residue was purified by silica gel column chromatography (elution solvent: a mixed solvent of hexane and ethyl acetate), and then the resulting solid was recrystallized from ethanol (15 mL) to obtain Compound 27 (70.0 mg, 0.228 mmol) as white crystals in a yield of 26%.
$^1$H NMR (δ ppm, DMSO-$d_6$): 8.08 (brs, 2H), 7.93 (d, J=1.0 Hz, 1H), 7.32 (dd, J=7.9 Hz, 7.3 Hz, 2H), 7.18 (d, J=3.3 Hz, 1H), 7.05 (d, J=7.9 Hz, 2H), 6.99 (s, 1H), 6.95 (t, J=7.3 Hz, 1H), 6.72 (dd, J=3.3 Hz, 1.0 Hz, 1H), 5.04 (s, 2H)
Mass (m/z): 307 ($M^+$+1)
IR (KBr, $cm^{-1}$): 1676, 1610, 1589, 1560, 1497, 1425, 1240, 744

| Elemental Analysis: as $C_{16}H_{13}N_5O_2$ | | | |
|---|---|---|---|
| Observed | C 62.63%, | H 4.29%, | N 22.87% |
| Calculated | C 62.58%, | H 4.26%, | N 22.79% |

Melting point: 247.2 - 247.4°C

Example 28

5-Amino-7-(3-bromophenoxymethyl)-2-(2-furyl)[1,2,4]triazolo[1,5-c]pyrimidine (Compound 28)

First, 5-(3,4-dimethoxybenzylamino)-2-(2-furyl)-7-hydroxymethyl[1,2,4]triazolo[1,5-c]pyrimidine (300 mg, 0.787 mmol) produced in Example 31 was dissolved in THF(30 mL), and triphenylphosphine (414 mg, 1.58 mmol), diethyl azodicarboxylate (0.249 mL, 1.58 mmol), and 3-bromophenol (273 mg, 1.58 mmol) were added to the resulting solution, followed by stirring at room temperature for 1 hour. Then, aqueous saturated sodium bicarbonate was added to the reaction solution, and extraction with chloroform was performed. The organic layer was dried over anhydrous magnesium sulfate, filtered, and then concentrated under reduced pressure. The residue was purified by silica gel column chromatography (elution solvent: a mixed solvent of hexane and ethyl acetate), and then the resulting solid was triturated with methanol to obtain 7-(3-bromophenoxymethyl)-5-(3,4-dimethoxybenzylamino)-2-(2-furyl)[1,2,4]triazolo[1,5-c]pyrimidine (382 mg, 0.691 mmol) as a white solid in a yield of 87%.
The resulting 7-(3-bromophenoxymethyl)-5-(3,4-dimethoxybenzylamino)-2-(2-furyl)[1,2,4]triazolo[1,5-c]pyrimidine was dissolved in trifluoroacetic acid (6 mL), and anisole (0.239 mL, 2.20 mmol) and trifluoromethanesulfonic acid (0.194 mL, 2.20 mmol) were added to the resulting solution, followed by stirring at room temperature for 12 hours. Then, a 4 mol/L aqueous solution of sodium hydroxide was added to the reaction solution, and the resulting mixture was subjected to extraction with chloroform. The organic layer was dried over anhydrous magnesium sulfate, filtered, and then concentrated under reduced pressure. The residue was triturated with diethyl ether, and the resulting solid was recrystallized from ethanol (15 mL) to obtain Compound 28 (196 mg, 0.494 mmol) as white crystals in a yield of 72%.

$^1H$ NMR (δ ppm, DMSO-$d_6$): 8.09 (brs, 2H), 7.94 (d, J=1.6 Hz, 1H), 7.25 (d, J=3.3 Hz, 1H), 7.05-7.25 (m, 4H), 7.02 (s, 1H), 6.73 (dd, J=3.3 Hz, 1.6 Hz, 1H), 5.08 (s, 2H)
Mass (m/z): 387($M^+$+2), 385($M^+$), 214
IR (KBr, $cm^{-1}$): 1666, 1652, 1608, 1578, 1473, 1223, 768

| Elemental Analysis: as $C_{16}H_{12}N_5O_2Br\cdot 0.3\ H_2O\cdot 0.2\ C_2H_5OH$ | | | |
|---|---|---|---|
| Observed | C 49.12%, | H 3.10%, | N 17.36% |
| Calculated | C 49.14%, | H 3.47%, | N 17.47% |

Melting point: 261.5 - 261.9°C

Example 29

5-Amino-7-[2-(1-hydroxy-1-methylethyl)phenoxymethyl]-2-(2-furyl)[1,2,4]triazolo[1,5-c]pyrimidine (Compound 29)

First, 5-(3,4-dimethoxybenzylamino)-2-(2-furyl)-7-hydroxymethyl[1,2,4]triazolo[1,5-c]pyrimidine (1.00 g, 2.62 mmol) produced in Example 31 was dissolved in THF (80 mL), and triphenylphosphine (1.38 g, 5.26 mmol), diethyl azodicarboxylate (0.828 mL, 5.26 mmol), and methyl salicylate (800 mg, 5.26 mmol) were added to the resulting solution, followed by stirring at room temperature for 1 hour. Then, aqueous saturated sodium bicarbonate was added to the reaction solution, and extraction with chloroform was performed. The organic layer was dried over anhydrous magnesium sulfate, filtered, and then concentrated under reduced pressure. The residue was purified by silica gel column chromatography (elution solvent: a mixed solvent of hexane and ethyl acetate), and the resulting solid was triturated with methanol to obtain 5-(3,4-dimethoxybenzylamino)-7-[(2-methoxycarbonylphenoxy)methyl]-2-(2-furyl)[1,2,4]triazolo[1,5-c]pyrimidine (630 mg, 1.22 mmol) as a white solid in a yield of 47%.

The obtained 5-(3,4-dimethoxybenzylamino)-7-[2-(1-hydroxy-1-methylethyl)phenoxymethyl]-2-(2-furyl)[1,2,4]triazolo[1,5-c]pyrimidine (600 mg, 1.16 mmol) was dissolved in THF (30 mL), and the resulting solution was cooled to 0°C in an argon atmosphere. Then, a THF solution (15.9 mL, 15.1 mmol) of 0.95 mol/L methylmagnesium bromide was added dropwise to the reaction solution, followed by stirring at room temperature for 3 hours. Then, distilled water was added to the reaction solution, and the resulting mixture was subjected to extraction with ethyl acetate. The organic layer was dried over anhydrous magnesium sulfate, filtered, and then concentrated under reduced pressure. The residue was purified by silica gel column chromatography (elution solvent: a mixed solvent of hexane and ethyl acetate) to obtain 5-(3,4-dimethoxybenzylamino)-7-[2-(1-hydroxy-1-methylethyl)phenoxymethyl]-2-(2-furyl)[1,2,4]triazolo[1,5-c]pyrimidine (399 mg, 0.774 mmol) as a white solid in a yield of 67%.

The obtained 5-(3,4-dimethoxybenzylamino)-7-[2-(1-hydroxy-1-methylethyl)phenoxymethyl]-2-(2-furyl)[1,2,4]triazolo[1,5-c]pyrimidine was dissolved in chloroform (36 mL), and distilled water (2 mL) and DDQ (529 mg, 2.33 mmol) were added to the resulting solution, followed by stirring at room temperature for 5 hours. The reaction solution was poured into a mixture of chloroform and aqueous saturated sodium bicarbonate, and then the mixture was subjected to separation. The organic layer was dried over anhydrous magnesium sulfate, filtered, and then concentrated under reduced pressure. The residue was recrystallized from ethanol (4 mL) to obtain Compound 29 (118 mg, 0.322 mmol) as white crystals in a yield of 42%.
$^1H$ NMR (δ ppm, DMSO-$d_6$): 8.08 (brs, 2H), 7.93 (d, J=1.7 Hz, 1H), 7.62 (d, J=7.6 Hz, 1H), 7.10-7.25 (m, 2H), 6.90-7.10 (m, 3H), 6.72 (dd, J=3.3 Hz, 1.7 Hz, 1H), 5.07 (s, 2H), 5.01 (s, 1H), 1.60 (s, 6H)
Mass (m/z): 366 ($M^+$+1)
IR (KBr, $cm^{-1}$): 3450, 1686, 1672, 1654, 1604, 1560, 1457, 1234, 1209

| Elemental Analysis: as $C_{19}H_{19}N_5O_3\cdot 0.3H_2O$ | | | |
|---|---|---|---|
| Observed | C 61.39%, | H 5.09%, | N 18.90% |
| Calculated | C 61.55%, | H 5.33%, | N 18.89% |

Melting point: 194.0 - 194.2°C

Example 30

5-Amino-7-(2-fluorophenoxymethyl)-2-(2-furyl)[1,2,4]triazolo[1,5-c]pyrimidine (Compound 30)

The reaction was carried out in a manner similar to that in Example 28 except that 3-bromophenol was replaced

by 2-fluorophenol. The resulting crude product was recrystallized from ethanol (9 mL) to obtain Compound 30 (118 mg, 0.363 mmol) as white crystals in a yield of 48%.
$^{1}H$ NMR (δ ppm, DMSO-$d_6$): 8.09 (brs, 2H), 7.92 (d, J=1.6 Hz, 1H), 7.25-7. 30 (m, 2H), 7.21 (d, J=3.3 Hz, 1H), 7.15 (m, 1H), 6.90-7.00 (m, 2H), 6.71 (dd, J=3.3 Hz, 1.6 Hz, 1H), 5.16 (s, 2H)
Mass (m/z): 325 ($M^+$), 214
IR (KBr, $cm^{-1}$): 1673, 1650, 1645, 1608, 1564, 1506, 1261, 744

| Elemental Analysis: as $C_{16}H_{12}N_5O_2F$ | | | |
|---|---|---|---|
| Observed | C 59.29%, | H 3.72%, | N 21.68% |
| Calculated | C 59.08%, | H 3.72%, | N 21.53% |

Melting point: 196.8 - 197.1°C

Example 31

5-Amino-2-(2-furyl)-7-(pyridin-3-yloxymethyl)[1,2,4]triazolo[1,5-c]pyrimidine (Compound 31)

First, 7-(tert-butyldimethylsilyloxymethyl)-5-(3,4-dimethoxybenzylamino)-2-(2-furyl)[1,2,4]triazolo[1,5-c]pyrimidine (2.66 g, 5.37 mmol) produced in Example 22 was dissolved in THF (60 mL), and a THF solution (8.05 mL, 8.05 mmol) of 1 mol/L tetrabutylammonium fluoride was added to the resulting solution, followed by stirring at room temperature for 1 hour. After the reaction solution was concentrated, the residue was triturated with methanol to obtain 5-(3,4-dimethoxybenzylamino)-2-(2-furyl)-7-hydroxymethyl[1,2,4]triazolo[1,5-c]pyrimidine (1.94 g, 5.09 mmol) as a white solid in a yield of 95%.
The obtained 5-(3,4-dimethoxybenzylamino)-2-(2-furyl)-7-hydroxymethyl[1,2,4]triazolo[1,5-c]pyrimidine (500 mg, 1.31 mmol) was dissolved in THF (15 mL), and 3-pyridinol (250 mg, 2.62 mmol) and (cyanomethylene)tributylphosphorane (632 mg, 2.62 mmol) were added to the resulting solution, followed by stirring at 120°C for 19 hours. Then, aqueous saturated sodium bicarbonate was added to the reaction solution, and extraction with chloroform was performed. The organic layer was dried over anhydrous magnesium sulfate, filtered, and then concentrated under reduced pressure. The residue was purified by silica gel column chromatography (elution solvent: a mixed solvent of hexane and ethyl acetate). The resulting solid was triturated with diethyl ether to obtain 5-(3,4-dimethoxybenzylamino)-2-(2-furyl)-7-(pyridin-3-yloxymethyl)[1,2,4]triazolo[1,5-c]pyrimidine (312 mg, 0.681 mmol) as a white solid in a yield of 52%.
The obtained 5-(3,4-dimethoxybenzylamino)-2-(2-furyl)-7-(3-pyridyloxymethyl)[1,2,4]triazolo[1,5-c]pyrimidine was dissolved in trifluoroacetic acid (6 mL), and anisole (0.333 mL, 3.03 mmol) and trifluoromethanesulfonic acid (0.268 mL, 3.03 mmol) were added to the resulting solution, followed by stirring at room temperature for 8 hours. Then, a 4 mol/L aqueous solution of sodium hydroxide was added to the reaction solution, and the resulting mixture was subjected to extraction with chloroform. The organic layer was dried over anhydrous magnesium sulfate, filtered, and then concentrated under reduced pressure. The residue was triturated with diethyl ether, and the resulting solid was recrystallized from a mixed solvent of ethanol (15 mL) and chloroform (4 mL) to obtain Compound 31 (147 mg, 0.477 mmol) as white crystals in a yield of 70%.
$^{1}H$ NMR (δ ppm, DMSO-$d_6$): 8.42 (d, J=2.6 Hz, 1H), 8.21 (dd, J=4.6 Hz, 1.4 Hz, 1H), 8.10 (brs, 2H), 7.93 (d, J=1.9 Hz, 1H), 7.49 (ddd, J=8.6 Hz, 2.6 Hz, 1.4 Hz, 1H), 7.35 (dd, J=8.6 Hz, 4.6 Hz, 1H), 7.18 (d, J=3.3 Hz, 1H), 7.04 (s, 1H), 6.72 (dd, J=3.3 Hz, 1.9 Hz, 1H), 5.12 (s, 2H)
Mass (m/z): 308 ($M^+$), 214
IR (KBr, $cm^{-1}$): 1678, 1651, 1608, 1560, 1427, 1273, 1232, 976, 746

| Elemental Analysis: as $C_{15}H_{12}N_6O_2$ | | | |
|---|---|---|---|
| Observed | C 58.14%, | H 4.02%, | N 27.27% |
| Calculated | C 58.44%, | H 3.92%, | N 27.26% |

Melting point: 253.5 - 254.0°C

Example 32

5-Amino-2-(2-furyl)-7-(pyridin-2-yloxymethyl)[1,2,4]triazolo[1,5-c]pyrimidine (Compound 32)

First, 5-(3,4-dimethoxybenzylamino)-2-(2-furyl)-7-hydroxymethyl[1,2,4]triazolo[1,5-c]pyrimidine (500 mg, 1.31 mmol) produced in Example 31 was dissolve din THF (15 mL), and 2-pyridinol (250 m g , 2.62 mmol) and (cyanomethylene)tributylphosphorane (632 mg, 2.62 mmol) were added to the resulting solution, followed by stirring at 120°C for 25 hours. Then, aqueous saturated sodium bicarbonate was added to the reaction solution, and extraction with chloroform was performed. The organic layer was dried over anhydrous magnesium sulfate, filtered, and then concentrated under reduced pressure. The residue was purified by silica gel column chromatography (elution solvent: a mixed solvent of chloroform and methanol) to obtain 5-(3,4-dimethoxybenzylamino)-2-(2-furyl)-7-(pyridin-2-yloxymethyl)[1,2,4]triazolo[1,5-c]pyrimidine (132 mg, 0.288 mmol) as a light brown amorphous substance in a yield of 22%, and also obtain 5-(3,4-dimethoxybenzylamino)-2-(2-furyl)-7-(2-oxopyridin-1-ylmethyl)[1,2,4]triazolo[1,5-c]pyrimidine (362 mg, 0.790 mmol) as a white solid in a yield of 60%.

The obtained 5-(3,4-dimethoxybenzylamino)-2-(2-furyl)-7-(pyridin-2-yloxymethyl)[1,2,4]triazolo[1,5-c]pyrimidine (67.8 mg, 0.148 mmol) was dissolved in trifluoroacetic acid (1 mL), and anisole (0.333 mL, 3.03 mmol) and trifluoromethanesulfonic acid (0.268 mL, 3.03 mmol) were added to the resulting solution, followed by stirring at room temperature for 5 hours. Then, a 4 mol/L aqueous solution of sodium hydroxide was added to the reaction solution, and the resulting mixture was subjected to extraction with chloroform. The organic layer was dried over anhydrous magnesium sulfate, filtered, and then concentrated under reduced pressure. The residue was triturated with diethyl ether, and the resulting solid was recrystallized from a mixed solvent of ethanol (10 mL) and DMF (1 mL) to obtain Compound 32 (29.7 mg, 0.0964 mmol) as white crystals in a yield of 65%.
$^1$H NMR (δ ppm, DMSO-$d_6$): 8.16 (d, J=5.0 Hz, 1H), 8.06 (brs, 2H), 7.93 (d, J=1.7 Hz, 1H), 7.78 (ddd, J=8.2 Hz, 7.3 Hz, 2.0 Hz, 1H), 7.16 (d, J=3. 3 Hz, 1H), 6.95-7.05 (m, 2H), 6.91 (s, 1H), 6.72 (dd, J=3.3 Hz, 1.7 Hz, 1H), 5.29 (s, 2H)
Mass (m/z): 309 ($M^+$+1)
IR (KBr, $cm^{-1}$): 1633, 1621, 1614, 1606, 1601, 1575, 1515, 1506, 1471, 1456, 1434, 1429, 1311, 1268, 1214, 732

| Elemental Analysis: as $C_{15}H_{12}N_6O_2 \cdot 0.3H_2O \cdot 0.1\ C_2H_5OH$ | | | |
|---|---|---|---|
| Observed | C 57.39%, | H 4.15%, | N 26.55% |
| Calculated | C 57.36%, | H 4.18%, | N 26.40% |

Melting point: 229.0 - 229.2°C

Example 33

5-Amino-2-(2-furyl)-7-(pyridin-4-yloxymethyl)[1,2,4]triazolo[1,5-c]pyrimidine (Compound 33)

The reaction was carried out in a manner similar to that in Example 31 except that 3-pyridinol was replaced by 4-pyridinol. The resulting crude product was recrystallized from ethanol (6 mL) to obtain Compound 33 (128 mg, 0.416 mmol) as white crystals in a yield of 39%.
$^1$H NMR (δ ppm, DMSO-$d_6$): 8.11 (brs, 2H), 7.93 (d, J=1.6 Hz, 1H), 7.73 (d, J=7.5 Hz, 2H), 7.18 (d, J=3.3 Hz, 1H), 6.75 (s, 1H), 6.71 (dd, J=3.3 Hz, 1.6 Hz, 1H), 6.13 (d, J=7.5 Hz, 2H), 5.02 (s, 2H)
Mass (m/z): 308 ($M^+$), 214
IR (KBr, $cm^{-1}$): 1655, 1649, 1632, 1562, 1549, 1508, 1425, 1329, 1198, 1182

| Elemental Analysis: as $C_{15}H_{12}N_6O_2 \cdot 0.4H_2O$ | | | |
|---|---|---|---|
| Observed | C 57.06%, | H 4.04%, | N 26.34% |
| Calculated | C 57.10%, | H 4.09%, | N 26.64% |

Melting point: > 300°C

Example 34

7-[(N-acetyl-3,4-dimethoxybenzylamino)methyl]-5-amino-2-(2-furyl)[1,2,4]triazolo[1,5-c]pyrimidine (Compound 34)

First, Compound 42 (250 mg, 0.66 mmol) produced in Example 42 was dissolved in chloroform (6 mL), and

EDC resin (1.32 g, 1.32 mmol), 1-hydroxybenzotriazole (101 mg, 0.66 mmol), and acetic acid (0.15 mL, 2.63 mmol) were added to the resulting solution, followed by stirring at room temperature for 72 hours. The reaction solution was filtered, and the filtered-off resin was washed with methanol. A mixture of the filtrate and the washing solution was concentrated, and the residue was dissolved in chloroform. The resulting solution was successively washed with aqueous saturated sodium bicarbonate and saturated brine. The organic layer was dried over anhydrous magnesium sulfate, filtered, and then concentrated under reduced pressure. The residue was recrystallized from a mixed solvent of diethyl ether (13 mL) and ethanol (1 mL) to obtain Compound 34 (29.7 mg, 0.0964 mmol) as white crystals in a yield of 55%.
$^1$H NMR (δ ppm, DMSO-$d_6$): 8.01 (brs, 1.2H), 7.95 (brs, 0.8H), 7.93 (d, J=1.0 Hz, 1H), 7.15-7.25 (m, 1H), 6.70-7.00 (m, 5H), 4.59 (s, 0.8H), 4.49 (s, 1.2H), 4.38 (s, 2H), 3.73 (s, 1.2H), 3.72 (s, 1.2H), 3.71 (s, 1.8H), 3.70 (s, 1.8H), 2.19 (s, 1.2H), 2.17 (s, 1.8H)
Mass (m/z): 422 ($M^+$), 215
IR (KBr, $cm^{-1}$): 1653, 1612, 1560, 1514, 1419, 1252, 1234, 1182, 748

| Elemental Analysis: as $C_{21}H_{22}N_6O_4 \cdot 0.5H_2O$ | | | |
|---|---|---|---|
| Observed | C 58.50%, | H 5.19%, | N 19.42% |
| Calculated | C 58.46%, | H 5.37%, | N 19.49% |

Melting point: 136.0 - 136.5°C

Example 35

5-Amino-7-[(N-methyl-3,4-dimethoxybenzylamino)methyl]-2-(2-furyl)[1,2,4]triazolo[1,5-c]pyrimidine monohydrochloride (Compound 35)

Compound 42 (200 mg, 0.53 mmol) produced in Example 42 was dissolved in methanol (16 mL), and a 35% aqueous solution of paraformaldehyde (448 mg, 5.22 mmol) was added to the resulting solution, followed by stirring at room temperature for 2 hours. Then, sodium borohydride (300 mg, 7.93 mmol) was added to the reaction solution, and the resulting mixture was stirred at room temperature for 2 hours. The reaction solution was subjected to extraction with chloroform, and then the organic layer was washed by saturated brine. The organic layer was dried over anhydrous magnesium sulfate, filtered, and then concentrated under reduced pressure. The residue was purified by silica gel column chromatography (elution solvent: a mixed solvent of chloroform and methanol), and an ethyl acetate solution of hydrogen chloride was added to the resulting crude product. The mixture was concentrated under reduced pressure, and the residue was recrystallized from a mixed solvent of diisopropyl ether (5 mL), isopropyl alcohol (2 mL), and ethyl acetate (4 mL) to obtain Compound 35 (130 mg, 0.33 mmol) as white crystals in a yield of 62%.
$^1$H NMR (δ ppm, DMSO-$d_6$): 10.83 (m, 1H), 8.14 (brs, 2H), 7.95 (d, J=1.6 Hz, 1H), 7.35 (d, J=1.7 Hz, 1H), 7.29 (s, 1H), 7.23 (d, J=3.3 Hz, 1H), 7. 15 (dd, J=8.2 Hz, 1.7 Hz, 1H), 7.00 (d, J=8.2 Hz, 1H), 6.74 (dd, J=3.3 Hz, 1.6 Hz, 1H), 4.37 (brs, 2H), 4.18 (s, 2H), 3.80 (s, 3H), 3.78 (s, 3H), 2.70 (s, 3H)
Mass (m/z): 394 ($M^+$), 215
IR (KBr, $cm^{-1}$): 1659, 1639, 1616, 1605, 1601, 1560, 1516, 1460, 1425, 1246, 1024

| Elemental Analysis: as $C_{20}H_{22}N_6O_3 \cdot 1.0HCl \cdot 0.1[(CH_3)_2CH]_2O \cdot 0.7\ H_2O$ | | | |
|---|---|---|---|
| Observed | C 54.57%, | H 6.11%, | N 18.46% |
| Calculated | C 54.53%, | H 5.73%, | N 18.52% |

Melting point: 153.4 - 153.9°C

Example 36

5-Amino-7-anilinomethyl-2-(2-furyl)[1,2,4]triazolo[1,5-c]pyrimidine (Compound 36)

First, Compound 41 (212 mg, 0.925 mmol) produced in Example 41 was dissolved in ethanol (12 mL), and aniline (0.192 mL, 2.11 mmol) was added to the resulting solution, followed by reflux for 5 hours. The reaction solution was concentrated under reduced pressure, and the residue was dissolved in methanol (12 mL). Then, sodium borohydride (105 mg, 2.77 mmol) was added to the resulting solution, and the mixture was stirred at room temperature for 2 hours. Then, distilled water added to the reaction mixture, and the precipitated solid was filtered off. The obtained solid was successively washed with methanol and chloroform, and then recrystallized from a mixed solvent of ethanol

(10 mL) and DMF (5 mL) to obtain Compound 36 (223 mg, 0.728 mmol) as white crystals in a yield of 79%.
$^1$H NMR (δ ppm, DMSO-$d_6$): 7.97 (brs, 2H), 7.90 (d, J=1.6 Hz, 1H), 7.13 (d, J=3.3 Hz, 1H), 7.06 (dd, J=8.8 Hz, 7.6 Hz, 2H), 6.78 (s, 1H), 6.69 (dd, J=3.3 Hz, 1.6 Hz, 1H), 6.58 (d, J=7.6 Hz, 2H), 6.53 (t, J=8.8 Hz, 1H), 6.31 (t, J=5.9 Hz, 1H), 4.21 (d, J=5.9 Hz, 2H)
Mass (m/z): 306 ($M^+$), 214
IR (KBr, $cm^{-1}$): 1670, 1662, 1612, 1600, 1560, 1506, 1419, 1331, 756

| Elemental Analysis: as $C_{16}H_{14}N_6O$ | | | |
|---|---|---|---|
| Observed | C 62.43%, | H 4.73%, | N 27.26% |
| Calculated | C 62.73%, | H 4.61%, | N 27.43% |

Melting point: 279.2 - 279.6°C

Example 37

5-Amino-2-(2-furyl)-7-(2-oxopyridin-1-ylmethyl)[1,2,4]triazolo[1,5-c]pyrimidine (Compound 37)

First, 5-(3,4-dimethoxybenzylamino)-2-(2-furyl)-7-(2-oxopyridin-1-ylmethyl)[1,2,4]triazolo[1,5-c]pyrimidine (317 mg, 0.692 mmol) produced in Example 32 was dissolved in trifluoroacetic acid (6 mL), and anisole (0.226 mL, 2.07 mmol) and trifluoromethanesulfonic acid (0.183 mL, 2.07 mmol) were added to the resulting solution, followed by stirring at room temperature for 2 hours. Then, a 4 mol/L aqueous solution of sodium hydroxide was added to the reaction solution, and extraction with chloroform was performed. The organic layer was dried over anhydrous magnesium sulfate, filtered, and then concentrated under reduced pressure. The residue was recrystallized from ethanol (10 mL) to obtain Compound 37 (184 mg, 0.595 mmol) as white crystals in a yield of 86%.
$^1$H NMR (δ ppm, DMSO-$d_6$): 8.04 (brs, 2H), 7.92 (d, J=1.6 Hz, 1H), 7.76 (dd, J=7.1 Hz, 1.7 Hz, 1H), 7.49 (ddd, J=8.6 Hz, 6.6 Hz, 1.7 Hz, 1H), 7.16 (d, J=3.3 Hz, 1H), 6.71 (dd, J=3.3 Hz, 1.6 Hz, 1H), 6.49 (s, 1H), 6.44 (dd, J=8.6 Hz, 1.3 Hz, 1H), 6.29 (ddd, J=7.1 Hz, 6.6 Hz, 1.3 Hz, 1H), 5.04 (s, 2H)
Mass (m/z): 308 ($M^+$)
IR (KBr, $cm^{-1}$): 1670, 1657, 1651, 1608, 1570, 1541, 1423, 1331, 764

| Elemental Analysis: as $C_{15}H_{12}N_6O_2 \cdot 0.1H_2O$ | | | |
|---|---|---|---|
| Observed | C 57.97%, | H 3.94%, | N 27.28% |
| Calculated | C 58.10%, | H 3.97%, | N 27.10% |

Melting point: 239.2 - 239.6°C

Example 38

7-Acetyl-5-amino-2-(2-furyl)[1,2,4]triazolo[1,5-c]pyrimidine (Compound 38)

The reaction was carried out in a manner similar to that in Example 18 except that 2-(3-tributylstannylphenyl)-1,3-dioxolane was replaced by tributyl(1-ethoxyvinyl)tin to obtain 5-(3,4-dimethoxybenzylamino)-7-(1-ethoxyvinyl)-2-(2-furyl)[1,2,4]triazolo[1,5-c]pyrimidine (1.38 g, 3.27 mmol) as a white solid in a yield of 63%.
The obtained 5-(3,4-dimethoxybenzylamino)-7-(1-ethoxyvinyl)-2-(2-furyl)[1,2,4]triazolo[1,5-c]pyrimidine (1.00 g, 2.37 mmol) was dissolved in a mixed solvent of THF (60 mL) and distilled water (10 mL), and conc. sulfuric acid (0.3 mL) was added to the resulting solution, followed by stirring at room temperature for 2 hours. The reaction solution was diluted with chloroform, and a 2 mol/L aqueous solution of potassium hydroxide was added to the solution to make it basic, followed by separation. The organic layer was dried over anhydrous magnesium sulfate, filtered, and then concentrated under reduced pressure to obtain 7-acetyl-5-(3,4-dimethoxybenzylamino)-2-(2-furyl)[1,2, 4]triazolo [1,5-c]pyrimidine as a crude product.
The obtained crude product was dissolved in dichloromethane (18 mL), and distilled water (1 mL) and DDQ (807 mg, 3.56 mmol) were added to the resulting solution, followed by stirring at room temperature for 4 hours. Then, chloroform and a 1 mol/L aqueous solution of potassium hydroxide were added to the reaction solution, followed by separation. The organic layer was dried over anhydrous magnesium sulfate, filtered, and then concentrated under reduced pressure. The residue was triturated with diethyl ether to obtain Compound 38 (403 mg, 1.66 mmol) as a yellowish-white solid in a yield of 70%. Furthermore, Compound 38 was recrystallized from ethanol to obtain yellowish-

white crystals.
$^1H$ NMR (δ ppm, DMSO-$d_6$): 8.26 (brs, 2H), 7.96 (d, J=1.6 Hz, 1H), 7.43 (s, 1H), 7.24 (d, J=3.3 Hz, 1H), 6.74 (dd, J=3.3 Hz, 1.6 Hz, 1H), 2.60 (s, 3H)
Mass (m/z): 243 ($M^+$)
IR (KBr, $cm^{-1}$): 1666, 1639, 1626, 1612, 1581, 1549, 1417, 1326, 1214

| Elemental Analysis: as $C_{11}H_9N_5O_2$ | | | |
|---|---|---|---|
| Observed | C 54.51%, | H 3.84%, | N 29.13% |
| Calculated | C 54.32%, | H 3.72%, | N 28.79% |

Melting point: 221.2 - 221.5°C

Example 39

5-Amino-7-benzoyl-2-(2-furyl)[1,2,4]triazolo[1,5-c]pyrimidine (Compound 39)

First, Compound G (1.00 g, 2.59 mmol) was dissolved in DMF (30 mL), and bistributyltin (2.37 g, 3.89 mmol) and bistriphenylphosphine palladium dichloride (91.0 mg, 0.129 mmol) were added to the resulting solution, followed by stirring at 100°C for 9 hours. The reaction solution was concentrated under reduced pressure, and the residue was purified by Florisil$^R$ chromatography (elution solvent: mixed solvent of hexane and ethyl acetate) to obtain 5-(3,4-dimethoxybenzylamino)-2-(2-furyl)-7-tributylstannyl[1,2,4]triazolo[1,5-c]pyrimidine (1.01 g, 1.58 mmol) as a colorless oily substance in a yield of 61%.

The obtained 5-(3,4-dimethoxybenzylamino)-2-(2-furyl)-7-tributylstannyl[1,2,4]triazolo[1,5-c]pyrimidine (500 mg, 0.781 mmol) was dissolved in THF (25 mL), and bistriphenylphosphine palladium dichloride (55.0 mg, 0.0781 mmol) and benzoyl chloride (0.180 mL, 1.55 mmol) were added to the resulting solution, followed by reflux for 10 hours. Then, aqueous sodium bicarbonate was added to the reaction solution to make it basic, and then extraction with ethyl acetate was performed. The organic layer was dried over anhydrous magnesium sulfate, filtered, and then concentrated under reduced pressure. The residue was purified by silica gel column chromatography (elution solvent: a mixed solvent of hexane and ethyl acetate) to obtain 7-benzoyl-5-(3,4-dimethoxybenzylamino)-2-(2-furyl)[1,2,4]triazolo[1,5-c]pyrimidine (281 mg, 0.617 mmol) as a white solid in a yield of 79%.

The obtained 7-benzoyl-5-(3,4-dimethoxybenzylamino)-2-(2-furyl)[1,2,4]triazolo[1,5-c]pyrimidine (230 mg, 0.505 mmol) was dissolved in dichloromethane (22.5 mL), and distilled water (1.2 mL) and DDQ (344 mg, 1.52 mmol) were added to the resulting solution, followed by stirring at room temperature for 12 hours. Then, the reaction solution was poured into a mixture of chloroform and aqueous sodium bicarbonate, and subjected to separation. The organic layer was dried over anhydrous magnesium sulfate, filtered, and then concentrated under reduced pressure. The residue was triturated with chloroform, and the resulting solid was recrystallized from ethanol (10 mL) to obtain Compound 39 (81.3 mg, 0.266 mmol) as white crystals in a yield of 53%.
$^1H$ NMR (δ ppm, DMSO-$d_6$): 8.27 (brs, 2H), 7.99 (d, J=7. 3 Hz, 2H), 7.98 (d, J=1.6 Hz, 1H), 7.69 (t, J=7.6 Hz, 1H), 7.56 (dd, J=7.6 Hz, 7.3 Hz, 2H), 7.38 (s, 1H), 7.26 (d, J=3.3 Hz, 1H), 6.75 (dd, J=3.3 Hz, 1.6 Hz, 1H)
Mass (m/z): 305 ($M^+$)
IR (KBr, $cm^{-1}$): 1691, 1631, 1626, 1606, 1579, 1547, 1414, 748

| Elemental Analysis: as $C_{16}H_{11}N_5O_2 \cdot 0.1\ H_2O$ | | | |
|---|---|---|---|
| Observed | C 62.52%, | H 3.59%, | N 22.92% |
| Calculated | C 62.58%, | H 3.68%, | N 22.81% |

Melting point: 229.2 - 229.7°C

Example 40

5-Amino-7-(4-fluorobenzoyl)-2-(2-furyl)[1,2,4]triazolo[1,5-c]pyrimidine (Compound 40)

The reaction was carried out in a manner similar to that in Example 39 except that benzoyl chloride was replaced by 4-fluorobenzoyl chloride. The residue was triturated with chloroform, and the resulting solid was recrystallized from a mixed solvent of ethanol (10 mL) and DMF (1 mL) to obtain Compound 40 (138 mg, 0.426 mmol) as white crystals in a yield of 36%.

$^1H$ NMR (δ ppm, DMSO-$d_6$): 8.28 (brs, 2H), 8.11 (dd, J=8.9 Hz, 5.6 Hz, 2H), 7.97 (d, J=1.6 Hz, 1H), 7.39 (s, 1H), 7.38 (dd, J=8.9 Hz, 8.9 Hz, 2H), 7.26 (d, J=3.3 Hz, 1H), 6.75 (dd, J=3.3 Hz, 1.6 Hz, 1H)
Mass (m/z): 323 ($M^+$)
IR (KBr, $cm^{-1}$): 1689, 1653, 1619, 1592, 1508, 1257, 1238, 762

| Elemental Analysis: as $C_{16}H_{10}N_5O_2F\cdot1.0\ H_2O$ | | | |
|---|---|---|---|
| Observed | C 56.35%, | H 3.72%, | N 20.43% |
| Calculated | C 56.31%, | H 3.54%, | N 20.52% |

Melting point: 228.3 - 228.5°C

Example 41

5-Amino-7-formyl-2-(2-furyl)[1,2,4]triazolo[1,5-c]pyrimidine (Compound 41)

First, Compound 22 (800 mg, 3.46 mmol) produced in Example 22 was dissolved in DMF (140 mL), and manganese dioxide (12.00 g, 138.4 mmol) was added to the resulting solution, followed by stirring at room temperature for 20 hours. The reaction solution was filtered through Celite, and the filtrate was concentrated under reduced pressure. The residue was triturated with diethyl ether, and the resulting solid was washed with methanol to obtain Compound 41 (550 mg, 2.40 mmol) as white crystals in a yield of 69%. Furthermore, Compound 41 was recrystallized from ethanol to obtain white crystals.
$^1H$ NMR (δ ppm, DMSO-$d_6$): 9.85 (s, 1H), 8.34 (brs, 2H), 7.98 (d, J=1.7 Hz, 1H), 7.56 (s, 1H), 7.25 (d, J=3.3 Hz, 1H), 6.75 (dd, J=3.3 Hz, 1.7 Hz, 1H)
Mass (m/z): 229 ($M^+$)
IR (KBr, $cm^{-1}$): 1702, 1672, 1626, 1604, 1568, 1508, 1421, 1221, 825, 762, 748

| Elemental Analysis: as $C_{10}H_7N_5O_2$ | | | |
|---|---|---|---|
| Observed | C 52.12%, | H 3.02%, | N 30.61% |
| Calculated | C 52.40%, | H 3.08%, | N 30.56% |

Melting point: 270°C (decomposed)

Example 42

5-Amino-7-(3,4-dimethoxybenzylaminomethyl)-2-(2-furyl)[1,2,4]triazolo[1,5-c]pyrimidine dihydrochloride (Compound 42)

The reaction was carried out in a manner similar to that in Example 3 except that Compound 2 was replaced by Compound 41. Then, an ethyl acetate solution of hydrogen chloride was added to the resulting crude product, and the mixture was concentrated under reduced pressure. The residue was recrystallized from ethanol (10 mL) to obtain Compound 42 (200 mg, 0.44 mmol) as white crystals in a yield of 41%.
$^1H$ NMR (δ ppm, DMSO-$d_6$): 9.75 (m, 2H), 8.15 (brs, 2H), 7.95 (d, J=1.7 Hz, 1H), 7.31 (d, J=1.6 Hz, 1H), 7.23 (d, J=3.3 Hz, 1H), 7.21 (s, 1H), 7.09 (dd, J=8.2 Hz, 1.6 Hz, 1H), 6.97 (d, J=8.2 Hz, 1H), 6.74 (dd, J=3.3 Hz, 1.7 Hz, 1H), 5.50 (brs, 1H), 4.20 (brs, 2H), 4.06 (brs, 2H), 3.78 (s, 3H), 3.77 (s, 3H)
Mass (m/z): 378 ($M^+$-2), 215
IR (KBr, $cm^{-1}$): 1670, 1637, 1616, 1568, 1520, 1460, 1267

| Elemental Analysis: as $C_{19}H_{20}N_6O_3\cdot2.0HCl\cdot1.5\ H_2O$ | | | |
|---|---|---|---|
| Observed | C 47.58%, | H 5.07%, | N 17.43% |
| Calculated | C 47.50%, | H 5.25%, | N 17.50% |

Melting point: 241.2 - 241.5°C

Example 43

5-Amino-7-[(3,5-dimethoxybenzylamino)methyl]-2-(2-furyl)[1,2,4]triazolo[1,5-c]pyrimidine dihydrochloride (Compound 43)

The reaction was carried out in a manner similar to that in Example 6 except that Compound 2 was replaced by Compound 41. Then, an ethyl acetate solution of hydrogen chloride was added to the resulting crude product, and the mixture was concentrated under reduced pressure. The residue was recrystallized from ethanol (6 mL) to obtain Compound 43 (350 mg, 0.77 mmol) as white crystals in a yield of 71%.
$^1$H NMR (δ ppm, DMSO-$d_6$): 9.76 (m, 2H), 8.15 (brs, 2H), 7.95 (d, J=1.6 Hz, 1H), 7.22 (d, J=3.3 Hz, 1H), 7.20 (s, 1H), 6.80 (d, J=2.0 Hz, 2H), 6. 74 (dd, J=3.3 Hz, 1.6 Hz, 1H), 6.53 (t, J=2.0 Hz, 1H), 6.10 (brs, 1H), 4.19 (brs, 2H), 4.08 (brs, 2H), 3.76 (s, 6H)
Mass (m/z): 380 ($M^+$), 215
IR (KBr, $cm^{-1}$): 1676, 1626, 1606, 1564, 1430, 1155

| Elemental Analysis: as $C_{19}H_{20}N_6O_3 \cdot 2.0HCl \cdot 0.3\ H_2O \cdot 0.2\ C_2H_5OH$ | | | |
|---|---|---|---|
| Observed | C 49.68%, | H 5.27%, | N 18.00% |
| Calculated | C 49.79%, | H 5.13%, | N 17.96% |

Melting point: 238.5 - 238.7°C

Example 44

5-Amino-7-[2-(2-pyridyl)ethylaminomethyl]-2-(2-furyl)[1,2,4]triazolo[1,5-c]pyrimidine dihydrochloride (Compound 44)

The reaction was carried out in a manner similar to that in Example 4 except that Compound 2 was replaced by Compound 41. Then, an ethyl acetate solution of hydrogen chloride was added to the resulting crude product, and the mixture was concentrated under reduced pressure. The residue was recrystallized from ethanol (10 mL) and DMF (2 mL) to obtain Compound 44 (92 mg, 0.22 mmol) as white crystals in a yield of 20%.
$^1$H NMR (δ ppm, DMSO-$d_6$): 9.76 (m, 2H), 8.73 (d, J=4.9 Hz, 1H), 8.22 (dd, J=7.9 Hz, 7.2 Hz, 1H), 8.15 (brs, 2H), 7.95 (d, J=1.6 Hz, 1H), 7.73 (d, J=7.9 Hz, 1H), 7.66 (dd, J=7.2 Hz, 4.9 Hz, 1H), 7.22 (d, J=3.3 Hz, 1H), 7.21 (s, 1H), 6.74 (dd, J=3.3 Hz, 1.6 Hz, 1H), 4.21 (s, 2H), 3.40-3.60 (m, 4H)
Mass (m/z): 335 ($M^+$), 215
IR (KBr, $cm^{-1}$): 1651, 1641, 1637, 1616, 1612, 1606, 1564, 1514

| Elemental Analysis: as $C_{17}H_{17}N_7O \cdot 2.0\ HCl \cdot 0.2\ H_2O$ | | | |
|---|---|---|---|
| Observed | C 49.66%, | H 4.73%, | N 23.66% |
| Calculated | C 49.57%, | H 4.75%, | N 23.80% |

Melting point: 249.5 - 249.7°C

Example 45

5-Amino-7-[2-(N-acetylamino)ethylaminomethyl]-2-(2-furyl)[1,2,4]triazolo[1,5-c]pyrimidine (Compound 45)

The reaction was carried out in a manner similar to that in Example 7 except that Compound 2 was replaced by Compound 41. Then, the resulting crude product was recrystallized from ethanol to obtain Compound 45 (40 mg, 0.13 mmol) as white crystals in a yield of 12%.
$^1$H NMR (δ ppm, DMSO-$d_6$): 7.92 (d, J=1.6 Hz, 1H), 7.88 (brs, 2H), 7.81 (t, J=5.9 Hz, 1H), 7.16 (d, J=3.3 Hz, 1H), 6.96 (s, 1H), 6.72 (dd, J=3.3 Hz, 1.6 Hz, 1H), 3.65 (s, 2H), 3.15 (dt, J=6.6 Hz, 5.9 Hz, 2H), 2.60 (t, J=6.6 Hz, 2H), 1.80 (s, 3H)
Mass (m/z): 315 ($M^+$), 214
IR (KBr, $cm^{-1}$): 1680, 1647, 1637, 1614, 1572, 1562, 1556, 1510, 1421, 748

| Elemental Analysis: as $C_{14}H_{17}N_7O_2$ | | | |
|---|---|---|---|
| Observed | C 53.38%, | H 5.53%, | N 31.14% |

(continued)

| Elemental Analysis: as $C_{14}H_{17}N_7O_2$ | | | |
|---|---|---|---|
| Calculated | C 53.33%, | H 5.43%, | N 31.09% |

Melting point: 190.5 - 190.9°C

Example 46

(±)-5-Amino-2-(2-furyl)-7-[(2-methoxy-1-methylethyl)aminomethyl][1,2,4]triazolo[1,5-c]pyrimidine dihydrochloride (Compound 46)

The reaction was carried out in a manner similar to that in Example 9 except that Compound 2 was replaced by Compound 41. Then, an ethyl acetate solution of hydrogen chloride was added to the resulting crude product, and the mixture was concentrated under reduced pressure. The residue was recrystallized from ethanol (5 mL) to obtain Compound 46 (101 mg, 0.27 mmol) as white crystals in a yield of 25%.
$^1$H NMR (δ ppm, DMSO-$d_6$): 9.00-9.10 (m, 2H), 7.88 (d, J=1.6 Hz, 1H), 7.87 (brs, 2H), 7.20 (s, 1H), 7.18 (d, J=3.3 Hz, 1H), 6.69 (dd, J=3.3 Hz, 1.6 Hz, 1H), 5.10 (brs, 1H), 4.16 (brs, 2H), 3.50-3.60 (m, 2H), 3.49 (m, 1H), 3.34 (s, 3H), 1.30 (d, J=6.6 Hz, 3H)
Mass (m/z): 257 ($M^+$), 214
IR (KBr, $cm^{-1}$): 1680, 1668, 1639, 1618, 1564, 1456, 1117, 977

| Elemental Analysis: as $C_{14}H_{18}N_6O_2 \cdot 2.0HCl \cdot 0.2\ H_2O$ | | | |
|---|---|---|---|
| Observed | C 44.42%, | H 5.57%, | N 22.03% |
| Calculated | C 44.38%, | H 5.43%, | N 22.18% |

Melting point: 191.4 - 192.2°C

Example 47

5-Amino-2-(2-furyl)-7-[3-(2-oxopyrrolidin-1-yl)propylaminomethyl][1,2,4]triazolo[1,5-c]pyrimidine dihydrochloride (Compound 47)

The reaction was carried out in a manner similar to that in Example 8 except that Compound 2 was replaced by Compound 41. Then, an ethyl acetate solution of hydrogen chloride was added to the resulting crude product, and the mixture was concentrated under reduced pressure. The residue was recrystallized from ethanol (6 mL) to obtain Compound 47 (181 mg, 0.42 mmol) as white crystals in a yield of 39%.
$^1$H NMR (δ ppm, DMSO-$d_6$): 9.50 (m, 2H), 8.13 (brs, 2H), 7.96 (d, J=1.7 Hz, 1H), 7.23 (s, 1H), 7.22 (d, J=3.3 Hz, 1H), 6.73 (dd, J=3.3 Hz, 1.7 Hz, 1H), 4.12 (brs, 2H), 3.35 (t, J=6.9 Hz, 2H), 3.26 (t, J=6.6 Hz, 2H), 2.95 (m, 2H), 2.25 (t, J=7.9 Hz, 2H), 1.80-2.00 (m, 4H)
Mass (m/z): 355 ($M^+$), 215
IR (KBr, $cm^{-1}$): 1668, 1657, 1641, 1612, 1510, 1456, 1423, 1335, 1288, 1224
Melting point: 229.1 - 229.8°C

Example 48

5-Amino-7-(4-ethylpiperazin-1-ylmethyl)-2-(2-furyl)[1,2,4]triazolo[1,5-c]pyrimidine trihydrochloride (Compound 48)

The reaction was carried out in a manner similar to that in Example 5 except that Compound 2 was replaced by Compound 41. Then, an ethyl acetate solution of hydrogen chloride was added to the resulting crude product, and the mixture was concentrated under reduced pressure. The residue was recrystallized from a mixed solvent of ethanol (10 mL) and DMF (5 mL) to obtain Compound 48 (75 mg, 0.17 mmol) as white crystals in a yield of 16%.
$^1$H NMR (δ ppm, DMSO-$d_6$): 11.87 (m, 1H), 8.17 (brs, 2H), 7.95 (d, J=1.6 Hz, 1H), 7.32 (s, 1H), 7.23 (d, J=3.3 Hz, 1H), 6.74 (dd, J=3.3 Hz, 1.6 Hz, 1H), 4.32 (s, 2H), 3.35-3.80 (m, 8H), 3.20 (q, J=6.2 Hz, 2H), 1.27 (t, J=6.2 Hz, 3H)
Mass (m/z): 327 ($M^+$), 215
IR (KBr, $cm^{-1}$): 2478, 1672, 1650, 1635, 1614, 1564, 1454, 973

| Elemental Analysis: as $C_{16}H_{21}N_7O \cdot 3.0\ HCl \cdot 0.1\ C_2H_5OH$ | | | |
|---|---|---|---|
| Observed | C 44.14%, | H 5.73%, | N 22.32% |
| Calculated | C 44.08%, | H 5.62%, | N 22.21% |

Melting point: 262.0 - 262.4°C

Example 49

5-Amino-2-(2-furyl)-7-[4-(2-methoxyethyl)piperazin-1-yl-methyl][1,2,4]triazolo[1,5-c]pyrimidine dihydrochloride (Compound 49)

The reaction was carried out in a manner similar to that in Example 48 except that 4-ethylpiperazine was replaced by 4-methoxyethylpiperazine. Then, an ethyl acetate solution of hydrogen chloride was added to the resulting crude product, and the mixture was concentrated under reduced pressure. The residue was recrystallized from a mixed solvent of ethanol (16 mL) and DMF (1 mL) to obtain Compound 49 (287 mg, 0.667 mmol) as white crystals in a yield of 51%.
$^1$H NMR (δ ppm, DMSO-$d_6$): 11.85 (m, 1H), 8.11 (brs, 2H), 7.96 (d, J=1.7 Hz, 1H), 7.32 (s, 1H), 7.24 (d, J=3.3 Hz, 1H), 6.74 (dd, J=3.3 Hz, 1.7 Hz, 1H), 4.25 (brs, 2H), 3.74 (t, J=4.0 Hz, 2H), 3.50-3.70 (m, 8H), 3.30-3.40 (m, 2H), 3.29 (s, 3H)
Mass (m/z): 358 ($M^+$+1)
IR (KBr, $cm^{-1}$): 2536, 1650, 1645, 1606, 1512, 1454, 1435, 1423, 1417

| Elemental Analysis: as $C_{17}H_{23}N_7O_2 \cdot 2.0HCl \cdot 0.2\ H_2O$ | | | |
|---|---|---|---|
| Observed | C 47.09%, | H 6.13%, | N 22.56% |
| Calculated | C 47.05%, | H 5.90%, | N 22.59% |

Melting point: 245.3 - 246.0°C

Example 50

5-Amino-7-[4-(cyclopropylmethyl)piperazin-1-ylmethyl]-2-(2-furyl)[1,2,4]triazolo[1,5-c]pyrimidine dihydrochloride (Compound 50)

The reaction was carried out in a manner similar to that in Example 48 except that 4-ethylpiperazine was replaced by 4-(cyclopropylmethyl)piperazine. Then, an ethyl acetate solution of hydrogen chloride was added to the resulting crude product, and the mixture was concentrated under reduced pressure. The residue was recrystallized from a mixed solvent of ethanol (14 mL) and DMF (3 mL) to obtain Compound 50 (223 mg, 0.523 mmol) as white crystals in a yield of 40%.
$^1$H NMR (δ ppm, DMSO-$d_6$): 11.61 (m, 1H), 8.15 (brs, 2H), 7.95 (d, J=1.6 Hz, 1H), 7.27 (s, 1H), 7.22 (d, J=3.3 Hz, 1H), 6.74 (dd, J=3.3 Hz, 1.6 Hz, 1H), 4.20 (brs, 2H), 3.30-3.80 (m, 8H), 3.07 (d, J=7.0 Hz, 2H), 1.10 (m, 1H), 0.65 (m, 2H), 0.43 (m, 2H)
Mass (m/z): 353($M^+$), 215
IR (KBr, $cm^{-1}$): 2550, 1643, 1601, 1506, 1454, 1417, 1323, 951

| Elemental Analysis: as $C_{18}H_{23}N_7O \cdot 2.0\ HCl \cdot 0.3\ H_2O$ | | | |
|---|---|---|---|
| Observed | C 50.05%, | H 6.38%, | N 22.71% |
| Calculated | C 50.07%, | H 5.98%, | N 22.71% |

Melting point: 270°C (decomposed)

Example 51

5-Amino-2-(2-furyl)-7-(2-thienyl)[1,2,4]triazolo[1,5-c]pyrimidine (Compound 51)

The reaction was carried out in a manner similar to that in Example 18 except that 2-(3-tributylstannylphenyl)-1,3-dioxolane was replaced by 2-(tributylstannyl)thiophene to obtain 5-(3,4-dimethoxybenzylamino)-2-(2-furyl)-7-(2-thienyl)[1,2,4]triazolo[1,5-c]pyrimidine (1.01 g, 2.33 mmol) as a white amorphous substance in a yield of 90%.

The obtained 5-(3,4-dimethoxybenzylamino)-7-(2-thienyl)-2-(2-furyl)[1,2,4]triazolo[1,5-c]pyrimidine was dissolved in dichloromethane (22.5 mL), and distilled water (1.25 mL) and DDQ (1.05 g, 4.63 mmol) were added to the resulting solution, followed by stirring at room temperature for 3 hours. Then, chloroform and a 1 mol/L aqueous solution of sodium hydroxide were added to the reaction solution, and the resulting mixture was subjected to separation. The organic layer was dried over anhydrous magnesium sulfate, filtered, and then concentrated under reduced pressure. The crude product was recrystallized from ethanol to obtain Compound 51 (366 mg, 1.29 mmol) as white crystals in a yield of 55%.

$^1$H NMR (δ ppm, DMSO-$d_6$): 8.05 (brs, 2H), 7.94 (d, J=1.6 Hz, 1H), 7.91 (d, J=3.7 Hz, 1H), 7.69 (d, J=5.0 Hz, 1H), 7.51 (s, 1H), 7.20 (d, J=3.3 Hz, 1H), 7.19 (dd, J=5.0 Hz, 3.7 Hz, 1H), 6.73 (dd, J=3.3 Hz, 1.6 Hz, 1H)

Mass (m/z): 283 ($M^+$)

IR (KBr, $cm^{-1}$): 1653, 1601, 1556, 1435, 1417

| Elemental Analysis: as $C_{13}H_9N_5OS$ | | | |
|---|---|---|---|
| Observed | C 55.13%, | H 3.42%, | N 24.39% |
| Calculated | C 55.11%, | H 3.20%, | N 24.72% |

Melting point: 232.2 - 232.3°C

Example 52

5-Amino-2-(2-furyl)-7-(2-pyridyl)[1,2,4]triazolo[1,5-c]pyrimidine (Compound 52)

The reaction was carried out in a manner similar to that in Example 18 except that 2-(3-tributylstannylphenyl)-1,3-dioxolane was replaced by tributylstannyl-2-pyridine. Then, the resulting crude product was recrystallized from ethanol (13 mL) to obtain Compound 52 (97.1 mg, 0.349 mmol) as white crystals in a yield of 25%.

$^1$H NMR (δ ppm, DMSO-$d_6$): 8.72 (d, J=4.3 Hz, 1H), 8.32 (d, J=7.6 Hz, 1H), 8.12 (brs, 2H), 7.99 (dd, J=7.6 Hz, 6.0 Hz, 1H), 7.95 (d, J=1.6 Hz, 1H), 7.84 (s, 1H), 7.49 (dd, J=6.0 Hz, 4.3 Hz, 1H), 7.24 (d, J=3.3 Hz, 1H), 6.74 (dd, J=3.3 Hz, 1.6 Hz, 1H)

Mass (m/z): 278 ($M^+$)

IR (KBr, $cm^{-1}$): 1660, 1655, 1597, 1559, 1510, 1410, 1225, 766, 741

| Elemental Analysis: as $C_{14}H_{10}N_6O \cdot 0.4\ H_2O \cdot 0.1\ C_2H_5OH$ | | | |
|---|---|---|---|
| Observed | C 58.75%, | H 3.67%, | N 28.90% |
| Calculated | C 58.80%, | H 3.96%, | N 28.97% |

Melting point: 274.5 - 275.0°C

Example 53

5-Amino-2-(2-furyl)-7-(3-pyridyl)[1,2,4]triazolo[1,5-c]pyrimidine (Compound 53)

The reaction was carried out in a manner similar to that in Example 18 except that 2-(3-tributylstannylphenyl)-1,3-dioxolane was replaced by butylstannyl-3-pyridine. Then, the resulting crude product was recrystallized from methanol (5 mL) to obtain Compound 53 (180 mg, 0.647 mmol) as white crystals in a yield of 50%.

$^1$H NMR (δ ppm, DMSO-$d_6$): 9.32 (d, J=1.6 Hz, 1H), 8.65 (d, J=5.0 Hz, 1H), 8.47 (dd, J=8.0 Hz, 1.6 Hz, 1H), 8.12 (brs, 2H), 7.95 (d, J=1.7 Hz, 1H), 7.68 (s, 1H), 7.55 (dd, J=8.0 Hz, 5.0 Hz, 1H), 7.23 (d, J=3.3 Hz, 1H), 6.74 (dd, J=3.3 Hz, 1.7 Hz, 1H)

Mass (m/z): 278 ($M^+$)

IR (KBr, $cm^{-1}$): 1668, 1655, 1601, 1560, 1508, 1331, 789

| Elemental Analysis: as $C_{14}H_{10}N_6O \cdot 0.3\ H_2O$ | | | |
|---|---|---|---|
| Observed | C 59.42%, | H 3.63%, | N 29.32% |
| Calculated | C 59.28%, | H 3.77%, | N 29.63% |

Melting point: 253.2 - 253.6°C

Example 54

5-Amino-2-(2-furyl)-7-(4-pyridyl)[1,2,4]triazolo[1,5-c]pyrimidine (Compound 54)

The reaction was carried out in a manner similar to that in Example 2 except that 2-formylphenylboric acid was replaced by 4-pyridylboric acid. Then, the resulting crude product was recrystallized from a mixed solvent of ethanol (16 mL) and DMF (8 mL) to obtain Compound 54 (455 mg, 1.54 mmol) as white crystals in a yield of 59%.
$^1$H NMR (δ ppm, DMSO-$d_6$): 8.72 (d, J=6.0 Hz, 2H), 8.18 (brs, 2H), 8.08 (d, J=6.0 Hz, 2H), 7.96 (d, J=1.6 Hz, 1H), 7.77 (s, 1H), 7.24 (d, J=3.3 Hz, 1H), 6.74 (dd, J=3.3 Hz, 1.7 Hz, 1H)
Mass (m/z): 278 ($M^+$)
IR (KBr, $cm^{-1}$): 1657, 1602, 1567, 1552, 1421, 1012, 744

| Elemental Analysis: as $C_{14}H_{10}N_6O \cdot 1.0\ H_2O$ | | | |
|---|---|---|---|
| Observed | C 56.92%, | H 4.17%, | N 28.31% |
| Calculated | C 56.75%, | H 4.08%, | N 28.36% |

Melting point: 279.2 - 279.9°C

Example 55

5-Amino-2-(2-furyl)-7-(2-furyl)[1,2,4]triazolo[1,5-c]pyrimidine (Compound 55)

The reaction was carried out in a manner similar to that in Example 18 except that 2-(3-tributylstannylphenyl)-1,3-dioxolane was replaced by 2-(tributylstannyl)furan. Then, the resulting crude product was recrystallized from ethanol (4 mL) to obtain Compound 55 (114 mg, 0.425 mmol) as white crystals in a yield of 27%.
$^1$H NMR (δ ppm, DMSO-$d_6$): 8.05 (brs, 2H), 7.93 (d, J=1.6 Hz, 1H), 7.89 (d, J=2.0 Hz, 1H), 7.21 (d, J=3.3 Hz, 1H), 7.20 (s, 1H), 7.08 (d, J=3.3 Hz, 1H), 6.72 (dd, J=3.3 Hz, 1.6 Hz, 1H), 6.68 (dd, J=3.3 Hz, 2.0 Hz, 1H)
Mass (m/z): 267 ($M^+$)
IR (KBr, $cm^{-1}$): 1658, 1614, 1602, 1591, 1552, 1545, 1504, 1419, 1328, 1217, 1178, 748

| Elemental Analysis: as $C_{13}H_9N_5O_2 \cdot 0.1H_2O$ | | | |
|---|---|---|---|
| Observed | C 57.97%, | H 3.32%, | N 26.26% |
| Calculated | C 58.04%, | H 3.45%, | N 26.03% |

Melting point: 250.2 - 250.4°C

Example 56

5-Amino-2-(2-furyl)-7-(5-pyrimidinyl)[1,2,4]triazolo[1,5-c]pyrimidine (Compound 56)

The reaction was carried out in a manner similar to that in Example 18 except that 2-(3-tributylstannylphenyl)-1,3-dioxolane was replaced by 5-(tributylstannyl)pyrimidine. Then, the resulting crude product was recrystallized from ethanol (10 mL) to obtain Compound 56 (117 mg, 0.419 mmol) as white crystals in a yield of 13%.
$^1$H NMR (δ ppm, DMSO-$d_6$): 9.48 (s, 2H), 9.27 (s, 1H), 8.23 (brs, 2H), 7.96 (d, J=1.7 Hz, 1H), 7.80 (s, 1H), 7.24 (d, J=3.3 Hz, 1H), 6.74 (dd, J=3.3 Hz, 1.7 Hz, 1H)
Mass (m/z): 279 ($M^+$)
IR (KBr, $cm^{-1}$): 1677, 1650, 1622, 1570, 744

| Elemental Analysis: as $C_{13}H_9N_7O \cdot 0.5\ H_2O$ | | | |
|---|---|---|---|
| Observed | C 54.23%, | H 3.37%, | N 33.80% |
| Calculated | C 54.17%, | H 3.50%, | N 34.01% |

Melting point: 271°C (decomposed)

Example 57

5-Amino-7-(3,5-dimethylisoxazol-4-yl)-2-(2-furyl)[1,2,4]triazolo[1,5-c]pyrimidine (Compound 57)

The reaction was carried out in a manner similar to that in Example 2 except that 2-formylphenylboric acid was replaced by 3,5-dimethylisoxazol-4-ylboric acid. Then, the resulting crude product was recrystallized from ethanol (15 mL) to obtain Compound 57 (415 mg, 1.40 mmol) as white crystals in a yield of 33%.
$^1$H NMR (δ ppm, DMSO-$d_6$): 8.06 (brs, 2H), 7.95 (d, J=1.7 Hz, 1H), 7.20 (d, J=3.3 Hz, 1H), 7.08 (s, 1H), 6.74 (dd, J=3.3 Hz, 1.7 Hz, 1H), 2.65 (s, 3H), 2.45 (s, 3H)
Mass (m/z): 296 ($M^+$), 281
IR (KBr, $cm^{-1}$): 1652, 1610, 1600, 1545, 1508, 1326, 1215

| Elemental Analysis: as $C_{14}H_{12}N_6O_2$ | | | |
|---|---|---|---|
| Observed | C 56.81%, | H 4.14%, | N 28.63% |
| Calculated | C 56.75%, | H 4.08%, | N 28.36% |

Melting point: 227.8 - 228.5°C

Example 58

5-Amino-7-(2-aminothiazol-4-yl)-2-(2-furyl)-[1,2,4]triazolo[1,5-c]pyrimidine (Compound 58)

First, 5-(3,4-dimethoxybenzylamino)-7-(1-ethoxyvinyl)-2-(2-furyl)[1,2,4]triazolo[1,5-c]pyrimidine (4.53 g, 10.8 mmol) produced in Example 38 was dissolved in a mixed solvent of THF (135 mL) and distilled water (135 mL), and N-bromosuccinimide (1.91 g, 10.8 mmol) was added to the resulting solution, followed by stirring at room temperature for 1 hour. The reaction solution was diluted with ethyl acetate, and aqueous saturated sodium bicarbonate was added to the solution to make it basic. Then, the solution was subjected to separation. The organic layer was dried over anhydrous magnesium sulfate, filtered, and then concentrated under reduced pressure to obtain 7-(2-bromoacetyl)-5-(3,4-dimethoxybenzylamino)-2-(2-furyl)[1,2,4]triazolo[1,5-c]pyrimidine (5.09 g, 10.7 mmol) as a crude product in a yield of 100%.

The resulting crude product (2.00 g, 4.20 mmol) was dissolved in dichloromethane (180 mL) and distilled water (10 mL), and DDQ (3.82 g, 16.8 mmol) was added to the resulting solution, followed by stirring at room temperature for 12 hours. Then, chloroform and aqueous saturated sodium bicarbonate were poured into the reaction solution, and separation was performed. The organic layer was dried over anhydrous magnesium sulfate, filtered, and then concentrated under reduced pressure. The residue was triturated with chloroform to obtain 5-amino-7-(2-bromoacetyl)-2-(2-furyl)[1,2,4]triazolo[1,5-c]pyrimidine (593 mg, 1.84 mmol) as a crude product in a yield of 44%.

The resulting crude product (500 mg, 1.55 mmol) was dissolved in ethanol (50 mL), and thiourea (236 mg, 3.10 mmol) was added to the resulting solution, followed by reflux for 3 hours. Then, the reaction solution was concentrated under reduced pressure, and the residue was washed with aqueous saturated sodium bicarbonate. The resulting crude product was recrystallized from a mixed solvent of ethanol (10 mL) and DMF (10 mL) to obtain Compound 58 (404 mg, 1.35 mmol) as yellow crystals in a yield of 87%.
$^1$H NMR (δ ppm, DMSO-$d_6$): 7.97 (s, 1H), 7.96 (brs, 2H), 7.94 (d, J=1.7 Hz, 1H), 7.27 (s, 1H), 7.21 (d, J=3.3 Hz, 1H), 7.20 (brs, 2H), 6.72 (dd, J=3.3 Hz, 1.7 Hz, 1H)
Mass (m/z): 299 ($M^+$)
IR (KBr, $cm^{-1}$): 3376, 1652, 1647, 1605, 1570, 1531
Melting point: 299.2 - 300.0°C

Example 59

5-Amino-2-(2-furyl)-7-[2-(2-morpholinoethylamino)thiazol-4-yl][1,2,4]triazolo[1,5-c]pyrimidine (Compound 59)

First, 5-amino-7-(2-bromoacetyl)-2-(2-furyl)[1,2,4]triazolo[1,5-c]pyrimidine (300 mg, 0.932 mmol) produced in Example 58 was dissolved in ethanol (30 mL), and 1-(2-morpholinoethyl)-2-thiourea (353 mg, 1.86 mmol) was added to the resulting solution, followed by reflux for 3 hours. Then, the reaction solution was concentrated under reduced pressure, and the residue was washed with aqueous saturated sodium bicarbonate. The resulting crude product was recrystallized from a mixed solvent of ethanol (12 mL) and DMF (6 mL) to obtain Compound 59 (275 mg, 0.667 mmol) as yellow crystals in a yield of 72%.

$^1H$ NMR (δ ppm, DMSO-$d_6$): 7.87 (d, J=1.4 Hz, 1H), 7.58 (brs, 2H), 7.35 (s, 1H), 7.23 (s, 1H), 7.16 (d, J=3.3 Hz, 1H), 6.68 (dd, J=3.3 Hz, 1.4 Hz, 1H), 3.55-3.60 (m, 4H), 3.44 (t, J=6.2 Hz, 2H), 2.58 (t, J=6.2 Hz, 2H), 2.40-2.50 (m, 4H)
Mass (m/z): 413 ($M^+$+1)
IR (KBr, $cm^{-1}$): 1662, 1654, 1646, 1600, 1569, 1560
Melting point: 274.7 - 275.0°C (decomposed)

Example 60

5-Amino-2-(2-furyl)-7-(1H-pyrazol-3-yl)[1,2,4]triazolo[1,5-c]pyrimidine (Compound 60)

First, 7-acetyl-5-(3,4-dimethoxybenzylamino)-2-(2-furyl)[1,2,4]triazolo[1,5-c]pyrimidine (1.00 g, 2.54 mmol) produced in Example 38 was dissolved in N,N-dimethylformamide dimethylacetal (20 mL), and the resulting solution was stirred at 140°C for 3 hours. After the reaction solution was concentrated under reduced pressure, the residue was dissolved in ethanol (30 mL), and hydrazine monohydrate (0.250 mL, 5.15 mmol) was added to the resulting solution, followed by reflux for 1 hour. Then, the reaction solution was stirred at room temperature overnight, and the precipitated solid was filtered off and then washed with ethanol to obtain 5-(3,4-dimethoxybenzylamino)-2-(2-furyl)-7-(1H-pyrazol-3-yl)[1,2,4]triazolo[1,5-c]pyrimidine as a crude product.

The resulting crude product was dissolved in chloroform (135 mL), and distilled water (7.5 mL) and DDQ (1.76 g, 8.64 mmol) were added to the resulting solution, followed by stirring at room temperature for 9 hours. Then, a 2 mol/L aqueous solution of sodium hydroxide was added to the reaction solution, and the precipitated solid was filtered off. The obtained solid was recrystallized from a mixed solvent of ethanol (10 mL) and DMF (5 mL) to obtain Compound 60 (175 mg, 0.655 mmol) as white crystals in a yield of 26%.
$^1H$ NMR (δ ppm, DMSO-$d_6$): 12.88 (brs, 1H), 7.87 (d, J=1.7 Hz, 1H), 7.70-7.72 (m, 3H), 7.40 (s, 1H), 7.17 (d, J=3.0 Hz, 1H), 6.80 (d, J=1.6 Hz, 1H), 6.69 (dd, J=3.0 Hz, 1.7 Hz, 1H)
Mass (m/z): 267 ($M^+$)
IR (KBr, $cm^{-1}$): 1645, 1610, 1525, 1510, 773

| Elemental Analysis: as $C_{12}H_9N_7O \cdot 0.1\ H_2O$ | | | |
|---|---|---|---|
| Observed | C 53.41%, | H 3.52%, | N 36.74% |
| Calculated | C 53.57%, | H 3.47%, | N 36.44% |

Melting point: > 300°C

Example 61

5-Amino-2-(2-furyl)-7-(4-hydroxymethylphenyl)[1,2,4]triazolo[1,5-c]pyrimidine (Compound 61)

First, 5-(3,4-dimethoxybenzylamino)-7-(4-formylphenyl)-2-(2-furyl)[1,2,4]triazolo[1,5-c]pyrimidine (1.00 g, 2.20 mmol) produced in Example 10 was dissolved in methanol (125 mL), and sodium borohydride (250 mg, 6.60 mmol) was added to the resulting solution, followed by stirring at 0°C for 1 hour. Then, the reaction solution was concentrated, and the residue was subjected to extraction with chloroform. The organic layer was dried over anhydrous magnesium sulfate, filtered, and then concentrated under reduced pressure. The residue was purified by silica gel column chromatography (elution solvent: mixed solvent of chloroform and methanol) to obtain 5-(3,4-dimethoxybenzylamino)-2-(2-furyl)-7-(4-hydroxymethylphenyl)[1,2,4]triazolo[1,5-c]pyrimidine (947 mg, 2.08 mmol) in a yield of 95%.

The obtained 5-(3,4-dimethoxybenzylamino)-2-(2-furyl)-7-(4-hydroxymethylphenyl)[1,2,4]triazolo[1,5-c]pyrimidine was dissolved in dichloromethane (90 mL) and distilled water (5 mL), and DDQ (1.45 g, 6.39 mmol) was added to the resulting solution, followed by stirring at room temperature for 2 hours. Then, the reaction solution was poured into ice-cold aqueous saturated sodium bicarbonate, and the precipitated solid was recrystallized from a mixed solvent of ethanol (15 mL) and DMF (6 mL) to obtain Compound 61 (200 mg, 0.651 mmol) as white crystals in a yield of 31%.
$^1H$ NMR (δ ppm, DMSO-$d_6$): 8.11 (d, J=8.3 Hz, 2H), 8.02 (brs, 2H), 7.95 (d, J=1.6 Hz, 1H), 7.53 (s, 1H), 7.43 (d, J=8.3 Hz, 2H), 7.21 (d, J=3.3 Hz, 1H), 6.73 (dd, J=3.3 Hz, 1.6 Hz, 1H), 5.29 (t, J=5.6 Hz, 1H), 4.57 (d, J=5.6 Hz, 2H)
Mass (m/z): 308 ($M^+$+1)
IR (KBr, $cm^{-1}$): 1653, 1649, 1610, 1578, 1560, 1514, 1419, 1406, 1335, 993

| Elemental Analysis: as $C_{16}H_{13}N_5O_2 \cdot 0.2\ H_2O$ | | | |
|---|---|---|---|
| Observed | C 61.92%, | H 4.34%, | N 22.45% |

(continued)

| Elemental Analysis: as $C_{16}H_{13}N_5O_2 \cdot 0.2\ H_2O$ | | | |
|---|---|---|---|
| Calculated | C 61.81%, | H 4.34%, | N 22.53% |

Melting point: 239.6 - 239.9°C

Example 62

5-Amino-2-(2-furyl)-7-(3-hydroxymethylpyridin-6-yl)[1,2,4]triazolo[1,5-c]pyrimidine (Compound 62)

Compound G (2.00 g, 5.19 mmol) was dissolved in DMF (50 mL), and 2-(3-pyridyl-4-tributylstannyl)-1,3-dioxolane (3.43 g, 7.79 mmol) and bistriphenylphosphine palladium dichloride (365 mg, 0.519 mmol) were added to the resulting solution, followed by stirring at 110°C for 6 hours. Then, an aqueous saturated solution of ammonium fluoride was added to the reaction solution, and extraction with chloroform was performed. The organic layer was dried over anhydrous magnesium sulfate, filtered, and then concentrated under reduced pressure. The residue was purified by silica gel column chromatography (elution solvent: mixed solvent of chloroform and methanol) to obtain 7-[3-(1,3-dioxolan-2-yl)pyridin-6-yl]-5-(3,4-dimethoxybenzylamino)-2-(2-furyl)[1,2,4]triazolo[1,5-c]pyrimidine (2.12 g, 4.24 mmol) as a brown oily substance in a yield of 82%.

The obtained 5-(3,4-dimethoxybenzylamino)-7-[3-(1,3-dioxolan-2-yl)pyridin-6-yl]-2-(2-furyl)[1,2,4]triazolo[1,5-c]pyrimidine was dissolved in THF (30 mL), and 2 mol/L hydrochloric acid (30 mL) was added to the resulting solution, followed by stirring at room temperature for 3 hours. Then, a 4 mol/L aqueous sodium hydroxide solution was added to the reaction solution, and extraction with chloroform was performed. The organic layer was dried over anhydrous magnesium sulfate, filtered, and then concentrated under reduced pressure. The residue was triturated with acetone to obtain 5-(3,4-dimethoxybenzylamino)-7-(3-formylpyridin-6-yl)-2-(2-furyl)[1,2,4]triazolo[1,5-c]pyrimidine (780 mg, 1.71 mmol) as a brown solid in a yield of 40%.

The obtained 5-(3,4-dimethoxybenzylamino)-7-(3-formyl-pyridin-6-yl)-2-(2-furyl)[1,2,4]triazolo[1,5-c]pyrimidine (620 mg, 1.36 mmol) was dissolved in a mixed solvent of methanol (62 mL) and chloroform (40 mL), and sodium borohydride (257 mg, 6.80 mmol) was added to the resulting solution, followed by stirring at room temperature for 1 hour. The reaction solution was concentrated, and the residue was subjected to extraction with chloroform. The organic layer was dried over anhydrous magnesium sulfate, filtered, and then concentrated under reduced pressure to obtain 5-(3,4-dimethoxybenzylamino)-2-(2-furyl)-7-(3-hydroxymethylpyridin-6-yl)[1,2,4]triazolo[1,5-c]pyrimidine (593 mg, 1.30 mmol) as a white solid in a yield of 96%.

The obtained 5-(3,4-dimethoxybenzylamino)-2-(2-furyl)-7-(3-hydroxymethylpyridin-6-yl)[1,2,4]triazolo[1,5-c]pyrimidine (401 mg, 0.879 mmol) was dissolved in trifluoroacetic acid (8 mL), and anisole (0.961 mL, 0.879 mmol) and trifluoromethanesulfonic acid (0.777 mL, 0.879 mmol) were added to the resulting solution, followed by stirring at room temperature for 1 hour. Then, ammonia water was added to the reaction solution, and the precipitated white solid was filtered off. The resulting solid was recrystallized from a mixed solvent of ethanol (12 mL) and DMF (9 mL) to obtain Compound 62 (152 mg, 0.493 mmol) as white crystals in a yield of 56%.

$^1$H NMR (δ ppm, DMSO-$d_6$): 8.66 (s, 1H), 8.30 (d, J=7.9 Hz, 1H), 8.10 (brs, 2H), 7.95 (d, J=1.6 Hz, 1H), 7.92 (d, J=7.9 Hz, 1H), 7.83 (s, 1H), 7.25 (d, J=3.3 Hz, 1H), 6.74 (dd, J=3.3 Hz, 1.6 Hz, 1H), 5.43 (t, J=5.2 Hz, 1H), 4.62 (d, J=5.2 Hz, 2H)

Mass (m/z): 309 ($M^+$+1)

IR (KBr, $cm^{-1}$): 1641, 1622, 1606, 1554, 1419, 1335, 1215, 1009

| Elemental Analysis: as $C_{15}H_{12}N_6O_2 \cdot 0.2\ H_2O$ | | | |
|---|---|---|---|
| Observed | C 57.74%, | H 4.19%, | N 26.76% |
| Calculated | C 57.76%, | H 4.01%, | N 26.94% |

Melting point: 292.8 - 293.2°C

Example 63

5-Amino-2-(2-furyl)-7-[4-(1-hydroxy-1-methylethyl)phenyl][1,2,4]triazolo[1,5-c]pyrimidine (Compound 63)

First, 5-(3,4-dimethoxybenzylamino)-7-(4-formylphenyl)-2-(2-furyl)[1,2,4]triazolo[1,5-c]pyrimidine (1.00 g,

2.20 mmol) produced in Example 10 was dissolved in a mixed solvent of ethanol (50 mL) and distilled water (10 mL), and silver nitrate (940 mg, 5.50 mmol) and a 0.5 mol/L aqueous solution of sodium hydroxide (22.0 mL, 11.0 mmol) were added to the resulting solution, followed by stirring at room temperature for 7 hours under light shielding. Then, ice and 2 mol/L hydrochloric acid were added to the reaction solution to make it acidic, and the solution was filtered with Celite. The filtrate was subjected to extraction with chloroform, and the organic layer was dried over anhydrous magnesium sulfate, filtered, and then concentrated under reduced pressure. The residue was triturated with methanol to obtain 5-(3,4-dimethoxybenzylamino)-7-(4-carboxyphenyl)-2-(2-furyl) [1,2,4]triazolo[1,5-c]pyrimidine (856 mg, 1.78 mmol) in a yield of 81%.

The obtained 7-(4-carboxyphenyl)-5-(3,4-dimethoxy-benzylamino)-2-(2-furyl)[1,2,4]triazolo[1,5-c]pyrimidine was dissolved in dichloromethane (50 mL), and oxalyl chloride (5.00 mL, 57.3 mmol) was added to the resulting solution, followed by reflux for 2 hours. Then, the reaction solution was concentrated under reduced pressure, and methanol (100 mL) was added to the residue, followed by stirring for 72 hours. Then, the reaction solution was concentrated under reduced pressure, and the residue was dissolved in ethyl acetate. The resulting solution was washed with aqueous sodium bicarbonate. The organic layer was dried over anhydrous magnesium sulfate, filtered, and then concentrated under reduced pressure. The residue was purified by silica gel column chromatography (elution solvent: mixed solvent of hexane and ethyl acetate) to obtain 5-(3,4-dimethoxybenzylamino)-2-(2-furyl)-7-(4-methoxycarbonylphenyl) [1,2,4]triazolo[1,5-c]pyrimidine (830 mg, 1.71 mmol) in a yield of 96%.

The obtained 7-(4-methoxycarbonylphenyl)-5-(3,4-dimethoxybenzylamino)-2-(2-furyl)[1,2,4]triazolo[1,5-c] pyrimidine (580 mg, 1.20 mmol) was dissolved in THF (30 mL), and the resulting solution was cooled to 0°C in an argon atmosphere. Then, a THF solution (14.3 mL, 15.0 mmol) of 0.95 mol/L methylmagnesium bromide was added dropwise to the reaction mixture, followed by stirring at room temperature for 3 hours. Then, distilled water was added to the reaction solution, and extraction with ethyl acetate was performed. The organic layer was dried over anhydrous magnesium sulfate, filtered, and then concentrated under reduced pressure to obtain 5-(3,4-dimethoxybenzylamino)-2-(2-furyl)-7-[4-(1-hydroxy-1-methylethyl)phenyl][1,2,4]triazolo[1,5-c]pyrimidine as a crude product.

The resulting crude produce was dissolved in dichloromethane (54 mL), and distilled water (3 mL) and DDQ (958 mg, 4.22 mmol) were added to the resulting solution, followed by stirring at room temperature for 3 hours. Then, the reaction solution was poured into a mixture of chloroform and aqueous saturated sodium bicarbonate, and separation was performed. The organic layer was dried over anhydrous magnesium sulfate, filtered, and then concentrated under reduced pressure. The residue was purified by silica gel column chromatography (elution solvent: mixed solvent of chloroform and methanol), and then recrystallized from ethanol (4 mL) to obtain Compound 63 (132 mg, 0.394 mmol) as white crystals in a yield of 33%.

$^1$H NMR (δ ppm, DMSO-$d_6$): 8.06 (d, J=8.2 Hz, 2H), 8.00 (brs, 2H), 7.95 (d, J=1.7 Hz, 1H), 7.57 (d, J=8.2 Hz, 2H), 7.51 (s, 1H), 7.20 (d, J=3.3 Hz, 1H), 6.74 (dd, J=3.3 Hz, 1.7 Hz, 1H), 5.10 (s, 1H), 1.47 (s, 6H)

Mass (m/z): 336 ($M^+$+1)

IR (KBr, $cm^{-1}$): 3500, 1660, 1655, 1599, 1550, 1228

| Elemental Analysis: as $C_{18}H_{17}N_5O_2$ | | | |
|---|---|---|---|
| Observed | C 64.51%, | H 5.32%, | N 20.55% |
| Calculated | C 64.47%, | H 5.11%, | N 20.88% |

Melting point: 246.0 - 246.6°C

Example 64

5-Amino-7-butyl-2-(2-furyl)[1,2,4]triazolo[1,5-c]pyrimidine (Compound 64)

First, 7-butyl-5-(3,4-dimethoxybenzylamino)-2-(2-furyl)[1,2,4]triazolo[1,5-c]pyrimidine (1.85 g, 4.54 mmol) produced in Example 22 was dissolved in dichloromethane (18 mL), and distilled water (1.0 mL) and DDQ (1.55 g, 6.81 mmol) were added to the resulting solution, followed by stirring at room temperature for 5 hours. Then, the reaction solution was diluted with chloroform, and a 2 mol/L aqueous solution of sodium hydroxide was added to the solution to make it basic, followed by separation. The organic layer was dried over anhydrous magnesium sulfate, filtered, and then concentrated under reduced pressure. The residue was triturated with diethyl ether, and the resulting solid was recrystallized from ethanol to obtain Compound 64 (118 mg, 0.46 mmol) as white crystals in a yield of 10%.

$^1$H NMR (δ ppm, DMSO-$d_6$): 7.91 (d, J=1.9 Hz, 1H), 7.85 (brs, 2H), 7.16 (d, J=3.3 Hz, 1H), 6.79 (s, 1H), 6.71 (dd, J=3.3 Hz, 1.9 Hz, 1H), 2.58 (t, J=7.7 Hz, 2H), 1.65 (m, 2H), 1.33 (m, 2H), 0.91 (t, J=7.3 Hz, 3H)

Mass (m/z): 257 ($M^+$)

IR (KBr, $cm^{-1}$): 1666, 1606, 1558, 1421, 1382, 748

| Elemental Analysis: as $C_{13}H_{15}N_5O \cdot 0.1\ H_2O$ | | | |
|---|---|---|---|
| Observed | C 60.14%, | H 5.74%, | N 27.34% |
| Calculated | C 60.26%, | H 5.91%, | N 27.03% |

Melting point: 155.0 - 155.3°C

Example 65

5-Amino-2-(2-furyl)-7-(2-phenylthienyl)[1,2,4]triazolo[1,5-c]pyrimidine (Compound 65)

The reaction was carried out in a manner similar to that in Example 18 except that 2-(3-tributylstannylphenyl)-1,3-dioxolane was replaced by 2-(phenylthienyl)-1-tributyltin to obtain 5-(3,4-dimethoxybenzylamino)-2-(2-furyl)-7-(2-phenylethynyl)[1,2,4]triazolo[1,5-c]pyrimidine (570 mg, 1.26 mmol) as a white amorphous substance in a yield of 70%.

The obtained 5-(3,4-dimethoxybenzylamino)-2-(2-furyl)-7-(2-phenylethynyl)[1,2,4]triazolo[1,5-c]pyrimidine was dissolved in dichloromethane (9 mL), and distilled water (0.5 mL) and DDQ (430 mg, 1.89 mmol) were added to the resulting solution, followed by stirring at room temperature for 6 hours. Then, the reaction solution was poured into a mixed solution of chloroform and a 1 mol/L aqueous solution of sodium hydroxide, and separation was performed. The organic layer was dried over anhydrous magnesium sulfate, filtered, and then concentrated under reduced pressure. The residue was triturated with methanol, and the resulting solid was recrystallized from a mixed solvent of ethanol (13 mL) and chloroform (4 mL) to obtain Compound 65 (160 mg, 0.530 mmol) as white crystals in a yield of 42%.

$^1$H NMR (δ ppm, DMSO-$d_6$): 8.16 (brs, 2H), 7.95 (d, J=2.0 Hz, 1H), 7.50-7. 60 (m, 2H), 7.40-7.50 (m, 3H), 7.26 (s, 1H), 7.23 (d, J=3.3 Hz, 1H), 6.74 (dd, J=3.3 Hz, 2.0 Hz, 1H)

Mass (m/z): 301 ($M^+$)

IR (KBr, $cm^{-1}$): 1645, 1599, 1538, 1323, 1234, 762, 744

| Elemental Analysis: as $C_{17}H_{11}N_5O \cdot 0.1\ H_2O$ | | | |
|---|---|---|---|
| Observed | C 67.16%, | H 3.65%, | N 23.32% |
| Calculated | C 67.36%, | H 3.72%, | N 23.10% |

Melting point: 246.5 - 247.3°C

Example 66

5-Amino-2-(2-furyl)-7-(2-phenylethyl)[1,2,4]triazolo[1,5-c]pyrimidine (Compound 66)

Compound 65 (600 mg, 1.99 mmol) was dissolved in ethanol (75 mL), and 10% palladium/carbon (60 mg) was added to the resulting solution, followed by stirring at 50°C for 2 hours in a hydrogen atmosphere. Then, the reaction solution was filtered with Celite, and the filtrate was concentrated under reduced pressure. The residue was purified by silica gel column chromatography (elution solvent: mixed solvent of hexane and ethyl acetate), and the resulting solid was recrystallized from ethanol (12 mL) to obtain Compound 66 (350 mg, 1.48 mmol) as white crystals in a yield of 74%.

$^1$H NMR (δ ppm, DMSO-$d_6$): 7.91 (brs, 2H), 7.90 (d, J=1.7 Hz, 1H), 7.20-7. 30 (m, 5H), 7.16 (d, J=3.3 Hz, 1H), 6.78 (s, 1H), 6.70 (dd, J=3.3 Hz, 1. 7 Hz, 1H), 2.80-3.00 (m, 4H)

Mass (m/z): 305 ($M^+$)

IR (KBr, $cm^{-1}$): 1666, 1606, 1556, 1510, 1421, 748

| Elemental Analysis: as $C_{17}H_{15}N_5O$ | | | |
|---|---|---|---|
| Observed | C 66.62%, | H 4.93%, | N 23.33% |
| Calculated | C 66.87%, | H 4.95%, | N 22.94% |

Melting point: 205.0 - 205.1°C

Example 67

5-Amino-2-(2-furyl)-7-(2-hydroxyethyl)[1,2,4]triazolo[1,5-c]pyrimidine (Compound 67)

Compound G (20.0 g, 51.9 mmol) was dissolved in toluene (300 mL), and vinyltributyltin (25.5 g, 77.8 mmol) and bistriphenylphosphine palladium dichloride (1.82 g, 2.59 mmol) were added to the resulting solution, followed by reflux for 3 hours. Then, the reaction solution was cooled to room temperature, and brine was added to the solution, followed by extraction with chloroform. The organic layer was dried over anhydrous magnesium sulfate, filtered, and then concentrated under reduced pressure. The residue was purified by silica gel column chromatography (elution solvent: mixed solvent of hexane and ethyl acetate) to obtain 5-(3,4-dimethoxybenzylamino)-2-(2-furyl)-7-vinyl[1,2,4]triazolo[1,5-c]pyrimidine (14.5 g, 38.5 mmol) as a white solid in a yield of 74%.

The obtained 5-(3,4-dimethoxybenzylamino)-2-(2-furyl)-7-vinyl[1,2,4]triazolo[1,5-c]pyrimidine (5.00 g, 13.3 mmol) was dissolved in THF (50 mL), and a THF solution (66.3 mL, 33.2 mmol) of 0.5 mol/L 9-borabicyclo[3.3.1]nonane was added dropwise to the resulting solution at room temperature under stirring at the same temperature. Furthermore, the resulting mixture was refluxed for 6 hours. Then, the reaction solution was cooled to 0°C, and ethanol (15 mL), a 6 mol/L aqueous solution of sodium hydroxide, and a 30% aqueous solution of hydrogen peroxide were added to the resulting solution. Then, an aqueous sodium hydrogen sulfite solution was added to the reaction solution, and aqueous sodium bicarbonate was added to the solution to make it basic, followed by extraction with chloroform. The organic layer was dried over anhydrous magnesium sulfate, filtered, and then concentrated under reduced pressure. The residue was purified by silica gel column chromatography (elution solvent: mixed solvent of chloroform and methanol) to obtain 5-(3,4-dimethoxybenzylamino)-2-(2-furyl)-7-(2-hydroxyethyl) [1,2,4]triazolo[1,5-c]pyrimidine as a white solid.

The obtained 5-(3,4-dimethoxybenzylamino)-2-(2-furyl)-7-(2-hydroxyethyl)[1,2,4]triazolo[1,5-c]pyrimidine was dissolved in dichloromethane (180 mL), and distilled water (10 mL) and DDQ (3.45 g, 15.2 mmol) were added to the resulting solution, followed by stirring at room temperature for 5 hours. The reaction solution was poured into a mixture of chloroform and aqueous sodium bicarbonate, and then the solution was subjected to separation. The organic layer was dried over anhydrous magnesium sulfate, filtered, and then concentrated under reduced pressure. The residue was triturated with chloroform, and the resulting solid was recrystallized from ethanol to obtain Compound 67 (421 mg, 1. 72 mmol) as a white solid in a yield of 13%.

$^1$H NMR (δ ppm, DMSO-$d_6$): 7.92 (d, J=1.6 Hz, 1H), 7.86 (brs, 2H), 7.17 (d, J=3.3 Hz, 1H), 6.81 (s, 1H), 6.71 (dd, J=3.3 Hz, 1.6 Hz, 1H), 4.65 (t, J=5.2 Hz, 1H), 3.74 (dt, J=6. 6 Hz, 5.2 Hz, 2H), 2.73 (t, J=6.6 Hz, 2H)

Mass (m/z): 245 ($M^+$), 215

IR (KBr, $cm^{-1}$): 3120, 3091, 1655, 1650, 1595, 1508, 773, 762

| Elemental Analysis: as $C_{11}H_{11}N_5O_2$ | | | |
|---|---|---|---|
| Observed | C 53.76%, | H 4.81%, | N 28.36% |
| Calculated | C 53.88%, | H 4.52%, | N 28.57% |

Melting point: 207.2 - 207.8°C

Example 68

5-Amino-7-[3-(cyclopropylmethylamino)-(E)-propan-1-en-1-yl]-2-(2-furyl)[1,2,4]triazolo[1,5-c]pyrimidine (Compound 68)

First, 5-(3,4-dimethoxybenzylamino)-2-(2-furyl)-7-vinyl[1,2,4]triazolo[1,5-c]pyrimidine (13.4 g, 35.6 mmol) produced in Example 67 was dissolved in THF (120 mL), and a 50% aqueous solution of 4-methylmorpholine-4-oxide (16.7 g, 71.2 mmol) and osmium tetraoxide (453 mg, 1.78 mmol) were added to the resulting solution, followed by stirring for 5 hours. Then, a 10% aqueous sodium solution of hydrogen sulfite was added to the resulting mixture, and the mixture was stirred for 2 hours. Then, the mixture was filtered with Celite, and the filtrate was subjected to extraction with chloroform. The organic layer was dried over anhydrous magnesium sulfate, filtered, and then concentrated under reduced pressure. The residue was purified by silica gel column chromatography (elution solvent: ethyl acetate). The resulting solid was triturated with methanol to obtain (±)-7-(1,2-dihydroxyethyl)-5-(3,4-dimethoxybenzylamino)-2-(2-furyl)[1,2,4]triazolo[1,5-c]pyrimidine (9.68 g, 23.6 mmol) as a white solid in a yield of 66%

The obtained (±)-7-(1,2-dihydroxyethyl)-5-(3,4-dimethoxybenzylamino)-2-(2-furyl)[1,2,4]triazolo[1,5-c]pyrimidine was dissolved in dichloromethane (450 mL) and distilled water (45 mL), and silica gel (31.0 g) and sodium metaperiodate (7.56 g, 35.3 mmol) were added to the resulting solution, followed by stirring at room temperature for 3 hours. Then, the reaction solution was filtered, and the filtrate was concentrated under reduced pressure. The residue

was triturated with methanol to obtain 5-(3,4-dimethoxybenzylamino)-7-formyl-2-(2-furyl)[1,2,4]triazolo[1,5-c]pyrimidine (8.06 g, 20.5 mmol) as a white solid in a yield of 87%.

Then, 60% sodium hydride (380 mg, 9.50 mmol) was suspended in THF (30 mL) in an argon stream, and ethyl diethylphosphonoacetate (1.88 mL, 9.50 mmol) was added to the resulting suspension, followed by stirring at 0°C for 3 minutes. Then, 5-(3,4-dimethoxybenzylamino)-7-formyl-2-(2-furyl)[1,2,4]triazolo[1,5-c]pyrimidine (1.20 g, 3.17 mmol) was added to the reaction mixture, followed by stirring at room temperature for 2 hours. Then, saturated brine was added to the reaction solution, and extraction with ethyl acetate was performed. The organic layer was dried over anhydrous magnesium sulfate, filtered, and then concentrated under reduced pressure. The residue was purified by silica gel column chromatography (elution solvent, ethyl acetate) to obtain ethyl 5-(3,4-dimethoxybenzylamino)-2-(2-furyl)[1,2,4]triazolo[1,5-c]pyrimidine-7-acrylate (1.35 g, 3.00 mmol) as white crystals in a yield of 95%.

The obtained ethyl 5-(3,4-dimethoxybenzylamino)-2-(2-furyl)[1,2,4]triazolo[1,5-c]pyrimidine-7-acrylate (1.30 g, 2.89 mmol) was dissolved in dichloromethane (20 mL), and the resulting solution was cooled to -78°C in an argon stream. Then, a toluene solution (8.68 mL, 8.68 mmol) of 1 mol/L diisopropyl aluminum hydride was added dropwise to the resulting mixture at the same temperature, and the mixture was gradually heated to 0°C, followed by stirring for 1 hour. Then, an aqueous saturated Rochelle salt solution was added to the reaction solution, and extraction with ethyl acetate was performed. The organic layer was dried over anhydrous magnesium sulfate, filtered, and then concentrated under reduced pressure. The residue was triturated with methanol to obtain 5-(3,4-dimethoxybenzylamino)-2-(2-furyl)-7-(3-hydroxy-(E)-prop-1-en-1-yl)[1,2,4]triazolo[1,5-c]pyrimidine (994 mg, 2.44 mmol) as a white solid in a yield of 84%.

The obtained 5-(3,4-dimethoxybenzylamino)-2-(2-furyl)-7-(3-hydroxy-(E)-prop-1-en-1-yl)[1,2,4]triazolo [1,5-c]pyrimidine was dissolved in dichloromethane (81 mL), and distilled water (4.5 mL) and DDQ (1.65 g, 7.29 mmol) were added to the resulting solution, followed by stirring at room temperature for 5 hours. The reaction solution was poured into a mixture of chloroform and aqueous saturated sodium bicarbonate, and the precipitated solid was filtered off. Then, the solid was successively washed with distilled water and chloroform to obtain 5-amino-2-(2-furyl)-7-(3-hydroxy-(E)-prop-1-en-1-yl)[1,2,4]triazolo[1,5-c]pyrimidine (457 mg, 1.78 mmol) as a brown solid in a yield of 87%.

The obtained 5-amino-2-(2-furyl)-7-(3-hydroxy-(E)-prop-1-en-1-yl)[1,2,4]triazolo[1,5-c]pyrimidine was dissolved in methanol (15 mL), and cyclopropylmethylamine (208 mg, 2.92 mmol) was added to the resulting solution, followed by reflux for 1 hour. Then, sodium borohydride (221 mg, 5.85 mmol) was added to the reaction solution, and the mixture was stirred at room temperature for 30 minutes. Then, the reaction solution was concentrated under reduced pressure, and the residue was purified by silica gel column chromatography (elution solvent: mixed solvent of chloroform and methanol). Then, an ethyl acetate solution of hydrogen chloride was added to the resulting white solid, and the mixture was concentrated under reduced pressure. The residue was recrystallized from ethanol (12 mL) to obtain Compound 68 (120 mg, 0.313 mmol) as white crystals in a yield of 18%.

$^1$H NMR (δ ppm, DMSO-$d_6$): 9.56 (m, 2H), 8.08 (brs, 2H), 7.95 (d, J=1.6 Hz, 1H), 7.21 (d, J=3.3 Hz, 1H), 7.00 (s, 1H), 6.75-6.85 (m, 2H), 6.73 (dd, J=3.3 Hz, 1.6 Hz, 1H), 3.78 (d, J=4.3 Hz, 2H), 2.81 (dd, J=5.6 Hz, 5.3 Hz, 2H), 1.03 (m, 1H), 0.53-0.61 (m, 2H), 0.38-0.41 (m, 2H)

Mass (m/z): 311 ($M^+$+1)

IR (KBr, $cm^{-1}$): 1691, 1635, 1629, 1608

| Elemental Analysis: as $C_{16}H_{18}N_6O·2.0\ HCl·0.8\ H_2O$ | | | |
|---|---|---|---|
| Observed | C 48.47%, | H 5.82%, | N 20.88% |
| Calculated | C 48.32%, | H 5.47%, | N 21.13% |

Melting point: 263.5 - 264.2°C

Example 69

5-Amino-2-(2-furyl)-7-[2-(1-hydroxycyclopentyl)ethynyl][1,2,4]triazolo[1,5-c]pyrimidine (Compound 69)

Compound G (1.25 g, 3.25 mmol) was dissolved in DMF (15 mL), and 2-(1-trimethylsilyloxycyclopentyl)ethynyltributyltin (2.29 g, 4.88 mmol) and bistriphenylphosphine palladium dichloride (230 mg, 0.325 mmol) were added to the resulting solution, followed by stirring at 110°C for 3 hours. The reaction solution was cooled to room temperature, and a THF solution (7.50 mL, 7.50 mmol) of 1 mol/L tetrabutylammonium fluoride was added to the solution, followed by stirring for 10 minutes. Then, distilled water was added to the reaction solution, and extraction with chloroform was performed. The organic layer was dried over anhydrous magnesium sulfate, filtered, and then concentrated under reduced pressure. The residue was purified by silica gel column chromatography (elution solvent: mixed solvent of chloroform and methanol) to obtain 5-(3,4-dimethoxybenzylamino)-2-(2-furyl)-7-[2-(1-hydroxycyclopentyl)ethynyl] [1,2,4]triazolo[1,5-c]pyrimidine as a brown oily substance.

The obtained 5-(3,4-dimethoxybenzylamino)-2-(2-furyl)-7-[2-(1-hydroxycyclopentyl)ethynyl][1,2,4]triazolo [1,5-c]pyrimidine was dissolved in chloroform (81 mL), and distilled water (4.5 mL) and DDQ (2.13 g, 9.75 mmol) were added to the resulting solution, followed by stirring at room temperature for 2 hours. Then, the reaction solution was poured into a mixture of chloroform and a 1 mol/L aqueous solution of sodium hydroxide, followed by separation. The organic layer was dried over anhydrous magnesium sulfate, filtered, and then concentrated under reduced pressure. The residue was triturated with chloroform, and the resulting solid was recrystallized from a mixed solvent of ethanol (12 mL) and DMF (8 mL) to obtain Compound 69 (536 mg, 1.73 mmol) as white crystals in a yield of 53%.
$^1$H NMR (δ ppm, DMSO-$d_6$): 8.08 (brs, 2H), 7.94 (d, J=1.6 Hz, 1H), 7.20 (d, J=3.3 Hz, 1H), 7.04 (s, 1H), 6.72 (dd, J=3.3 Hz, 1.6 Hz, 1H), 5.47 (s, 1H), 1.60-2.00 (m, 8H)
Mass (m/z): 310 ($M^+$+1)
IR (KBr, $cm^{-1}$): 1666, 1650, 1637, 1606, 1589, 1551, 1419, 1227, 1182, 746

| Elemental Analysis: as $C_{16}H_{15}N_5O_2$ | | | |
|---|---|---|---|
| Observed | C 62.37%, | H 5.03%, | N 22.73% |
| Calculated | C 62.13%, | H 4.89%, | N 22.64% |

Melting point: 249.5 - 249.9°C

Example 70

5-Amino-7-[1-(cyclopropylmethyl)-1,2,3,6-tetrahydropyridin-4-yl)-2-(2-furyl)[1,2,4]triazolo[1,5-c]pyrimidine (Compound 70)

Compound G (5.30 g, 13.7 mmol) was dissolved in toluene (300 mL), and tert-butyl 4-(tributylstannyl-3,6-dihydro-2H-pyridine-1-carboxylate (9.70 g, 20.6 mmol) and bistriphenylphosphine palladium dichloride (1.00 g, 1.37 mmol) were added to the resulting solution, followed by reflux for 6 hours. Then, the reaction solution was cooled to room temperature, and ethyl acetate and a 2 mol/L aqueous solution of ammonium fluoride were added to the solution, followed by stirring. The reaction solution was filtered with Celite, and the filtrate was subjected to separation. The organic layer was dried over anhydrous magnesium sulfate, filtered, and then concentrated under reduced pressure. The residue was purified by silica gel column chromatography (elution solvent: mixed solvent of hexane and ethyl acetate) to obtain 7-[1-(tert-butoxycarbonyl)-1,2,3,6-tetrahydropyridin-4-yl]-5-(3,4-dimethoxybenzylamino)-2-(2-furyl) [1,2,4]triazolo[1,5-c]pyrimidine (4.19 g, 7.87 mmol) as a light-yellow amorphous substance in a yield of 57%.
The obtained 7-[1-(tert-butoxycarbonyl)-1,2,3,6-tetrahydropyridin-4-yl]-5-(3,4-dimethoxybenzylamino)-2-(2-furyl)[1,2,4]triazolo[1,5-c]pyrimidine (3.90 g, 7.31 mmol) was dissolved in trifluoroacetic acid (50 mL), and anisole (2.40 mL, 22.0 mmol) and trifluoromethanesulfonic acid (2.00 mL, 22.0 mmol) were added to the resulting solution, followed by stirring at room temperature for 4 hours. Then, diethyl ether and ammonia water were added to the reaction solution, and the precipitated solid was filtered off. The solid was recrystallize3d from ethanol to obtain 5-amino-2-(2-furyl)-7-(1,2,3,6-tetrahydropyridin-4-yl)[1,2,4]triazolo[1,5-c]pyrimidine (1.04 g, 3.68 mmol) as yellow crystals in a yield of 50%.
The obtained 5-amino-2-(2-furyl)-7-(1,2,3,6-tetrahydropyridin-4-yl)[1,2,4]triazolo[1,5-c]pyrimidine (300 mg, 1.06 mmol) was dissolved in a mixed solvent of dichloromethane (10 mL) and acetic acid (1 mL), and cyclopropane carboaldehyde (0.158 mL, 2.13 mmol) and sodium triacetoxyborohydride (673 mg, 3.18 mmol) were added to the resulting solution, followed by stirring at room temperature for 6 hours. Then, a 2 mol/L aqueous solution of sodium hydroxide was added to the reaction solution, and extraction with chloroform was performed. The organic layer was dried over anhydrous magnesium sulfate, filtered, and then concentrated under reduced pressure. The residue was purified by silica gel column chromatography (elution solvent: mixed solvent of chloroform and methanol), and the resulting white solid was recrystallized from ethanol (10 mL) to obtain Compound 70 (190 mg, 0.505 mmol) as white crystals in a yield of 48%.
$^1$H NMR (δ ppm, DMSO-$d_6$): 7.92 (d, J=1.6 Hz, 1H), 7.82 (brs, 2H), 7.17 (d, J=3.3 Hz, 1H), 6.92 (s, 1H), 6.89 (m, 1H), 6.72 (dd, J=3.3 Hz, 1.6 Hz, 1H), 3.20-3.23 (m, 2H), 2.66-2.70 (m, 2H), 2.55-2.60 (m, 2H), 2.30 (d, J=6.6 Hz, 2H), 0.87 (m, 1H), 0.45-0.55 (m, 2H), 0.10-0.15 (m, 2H)
Mass (m/z): 337 ($M^+$+1)
IR (KBr, $cm^{-1}$): 1664, 1647, 1641, 1598, 1425, 1225

| Elemental Analysis: as $C_{18}H_{20}N_6O \cdot 0.3\ H_2O$ | | | |
|---|---|---|---|
| Observed | C 63.48%, | H 6.01%, | N 24.32% |

(continued)

| Elemental Analysis: as $C_{18}H_{20}N_6O \cdot 0.3\ H_2O$ | | | |
|---|---|---|---|
| Calculated | C 63.25%, | H 6.07%, | N 24.59% |

Melting point: 196.9 - 197.4°C

Example 71

5-Amino-7-[1-(cyclopropylmethyl)piperidin-4-yl]-2-(2-furyl)[1,2,4]triazolo[1,5-c]pyrimidine (Compound 71)

First, 5-amino-2-(2-furyl)-7-(1,2,3,6-tetrahydropyridin-4-yl)[1,2,4]triazolo[1,5-c]pyrimidine (900 mg, 3.19 mmol) produced in Example 70 was dissolved in ethanol (60 mL), and 10% palladium/carbon (50 mg) was added to the resulting solution, followed by stirring at 60°C for 2 hours in a hydrogen atmosphere. Then, the reaction solution was filtered with Celite, and the filtrate was concentrated under reduced pressure to obtain 5-amino-2-(2-furyl)-7-(piperidin-4-yl)[1,2,4]triazolo[1,5-c]pyrimidine (750 mg, 2.64 mmol) as a crude product in a yield of 83%.

The obtained crude product (300 mg, 1.06 mmol) was dissolved in a mixed solvent of dichloromethane (20 mL) and acetic acid (2 mL), and cyclopropane carboaldehyde (0.120 mL, 1.58 mmol) and sodium triacetoxyborohydride (450 mg, 2.12 mmol) were added to the resulting solution, followed by stirring at room temperature for 3 hours. Then, a 2 mol/L aqueous solution of sodium hydroxide was added to the reaction solution, and extraction with chloroform was performed. The organic layer was dried over anhydrous magnesium sulfate, filtered, and then concentrated under reduced pressure. The residue was purified by silica gel column chromatography (elution solvent: mixed solvent of chloroform and methanol), and the resulting white solid was recrystallized from ethanol (5 mL) to obtain Compound 71 (100 mg, 0.296 mmol) as white crystals in a yield of 28%.
$^1$H NMR (δ ppm, DMSO-$d_6$): 7.92 (d, J=1.6 Hz, 1H), 7.84 (brs, 2H), 7.16 (d, J=3.3 Hz, 1H), 6.80 (s, 1H), 6.71 (dd, J=3.3 Hz, 1.6 Hz, 1H), 3.05-3.10 (m, 2H), 2.47 (m, 1H), 2.20 (d, J=6.6 Hz, 2H), 1.60-2.10 (m, 6H), 0.83 (m, 1H), 0.40-0.50 (m, 2H), 0.05-0.10 (m, 2H)
Mass (m/z): 338 ($M^+$+1), 323
IR (KBr, $cm^{-1}$): 1664, 1606, 1560, 1417, 1328

| Elemental Analysis: as $C_{18}H_{22}N_6O \cdot 0.1\ H_2O$ | | | |
|---|---|---|---|
| Observed | C 63.24%, | H 6.93%, | N 24.76% |
| Calculated | C 63.55%, | H 6.58%, | N 24.70% |

Melting point: 186.4 - 186.6°C

Example 72

5-Amino-2-(2-furyl)-7-[1-(1,2,3-thiadiazol-4-yl)methyl-1,2,3,6-tetrahydropyridin-4-yl][1,2,4]triazolo[1,5-c]pyrimidine (compound 72)

The reaction was carried out in a manner similar to that in Example 70 except that cyclopropane carboaldehyde was replaced by 1,2,3-thiadiazole-4-carboaldehyde. Then, the resulting crude product was recrystallized from a mixed solvent of ethanol (10 mL) and DMF(2 mL) to obtain Compound 72 (144 mg, 0.379 mmol) as white crystals in a yield of 36%.
$^1$H NMR (δ ppm, DMSO-$d_6$): 9.11 (s, 1H), 7.92 (d, J=1.6 Hz, 1H), 7.81 (brs, 2H), 7.16 (d, J=3.3 Hz, 1H), 6.92 (s, 1H), 6.88 (m, 1H), 6.71 (dd, J=3.3 Hz, 1.6 Hz, 1H), 4.18 (s, 2H), 3.25-3.30 (m, 2H), 2.70-2.75 (m, 2H), 2.55-2.60 (m, 2H)
Mass (m/z): 381 ($M^+$+1)
IR (KBr, $cm^{-1}$): 1664, 1654, 1598, 1560, 1508, 1227

| Elemental Analysis: as $C_{17}H_{16}N_8OS \cdot 0.3\ C_2H_5OH \cdot 0.1\ H_2O$ | | | |
|---|---|---|---|
| Observed | C 53.31%, | H 4.20%, | N 28.25% |
| Calculated | C 53.38%, | H 4.58%, | N 28.29% |

Melting point: 227.8 - 228.2°C

Example 73

5-Amino-2-(2-furyl)-7-[1-(2-methoxyethyl)-1,2,3,6-tetrahydropyridin-4-yl][1,2,4]triazolo[1,5-c]pyrimidine (Compound 73)

First, 5-amino-2-(2-furyl)-7-(1,2,3,6-tetrahydropyridin-4-yl)[1,2,4]triazolo[1,5-c]pyrimidine (600 mg, 2.13 mmol) produced in Example 70 was dissolved in DMF (10 mL), and 2-methoxy1-bromoethane (0.300 mL, 3.19 mmol) and triethylamine (0.890 mL, 6.38 mmol) were added to the resulting solution, followed by stirring at room temperature for 11 hours. Then, the reaction solution was concentrated under reduced pressure, and the residue was purified by silica gel column chromatography (elution solvent: mixed solvent of chloroform and methanol), and the resulting white solid was recrystallized from ethanol (5 mL) to obtain Compound 73 (90 mg, 0.264 mmol) as white crystals in a yield of 12%.
$^1$H NMR ($\delta$ ppm, DMSO-$d_6$): 7.91 (d, J=1.6 Hz, 1H), 7.80 (brs, 2H), 7.16 (d, J=3.3 Hz, 1H), 6.91 (s, 1H), 6.86 (m, 1H), 6.70 (dd, J=3.3 Hz, 1.6 Hz, 1H), 3.48 (t, J=5.9 Hz, 2H), 3.32 (s, 3H), 3.15-3.25 (m, 2H), 2.60-2.70 (m, 2H), 2.60 (t, J=5.9 Hz, 2H), 2.45-2.55 (m, 2H)
Mass (m/z): 340 ($M^+$+1)
IR (KBr, $cm^{-1}$): 1662, 1656, 1647, 1635, 1608, 1558, 1550

| Elemental Analysis: as $C_{17}H_{20}N_6O_2 \cdot 0.4\ H_2O$ | | | |
|---|---|---|---|
| Observed | C 58.88%, | H 5.96%, | N 24.26% |
| Calculated | C 58.74%, | H 6.03%, | N 24.18% |

Melting point: 164.2 - 164.6°C

Example 74

5-Amino-7-[1-(2-methyl-2-hydroxypropyl)-1,2,3,6-tetrahydropyridin-4-yl]-2-(2-furyl)[1,2,4]triazolo[1,5-c]pyrimidine (Compound 74)

The reaction was carried out in a manner similar to that in Example 73 except that 2-methoxyl-bromoethane was replaced by ethyl 2-bromoacetate. Then, the resulting crude product was purified by silica gel column chromatography (elution solvent: mixed solvent of chloroform and methanol). The resulting solid was triturated with diethyl ether to obtain 5-amino-7-[1-(ethoxycarbonylmethyl)-1,2,3,6-tetrahydropyridin-4-yl]-2-(2-furyl)[1,2,4]triazolo[1,5-c]pyrimidine (1.70 g, 4.63 mmol) as a white solid in a yield of 65%.
The obtained 5-amino-7-[1-(ethoxycarbonylmethyl)-1,2,3,6-tetrahydropyridin-4-yl]-2-(2-furyl)[1,2,4]triazolo[1,5-c]pyrimidine (500 mg, 1.36 mmol) was dissolved in THF (50 mL), and the resulting solution was added dropwise at 0°C to a solution prepared by diluting a THF solution (28.6 mL, 27.2 mmol) of 0.95 mol/L methyl magnesium bromide with THF (20 mL). Furthermore, the reaction mixture was stirred at room temperature for 10 minutes, and then an aqueous saturated solution of ammonium chloride and distilled water were added to the reaction solution, followed by extraction with chloroform. The organic layer was dried over anhydrous magnesium sulfate, filtered, and then concentrated under reduced pressure. The residue was dissolved in THF (12 mL), and DBU (2.35 mL, 1.71 mmol) was added to the resulting solution, followed by stirring at room temperature for 2 hours. Then, the reaction solution was concentrated under reduced pressure, and the residue was purified by silica gel column chromatography (elution solvent: mixed solvent of chloroform and methanol). The resulting white solid was recrystallized from ethanol (6 mL) to obtain Compound 74 (120 mg, 0.340 mmol) as white crystals in a yield of 25%.
$^1$H NMR ($\delta$ ppm, DMSO-$d_6$) : 7.92 (d, J=1.7 Hz, 1H), 7.80 (brs, 2H), 7.17 (d, J=3.3 Hz, 1H), 6.91 (s, 1H), 6.87 (m, 1H), 6.71 (dd, J=3.3 Hz, 1.7 Hz, 1H), 4.12 (s, 1H), 3.20-3.30 (m, 2H), 2.70-2.80 (m, 2H), 2.40-2.55 (m, 2H), 2.34 (s, 2H), 1.12 (s, 6H)
Mass (m/z): 355 ($M^+$+1)
IR (KBr, $cm^{-1}$): 1666, 1652, 1647, 1602, 1558, 1551, 1457, 1419, 1124, 752

| Elemental Analysis: as $C_{18}H_{22}N_6O_2 \cdot 0.3\ H_2O$ | | | |
|---|---|---|---|
| Observed | C 59.99%, | H 6.47%, | N 23.31% |
| Calculated | C 60.09%, | H 6.33%, | N 23.36% |

Melting point: 197.8 - 198.1°C

Example 75

5-Amino-2-(2-furyl)-7-[1-(2-hydroxyethyl)-1,2,3,6-tetrahydropyridin-4-yl][1,2,4]triazolo[1,5-c]pyrimidine (Compound 75)

First, 5-amino-7-[1-(ethoxycarbonylmethyl)-1,2,3,6-tetrahydropyridin-4-yl]-2-(2-furyl)[1,2,4]triazolo[1,5-c]pyrimidine (500 mg, 1.36 mmol) produced in Example 74 was dissolved in THF (40 mL), and the resulting solution was stirred at -70°C in an argon stream. Then, a hexane solution (17.2 mL, 16.4 mmol) of 0.95 mol/L diisopropyl aluminum hydride was added dropwise to the reaction mixture at the same temperature, followed by further stirring at the same temperature for 1 hour. Then, an aqueous saturated solution of Rochelle salt was added to the reaction solution, and extraction with chloroform was performed. The organic layer was dried over anhydrous magnesium sulfate, filtered, and then concentrated under reduced pressure. The residue was dissolved in methanol (10 mL), and sodium borohydride (257 mg, 6.80 mmol) was added to the resulting solution, followed by stirring at room temperature for 2 hours. Then, the reaction solution was concentrated under reduced pressure, and the residue was washed with distilled water. The resulting solid was recrystallized from a mixed solvent of ethanol (10 mL) and DMF (2 mL) to obtain Compound 75 (244 mg, 0.748 mmol) as white crystals in a yield of 55%.
$^1$H NMR (δ ppm, DMSO-$d_6$): 7.93 (d, J=1.6 Hz, 1H), 7.82 (brs, 2H), 7.17 (d, J=3.3 Hz, 1H), 6.92 (s, 1H), 6.88 (m, 1H), 6.71 (dd, J=3.3 Hz, 1.6 Hz, 1H), 4.45 (t, J=5.2 Hz, 1H), 3.56 (dt, J=5.6 Hz, 5.2 Hz, 2H), 3.15-3.25 (m, 2H), 2.60-2.65 (m, 2H), 2.40-2.60 (m, 4H)
Mass (m/z): 327 ($M^+$+1)
IR (KBr, $cm^{-1}$): 1643, 1635, 1604, 1556, 1510, 1413, 1329, 1215, 1038, 746

| Elemental Analysis: as $C_{16}H_{18}N_6O_2 \cdot 0.2\ H_2O$ | | | |
|---|---|---|---|
| Observed | C 58.11%, | H 5.93%, | N 25.60% |
| Calculated | C 58.24%, | H 5.62%, | N 25.47% |

Melting point: 199.2 - 199.9°C

Example 76

5-Amino-7-[1-(3-methyl-3-hydroxybutyl)-1,2,3,6-tetrahydropyridin-4-yl]-2-(2-furyl)[1,2,4]triazola[1,5-c]pyrimidine (Compound 76)

The reaction was carried out in a manner similar to that in Example 73 except that 2-methoxy-1-bromoethane was replaced by 3-dimethyl-3-hydroxy-1-butyl methanesulfonate. Then, the resulting crude product was purified by silica gel column chromatography (elution solvent: mixed solvent of chloroform and methanol). The resulting white solid was recrystallized from ethanol (10 mL) to obtain Compound 76 (719 mg, 1.95 mmol) in a yield of 55%.
$^1$H NMR (δ ppm, DMSO-$d_6$): 7.92 (d, J=1.7 Hz, 1H), 7.82 (brs, 2H), 7.17 (d, J=3.3 Hz, 1H), 6.91 (s, 1H), 6.88 (m, 1H), 6.72 (dd, J=3.3 Hz, 1.7 Hz, 1H), 4.59 (s, 1H), 3.10-3.20 (m, 2H), 2.60-2.70 (m, 2H), 2.40-2.60 (m, 4H), 1.59 (t, J=7.2 Hz, 2H), 1.11 (s, 6H)
Mass (m/z): 369 ($M^+$+1)
IR (KBr, $cm^{-1}$): 1641, 1608, 1554, 1512, 1425, 1331, 1219, 1182, 744

| Elemental Analysis: as $C_{19}H_{24}N_6O_2 \cdot 0.6\ C_2H_5OH$ | | | |
|---|---|---|---|
| Observed | C 61.23%, | H 7.08%, | N 21.39% |
| Calculated | C 61.26%, | H 7.02%, | N 21.22% |

Melting point: 183.5 - 184.0°C

Example 77

5-Amino-2-(2-furyl)[1,2,4]triazolo[1,5-c]pyrimidine-7-carboxylic acid (2-acetylaminoethyl)amide (Compound 77)

Compound 41 (4.53 g, 19.8 mmol) was dissolved in acetic acid (160 mL), and a solution of sulfamic acid (3.46 g, 35.7 mmol) in distilled water (40 mL) was added to the resulting solution. Then, sodium hypochlorite (2.51 g, 27.7 mmol) was added to the mixture, followed by stirring at room temperature for 4 hours. Then, distilled water and 2 mol/L hydrochloric acid were added to the reaction solution, and the precipitated solid was filtered off and dried to obtain 5-amino-2-(2-furyl)[1,2,4]triazolo[1,5-c]pyrimidine-7-carboxylic acid (4.33 g, 17.7 mmol) as a white solid in a yield of 89%.

The obtained 5-amino-2-(2-furyl)[1,2,4]triazolo[1,5-c]pyrimidine-7-carboxylic acid (500 mg, 2.04 mmol) was dissolved in DMF (50 mL), and N-acetylethylenediamine (313 mg, 3.06 mmol), 1-ethyl-3-[3-(dimethylamino)propyl] carbodiimide monohydrochloride (782 mg, 4.08 mmol), and 1-hydroxybenzotriazole (312 mg, 2.04 mmol) were added to the resulting solution, followed by stirring at room temperature for 12 hours. Then, chloroform (30 mL) and aldehyde resin (produced by Sigma-Aldrich Co., 1.0 g) were added to the reaction solution, followed by further stirring at room temperature for 12 hours. The resin was filtered off from the reaction solution, and the filtrate was concentrated. The residue was triturated with chloroform, and then recrystallized from a mixed solvent of ethanol (20 mL) and DMF (1 mL) to obtain Compound 77 (319 mg, 0.970 mmol) as white crystals in a yield of 48%.
$^1$H NMR (δ ppm, DMSO-$d_6$): 8.37 (t, J=6.0 Hz, 1H), 8.15 (brs, 2H), 8.00 (t, J=5.3 Hz, 1H), 7.95 (d, J=1.6 Hz, 1H), 7.46 (s, 1H), 7.24 (d, J=3.3 Hz, 1H), 6.74 (dd, J=3.3 Hz, 1.6 Hz, 1H), 3.38 (dt, J=5.9 Hz, 5.3 Hz, 2H), 3.21 (dt, J=6.0 Hz, 5.9 Hz, 2H), 1.82 (s, 3H)
Mass (m/z): 330 ($M^+$+1)
IR (KBr, $cm^{-1}$): 1654, 1637, 1560, 1540

| Elemental Analysis: as $C_{14}H_{15}N_7O_3 \cdot 0.8\ H_2O$ | | | |
|---|---|---|---|
| Observed | C 48.97%, | H 4.60%, | N 28.26% |
| Calculated | C 48.92%, | H 4.87%, | N 28.52% |

Melting point: 233.8 - 234.0°C

Example 78

5-Amino-2-(2-furyl)[1,2,4]triazolo[1,5-c]pyrimidine-7-carboxylic acid (N-benzyl-N-methyl)amide (Compound 78)

The reaction was carried out in a manner similar to that in Example 77 except that N-acetylethylenediamine was replaced by N-methylbenzylamine. Then, an ethyl acetate solution of hydrogen chloride was added to the resulting crude product, and the mixture was concentrated under reduced pressure. The residue was recrystallized from ethanol (18 mL) to obtain Compound 78 (223 mg, 0.579 mmol) as white crystals in a yield of 28%.
$^1$H NMR (δ ppm, DMSO-$d_6$): 8.96 (brs, 1H), 8.23 (brs, 2H), 7.96 (m, 1H), 7.25-7.40 (m, 5H), 7.22 (m, 1H), 7.10 (s, 0.6H), 7.08 (s, 0.4H), 6.74 (m, 1H), 4.67 (s, 1.2H), 4.55 (s, 0.8H), 2.90 (s, 1.8H), 2.85 (s, 1.2H)
Mass (m/z): 348 ($M^+$+1)
IR (KBr, $cm^{-1}$): 1685, 1653, 1637, 1612, 1560, 1508

| Elemental Analysis: as $C_{18}H_{16}N_6O_2 \cdot 1.0\ HCl$ | | | |
|---|---|---|---|
| Observed | C 56.19%, | H 4.52%, | N 21.61% |
| Calculated | C 56.18%, | H 4.45%, | N 21.84% |

Melting point: 188.5 - 189.1°C

Example 79

5-Amino-2-(2-furyl)[1,2,4]triazolo[1,5-c]pyrimidine-7-carboxylic acid {N-benzyl-N-[2-(dimethylamino)ethyl]}amide (Compound 79)

The reaction was carried out in a manner similar to that in Example 77 except that N-acetylethylenediamine was replaced by N-benzyl-2-(dimethylamino)ethylamine. The resulting crude product was recrystallized from ethanol (14 mL) to obtain Compound 79 (403 mg, 0.994 mmol) as white crystals in a yield of 61%.
$^1$H NMR (δ ppm, DMSO-$d_6$): 8.22 (brs, 2H), 7.93-7.97 (m, 1H), 7.20-7.40 (m, 5H), 7.22 (d, J=3.3 Hz, 0.6H), 7.20 (d, J=3.3 Hz, 0.4H), 7.10 (s, 0.6H), 7.03 (s, 0.4H), 6.70-6.75 (m, 1H), 4.71 (s, 1.2H), 4.58 (s, 0.8H), 3.30-3.40 (m, 2H), 2.30-2.45 (m, 2H), 2.13 (s, 2.4H), 1.97 (s, 3.6H)
Mass (m/z): 406 ($M^+$+1)
IR (KBr, $cm^{-1}$): 1670, 1639, 1606, 1576, 1508, 1471, 1423, 1221

| Elemental Analysis: as $C_{21}H_{23}N_7O_2 \cdot 0.2\ H_2O$ | | | |
|---|---|---|---|
| Observed | C 61.46%, | H 5.91%, | N 24.13% |
| Calculated | C 61.66%, | H 5.77%, | N 23.97% |

Melting point: 215.2 - 215.4°C

Example 80

5-Amino-2-(2-furyl)[1,2,4]triazolo[1,5-c]pyrimidine-7-carboxylic acid (3,4-dimethoxybenzyl)amide (Compound 80)

The reaction was carried out in a manner similar to that in Example 77 except that N-acetylethylenediamine was replaced by veratrylamine. The resulting crude product was recrystallized from a mixed solvent of ethanol (12 mL) and DMF (1 mL) to obtain Compound 80 (320 mg, 0.812 mmol) as white crystals in a yield of 40%.
$^1$H NMR (δ ppm, DMSO-$d_6$): 8.54 (t, J=6.0 Hz, 1H), 8.16 (brs, 2H), 7.95 (d, J=1.6 Hz, 1H), 7.49 (s, 1H), 7.24 (d, J=3.3 Hz, 1H), 6.97 (s, 1H), 6.92 (d, J=8.5 Hz, 1H), 6.88 (d, J=8.5 Hz, 1H), 6.74 (dd, J=3.3 Hz, 1.6 Hz, 1H), 4.44 (d, J=6.0 Hz, 2H), 3.74 (s, 3H), 3.73 (s, 3H)
Mass (m/z): 394 ($M^+$)
IR (KBr, $cm^{-1}$): 1677, 1645, 1619, 1523, 1515, 1261

| Elemental Analysis: as $C_{19}H_{18}N_6O_4 \cdot 0.1H_2O$ | | | |
|---|---|---|---|
| Observed | C 57.73%, | H 5.04%, | N 21.36% |
| Calculated | C 57.60%, | H 4.63%, | N 21.21% |

Melting point: 182.1 - 182.4°C

Example 81

5-Amino-2-(2-furyl)[1,2,4]triazolo[1,5-c]pyrimidine-7-carboxylic acid [N-(3,4-dimethoxybenzyl)-N-methyl]amide (Compound 81)

The reaction was carried out in a manner similar to that in Example 77 except that N-acetylethylenediamine was replaced by N-methyl-3,4-dimethoxybenzylamine. Then, an ethyl acetate solution of hydrogen chloride was added to the resulting crude product, and the precipitated crystals were recrystallized from a mixed solvent of ethanol (12 mL) and DMF (3 mL) to obtain Compound 81 (231 mg, 0.520 mmol) as white crystals in a yield of 32%.
$^1$H NMR (δ ppm, DMSO-$d_6$): 8.21 (brs, 2H), 7.95 (d, J=1.6 Hz, 1H), 7.22 (d, J=3.3 Hz, 0.5H), 7.20 (d, J=3.3 Hz, 0.5H), 7.08 (s, 0.5H), 7.07 (s, 0. 5H), 6.80-7.00 (m, 3H), 6.74 (dd, J=3.3 Hz, 1.6 Hz, 1H), 4.58 (s, 1H), 4.46 (s, 1H), 3.77 (s, 1.5H), 3.76 (s, 1.5H), 3.73 (s, 3H), 2.87 (s, 1.5H), 2.85 (s, 1.5H)
Mass (m/z): 409 ($M^+$+1)
IR (KBr, $cm^{-1}$): 1651, 1637, 1556, 1512, 1425, 1419, 1317, 1236, 763

| Elemental Analysis: as $C_{20}H_{20}N_6O_4 \cdot 1.0\ HCl \cdot 0.1\ H_2O$ | | | |
|---|---|---|---|
| Observed | C 53.81%, | H 4.81%, | N 18.93% |
| Calculated | C 53.78%, | H 4.78%, | N 18.81% |

Melting point: 190.3 - 190.8°C

Example 82

5-Amino-2-(2-furyl)[1,2,4]triazolo[1,5-c]pyrimidine-7-carboxylic acid (3,4-difluorobenzyl)amide (Compound 82)

The reaction was carried out in a manner similar to that in Example 77 except that N-acetylethylenediamine was replaced by 3,4-difluorobenzylamine. Then, the resulting crude product was recrystallized from a mixed solvent of ethanol (14 mL) and DMF (3 mL) to obtain Compound 82 (440 mg, 1.21 mmol) as white crystals in a yield of 74%.
$^1$H NMR (δ ppm, DMSO-$d_6$): 8.86 (t, J=6.3 Hz, 1H), 8.15 (brs, 2H), 7.96 (d, J=1.6 Hz, 1H), 7.49 (s, 1H), 7.30-7.42 (m,

2H), 7.25 (d, J=3.3 Hz, 1H), 7.20 (m, 1H), 6.75 (dd, J=3.3 Hz, 1.6 Hz, 1H), 4.50 (d, J=6.3 Hz, 2H)
Mass (m/z): 371 ($M^+$+1)
IR (KBr, $cm^{-1}$): 1670, 1653, 1637, 1616, 1606, 1576, 1560, 1508, 1473, 1423, 1221

| Elemental Analysis: as $C_{17}H_{12}F_2N_6O_2$ | | | |
|---|---|---|---|
| Observed | C 55.50%, | H 3.27%, | N 22.90% |
| Calculated | C 55.14%, | H 3.27%, | N 22.69% |

Melting point: 254.5 - 254.9°C

Example 83

5-Amino-2-(2-furyl)[1,2,4]triazolo[1,5-c]pyrimidine-7-carboxylic acid (3-pyridylmethyl)amide (Compound 83)

The reaction was carried out in a manner similar to that in Example 77 except that N-acetylethylenediamine was replaced by 3-picolylamine. Then, the resulting crude product was recrystallized from a mixed solvent of ethanol (15 mL) and DMF (2 mL) to obtain Compound 83 (450 mg, 1.34 mmol) as white crystals in a yield of 66%.
$^1$H NMR (δ ppm, DMSO-$d_6$): 8.88 (t, J=8.3 Hz, 1H), 8.59 (d, J=1.6 Hz, 1H), 8.48 (dd, J=4.9 Hz, 1.6 Hz, 1H), 8.15 (brs, 2H), 7.96 (d, J=1.6 Hz, 1H), 7.77 (d, J=8.0 Hz, 1H), 7.51 (s, 1H), 7.37 (dd, J=8.0 Hz, 4.9 Hz, 1H), 7.25 (d, J=3.3 Hz, 1H), 6.74 (dd, J=3.3 Hz, 1.6 Hz, 1H), 4.56 (d, J=8.3 Hz, 2H)
Mass (m/z): 335 ($M^+$)
IR (KBr, $cm^{-1}$): 1668, 1655, 1608, 1567, 1527, 1510, 1498, 1331, 762

| Elemental Analysis: as $C_{16}H_{13}N_7O_2$ | | | |
|---|---|---|---|
| Observed | C 56.98%, | H 3.99%, | N 29.59% |
| Calculated | C 57.31%, | H 3.91%, | N 29.24% |

Melting point: 229.7 - 230°C

Example 84

5-Amino-2-(2-furyl)[1,2,4]triazolo[1,5-c]pyrimidine-7-carboxylic acid [2-(2-pyridyl)ethyl]amide (Compound 84)

The reaction was carried out in a manner similar to that in Example 77 except that N-acetylethylenediamine was replaced by 2-(2-aminoethyl)pyridine. Then, the resulting crude product was recrystallized from a mixed solvent of ethanol (12 mL) and DMF (2 mL) to obtain Compound 84 (524 mg, 1.50 mmol) as white crystals in a yield of 74%.
$^1$H NMR (δ ppm, DMSO-$d_6$): 8.55-8.65 (m, 2H), 8.16 (brs, 2H), 7.95 (d, J=2.0 Hz, 1H), 7.72 (dd, J=7.6 Hz, 5.6 Hz, 1H), 7.46 (s, 1H), 7.25-7.30 (m, 2H), 7.24 (d, J=3.3 Hz, 1H), 6.74 (dd, J=3.3 Hz, 2.0 Hz, 1H), 3.71 (dt, J=6.9 Hz, 6.3 Hz, 2H), 3.03 (t, J=6.9 Hz, 2H)
Mass (m/z): 350 ($M^+$+1)
IR (KBr, $cm^{-1}$): 1655, 1645, 1623, 1606, 1576, 1535

| Elemental Analysis: as $C_{17}H_{15}N_7O_2 \cdot 0.5H_2O$ | | | |
|---|---|---|---|
| Observed | C 57.09%, | H 4.51%, | N 27.23% |
| Calculated | C 56.98%, | H 4.50%, | N 27.36% |

Melting point: 200.3 - 200.5°C

Example 85

5-Amino-2-(2-furyl)[1,2,4]triazolo[1,5-c]pyrimidine-7-carboxylic acid {N-[2-(2-pyridyl)ethyl]-N-methyl}amide (Compound 85)

The reaction was carried out in a manner similar to that in Example 77 except that N-acetylethylenediamine was replaced by N-methyl-2-(2-pyridyl)ethylamine. Then, the resulting crude product was recrystallized from ethanol

(6 mL) to obtain Compound 85 (379 mg, 1.25 mmol) as white crystals in a yield of 76%.
$^1H$ NMR (δ ppm, DMSO-$d_6$): 8.54 (d, J=4.3 Hz, 0.5H), 8.33 (d, J=4.2 Hz, 0. 5H), 8.18 (brs, 2H), 7.95 (d, J=1.6 Hz, 1H), 7.75 (dd, J=7.6 Hz, 5.9 Hz, 0.5H), 7.62 (dd, J=7.6 Hz, 5.9 Hz, 0.5H), 7.36 (d, J=7.6 Hz, 0.5H), 7.10-7.30 (m, 2.5H), 6.92 (s, 0.5H), 6.74 (dd, J=3.3 Hz, 1.6 Hz, 1H), 6.67 (s, 0.5H), 3.74 (t, J=7.6 Hz, 1H), 3.67 (t, J=7.6 Hz, 1H), 3.05 (t, J=7. 6 Hz, 2H), 2.98 (s, 1.5H), 2.92 (s, 1.5 H)
Mass (m/z): 364 ($M^+$+1)
IR (KBr, $cm^{-1}$): 1653, 1637, 1623, 1616, 1610, 1560, 1508, 1500

| Elemental Analysis: as $C_{18}H_{17}N_7O_2 \cdot 0.5\ H_2O$ | | | |
|---|---|---|---|
| Observed | C 58.19%, | H 4.77%, | N 26.34% |
| Calculated | C 58.06%, | H 4.87%, | N 26.33% |

Melting point: 151.5 - 152.0°C

Example 86

5-Amino-2-(2-furyl)[1,2,4]triazolo[1,5-c]pyrimidine-7-carboxylic acid (2-methoxyethyl)amide (Compound 86)

The reaction was carried out in a manner similar to that in Example 77 except that N-acetylethylenediamine was replaced by 2-methoxyethylamine. Then, the resulting crude product was recrystallized from ethanol (6 mL) to obtain Compound 86 (231 mg, 0.764 mmol) as white crystals in a yield of 47%.
$^1H$ NMR (δ ppm, DMSO-$d_6$): 8.15-8.30 (m, 3H), 7.95 (d, J=1.6 Hz, 1H), 7.47 (s, 1H), 7.24 (d, J=3.3 Hz, 1H), 6.74 (dd, J=3.3 Hz, 1.6 Hz, 1H), 3.45-3.60 (m, 4H), 3.32 (s, 3H)
Mass (m/z): 303 ($M^+$+1)
IR (KBr, $cm^{-1}$): 1683, 1670, 1662, 1653, 1647, 1601, 1560, 1541, 1508, 1419

| Elemental Analysis: as $C_{13}H_{14}N_6O_3$ | | | |
|---|---|---|---|
| Observed | C 51.69%, | H 4.76%, | N 27.83% |
| Calculated | C 51.65%, | H 4.67%, | N 27.80% |

Melting point: 163.4 - 163.6°C

Example 87

5-Amino-2-(2-furyl)[1,2,4]triazolo[1,5-c]pyrimidine-7-carboxylic acid [N-(2-methoxyethyl)-N-methyl]amide (Compound 87)

The reaction was carried out in a manner similar to that in Example 77 except that N-acetylethylenediamine was replaced by N-(2-methoxy-1-ethyl)-N-methylamine. Then, the resulting crude product was recrystallized from ethanol (2 mL) to obtain Compound 87 (318 mg, 1.01 mmol) as white crystals in a yield of 62%.
$^1H$ NMR (δ ppm, DMSO-$d_6$): 8.19 (brs, 2H), 7.95 (d, J=1.6 Hz, 1H), 7.23 (d, J=3.3 Hz, 1H), 7.00 (s, 1H), 6.74 (dd, J=3.3 Hz, 1.6 Hz, 1H), 3.55-3.60 (m, 2H), 3.40-3.50 (m, 2H), 3.30 (s, 1.5H), 3.21 (s, 1.5H), 2.99 (s, 3H)
Mass (m/z): 317 ($M^+$+1)
IR (KBr, $cm^{-1}$): 1662, 1641, 1599, 1560, 1458, 1425, 1325, 1122

| Elemental Analysis: as $C_{14}H_{16}N_6O_3 \cdot 0.1\ H_2O$ | | | |
|---|---|---|---|
| Observed | C 53.01%, | H 5.17%, | N 26.90% |
| Calculated | C 53.16%, | H 5.10%, | N 26.57% |

Melting point: 143.5 - 143.8°C

Example 88

5-Amino-2-(2-furyl)[1,2,4]triazolo[1,5-c]pyrimidine-7-carboxylic acid [N,N-bis(2-methoxyethyl)]amide (Compound 88)

The reaction was carried out in a manner similar to that in Example 77 except that N-acetylethylenediamine was replaced by N,N-bis(2-methoxyethyl)amine. Then, the resulting crude product was recrystallized from ethanol (8 mL) to obtain Compound 88 (365 mg, 1.01 mmol) as white crystals in a yield of 62%.
$^1$H NMR (δ ppm, DMSO-$d_6$): 8.20 (brs, 2H), 7.95 (d, J=1.6 Hz, 1H), 7.22 (d, J=3.3 Hz, 1H), 6.99 (s, 1H), 6.74 (dd, J=3.3 Hz, 1.6 Hz, 1H), 3.60 (t, J=5.5 Hz, 2H), 3.40-3.50 (m, 6H), 3.29 (s, 3H), 3.18 (s, 3H)
Mass (m/z): 361 ($M^+$+1)
IR (KBr, $cm^{-1}$): 1670, 1652, 1635, 1608, 1569, 1506, 1466, 1452, 1419, 1120

| Elemental Analysis: as $C_{16}H_{20}N_6O_4$ | | | |
|---|---|---|---|
| Observed | C 53.45%, | H 5.86%, | N 23.47% |
| Calculated | C 53.33%, | H 5.59%, | N 23.32% |

Melting point: 190.5 - 190.8°C

Example 89

5-Amino-2-(2-furyl)[1,2,4]triazolo[1,5-c]pyrimidine-7-carboxylic acid isopropylamide (Compound 89)

The reaction was carried out in a manner similar to that in Example 77 except that N-acetylethylenediamine was replaced by isopropylamine. Then, the resulting crude product was recrystallized from ethanol (20 mL) to obtain Compound 89 (166 mg, 0.579 mmol) as white crystals in a yield of 36%.
$^1$H NMR (δ ppm, DMSO-$d_6$): 8.19 (brs, 2H), 7.98 (d, J=8.6 Hz, 1H), 7.95 (d, J=1.6 Hz, 1H), 7.45 (s, 1H), 7.24 (d, J=3.3 Hz, 1H), 6.74 (dd, J=3.3 Hz, 1.6 Hz, 1H), 4.06 (m, 1H), 1.20 (d, J=6.6 Hz, 6H)
Mass (m/z): 287 ($M^+$+1)
IR (KBr, $cm^{-1}$): 1672, 1653, 1641, 1594, 1540, 1527, 1508

| Elemental Analysis: as $C_{13}H_{14}N_6O_2 \cdot 0.3H_2O$ | | | |
|---|---|---|---|
| Observed | C 53.45%, | H 4.75%, | N 28.52% |
| Calculated | C 53.53%, | H 5.04%, | N 28.81% |

Melting point: 221.6 - 221.9°C

Example 90

5-Amino-2-(2-furyl)[1,2,4]triazolo[1,5-c]pyrimidine-7-carboxylic acid [3-(1-imidazolyl)propyl]amide (Compound 90)

The reaction was carried out in a manner similar to that in Example 77 except that N-acetylethylenediamine was replaced by 3-(1-imidazolyl)propylamine. Then, the resulting crude product was recrystallized from ethanol (20 mL) to obtain Compound 90 (197 mg, 0.558 mmol) as white crystals in a yield of 36%.
$^1$H NMR (δ ppm, DMSO-$d_6$): 8.27 (t, J=6.6 Hz, 1H), 7.80-7.90 (m, 3H), 7.63 (s, 1H), 7.47 (s, 1H), 7.21 (d, J=3.3 Hz, 1H), 7.17 (s, 1H), 6.89 (s, 1H), 6.71 (dd, J=3.3 Hz, 2.0 Hz, 1H), 4.03 (t, J=7.0 Hz, 2H), 3.32 (dt, J=6.6 Hz, 6.6 Hz, 2H), 2.01 (tt, J=7.0 Hz, 6.6 Hz, 2H)
Mass (m/z): 353 ($M^+$+1)
IR (KBr, $cm^{-1}$): 1662, 1657, 1649, 1616, 1566, 1531, 1508, 1223

| Elemental Analysis: as $C_{16}H_{16}N_8O_2 \cdot 0.8H_2O$ | | | |
|---|---|---|---|
| Observed | C 52.49%, | H 4.92%, | N 30.49% |
| Calculated | C 52.40%, | H 4.84%, | N 30.55% |

Melting point: 198.5 - 198.8°C

Example 91

5-Amino-2-(2-furyl)[1,2,4]triazolo[1,5-c]pyrimidine-7-carboxylic acid benzylamide (Compound 91)

The reaction was carried out in a manner similar to that in Example 77 except that N-acetylethylenediamine was replaced by benzylamine. Then, the resulting crude product was recrystallized from ethanol (13 mL) to obtain Compound 91 (364 mg, 1.08 mmol) as white crystals in a yield of 66%.
$^1$H NMR (δ ppm, DMSO-$d_6$): 8.71 (t, J=6.3 Hz, 1H), 8.16 (brs, 2H), 7.95 (d, J=1.7 Hz, 1H), 7.49 (s, 1H), 7.30-7.40 (m, 5H), 7.25 (d, J=3.3 Hz, 1H), 6.74 (dd, J=3.3 Hz, 1.7 Hz, 1H), 4.52 (d, J=6.3 Hz, 2H)
Mass (m/z): 335 ($M^+$+1)
IR (KBr, $cm^{-1}$): 1645, 1606, 1560, 1539, 1417, 1221, 761, 746

| Elemental Analysis: as $C_{17}H_{14}N_6O_2 \cdot 0.1H_2O$ | | | |
|---|---|---|---|
| Observed | C 60.82%, | H 4.22%, | N 25.21% |
| Calculated | C 60.74%, | H 4.26%, | N 25.00% |

Melting point: 224.9 - 225.1°C

Example 92

5-Amino-2-(2-furyl)[1,2,4]triazolo[1,5-c]pyrimidine-7-carboxylic acid [3-(2-oxopyridin-1-yl)propyl]amide (Compound 92)

The reaction was carried out in a manner similar to that in Example 77 except that N-acetylethylenediamine was replaced by 1-(3-aminopropyl)-2-pyrolidone. Then, the resulting crude product was recrystallized from ethanol (10 mL) to obtain Compound 92 (409 mg, 1.11 mmol) as white crystals in a yield of 54%.
$^1$H NMR (δ ppm, DMSO-$d_6$): 8.38 (t, J=6.3 Hz, 1H), 8.16 (brs, 2H), 7.96 (d, J=1.6 Hz, 1H), 7.47 (s, 1H), 7.25 (d, J=3.3 Hz, 1H), 6.74 (dd, J=3.3 Hz, 1.6 Hz, 1H), 3.20-3.40 (m, 6H), 2.24 (t, J=8.3 Hz, 2H), 1.93 (m, 2H), 1.73 (m, 2H)
Mass (m/z): 369 ($M^+$+1)
IR (KBr, $cm^{-1}$): 1668, 1650, 1608, 1539, 1511, 1417, 744

| Elemental Analysis: as $C_{17}H_{19}N_7O_3$ | | | |
|---|---|---|---|
| Observed | C 55.22%, | H 5.28%, | N 26.68% |
| Calculated | C 55.28%, | H 5.18%, | N 26.54% |

Melting point: 181.5 - 182.2°C

Example 93

5-Amino-2-(2-furyl)[1,2,4]triazolo[1,5-c]pyrimidine-7-carboxylic acid cyclohexylmethylamide (Compound 93)

The reaction was carried out in a manner similar to that in Example 77 except that N-acetylethylenediamine was replaced by cyclohexylmethylamine. Then, the resulting crude product was recrystallized from ethanol (6 mL) to obtain Compound 93 (194 mg, 0.571 mmol) as white crystals in a yield of 35%.
$^1$H NMR (δ ppm, DMSO-$d_6$): 8.24 (t, J=6.2 Hz, 1H), 8.18 (brs, 2H), 7.95 (d, J=1.6 Hz, 1H), 7.45 (s, 1H), 7.24 (d, J=3.3 Hz, 1H), 6.74 (dd, J=3.3 Hz, 1.6 Hz, 1H), 3.18 (dd, J=6.6 Hz, 6.2 Hz, 2H), 1.40-1.80 (m, 6H), 1.05-1.30 (m, 3H), 0.80-1.00 (m, 2H)
Mass (m/z): 341 ($M^+$+1)
IR (KBr, $cm^{-1}$): 1664, 1641, 1602, 1538, 1510, 1321

| Elemental Analysis: as $C_{17}H_{20}N_6O_2$ | | | |
|---|---|---|---|
| Observed | C 59.92%, | H 6.04%, | N 24.64% |
| Calculated | C 59.99%, | H 5.92%, | N 24.69% |

Melting point: 273.5 - 274.0°C

Example 94

5-Amino-2-(2-furyl)[1,2,4]triazolo[1,5-c]pyrimidine-7-carboxylic acid (1,2,3,4-tetrahydroisoquinolyl)amide (Compound 94)

The reaction was carried out in a manner similar to that in Example 77 except that N-acetylethylenediamine was replaced by 1,2,3,4-tetradydroisoquinoline. Then, the resulting crude product was recrystallized from ethanol (12 mL) to obtain Compound 94 (404 mg, 1.12 mmol) as white crystals in a yield of 55%.
$^1$H NMR (δ ppm, DMSO-$d_6$): 8.20 (brs, 2H), 7.95 (d, J=2.0 Hz, 1H), 7.00-7. 25 (m, 6H), 6.74 (dd, J=3.3 Hz, 2.0 Hz, 1H), 4.77 (s, 1.2H), 4.63 (s, 0. 8H), 3.80-3.90 (m, 0.8H), 3.60-3.70 (m, 1.2H), 2.85-2.88 (m, 2H)
Mass (m/z): 360 ($M^+$)
IR (KBr, $cm^{-1}$): 1653, 1646, 1633, 1506, 1456, 1221

| Elemental Analysis: as $C_{19}H_{16}N_6O_2 \cdot 0.3\ H_2O$ | | | |
|---|---|---|---|
| Observed | C 62.35%, | H 4.27%, | N 23.17% |
| Calculated | C 62.39%, | H 4.57%, | N 22.98% |

Melting point: 203.0 - 203.2°C

Example 95

5-Amino-2-(2-furyl)[1,2,4]triazolo[1,5-c]pyrimidine-7-carboxylic acid [4-(2-methoxyethyl)piperazinyl]amide (Compound 95)

The reaction was carried out in a manner similar to that in Example 77 except that N-acetylethylenediamine was replaced by 4-(2-methoxyethyl)piperazine. Then, the resulting crude product was recrystallized from ethanol (5 mL) to obtain Compound 95 (325 mg, 0.875 mmol) as white crystals in a yield of 43%.
$^1$H NMR (δ ppm, DMSO-$d_6$): 8.19 (brs, 2H), 7.95 (d, J=1.7 Hz, 1H), 7.23 (d, J=3.3 Hz, 1H), 7.02 (s, 1H), 6.74 (dd, J=3.3 Hz, 1.7 Hz, 1H), 3.61 (m, 2H), 3.30-3.50 (m, 4H), 3.23 (s, 3H), 2.40-2.60 (m, 6H)
Mass (m/z): 371 ($M^+$+1), 326
IR (KBr, $cm^{-1}$): 1654, 1606, 1479, 1467, 1421, 1330

| Elemental Analysis: as $C_{17}H_{21}N_7O_3$ | | | |
|---|---|---|---|
| Observed | C 54.75%, | H 5.92%, | N 26.28% |
| Calculated | C 54.98%, | H 5.70%, | N 26.40% |

Melting point: 211.8 - 212.0°C

Example 96

5-Amino-2-(2-furyl)[1,2,4]triazolo[1,5-c]pyrimidine-7-carboxylic acid (4-cyclopropylmethyl)piperazinylamide (Compound 96)

First, 5-amino-2-(2-furyl)[1,2,4]triazolo[1,5-c]pyrimidine-7-carboxylic acid (500 mg, 2.04 mmol) produced in Example 77 was dissolved in DMF (50 mL), and 4-cyclopropylmethylpiperazine (429 mg, 3.06 mmol), 1-ethyl-3-[3-(dimethylamino)propyl]carbodiimide monohydrochloride (782 mg, 4.08 mmol), and 1-hydroxybenzotriazole (312 mg, 2.04 mmol) were added to the resulting solution, followed by stirring at room temperature for 12 hours. Then, THF (30 mL), acid chloride resin [3.0 g, the acid chloride resin being prepared by the method described in the document (Tetrahedron Letters, Vol. 37, No. 40, p. 7193 (1996))] and polyvinylpyridine resin (produced by Aldrich Co., 3.0 g) were added to the reaction solution, followed by further stirring at room temperature for 12 hours. The resins were filtered off from the reaction solution, and the filtrate was concentrated. The residue was recrystallized from a mixed solvent of ethanol (10 mL) and DMF (10 mL) to obtain Compound 96 (420 mg, 1.14 mmol) as white crystals in a yield of 56%.
$^1$H NMR (δ ppm, DMSO-$d_6$): 8.18 (brs, 2H), 7.95 (d, J=1.7 Hz, 1H), 7.22 (d, J=3.3 Hz, 1H), 7.00 (s, 1H), 6.73 (dd, J=3.3 Hz, 1.7 Hz, 1H), 3.50-3.65 (m, 2H), 3.40-3.45 (m, 2H), 2.40-2.50 (m, 4H), 2.21 (d, J=6.6 Hz, 2H), 0.83 (m, 1H), 0.43-0.49 (m, 2H), 0.06-0.08 (m, 2H)

Mass (m/z): 368 ($M^+$+1)
IR (KBr, $cm^{-1}$): 1656, 1641, 1633, 1616, 1610, 1558, 1003

| Elemental Analysis: as $C_{18}H_{21}N_7O_2 \cdot 0.4\ H_2O$ | | | |
|---|---|---|---|
| Observed | C 57.56%, | H 5.95%, | N 26.59% |
| Calculated | C 57.71%, | H 5.87%, | N 26.17% |

Melting point: 299.2 - 299.8°C

Example 97

5-Amino-7-[1-(2,2,2-trifluoroethyl)-1,2,5,6-tetrahydropyridin-4-yl]-2-(2-furyl)[1,2,4]triazolo[1,5-c]pyrimidine (Compound 97)

The reaction was carried out in a manner similar to that in Example 73 except that 2-methoxy-1-bromoethane was replaced by 2,2,2-trifluoroethyl trifluoromethanesulfonate. Then, the resulting residue was purified by silica gel column chromatography (elution solvent: mixed solvent of chloroform and methanol). The resulting white solid was recrystallized from a mixed solvent of ethanol (11 mL) and methanol (3 mL) to obtain Compound 97 (300 mg, 0.824 mmol) as yellow crystals in a yield of 47%.
$^1H$ NMR (δ ppm, DMSO-$d_6$): 7.93 (d, J=1.0 Hz, 1H), 7.83 (brs, 2H), 7.18 (d, J=3.3 Hz, 1H), 6.94 (s, 1H), 6.86 (m, 1H), 6.72 (dd, J=3.3 Hz, 1.0 Hz, 1H), 3.20-3.43 (m, 6H), 2.80-2.95 (m, 2H)
Mass (m/z): 365 ($M^+$+1)
IR (KBr, $cm^{-1}$): 1664, 1598, 1551, 1512, 1429, 1331, 1272, 1145

| Elemental Analysis: as $C_{16}H_{15}N_6F_3O$ | | | |
|---|---|---|---|
| Observed | C 52.67%, | H 4.15%, | N 22.92% |
| Calculated | C 52.75%, | H 4.15%, | N 23.07% |

Melting point: 247.5 - 248.2°C (decomposed)

Example 98

5-Amino-2-(2-furyl)[1,2,4]triazolo[1,5-c]pyrimidine-7-carboxylic acid ethylamide (Compound 98)

The reaction was carried out in a manner similar to that in Example 77 except that N-acetylethylenediamine was replaced by ethylamine. Then, the resulting crude product was recrystallized from ethanol (3 mL) to obtain Compound 98 (193 mg, 0.709 mmol) as white crystals in a yield of 50%.
$^1H$ NMR (δ ppm, DMSO-$d_6$): 8.27 (t, J=6.0 Hz, 1H), 8.15 (brs, 2H), 7.95 (d, J=1.0 Hz, 1H), 7.46 (s, 1H), 7.24 (d, J=3.3 Hz, 1H), 6.74 (dd, J=3.3 Hz, 1.0 Hz, 1H), 3.34 (m, 2H), 1.15 (t, J=7.0 Hz, 3H)
Mass (m/z): 273 ($M^+$+1)
IR (KBr, $cm^{-1}$): 1672, 1653, 1641, 1594, 1540, 1527, 1508

| Elemental Analysis: as $C_{12}H_{12}N_6O_2 \cdot 0.2H_2O$ | | | |
|---|---|---|---|
| Observed | C 52.36%, | H 4.55%, | N 30.19% |
| Calculated | C 52.25%, | H 4.53%, | N 30.46% |

Melting point: 178.5 - 178.9°C

Example 99

5-Amino-2-(2-furyl)[1,2,4]triazolo[1,5-c]pyrimidine-7-carboxylic acid (N-benzyl-N-ethyl)amide (Compound 99)

The reaction was carried out in a manner similar to that in Example 77 except that N-acetylethylenediamine was replaced by N-ethylbenzylamine. Then, the resulting crude product was recrystallized from a mixed solvent of hexane and ethyl acetate to obtain Compound 99 (195 mg, 0.539 mmol) as light yellow crystals in a yield of 22%.

$^1$H NMR (δ ppm, DMSO-$d_6$, 100°C): 7.85 (dd, J=1.8 Hz, 0.9 Hz, 1H), 7.81 (brs, 2H), 7.22-7.40 (m, 5H), 7.17 (dd, J=0.9 Hz, 3.4 Hz, 1H), 7.02 (s, 1H), 6.67 (dd, J=3.4 Hz, 1.8 Hz, 1H), 4.63 (s, 2H), 3.34 (q, J=7.0 Hz, 2H), 1.08 (t, J=7.0 Hz, 3H)

Example 100

5-Amino-2-(2-furyl)[1,2,4]triazolo[1,5-c]pyrimidine-7-carboxylic acid (N-benzyl-N-isopropyl)amide (Compound 100)

The reaction was carried out in a manner similar to that in Example 77 except that N-acetylethylenediamine was replaced by N-isopropylbenzylamine. Then, the resulting crude product was recrystallized from a mixed solvent of hexane and ethyl acetate to obtain Compound 100 (150 mg, 0.466 mmol) as light yellow crystals in a yield of 19%.
$^1$H NMR (δ ppm, DMSO-$d_6$, 100°C): 7.85 (dd, J=1.8 Hz, 0.9 Hz, 1H), 7.81 (brs, 2H), 7.18-7.37 (m, 5H), 7.16 (dd, J=0.9 Hz, 3.4 Hz, 1H), 6.99 (s, 1H), 6.68 (dd, J=3.4 Hz, 1.8 Hz, 1H), 4.60 (s, 2H), 4.21 (brs, 1H), 1.13 (d, J=6.6 Hz, 6H)

Example 101

5-Amino-2-(2-furyl)[1,2,4]triazolo[1,5-c]pyrimidine-7-carboxylic acid [N-(2-furylmethyl)-N-methyl]amide (Compound 101)

The reaction was carried out in a manner similar to that in Example 77 except that N-acetylethylenediamine was replaced by N-methylfurfurylamine. Then, the resulting crude product was recrystallized from a mixed solvent of hexane and ethyl acetate to obtain Compound 101 (199 mg, 0.744 mmol) as white crystals in a yield of 61%.
$^1$H NMR (δ ppm, DMSO-$d_6$, 100°C): 7.85 (d, J=1.7 Hz, 1H), 7.17 (d, J=3.4 Hz, 1H), 7.01 (s, 1H), 6.68 (dd, J=3.4 Hz, 1.7 Hz, 1H), 6.39 (s, 1H), 6.35 (brs, 1H), 4.64 (s, 2H), 2.95 (s, 3H)

Example 102

5-Amino-2-(2-furyl)[1,2,4]triazolo[1,5-c]pyrimidine-7-carboxylic acid [N-ethyl-N-(4-pyridylmethyl)]amide (Compound 102)

The reaction was carried out in a manner similar to that in Example 77 except that N-acetylethylenediamine was replaced by N-ethyl-4-pyridylmethylamine. Then, the resulting crude product was recrystallized from a mixed solvent of methanol and ethyl acetate to obtain Compound 102 (332 mg, 0.941 mmol) as white crystals in a yield of 48%.
$^1$H NMR (δ ppm, DMSO-$d_6$, 80°C): 8.55 (brs, 1H), 8.49 (d, J=4.5 Hz, 1H), 7.98 (brs, 2H), 7.88 (d, J=1.8 Hz, 1H), 7.78 (d, J=7.5 Hz, 1H), 7.36 (dd, J=7.5 Hz, 4.5 Hz, 1H), 7.19 (d, J=3.4 Hz, 1H), 7.10 (s, 1H), 6.69 (dd, J=3.4 Hz, 1.8 Hz, 1H), 4.74 (s, 2H), 3.64 (brs, 2H), 2.89 (brs, 2H)

Example 103

5-Amino-2-(2-furyl)[1,2,4]triazolo[1,5-c]pyrimidine-7-carboxylic acid [N-(2-cyanoethyl)-N-(3-pyridylmethyl)]amide (Compound 103)

The reaction was carried out in a manner similar to that in Example 77 except that N-acetylethylenediamine was replaced by 3-(3-pyridylmethylamino)propionitrile. Then, the resulting crude product was recrystallized from a mixed solvent of methanol and ethyl acetate to obtain Compound 103 (274 mg, 0.699 mmol) as white crystals in a yield of 29%.
$^1$H NMR (δ ppm, DMSO-$d_6$, 80°C): 8.57 (brs, 1H), 8.49 (d, J=4.5 Hz, 1H), 7.98 (brs, 2H), 7.88 (d, J=1.8 Hz, 1H), 7.78 (d, J=7.5 Hz, 1H), 7.36 (dd, J=7.5 Hz, 4.5 Hz, 1H), 7.19 (d, J=3.4 Hz, 1H), 7.10 (s, 1H), 6.69 (dd, J=3.4 Hz, 1.8 Hz, 1H), 4.75 (s, 2H), 3.64 (brs, 2H), 2.89 (brs, 2H)

Example 104

5-Amino-2-(2-furyl)[1,2,4]triazolo[1,5-c]pyrimidine-7-carboxylic acid [N-methyl-N-(2-pyridylmethyl)]amide (Compound 104)

The reaction was carried out in a manner similar to that in Example 77 except that N-acetylethylenediamine was replaced by N-methyl-2-pyridylmethylamine. Then, the resulting crude product was recrystallized from a mixed solvent of hexane and ethyl acetate to obtain Compound 104 (329 mg, 0.976 mmol) as white crystals in a yield of 80%.
$^1$H NMR (δ ppm, DMSO-$d_6$, 80°C): 8.52 (brs, 1H), 7.92 (brs, 2H), 7.87 (brs, 1H), 7.76 (brs, 1H), 7.35 (d, J=7.9 Hz, 1H),

7.23-7.30 (m, 1H), 7.17 (brs, 1H), 7.04 (s, 1H), 6.68 (dd, J=3.4 Hz, 1.8 Hz, 1H), 4.72 (s, 2H), 3.00 (s, 3H)

Example 105

5-Amino-2-(2-furyl)[1,2,4]triazolo[1,5-c]pyrimidine-7-carboxylic acid [N-methyl-N-(3-pyridylmethyl)]amide (Compound 105)

The reaction was carried out in a manner similar to that in Example 77 except that N-acetylethylenediamine was replaced by N-methyl-3-pyridylmethylamine. Then, the resulting crude product was recrystallized from a mixed solvent of methanol and ethyl acetate to obtain Compound 105 (201 mg, 0.598 mmol) as white crystals in a yield of 31%.
$^1$H NMR (δ ppm, DMSO-$d_6$, 80°C): 8.54 (brs, 1H), 8.49 (brs, 1H), 7.92 (brs, 2H), 7.87 (d, J=1.7 Hz, 1H), 7.76 (d, J=6.6 Hz, 1H), 7.37 (brs, 1H), 7.18 (d, J=3.3 Hz, 1H), 7.06 (s, 1H), 6.69 (dd, J=3.3 Hz, 1.7 Hz, 1H), 4.66 (s, 2H), 2.93 (s, 3H)

Example 106

5-Amino-2-(2-furyl)[1,2,4]triazolo[1,5-c]pyrimidine-7-carboxylic acid [N-methyl-N-(4-pyridylmethyl)]amide (Compound 106)

The reaction was carried out in a manner similar to that in Example 77 except that N-acetylethylenediamine was replaced by N-methyl-4-pyridylmethylamine. Then, the resulting crude product was recrystallized from a mixed solvent of methanol and ethyl acetate to obtain Compound 106 (374 mg, 1.11 mmol) as white crystals in a yield of 66%.
$^1$H NMR (δ ppm, DMSO-$d_6$, 80°C): 8.53 (brs, 2H), 7.92 (brs, 2H), 7.87 (brs, 1H), 7.31 (d, J=5.0 Hz, 2H), 7.17 (brs, 1H), 7.09 (s, 1H), 6.69 (brs, 1H), 4.66 (s, 2H), 2.95 (s, 3H)

Example 107

5-Amino-2-(2-furyl)[1,2,4]triazolo[1,5-c]pyrimidine-7-carboxylic acid [N-(1,3-benzodioxol-5-ylmethyl)-N-methyl]amide monohydrochloride (Compound 107)

The reaction was carried out in a manner similar to that in Example 77 except that N-acetylethylenediamine was replaced by N-methyl4-(1,2-methylenedioxy)benzylamine. Then, an ethyl acetate solution of hydrogen chloride was added to the resulting crude product. The precipitated crystals were recrystallized from a mixed solvent of ethanol and ethyl acetate to obtain Compound 107 (442 mg, 1.02 mmol) as white crystals in a yield of 84%.
$^1$H NMR (δ ppm, DMSO-$d_6$, 80°C): 7.90 (brs, 2H), 7.89 (d, J=1.8 Hz, 1H), 7.20 (d, J=3.3 Hz, 1H), 7.07 (s, 1H), 6.75-6.78 (m, 3H), 6.71 (dd, J=3.3 Hz, 1.8 Hz, 1H), 6.00 (s, 2H), 4.54 (s, 2H)

Example 108

5-Amino-2-(2-furyl)[1,2,4]triazolo[1,5-c]pyrimidine-7-caroboxylic acid [N-(4-methoxybenzyl)-N-methyl]amide monohydrochloride (Compound 108)

The reaction was carried out in a manner similar to that in Example 77 except that N-acetylethylenediamine was replaced by N-methyl-4-methoxybenzylamine. Then, an ethyl acetate solution of hydrogen chloride was added to the resulting crude product. The precipitated crystals were recrystallized from a mixed solvent of ethanol and ethyl acetate to obtain Compound 108 (381 mg, 0.915 mmol) as white crystals in a yield of 75%.
$^1$H NMR (δ ppm, DMSO-$d_6$, 80°C): 7.90 (brs, 2H), 7.87 (d, J=1.8 Hz, 1H), 7.25 (d, J=7.7 Hz, 2H), 7.18 (d, J=3.5 Hz, 1H), 7.04 (s, 1H), 6.91 (dd, J=7.7 Hz, 2H), 6.69 (dd, J=3.5 Hz, J=1.8 Hz, 1H), 4.54 (s, 2H), 3.75 (s, 3H)

Example 109

5-Amino-2-(2-furyl)[1,2,4]triazolo[1,5-c]pyrimidine-7-carboxylic acid [N-(3-methoxybenzyl)-N-methyl]amide monohydrochloride (Compound 109)

The reaction was carried out in a manner similar to that in Example 77 except that N-acetylethylenediamine was replaced by N-methyl-3-methoxybenzylamine. Then, an ethyl acetate solution of hydrogen chloride was added to the resulting crude product. The precipitated crystals were recrystallized from a mixed solvent of ethanol and ethyl acetate to obtain Compound 109 (292 mg, 0.708 mmol) as white crystals in a yield of 58%.
$^1$H NMR (δ ppm, DMSO-$d_6$, 80°C): 7.89 (brs, 2H), 7.87 (d, J=1.8 Hz, 1H), 7.22-7.30 (m, 1H), 7.18 (d, J=3.5 Hz, 1H),

7.04 (s, 1H), 6.75-6.95 (m, 3H), 6.68 (dd, J=3.5 Hz, J=1.8 Hz, 1H), 4.60 (s, 2H), 3.76 (s, 3H)

Example 110

5-Amino-2-(2-furyl)[1,2,4]triazolo[1,5-c]pyrimidine-7-carboxylic acid [N-(2-methoxybenzyl)-N-methyl]amide monohydrochloride (Compound 110)

The reaction was carried out in a manner similar to that in Example 77 except that N-acetylethylenediamine was replaced by N-methyl-2-methoxybenzylamine. Then, an ethyl acetate solution of hydrogen chloride was added to the resulting crude product. The precipitated crystals were recrystallized from a mixed solvent of ethanol and ethyl acetate to obtain Compound 110 (233 mg, 0.561 mmol) as white crystals in a yield of 46%.
$^1$H NMR (δ ppm, DMSO-$d_6$, 100°C): 7.85 (d, J=1.8 Hz, 1H), 7.79 (brs, 2H), 7.20-7.30 (m, 2H), 7.17 (d, J=3.5 Hz, 1H), 6.90-7.03 (m, 2H), 6.68 (dd, J=3.5 Hz, J=1.8 Hz, 1H), 4.62 (s, 2H), 3.76 (s, 3H), 2.91 (s, 3H)

Example 111

5-Amino-2-(2-furyl)[1,2,4]triazolo[1,5-c]pyrimidine-7-carboxylic acid [N-methyl-N-(2-quinolylmethyl)]amide monohydrochloride (Compound 111)

The reaction was carried out in a manner similar to that in Example 77 except that N-acetylethylenediamine was replaced by N-methyl-2-quinolylmethylamine. Then, an ethyl acetate solution of hydrogen chloride was added to the resulting crude product. The precipitated crystals were recrystallized from a mixed solvent of ethanol and ethyl acetate to obtain Compound 111 (407 mg, 1.02 mmol) as brown crystals in a yield of 61%.
$^1$H NMR (δ ppm, DMSO-$d_6$, 80°C): 8.54 (brs, 1H), 8.08 (brs, 3H), 7.87 (brs, 2H), 7.65 (brs, 3H), 7.14 (brs, 2H), 6.68 (brs, 1H), 5.02 (s, 2H), 3.10 (brs, 3H)

Example 112

5-Amino-2-(2-furyl)[1,2,4]triazolo[1,5-c]pyrimidine-7-carboxylic acid [N-methyl-N-(3-quinolylmethyl)]amide monohydrochloride (Compound 112)

The reaction was carried out in a manner similar to that in Example 77 except that N-acetylethylenediamine was replaced by N-methyl-3-quinolylmethylamine. Then, an ethyl acetate solution of hydrogen chloride was added to the resulting crude product. The precipitated crystals were recrystallized from ethyl acetate to obtain Compound 112 (647 mg, 1.75 mmol) as light yellow crystals in a yield of 84%.
$^1$H NMR (δ ppm, DMSO-$d_6$, 80°C): 9.07 (s, 1H), 8.65 (s, 1H), 8.16-8.24 (m, 2H), 7.92 (t, J=7.0 Hz, 1H), 7.88 (d, J=1.7 Hz, 1H), 7.76 (t, J=7.0 Hz, 1H), 7.18 (d, J=3.5 Hz, 1H), 7.16 (s, 1H), 6.69 (dd, J=3.5 Hz, 1.7 Hz, 1H), 4.91 (s, 2H), 3.02 (s, 3H)

Example 113

5-Amino-2-(2-furyl)[1,2,4]triazolo[1,5-c]pyrimidine-7-carboxylic acid [N-methyl-N-(4-quinolylmethyl)]amide (Compound 113)

The reaction was carried out in a manner similar to that in Example 77 except that N-acetylethylenediamine was replaced by N-methyl-4-quinolylmethylamine. Then, the resulting crude product was recrystallized from a mixed solvent of methanol and ethyl acetate to obtain Compound 113 (276 mg, 0.592 mmol) as white crystals in a yield of 35%.
$^1$H NMR (δ ppm, DMSO-$d_6$, 80°C): 8.89 (d, J=4.4 Hz, 1H), 8.21 (brs, 1H), 8.04 (brs, 1H), 7.92 (s, 2H), 7.78 (brs, 1H), 7.66 (brs, 1H), 7.45(d, J=4.4 Hz, 1H), 7.17 (brs, 1H), 7.12 (brs, 1H), 6.68 (brs, 1H), 5.20 (s, 3H), 3.02 (s, 3H)

Example 114

5-Amino-2-(2-furyl)[1,2,4]triazolo[1,5-c]pyrimidine-7-carboxylic acid [N-isopropyl-N-(3-pyridylmethyl)]amide (Compound 114)

The reaction was carried out in a manner similar to that in Example 77 except that N-acetylethylenediamine was replaced by N-isopropyl-3-pyridylmethylamine. Then, the resulting crude product was recrystallized from a mixed solvent of ethanol and ethyl acetate to obtain Compound 114 (376 mg, 1.00 mmol) as white crystals in a yield of 49%.

$^1$H NMR (δ ppm, DMSO-$d_6$, 80°C): 8.57 (s, 1H), 8.42 (d, J=3.8 Hz, 1H), 7.93 (s, 2H), 7.87 (brs, 1H), 7.31 (dd, J=7.6 Hz, 3.8 Hz, 1H), 7.18 (d, J=3.3 Hz, 1H), 7.04 (s, 1H), 6.68 (dd, J=3.3 Hz, 1.8 Hz, 1H), 4.62 (s, 2H), 4.15 (brs, 1H), 1.14 (d, J=6.6 Hz, 6H)

Example 115

5-Amino-2-(2-furyl)[1,2,4]triazolo[1,5-c]pyrimidine-7-carboxylic acid [N-methyl-N-(1-methylpyrrol-3-ylmethyl)]amide (Compound 115)

The reaction was carried out in a manner similar to that in Example 77 except that N-acetylethylenediamine was replaced by N-methyl-1-methylpyrrol-3-ylmethylamine. Then, the resulting crude product was recrystallized from ethyl acetate to obtain Compound 115 (411 mg, 1.17 mmol) as white crystals in a yield of 55%.
$^1$H NMR (δ ppm, DMSO-$d_6$, 80°C): 7.91 (s, 2H), 7.88 (d, J=1.1 Hz, 1H), 7.18 (d, J=3.5 Hz, 1H), 7.02 (s, 1H), 6.69 (dd, J=3.5 Hz, 1.1 Hz, 1H), 6.68 (brs, 1H), 6.04 (brs, 1H), 5.93 (dd, J=3.1 Hz, 2.9 Hz, 1H), 4.63 (s, 2H), 3.57 (s, 3H), 2.84 (s, 3H)

Example 116

5-Amino-2-(2-furyl)[1,2,4]triazolo[1,5-c]pyrimidine-7-carboxylic acid [N-isopropyl-N-(1-methylpyrrol-3-ylmethyl)]amide (Compound 116)

The reaction was carried out in a manner similar to that in Example 77 except that N-acetylethylenediamine was replaced by N-isopropyl-1-methylpyrrol-3-ylmethylamine. Then, the resulting crude product was recrystallized from ethyl acetate to obtain Compound 116 (565 mg, 1.50 mmol) as white crystals in a yield of 72%.
$^1$H NMR (δ ppm, DMSO-$d_6$, 80°C): 7.89 (s, 2H), 7.87 (d, J=1.1 Hz, 1H), 7.17 (d, J=3.5 Hz, 1H), 6.95 (s, 1H), 6.69 (dd, J=3.5 Hz, 1.1 Hz, 1H), 6.59 (dd, J=2.9 Hz, 1.7 Hz, 1H), 5.98 (dd, J=3.1 Hz, 1.7 Hz, 1H), 5.88 (dd, J=3.1 Hz, 2.9 Hz, 1H), 4.58 (s, 2H), 4.10 (brs, 1H), 3.56 (s, 3H), 1.11 (d, J=6.6 Hz, 6H)

Example 117

5-Amino-2-(2-furyl)[1,2,4]triazolo[1,5-c]pyrimidine-7-carboxylic acid (N-methyl-N-phenyl)amide (Compound 117)

The reaction was carried out in a manner similar to that in Example 77 except that N-acetylethylenediamine was replaced by N-methylaniline. Then, the resulting crude product was recrystallized from 2-propanol to obtain Compound 117 (131 mg, 0.354 mmol) as white crystals in a yield of 29%.
$^1$H NMR (δ ppm, DMSO-$d_6$, 80°C): 7.85 (d, J=1.7 Hz, 1H), 7.75 (brs, 2H), 7.15-7.33 (m, 5H), 7.13 (d, J=3.3 Hz, 1H), 6.90 (s, 1H), 6.66 (dd, J=3.4 Hz, 1.7 Hz, 1H), 4.54 (s, 2H)

Example 118

5-Amino-2-(2-furyl)[1,2,4]triazolo[1,5-c]pyrimidine-7-carboxylic acid [N-(6-chloropyridin-3-yl)-N-methyl]amide (Compound 118)

The reaction was carried out in a manner similar to that in Example 77 except that N-acetylethylenediamine was replaced by N-methyl-6-chloropyridin-3-ylamine. Then, the resulting crude product was recrystallized from a mixed solvent of methanol and ethyl acetate to obtain Compound 118 (117 mg, 0.319 mmol) as white crystals in a yield of 14%.
$^1$H NMR (δ ppm, DMSO-$d_6$, 80°C) : 8.32 (d, J=2.8 Hz, 1H), 7.87 (d, J=1.7 Hz, 1H), 7.80 (brs, 2H), 7.79 (dd, J=8.6 Hz, J=2.8 Hz, 1H), 7.43 (d, J=8.6 Hz, 1H), 7.16 (d, J=3.5 Hz, 1H), 7.10 (s, 1H), 6.68 (dd, J=3.5 Hz, 1.7 Hz, 1H), 3.39 (s, 3H)

Example 119

5-Amino-2-(2-furyl)[1,2,4]triazolo[1,5-c]pyrimidine-7-carboxylic acid [N-(2-fluorobenzyl)-N-methyl]amide monohydrochloride (Compound 119)

The reaction was carried out in a manner similar to that in Example 77 except that N-acetylethylenediamine was replaced by N-methyl-2-fluorobenzylamine. Then, an ethyl acetate solution of hydrogen chloride was added to the resulting crude product. The precipitated crystals were recrystallized from a mixed solvent of methanol and ethyl acetate to obtain Compound 119 (134 mg, 0.329 mmol) as white crystals in a yield of 27%.

$^1H$ NMR (δ ppm, DMSO-$d_6$, 80°C): 7.95 (brs, 2H), 7.88 (d, J=1.8 Hz, 1H), 7.26-7.45 (m, 2H), 7.10-7.25 (m, 2H), 7.18 (d, J=3.5 Hz, 1H), 7.04 (s, 1H), 6.69 (dd, J=3.5 Hz, 1.8 Hz, 1H), 4.71 (s, 2H), 4.32 (s, 3H)

Example 120

5-Amino-2-(2-furyl)[1,2,4]triazolo[1,5-c]pyrimidine-7-carboxylic acid [N-(3-fluorobenzyl)-N-methyl]amide monohydrochloride (Compound 120)

The reaction was carried out in a manner similar to that in Example 77 except that N-acetylethylenediamine was replaced by N-methyl-3-fluorobenzylamine. Then, an ethyl acetate solution of hydrogen chloride was added to the resulting crude product. The precipitated crystals were recrystallized from a mixed solvent of methanol and ethyl acetate to obtain Compound 120 (77 mg, 0.195 mmol) as white crystals in a yield of 16%.
$^1H$ NMR (δ ppm, DMSO-$d_6$, 80°C): 7.90 (brs, 2H), 7.87 (d, J=1.7 Hz, 1H), 7.28-7.48 (m, 2H), 7.18 (d, J=3.3 Hz, 1H), 7.07 (s, 1H), 7.00-7.23 (m, 2H), 6.69 (dd, J=3.3 Hz, 1.7 Hz, 1H), 4.65 (s, 2H), 3.99 (s, 3H)

Example 121

5-Amino-2-(2-furyl)[1,2,4]triazolo[1,5-c]pyrimidine-7-carboxylic acid [N-(4-fluorobenzyl)-N-methyl]amide monohydrochloride (Compound 121)

The reaction was carried out in a manner similar to that in Example 77 except that N-acetylethylenediamine was replaced by N-methyl-4-fluorobenzylamine. Then, an ethyl acetate solution of hydrogen chloride was added to the resulting crude product. The precipitated crystals were recrystallized from a mixed solvent of methanol and ethyl acetate to obtain Compound 121 (127 mg, 0.329 mmol) as white crystals in a yield of 27%.
$^1H$ NMR (δ ppm, DMSO-$d_6$, 80°C): 7.91 (brs, 2H), 7.88 (d, J=1.7 Hz, 1H), 7.30-7.44 (m, 2H), 7.18 (d, J=3.3 Hz, 1H), 7.08-7.23 (m, 2H), 7.06 (s, 1H), 6.69 (dd, J=3.3 Hz, 1.7 Hz, 1H), 5.30 (s, 3H), 4.60 (s, 2H)

Example 122

5-Amino-2-(2-furyl)[1,2,4]triazolo[1,5-c]pyrimidine-7-carboxylic acid [N-(2-chlorobenzyl)-N-methyl]amide monohydrochloride (Compound 122)

The reaction was carried out in a manner similar to that in Example 77 except that N-acetylethylenediamine was replaced by N-methyl-2-chlorobenzylamine. Then, an ethyl acetate solution of hydrogen chloride was added to the resulting crude product. The precipitated crystals were recrystallized from a mixed solvent of methanol and ethyl acetate to obtain Compound 122 (91 mg, 0.220 mmol) as white crystals in a yield of 18%.
$^1H$ NMR (δ ppm, DMSO-$d_6$, 80°C): 7.93 (brs, 2H), 7.88 (d, J=1.7 Hz, 1H), 7.28-7.53 (m, 4H), 7.18 (d, J=3.3 Hz, 1H), 7.06 (s, 1H), 6.69 (dd, J=3.3 Hz, 1.7 Hz, 1H), 4.75 (s, 2H), 3.79 (s, 3H)

Example 123

5-Amino-2-(2-furyl)[1,2,4]triazolo[1,5-c]pyrimidine-7-carboxylic acid [N-(4-chlorobenzyl)-N-methyl]amide monohydrochloride (Compound 123)

The reaction was carried out in a manner similar to that in Example 77 except that N-acetylethylenediamine was replaced by N-methyl-4-chlorobenzylamine. Then, an ethyl acetate solution of hydrogen chloride was added to the resulting crude product. The precipitated crystals were recrystallized from 2-propanol to obtain Compound 123 (144 mg, 0.342 mmol) as white crystals in a yield of 28%.
$^1H$ NMR (δ ppm, DMSO-$d_6$, 80°C): 7.90 (brs, 2H), 7.87 (dd, J=1.8 Hz, J=0.8 Hz, 1H), 7.33-7.48 (m, 4H), 7.18 (dd, J=3.3 Hz, J=0.8 Hz, 1H), 7.06 (s, 1H), 6.68 (dd, J=3.3 Hz, 1.8 Hz, 1H), 4.61 (s, 2H), 4.47 (s, 3H)

Example 124

5-Amino-2-(2-furyl)[1,2,4]triazolo[1,5-c]pyrimidine-7-carboxylic acid [N-methyl-N-(6-methylpyridin-2-ylmethyl)]amide (Compound 124)

The reaction was carried out in a manner similar to that in Example 77 except that N-acetylethylenediamine was replaced by N-methyl-6-methylpyridin-2-ylmethylamine. Then, the resulting crude product was recrystallized from

ethyl acetate to obtain Compound 124 (277 mg, 0.832 mmol) as white crystals in a yield of 64%.
$^1$H NMR (δ ppm, DMSO-$d_6$, 80°C): 7.82-7.96 (m, 3H), 7.56-7.70 (m, 1H), 7.05-7.20 (m, 3H), 7.04 (s, 1H), 6.64-6.71 (m, 1H), 4.66 (s, 2H), 2.98 (s, 3H), 2.48 (s, 3H)

Example 125

5-Amino-2-(2-furyl)[1,2,4]triazolo[1,5-c]pyrimidine-7-carboxylic acid [N-isopropyl-N-(4-pyridylmethyl)]amide (Compound 125)

The reaction was carried out in a manner similar to that in Example 77 except that N-acetylethylenediamine was replaced by N-isopropyl-4-pyridylmethylamine. Then, the resulting crude product was recrystallized from a mixed solvent of methanol and ethyl acetate to obtain Compound 125 (174 mg, 0.458 mmol) as white crystals in a yield of 25%.
$^1$H NMR (δ ppm, DMSO-$d_6$, 80°C): 8.50 (d, J=5.4 Hz, 2H), 7.93 (brs, 1H), 7.88 (d, J=1.8 Hz, 1H), 7.34 (d, J=5.4 Hz, 2H), 7.18 (d, J=3.1 Hz, 1H), 7.07 (s, 1H), 6.70 (dd, J=3.1 Hz, 1.8 Hz, 1H), 4.61 (s, 2H), 4.19 (brs, 1H), 1.13 (d, J=6.6 Hz, 1H)

Example 126

5-Amino-2-(2-furyl)[1,2,4]triazolo[1,5-c]pyrimidine-7-carboxylic acid [N-(2-methoxyethyl)-N-(3-pyridylmethyl)]amide (Compound 126)

The reaction was carried out in a manner similar to that in Example 77 except that N-acetylethylenediamine was replaced by N-(2-methoxyethyl)-3-pyridylmethylamine. Then, the resulting crude product was recrystallized from ethyl acetate to obtain Compound 126 (318 mg, 0.816 mmol) as white crystals in a yield of 41%.
$^1$H NMR (δ ppm, DMSO-$d_6$, 80°C): 8.55 (brs, 1H), 8.46 (d, J=3.3 Hz, 1H), 7.93 (brs, 2H), 7.87 (d, J=1.8 Hz, 1H), 7.75 (d, J=7.9 Hz, 1H), 7.31 (dd, J=7.9 Hz, 3.3 Hz, 2H), 7.18 (d, J=3.5 Hz, 1H), 7.06 (s, 1H), 6.69 (dd, J=3.5 Hz, 1.8 Hz, 1H), 4.73 (s, 2H), 3.51 (s, 3H), 3.18 (brs, 2H), 3.06 (brs, 2H)

Example 127

5-Amino-2-(2-furyl)[1,2,4]triazolo[1,5-c]pyrimidine-7-carboxylic acid [N-ethyl-N-(3-pyridylmethyl)]amide (Compound 127)

The reaction was carried out in a manner similar to that in Example 77 except that N-acetylethylenediamine was replaced by N-ethyl-3-pyridylmethylamine. Then, the resulting crude product was recrystallized from a mixed solvent of methanol and ethyl acetate to obtain Compound 127 (312 mg, 0.465 mmol) as white crystals in a yield of 28%.
$^1$H NMR (δ ppm, DMSO-$d_6$, 80°C): 8.55 (brs, 1H), 8.48 (d, J=4.6 Hz, 1H), 7.91 (brs, 1H), 7.87 (d, J=1.6 Hz, 1H), 7.77 (d, J=7.7 Hz, 1H), 7.35 (dd, J=7.7 Hz, 4.6 Hz, 1H), 7.18 (d, J=3.5 Hz, 1H), 7.06 (s, 1H), 6.69 (dd, J=3.5 Hz, 1.6 Hz, 1H), 4.66 (s, 2H), 3.36 (q, J=7.1 Hz, 2H), 1.09 (t, J=7.1 Hz, 3H)

Example 128

5-Amino-2-(2-furyl)[1,2,4]triazolo[1,5-c]pyrimidine-7-carboxylic acid [N-(2-chloropyridin-3-ylmethyl)-N-methyl]amide (Compound 128)

The reaction was carried out in a manner similar to that in Example 77 except that N-acetylethylenediamine was replaced by N-methyl-2-chloropyridin-3-ylmethylamine. Then, the resulting crude product was recrystallized from a mixed solvent of methanol and ethyl acetate to obtain Compound 128 (159 mg, 0.416 mmol) as white crystals in a yield of 20%.
$^1$H NMR (δ ppm, DMSO-$d_6$, 80°C): 8.34 (brs, 1H), 7.91 (brs, 2H), 7.88 (d, J=1.7 Hz, 1H), 7.84 (brs, 1H), 7.46 (dd, J=7.5 Hz, 4. 8 Hz, 1H), 7.01 (brs, 1H), 6. 69 (dd, J=3. 3 Hz, 1. 7 Hz, 1H), 4.74 (s, 2H), 3.00 (s, 3H))

Example 129

5-Amino-2-(2-furyl)[1,2,4]triazolo[1,5-c]pyrimidine-7-carboxylic acid [N-ethyl-N-(2-chloropyridin-3-ylmethyl)]amide (Compound 129)

The reaction was carried out in a manner similar to that in Example 77 except that N-acetylethylenediamine

was replaced by N-ethyl-2-chloropyridin-3-ylmethylamine. Then, the resulting crude product was recrystallized from a mixed solvent of methanol and ethyl acetate to obtain Compound 129 (250 mg, 0.636 mmol) as light yellow crystals in a yield of 30%.
$^1H$ NMR (δ ppm, DMSO-$d_6$, 80°C): 8.33 (brs, 1H), 7.93 (brs, 2H), 7.88 (d, J=1.7 Hz, 1H), 7.83 (brs, 1H), 7.44 (dd, J=7.5 Hz, 4.8 Hz, 1H), 7.18 (d, J=3.3 Hz, 1H), 6.99 (brs, 1H), 6.69 (dd, J=3.3 Hz, 1.7 Hz, 1H), 4.72 (s, 2H), 3.42 (q, J=7.0 Hz, 2H), 1.12 (t, J=7.0 Hz, 3H)

Example 130

5-Amino-2-(2-furyl)[1,2,4]triazolo[1,5-c]pyrimidine-7-carboxylic acid [N-methyl-N-(6-methylpyridin-3-ylmethyl)]amide (Compound 130)

The reaction was carried out in a manner similar to that in Example 77 except that N-acetylethylenediamine was replaced by N-(6-methylpyridin-3-ylmethyl)methylamine. Then, the resulting crude product was recrystallized from ethyl acetate to obtain Compound 130 (65 mg, 0.178 mmol) as white crystals in a yield of 28%.
$^1H$ NMR (δ ppm, DMSO-$d_6$, 80°C): 8.41 (brs, 1H), 7.91 (brs, 2H), 7.87 (d, J=1.8 Hz, 1H), 7.64 (dd, J=8.1 Hz, 2.0 Hz, 1H), 7.22 (d, J=7.8 Hz, 1H), 7.19 (d, J=3.5 Hz, 1H), 7.06 (s, 1H), 6. 69 (dd, J=3. 5 Hz, 1. 8 Hz, 1H), 4. 60 (s, 2H), 2. 90 (s, 3H), 2.45 (s, 3H)

Example 131

5-Amino-2-(2-furyl)[1,2,4]triazolo[1,5-c]pyrimidine-7-carboxylic acid [N-methyl-N-(6-methoxypyridin-3-ylmethyl)] amide (Compound 131)

The reaction was carried out in a manner similar to that in Example 77 except that N-acetylethylenediamine was replaced by N-methyl-6-methoxypyridin-3-ylmethylamine. Then, the resulting crude product was recrystallized from a mixed solvent of ethanol and ethyl acetate to obtain Compound 131 (216 mg, 0.560 mmol) as white crystals in a yield of 25%.
$^1H$ NMR (δ ppm, DMSO-$d_6$, 80°C): 8.13 (brs, 1H), 7.92 (brs, 2H), 7.87 (dd, J=1.7 Hz, 0.7 Hz, 1H), 7.71 (d, J=8.3 Hz, 1H), 7.19 (dd, J=3.3 Hz, 0.7 Hz, 1H), 7.07 (s, 1H), 6.79 (d, J=8.3 Hz, 1H), 6.69 (dd, J=3.3 Hz, 1.7 Hz, 1H), 4.56 (s, 2H), 3.86 (s, 3H), 2.90 (s, 3H)

Example 132

5-Amino-2-(2-furyl)[1,2,4]triazolo[1,5-c]pyrimidine-7-carboxylic acid [N-ethyl-N-(6-methoxypyridin-3-ylmethyl)]amide (Compound 132)

The reaction was carried out in a manner similar to that in Example 77 except that N-acetylethylenediamine was replaced by N-ethyl-6-methoxypyridin-3-ylmethylamine. Then, the resulting crude product was recrystallized from ethyl acetate to obtain Compound 132 (247 mg, 0.627 mmol) as white crystals in a yield of 35%.
$^1H$ NMR (δ ppm, DMSO-$d_6$, 80°C): 8.14 (brs, 2H), 7.93 (brs, 2H), 7.88 (dd, J=1.9 Hz, 1.0 Hz, 1H), 7.72 (d, J=8.3 Hz, 1H), 7.19 (dd, J=3.3 Hz, 1.0 Hz, 1H), 7.05 (s, 1H), 6.78 (d, J=8.3 Hz, 1H), 6.69 (dd, J=3.3 Hz, 1.9 Hz, 1H), 4.57 (s, 2H), 3.85 (s, 3H), 3.33 (q, J=6.9 Hz, 2H), 1.09 (t, J=6.9 Hz, 3H)

Example 133

5-Amino-2-(2-furyl)[1,2,4]triazolo[1,5-c]pyrimidine-7-carboxylic acid [N-(2-pyridylmethyl)-N-(2,2,2-trifluoroethyl)] amide (Compound 133)

The reaction was carried out in a manner similar to that in Example 77 except that N-acetylethylenediamine was replaced by N-(2-pyridylmethyl)-2,2,2-trifluoroethylamine. Then, the resulting crude product was recrystallized from a mixed solvent of hexane and ethyl acetate to obtain Compound 131 (201 mg, 0.475 mmol) as white crystals in a yield of 22%.
$^1H$ NMR (δ ppm, DMSO-$d_6$, 80°C): 8.50 (d, J=3.5 Hz, 1H), 7.99 (s, 2H), 7.87 (d, J=0.9 Hz, 1H), 7.68-7.78 (m, 1H), 7.23-7.35 (m, 2H), 7.17 (d, J=3.3 Hz, 1H), 6.99 (brs, 1H), 6.68 (dd, J=3.3 Hz, 0.9 Hz, 1H), 4.90 (s, 2H), 4.36 (brs, 2H)

Example 134

5-Amino-2-(2-furyl)[1,2,4]triazolo[1,5-c]pyrimidine-7-carboxylic acid [N-methyl-N-(2-methylpyridin-3-ylmethyl)]amide (Compound 134)

The reaction was carried out in a manner similar to that in Example 77 except that N-acetylethylenediamine was replaced by N-methyl-2-methylpyridin-3-ylmethylamine. Then, the resulting crude product was recrystallized from a mixed solvent of methanol and ethyl acetate to obtain Compound 134 (226 mg, 0.632 mmol) as white crystals in a yield of 40%.
$^1$H NMR (δ ppm, DMSO-$d_6$, 80°C): 8.34 (brs, 1H), 7.87 (brs, 3H), 7.60 (d, J=7.6 Hz, 1H), 7.15-7.25 (m, 2H), 7.06 (s, 1H), 6.69 (brs, 1H), 4.68 (s, 2H), 2.92 (s, 3H), 2.49 (s, 3H)

Example 135

5-Amino-2-(2-furyl)[1,2,4]triazolo[1,5-c]pyrimidine-7-carboxylic acid [N-(6-chloropyridin-3-ylmethyl)-N-methyl]amide (Compound 135)

The reaction was carried out in a manner similar to that in Example 77 except that N-acetylethylenediamine was replaced by N-methyl-6-chloropyridin-3-ylmethylamine. Then, the resulting crude product was recrystallized from a mixed solvent of hexane and ethyl acetate to obtain Compound 135 (296 mg, 0.779 mmol) as white crystals in a yield of 44%.
$^1$H NMR (δ ppm, DMSO-$d_6$, 80°C): 8.39 (s, 1H), 7.92 (brs, 2H), 7.88 (dd, J=1.8 Hz, 0.7 Hz, 1H), 7.82-7.90 (m, 1H), 7.48 (d, J=7.0 Hz, 1H), 7.19 (dd, J=3.5 Hz, 0.7 Hz, 1H), 7.09 (s, 1H), 6.69 (dd, J=3.5 Hz, 1.8 Hz, 1H), 4.66 (s, 2H), 2.94 (s, 3H)

Example 136

5-Amino-2-(2-furyl)[1,2,4]triazolo[1,5-c]pyrimidine-7-carboxylic acid {N-[(6-chloro)pyridin-3-ylmethyl]-N-ethyl}amide (Compound 136)

The reaction was carried out in a manner similar to that in Example 77 except that N-acetylethylenediamine was replaced by N-ethyl-6-chloropyridin-3-ylmethylamine. Then, the resulting crude product was recrystallized from a mixed solvent of hexane and ethyl acetate to obtain Compound 136 (349 mg, 0.870 mmol) as white crystals in a yield of 47%.
$^1$H NMR (δ ppm, DMSO-$d_6$, 80°C): 8.40 (s, 1H), 7.93 (brs, 2H), 7.88 (dd, J=1.8 Hz, 0.7 Hz, 1H), 7.82-7.90 (m, 1H), 7.47 (d, J=8.1 Hz, 1H), 7.34 (dd, J=3.5 Hz, 0.7 Hz, 1H), 7.08 (s, 1H), 6.69 (dd, J=3.5 Hz, 1.8 Hz, 1H), 4.66 (s, 2H), 3.36 (q, J=7.0 Hz, 2H), 1.10 (t, J=7.0 Hz, 3H)

Example 137

5-Amino-2-(2-furyl)[1,2,4]triazolo[1,5-c]pyrimidine-7-carboxylic acid [N-(2-methoxypyridin-3-ylmethyl)-N-methyl] amide (Compound 137)

The reaction was carried out in a manner similar to that in Example 77 except that N-acetylethylenediamine was replaced by N-methyl-2-methoxypyridin-3-ylmethylamine. Then, the resulting crude product was recrystallized from a mixed solvent of hexane and ethyl acetate to obtain Compound 137 (350 mg, 0.926 mmol) as white crystals in a yield of 49%.
$^1$H NMR (δ ppm, DMSO-$d_6$, 80°C): 8.09 (brs, 1H), 7.91 (brs, 2H), 7.88 (d, J=1.7 Hz, 1H), 7.18 (d, J=3.3 Hz, 1H), 7.00 (dd, J=7.2 Hz, 3.9 Hz, 1H), 6.69 (dd, J=3.3 Hz, 1.7 Hz, 1H), 4.59 (s, 2H), 3.90 (brs, 3H), 2.96 (s, 3H)

Example 138

5-Amino-2-(2-furyl)[1,2,4]triazolo[1,5-c]pyrimidine-7-carboxylic acid {N-methyl-N-[2-(3-pyridyl)ethyl]}amide (Compound 138)

The reaction was carried out in a manner similar to that in Example 77 except that N-acetylethylenediamine was replaced by N-methyl-2-(3-pyridyl)ethylamine. Then, the resulting crude product was recrystallized from a mixed solvent of methanol and ethyl acetate to obtain Compound 138 (100 mg, 0.220 mmol) as white crystals in a yield of 18%.

$^1$H NMR (δ ppm, DMSO-$d_6$, 80°C): 8.40 (brs, 2H), 7.90 (brs, 3H), 7.50 (brs, 1H), 7.20 (brs, 2H), 6.70 (brs, 2H), 3.60 (brs, 2H), 3.10 (brs, 2H), 2.97 (s, 3H)

Example 139

5-Amino-2-(2-furyl)[1,2,4]triazolo[1,5-c]pyrimidine-7-carboxylic acid {N-methyl-N-[2-(4-pyridyl)ethyl]}amide (Compound 139)

The reaction was carried out in a manner similar to that in Example 77 except that N-acetylethylenediamine was replaced by N-methyl-2-(4-pyridyl)ethylamine. Then, the resulting crude product was recrystallized from a mixed solvent of methanol and ethyl acetate to obtain Compound 139 (100 mg, 0.268 mmol) as white crystals in a yield of 22%.
$^1$H NMR (δ ppm, DMSO-$d_6$, 24°C): 8.53 (brs, 0.4H), 8.45 (brs, 0.4H), 8.37 (brs, 1.2H), 8.19 (brs, 2H), 7.95 (d, J=1.8 Hz, 1H), 7.74 (d, J=7.0 Hz, 1H), 7.49 (d, J=7.0 Hz, 1H), 7.20-7.26 (m, 1.6H), 6.87 (s, 0.4H), 6.73 (dd, J=3.3 Hz, 1.8 Hz, 1H), 6.63 (s, 0.6H), 3.64 (t, J=7.5 Hz, 0.4H), 3.52 (t, J=7.5 Hz, 0.6H), 3.01 (s, 0.6H), 2.93 (s, 0.4H), 2.88-3.03 (m, 2H)

Example 140

5-Amino-2-(2-furyl)[1,2,4]triazolo[1,5-c]pyrimidin-7-yl morpholino ketone (Compound 140)

The reaction was carried out in a manner similar to that in Example 77 except that N-acetylethylenediamine was replaced by morpholine. Then, the resulting crude product was recrystallized from a mixed solvent of hexane and ethyl acetate to obtain Compound 140 (165 mg, 0.781 mmol) as light yellow crystals in a yield of 64%.
$^1$H NMR (δ ppm, DMSO-$d_6$, 24°C): 8.18 (brs, 2H), 7.95 (dd, J=1.8 Hz, 0.9 Hz, 1H), 7.21 (d, J=3.4 Hz, 0.8 Hz, 1H), 7.05 (s, 1H), 6.73 (dd, J=3.4 Hz, 1.8 Hz, 1H), 3.40-3.70 (m, 8H)

Example 141

5-Amino-2-(2-furyl)[1,2,4]triazolo[1,5-c]pyrimidin-7-yl cis-2,6-dimethylmorpholin-4-yl ketone (Compound 141)

The reaction was carried out in a manner similar to that in Example 77 except that N-acetylethylenediamine was replaced by cis-2,6-dimethylmorpholine. Then, the resulting crude product was recrystallized from a mixed solvent of hexane and ethyl acetate to obtain Compound 141 (226 mg, 0.781 mmol) as light yellow crystals in a yield of 64%.
$^1$H NMR (δ ppm, DMSO-$d_6$, 24°C): 8.17 (brs, 1H), 7.94 (brs, 1H), 7.21 (d, J=3.4 Hz, 1H), 7.02 (s, 1H), 6.73 (dd, J=3.4 Hz, 1.8 Hz, 1H), 4.35 (d, J=12.4 Hz, 1H), 3.69 (d, J=12.4 Hz, 1H), 3.40-3.60 (m, 2H), 2.65-2.79 (m, 1H), 2.40-2.50 (m, 1H), 1.15 (d, J=6.3 Hz, 3H), 1.00 (d, J=6.3 Hz, 3H)

Example 142

5-Amino-2-(2-furyl)[1,2,4]triazolo[1,5-c]pyrimidin-7-yl piperidino ketone (Compound 142)

The reaction was carried out in a manner similar to that in Example 77 except that N-acetylethylenediamine was replaced by piperidine. Then, the resulting crude product was recrystallized from a mixed solvent of hexane and ethyl acetate to obtain Compound 142 (144 mg, 0.459 mmol) as white crystals in a yield of 45%.
$^1$H NMR (δ ppm, DMSO-$d_6$, 100°C): 7.84 (dd, J=1.7 Hz, 0.8 Hz, 1H), 7.77 (brs, 2H), 7.17 (dd, J=3.3 Hz, 0.8 Hz, 1H), 6.95 (s, 1H), 6.68 (dd, J=3.3 Hz, 1.7 Hz, 1H), 3.46 (brs, 4H), 1.45-1.70 (m, 6H)

Example 143

5-Amino-2-(2-furyl)[1,2,4]triazolo[1,5-c]pyrimidin-7-yl 4-methylpiperidino ketone (Compound 143)

The reaction was carried out in a manner similar to that in Example 77 except that N-acetylethylenediamine was replaced by 4-methylpiperidine. Then, the resulting crude product was recrystallized from a mixed solvent of hexane and ethyl acetate to obtain Compound 143 (199 mg, 0.842 mmol) as light yellow crystals in a yield of 69%.
$^1$H NMR (δ ppm, DMSO-$d_6$, 100°C): 7.85 (d, J=1.7 Hz, 1H), 7.84 (brs, 2H), 7.16 (d, J=3.3 Hz, 1H), 6.95 (s, 1H), 6.68 (dd, J=3.3 Hz, 1.7 Hz, 1H), 4.00 (brs, 4H), 2.90 (brs, 1H), 1.58-1.73 (m, 2H), 1.05-1.20 (m, 2H), 0.94 (d, J=6.2 Hz, 3H)

Example 144

5-Amino-2-(2-furyl)[1,2,4]triazolo[1,5-c]pyrimidin-7-yl 4-methoxypiperidino ketone (Compound 144)

The reaction was carried out in a manner similar to that in Example 77 except that N-acetylethylenediamine was replaced by 4-methoxypiperidine. Then, the resulting crude product was recrystallized from a mixed solvent of ethanol and ethyl acetate to obtain Compound 144 (68 mg, 0.208 mmol) as white crystals in a yield of 26%.
$^1$H NMR (δ ppm, DMSO-$d_6$, 25°C): 8.16 (brs, 2H), 7.94 (dd, J=2.0 Hz, 0.7 Hz, 1H), 7.21 (dd, J=3.3 Hz, 0.7 Hz, 1H), 7.01 (s, 1H), 6.73 (dd, J=3.3 Hz, 2.0 Hz, 1H), 3.85-3.96 (m, 1H), 3.40-3.58 (m, 2H), 3.26 (s, 3H), 3.11-3.38 (m, 2H), 1.74-1.94 (m, 2H), 1.37-1.54 (m, 2H)

Example 145

5-Amino-2-(2-furyl)[1,2,4]triazolo[1,5-c]pyrimidin-7-yl 3-methylpiperidino ketone (Compound 145)

The reaction was carried out in a manner similar to that in Example 77 except that N-acetylethylenediamine was replaced by 3-methylpiperidine. Then, the resulting crude product was recrystallized from a mixed solvent of hexane and ethyl acetate to obtain Compound 145 (149 mg, 0.573 mmol) as light yellow crystals in a yield of 47%.
$^1$H NMR (δ ppm, DMSO-$d_6$, 100°C): 7.85 (d, J=1.8 Hz, 1H), 7.77 (brs, 2H), 7.17 (d, J=3.3 Hz, 1H), 6.95 (s, 1H), 6.68 (dd, J=3.3 Hz, 1.8 Hz, 1H), 3.80 (brs, 2H), 2.90 (brs, 1H), 2.65 (brs, 1H), 1.56-1.70 (m, 2H), 1.38-1.55 (m, 1H), 1.10-1.28 (m, 1H), 0.85 (s, 3H)

Example 146

5-Amino-2-(2-furyl)[1,2,4]triazolo[1,5-c]pyrimidin-7-yl perhydro-1,4-oxazepinyl ketone (Compound 146)

The reaction was carried out in a manner similar to that in Example 77 except that N-acetylethylenediamine was replaced by homomorpholine. Then, the resulting crude product was recrystallized from methanol to obtain Compound 146 (148 mg, 1.18 mmol) as white crystals in a yield of 97%.
$^1$H NMR (δ ppm, DMSO-$d_6$, 100°C): 7.85 (dd, J=1.7 Hz, 0.9 Hz, 1H), 7.79 (brs, 2H), 7.17 (d, J=3.4 Hz, 0.9 Hz, 1H), 7.00 (s, 1H), 6.68 (dd, J=3.4 Hz, 1.7 Hz, 1H), 3.68-3.75 (m, 8H), 2.00-2.21 (m, 2H)

Example 147

5-Amino-2-(2-furyl)[1,2,4]triazolo[1,5-c]pyrimidin-7-yl 2-(3-pyridyl)piperidino ketone monohydrochloride (Compound 147)

The reaction was carried out in a manner similar to that in Example 77 except that N-acetylethylenediamine was replaced by 2-(3-pyridyl)piperidine. Then, an ethyl acetate solution of hydrogen chloride was added to the resulting crude product. The precipitated crystals were recrystallized from a mixed solvent of ethanol and ethyl acetate to obtain Compound 147 (230 mg, 0.588 mmol) as brown crystals in a yield of 47%.
$^1$H NMR (δ ppm, DMSO-$d_6$, 80°C): 8.71 (brs, 1H), 8.18 (d, J=6.7 Hz, 1H), 7.88 (brs, 1H), 7.87 (d, J=1.7 Hz, 1H), 7.77 (brs, 1H), 7.18 (d, J=3.3 Hz, 1H), 7.11 (s, 1H), 6.69 (dd, J=3.3 Hz, 1.7 Hz, 1H), 3.30 (brs, 2H), 2.80 (brs, 1H), 1.34-1.75 (m, 6H)

Example 148

5-Amino-2-(2-furyl)[1,2,4]triazolo[1,5-c]pyrimidin-7-yl pyrrolidinyl ketone (Compound 148)

The reaction was carried out in a manner similar to that in Example 77 except that N-acetylethylenediamine was replaced by pyrrolidine. Then, the resulting crude product was recrystallized from a mixed solvent of hexane and ethyl acetate to obtain Compound 148 (90 mg, 0.296 mmol) as white crystals in a yield of 29%.
$^1$H NMR (δ ppm, DMSO-$d_6$, 100°C): 7.85 (dd, J=1.7 Hz, 0.9 Hz, 1H), 7.75 (brs, 2H), 7.18 (dd, J=3.4 Hz, 0.9 Hz, 1H), 7.12 (s, 1H), 6.69 (dd, J=3.4 Hz, 1.7 Hz, 1H), 3.51 (brs, 4H), 1.80-1.95 (m, 4H)

Example 149

(2R)-(2-Methoxymethyl)pyrrolidinyl 5-amino-2-(2-furyl)-7-[1,2,4]triazolo[1,5-c]pyrimidin-7-yl ketone (Compound 149)

The reaction was carried out in a manner similar to that in Example 77 except that N-acetylethylenediamine was replaced by (2S)-(+)-methoxymethylpyrrolidine. Then, the resulting crude product was recrystallized from a mixed solvent of hexane and ethyl acetate to obtain Compound 149 (148 mg, 0.769 mmol) as white crystals in a yield of 63%.
$^1$H NMR (δ ppm, DMSO-$d_6$, 100°C): 7.85 (dd, J=1.7 Hz, 0.8 Hz, 1H), 7.78 (brs, 2H), 7.18 (dd, J=3.4 Hz, 0.8 Hz, 1H), 7.09 (s, 1H), 6.68 (dd, J=3.4 Hz, 1.7 Hz, 1H), 4.35 (brs, 1H), 3.57 (brs, 1H), 3.23 (brs, 2H), 1.71-2.00 (m, 3H)

Example 150

5-Amino-2-(2-furyl)[1,2,4]triazolo[1,5-c]pyrimidin-7-yl (4-hydroxy-4-phenyl)piperidino ketone (Compound 150)

The reaction was carried out in a manner similar to that in Example 77 except that N-acetylethylenediamine was replaced by 4-hydroxy-4-phenylpiperidine. Then, the resulting crude product was recrystallized from a mixed solvent of 2-propanol and ethyl acetate to obtain Compound 150 (173 mg, 0.427 mmol) as white crystals in a yield of 35%.
$^1$H NMR (δ ppm, DMSO-$d_6$, 80°C): 7.88 (brs, 3H), 7.51 (d, J=7.3 Hz, 2H), 7.33 (t, J=7.3 Hz, 2H), 7.21 (t, J=7.3 Hz, 1H), 7.18 (d, J=3.3 Hz, 1H), 7.06 (s, 1H), 6.69 (brs, 1H), 4.39 (brs, 1H), 3.15-3.87 (m, 4H), 1.85-2.10 (m, 2H), 1.52-1.85 (m, 2H)

Example 151

5-Amino-2-(2-furyl)[1,2,4]triazolo[1,5-c]pyrimidin-7-yl cis-decahydroisoquinolyl ketone (Compound 151)

The reaction was carried out in a manner similar to that in Example 77 except that N-acetylethylenediamine was replaced by cis-decahydroisoquinoline. Then, the resulting crude product was recrystallized from ethyl acetate to obtain Compound 151 (239 mg, 0.653 mmol) as white crystals in a yield of 51%.
$^1$H NMR (δ ppm, DMSO-$d_6$, 28°C): 8.14 (brs, 2H), 7.94 (d, J=1.7 Hz, 1H), 7.21 (d, J=3.3 Hz, 1H), 6.98 (s, 0.6 Hz), 6.93 (s, 0.4H), 6.73 (dd, J=3.3 Hz, J=1.7 Hz, 1H), 3.90-4.15 (m, 1H), 3.40-3.60 (m, 1H), 3.00-3.25 (m, 2H), 1.10-1.95 (m, 12H)

Example 152

5-Amino-2-(2-furyl)[1,2,4]triazolo[1,5-c]pyrimidin-7-yl trans-decahydroisoquinolyl ketone (Compound 152)

The reaction was carried out in a manner similar to that in Example 77 except that N-acetylethylenediamine was replaced by trans-decahydroisoquinoline. Then, the resulting crude product was recrystallized from ethyl acetate to obtain Compound 152 (194 mg, 0.533 mmol) as white crystals in a yield of 42%.
$^1$H NMR (δ ppm, DMSO-$d_6$, 25°C): 8.13 (brs, 2H), 7.93 (dd, J=1.7 Hz, 0.7 Hz, 1H), 7.19 (dd, J=3.5 Hz, 0.7 Hz, 1H), 6.71 (dd, J=3.5 Hz, 1.7 Hz), 4.50 (d, J=12.9 Hz, 0.5H), 4.33 (d, J=12.9 Hz, 0.5H), 3.67 (d, J=12.9 Hz, 0.5H), 3.53 (d, J=12.9 Hz, 0.5H), 2.99 (t, J=11.6 Hz, 0.5H), 2.68 (t, J=11.6 Hz, 0.5H), 2.64 (t, J=11.5 Hz, 0.3H), 2.34 (t, J=11.5 Hz, 0.7H), 0.75-1.80 (m, 12H)

Example 153

5-Amino-2-(2-furyl)[1,2,4]triazolo[1,5-c]pyrimidin-7-yl 3,5-dimethylpiperidino ketone (Compound 153)

The reaction was carried out in a manner similar to that in Example 77 except that N-acetylethylenediamine was replaced by 3,5-dimethylpiperidine. Then, the resulting crude product was recrystallized from ethyl acetate to obtain Compound 153 (114 mg, 0.305 mmol) as white crystals in a yield of 25%.
$^1$H NMR (δ ppm, DMSO-$d_6$, 80°C): 7.87 (brs, 3H), 7.18 (d, J=3.1 Hz, 1H), 6.95 (s, 1 Hz), 6.69 (dd, J=3.1 Hz, J=1.8 Hz, 1H), 4.41 (brs, 2H), 3.65 (brs, 2H), 2.26 (brs, 2H), 1.60 (brs, 2H), 0.85 (brs, 6H)

Example 154

5-Amino-2-(2-furyl)[1,2,4]triazolo[1,5-c]pyrimidin-7-yl perhydroazepinyl ketone (Compound 154)

The reaction was carried out in a manner similar to that in Example 77 except that N-acetylethylenediamine

was replaced by hexamethyleneimine. Then, the resulting crude product was recrystallized from a mixed solvent of hexane and ethyl acetate to obtain Compound 154 (227 mg, 0.699 mmol) as white crystals in a yield of 55%.
$^1$H NMR (δ ppm, DMSO-$d_6$, 25°C): 8.15 (brs, 2H), 7.94 (d, J=1.7 Hz, 1H), 7.21 (d, J=3.3 Hz, 1H), 6.98 (s, 1 Hz), 6.73 (dd, J=3.3 Hz, J=1.7 Hz, 1H), 3.54 (t, J=5.0 Hz, 2H), 3.37 (t, J=5.0 Hz, H), 1.49-1.78 (m, 8H)

Example 155

[5-Amino-2-(2-furyl)[1,2,4]triazolo[1,5-c]pyrimidin-7-yl]carbonyl-4-phenylpiperidine-4-carbonitrile (Compound 155)

The reaction was carried out in a manner similar to that in Example 77 except that N-acetylethylenediamine was replaced by 4-cyano-4-phenylpiperidine. Then, the resulting crude product was recrystallized from a mixed solvent of hexane and ethyl acetate to obtain Compound 155 (145 mg, 0.346 mmol) as white crystals in a yield of 27%.
$^1$H NMR (δ ppm, DMSO-$d_6$, 27°C): 8.19 (brs, 2H), 7.94 (dd, J=1.8 Hz, 0.7 Hz, 1H), 7.59 (d, J= 7.4 Hz, 1H), 7.47 (dd, J=7.4 Hz, 7.2 Hz, 1H), 7.39 (t, J=7.2 Hz, 1H), 7.21 (dd, J=3.5 Hz, 0.7 Hz, 1H), 7.15 (s, 1H), 6.73 (dd, J=3.5 Hz, 1.8 Hz, 1H), 4.63 4.72 (m, 1H), 3.92-4.02 (m, 1H), 3.26-3.39 (m, 1H), 3.01-3.14 (m, 1H), 2.23-2.32 (m, 1H), 2.00-2.20 (m, 3H)

Example 156

5-Amino-2-(2-furyl)[1,2,4]triazolo[1,5-c]pyrimidin-7-yl 4-hydroxypiperidino ketone (Compound 156)

The reaction was carried out in a manner similar to that in Example 77 except that N-acetylethylenediamine was replaced by 4-hydroxypiperidine. Then, the resulting crude product was triturated with chloroform to obtain Compound 156 (180 mg, 0.500 mmol) as white crystals in a yield of 41%. $^1$H NMR (δ ppm, DMSO-$d_6$, 80°C): 7.87 (dd, J=1.6 Hz, 0.8 Hz, 1H), 7.80 (brs, 2H), 7.18 (dd, J=3.5 Hz, 0.8 Hz, 1H), 6.96 (s, 1H), 6.68 (dd, J=3.5 Hz, 1.6 Hz, 1H), 4.51 (d, J=4.0 Hz, 1H), 3.77 (m, 2H), 3.70 (brs, 1H), 3.11-3.29 (m, 2H), 1.69-1.85 (m, 2H), 1.31-1.49 (m, 2H)

Example 157

5-Amino-2-(2-furyl)[1,2,4]triazolo[1,5-c]pyrimidin-7-yl 1,4-dioxa-8-azaspiro[4,5]decanyl ketone (Compound 157)

The reaction was carried out in a manner similar to that in Example 77 except that N-acetylethylenediamine was replaced by 1,4-dioxa-8-azaspiro[4,5]decane. Then, the resulting crude product was recrystallized from a mixed solvent of methanol and ethyl acetate to obtain Compound 157 (180 mg, 0.488 mmol) as white crystals in a yield of 40%.
$^1$H NMR (δ ppm, DMSO-$d_6$, 80°C): 7.88 (d, J=1.7 Hz, 1H), 7.80 (brs, 2H), 7.84 (brs, 2H), 7.18 (d, J=3.3 Hz, 1H), 7.03 (s, 1H), 6.70 (dd, J=3.3 Hz, 1.7 Hz, 1H), 3.91 (brs, 4H), 3.55 (brs, 4H), 1.51-1.56 (m, 4H)

Example 158

5-Amino-2-(2-furyl)[1,2,4]triazolo[1,5-c]pyrimidin-7-yl 4-benzylpiperidino ketone (Compound 158)

The reaction was carried out in a manner similar to that in Example 77 except that N-acetylethylenediamine was replaced by 4-benzylpiperidine. Then, the resulting crude product was recrystallized from ethyl acetate to obtain Compound 158 (137 mg, 0.342 mmol) as white crystals in a yield of 28%.
$^1$H NMR (δ ppm, DMSO-$d_6$, 80°C) : 7.87 (dd, J=1.8 Hz, 0. 8 Hz, 1H), 7.85 (brs, 2H), 7.22-7.30 (m, 2H), 7.17 (dd, J=3.5 Hz, 0.8 Hz, 1H), 7.12-7.21 (m, 3H), 6.95 (s, 1H), 6.68 (dd, J=3.5 Hz, 1.8 Hz, 1H), 4.34 (brs, 1H), 3.78 (brs, 1H), 2.86 (brs, 1H), 2.56 (d, J=7.1 Hz, 1H), 1.73-1.95 (m, 0.3H), 1.53-1.72 (m, 0.7H), 1.09-1.28 (m, 2H)

Example 159

5-Amino-2-(2-furyl)[1,2,4]triazolo[1,5-c]pyrimidin-7-yl 4-(2-hydroxyethyl)piperidino ketone (Compound 159)

The reaction was carried out in a manner similar to that in Example 77 except that N-acetylethylenediamine was replaced by 4-piperidineethanol. Then, the resulting crude product was recrystallized from ethanol to obtain Compound 159 (152 mg, 0.427 mmol) as white crystals in a yield of 35%. $^1$H NMR (δ ppm, DMSO-$d_6$, 80°C): 7.87 (d, J=1.7 Hz, 1H), 7.86 (brs, 2H), 7.18 (d, J=3.4 Hz, 1H), 6.96 (s, 1H), 6.69 (dd, J=3.4 Hz, 1.7 Hz, 1H), 4.38 (brs, 1H), 4.00-4.15 (m, 2H), 3.74 (brs, 1H), 3.40-3.53 (m, 2H), 2.90 (brs, 2H), 1.55-1.80 (m, 2H), 1.35-1.45 (m, 2H), 1.01-1.25 (m, 2H)

Example 160

5-Amino-2-(2-furyl)[1,2,4]triazolo[1,5-c]pyrimidin-7-yl 4-piperidinopiperidino ketone (Compound 160)

The reaction was carried out in a manner similar to that in Example 77 except that N-acetylethylenediamine was replaced by 4-piperidinopiperidine. Then, the resulting crude product was recrystallized from ethanol-ethyl acetate to obtain Compound 160 (173 mg, 0.439 mmol) as white crystals in a yield of 36%.
$^1$H NMR (δ ppm, DMSO-$d_6$, 80°C): 7.87 (brs, 3H), 7.17 (d, J=3.5 Hz, 1H), 6.97 (s, 1H), 6.69 (dd, J=3.5 Hz, 1.7 Hz, 1H), 4.40 (brs, 1H), 3.79 (brs, 1H), 2.89 (brs, 6H), 2.43 (brs, 1H), 1.60-1.87 (m, 1H), 1.13-1.58 (m, 9H)

Example 161

[5-Amino-2-(2-furyl)[1,2,4]triazolo[1,5-c]pyrimidine-7-carbonyl]piperidine-4-carboxylic acid amide (Compound 161)

The reaction was carried out in a manner similar to that in Example 77 except that N-acetylethylenediamine was replaced by piperidine-4-carboxylic acid amide. Then, the resulting crude product was triturated with ethyl acetate to obtain Compound 161 (213 mg, 0.598 mmol) as white crystals in a yield of 49%.
$^1$H NMR (δ ppm, DMSO-$d_6$, 80°C): 7.87 (brs, 3H), 7.17 (d, J=3.3 Hz, 1H), 6.97 (s, 1H), 6.80 (brs, 2H), 6.69 (dd, J=3.3 Hz, 1.8 Hz, 1H), 4.31 (brs, 0.5H), 3.78 (brs, 0.5H), 2.80-3.20 (m, 2H), 2.25-2.30 (m, 2H), 1.45-1.80 (m, 4H)

Example 162

5-Amino-2-(2-furyl)[1,2,4]triazolo[1,5-c]pyrimidin-7-yl 4-benzyl-4-hydroxypiperidino ketone (Compound 162)

The reaction was carried out in a manner similar to that in Example 77 except that N-acetylethylenediamine was replaced by 4-benzyl-4-hydroxypiperidine. Then, the resulting crude product was recrystallized from a mixed solvent of methanol and ethyl acetate to obtain Compound 162 (101 mg, 0.244 mmol) as white crystals in a yield of 20%.
$^1$H NMR (δ ppm, DMSO-$d_6$, 80°C): 7.87 (dd, J=1.7 Hz, 0.7 Hz, 1H), 7.86 (brs, 2H), 7.20-7.32 (m, 5H), 7.18 (dd, J=3.3 Hz, 0.7 Hz, 1H), 6.94 (s, 1H), 6.69 (dd, J=3.3 Hz, 1.7 Hz, 1H), 4.25 (s, 1H), 4.16 (brs, 1H), 3.52 (brs, 1H), 3.20 (brs, 2H), 2.73 (s, 2H), 1.31-1.60 (m, 4H)

Preparation Example 1

Tablet

A tablet comprising the following composition is prepared by a conventional method.

| | |
|---|---|
| Compound 69 | 10 mg |
| Lactose | 30 mg |
| Potato starch | 15 mg |
| Polyvinyl alcohol | 1.5 mg |
| Magnesium stearate | 0.5 mg |

Preparation Example 2

Tablet

A tablet comprising the following prescription is prepared by a conventional method.

| | |
|---|---|
| Compound 73 | 10 mg |
| Lactose | 30 mg |
| Potato starch | 15 mg |
| Polyvinyl alcohol | 1.5 mg |
| Magnesium stearate | 0.5 mg |

Preparation Example 3

Tablet

A tablet comprising the following prescription is prepared by a conventional method.

| | |
|---|---|
| Compound 76 | 10 mg |
| Lactose | 30 mg |
| Potato starch | 15 mg |
| Polyvinyl alcohol | 1.5 mg |
| Magnesium stearate | 0.5 mg |

Preparation Example 4

Tablet

A tablet comprising the following prescription is prepared by a conventional method.

| | |
|---|---|
| Compound 84 | 10 mg |
| Lactose | 30 mg |
| Potato starch | 15 mg |
| Polyvinyl alcohol | 1.5 mg |
| Magnesium stearate | 0.5 mg |

Preparation Example 5

Tablet

A tablet comprising the following prescription is prepared by a conventional method.

| | |
|---|---|
| Compound 89 | 10 mg |
| Lactose | 30 mg |
| Potato starch | 15 mg |
| Polyvinyl alcohol | 1.5 mg |
| Magnesium stearate | 0.5 mg |

Preparation Example 6

Injection

An injection comprising the following prescription is prepared by a conventional method.

| | |
|---|---|
| Compound 69 | 2 mg |
| Purified soybean oil | 200 mg |
| Purified egg-yolk lecithin | 24 mg |
| Injectable glycerin | 50 mg |
| Distilled water for injection | 0.72 mL |

Preparation Example 7

Injection

An injection comprising the following prescription is prepared by a conventional method.

| | |
|---|---|
| Compound 73 | 2 mg |
| Purified soybean oil | 200 mg |
| Purified egg-yolk lecithin | 24 mg |
| Injectable glycerin | 50 mg |
| Distilled water for injection | 0.72 mL |

Preparation Example 8

Injection

An injection comprising the following prescription is prepared by a conventional method.

| | |
|---|---|
| Compound 76 | 2 mg |
| Purified soybean oil | 200 mg |
| Purified egg-yolk lecithin | 24 mg |
| Injectable glycerin | 50 mg |
| Distilled water for injection | 0.72 mL |

Preparation Example 9

Injection

An injection comprising the following prescription is prepared by a conventional method.

| | |
|---|---|
| Compound 84 | 2 mg |
| Purified soybean oil | 200 mg |
| Purified egg-yolk lecithin | 24 mg |
| Injectable glycerin | 50 mg |
| Distilled water for injection | 0.72 mL |

Preparation Example 10

Injection

An injection comprising the following prescription is prepared by a conventional method.

| | |
|---|---|
| Compound 89 | 2 mg |
| Purified soybean oil | 200 mg |
| Purified egg-yolk lecithin | 24 mg |
| Injectable glycerin | 50 mg |
| Distilled water for injection | 0.72 mL |

Industrial Applicability

The present invention provides novel triazolopyrimidine derivatives and pharmaceutically acceptable salts thereof which have adenosine $A_{2A}$ antagonism and are useful for treating and/or preventing a disease (for example, Parkinson's disease, dementia including senile dementia, depression or the like) induced by hyperactivity of adenosine $A_{2A}$ receptors.

## Claims

**1.** A [1,2,4]triazolo[1,5-c]pyrimidine derivative represented by formula (I):

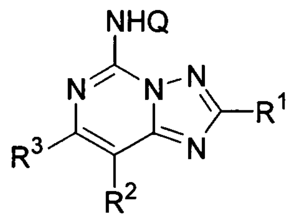

(I)

{wherein
$R^1$ represents substituted or unsubstituted aryl, or a substituted or unsubstituted aromatic heterocyclic group;
$R^2$ represents a hydrogen atom, halogen, lower alkyl, lower alkanoyl, aroyl, substituted or unsubstituted aryl, or a substituted or unsubstituted aromatic heterocyclic group;
$R^3$ represents the following:

1) lower alkyl or hydroxy-substituted lower alkyl;
2) lower cycloalkyl;
3) formyl;
4) substituted or unsubstituted lower alkanoyl;
5) substituted or unsubstituted aroyl;
6) formula ($A^3$)

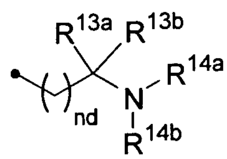

($A^3$)

[wherein
nd represents an integer of 0 to 3;
$R^{13a}$ and $R^{13b}$ may be the same or different and each represent a hydrogen atom, halogen, lower alkyl, lower cycloalkyl, substituted or unsubstituted lower alkanoyl, substituted or unsubstituted aralkyl, substituted or unsubstituted aryl, a substituted or unsubstituted aromatic heterocyclic group, substituted or unsubstituted aroyl, lower alkoxycarbonyl, or lower alkoxy-substituted lower alkyl; $R^{13a}$ and $R^{13b}$ form a lower cycloalkane ring together with the adjacent carbon atom; or $R^{13a}$ and $R^{13b}$ are combined together to represent an oxygen atom or a sulfur atom; and
$R^{14a}$ and $R^{14b}$ may be the same or different and each represent a hydrogen atom, substituted or unsubstituted lower alkyl, lower cycloalkyl, substituted or unsubstituted lower alkanoyl, substituted or unsubstituted aryl, a substituted or unsubstituted aromatic heterocyclic group, substituted or unsubstituted aroyl, lower alkoxycarbonyl, formyl, or formula ($B^1$)

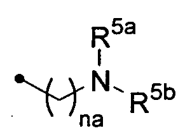

($B^1$)

(wherein
na represents an integer of 2 to 5; and
$R^{5a}$ and $R^{5b}$ may be the same or different and each represent a hydrogen atom, lower alkyl, lower cycloalkyl, substituted or unsubstituted lower alkanoyl, substituted or unsubstituted aralkyl, substituted or unsubstituted aryl, a substituted or unsubstituted aromatic heterocyclic group, substituted or unsubstituted aroyl, lower alkoxycarbonyl, lower alkoxy-substituted lower alkyl, or formyl; or $R^{5a}$ and $R^{5b}$ form a substituted or unsubstituted heterocyclic group together with the adjacent nitrogen atom); or
$R^{14a}$ and $R^{14b}$ form a substituted or unsubstituted heterocyclic group together with the adjacent nitrogen atom];
7) formula ($C^3$)

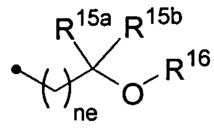

**(C$^3$)**

(wherein

ne, $R^{15a}$ and $R^{15b}$ have the same meanings as the above-described nd, $R^{13a}$ and $R^{13b}$, respectively; and

$R^{16}$ represents a hydrogen atom, lower alkyl, lower cycloalkyl, substituted or unsubstituted lower alkanoyl, substituted or unsubstituted aralkyl, substituted or unsubstituted aryl, a substituted or unsubstituted aromatic heterocyclic group, substituted or unsubstituted aroyl, or lower alkoxy-substituted lower alkyl);

8) formula (E$^1$)

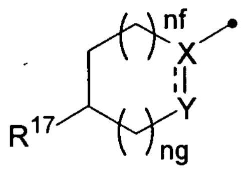

**(E$^1$)**

[wherein

nf represents an integer of 0 to 3;

ng represents an integer of 1 to 4;

X---Y represents $CR^{18}$-$CH_2$ (wherein $R^{18}$ represents a hydrogen atom, hydroxy, halogen, nitro, cyano, trifluoromethyl, lower alkyl, lower alkoxy, lower alkanoyl, or lower alkoxycarbonyl), or C=CH; and

$R^{17}$ represents a hydrogen atom, substituted or unsubstituted lower alkyl, substituted or unsubstituted lower alkanoyl, substituted or unsubstituted lower cycloalkyl, substituted or unsubstituted aryl, a substituted or unsubstituted aromatic heterocyclic group, substituted or unsubstituted aroyl, lower alkoxycarbonyl, or formyl];

9) formula (F$^1$)

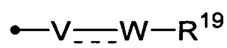

**(F$^1$)**

[wherein

V---W represents $CR^{20}$=$CR^{21}$ (wherein $R^{20}$ and $R^{21}$ may be the same or different and each represent a hydrogen atom, substituted or unsubstituted lower alkyl, substituted or unsubstituted lower alkanoyl, substituted or unsubstituted lower cycloalkyl, substituted or unsubstituted aryl, a substituted or unsubstituted aromatic heterocyclic group, substituted or unsubstituted aroyl, or lower alkoxycarbonyl) or C≡C; and

$R^{19}$ represents a hydrogen atom, substituted or unsubstituted lower alkyl, substituted or unsubstituted lower alkanoyl, substituted or unsubstituted lower cycloalkyl, substituted or unsubstituted aryl, a substituted or unsubstituted aromatic heterocyclic group, substituted or unsubstituted aroyl, lower alkoxycarbonyl, or formula (A$^4$)

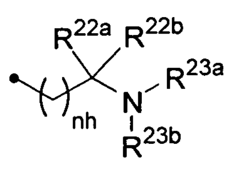

**(A$^4$)**

(wherein

nh, $R^{22a}$, $R^{22b}$, $R^{23a}$ and $R^{23b}$ have the same meanings as the above-described nd, $R^{13a}$, $R^{13b}$, $R^{14a}$ and $R^{14b}$, respectively),

provided that $R^{19}$ is not substituted or unsubstituted aryl when V---W is CH=CH];

10) aryl substituted with a substituent selected from the group consisting of

$-CH_2NHR^{4a}$ [wherein $R^{4a}$ represents substituted or unsubstituted lower alkyl, lower cycloalkyl, substituted or unsubstituted lower alkanoyl, substituted or unsubstituted aryl, a substituted or unsubstituted aromatic heterocyclic group, substituted or unsubstituted aroyl, lower alkoxycarbonyl, formyl, or formula ($B^1$)

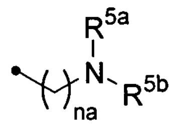

**($B^1$)**

(wherein na, $R^{5a}$ and $R^{5b}$ have the same meanings as defined above, respectively)],

$-(CH_2)_{nb}-C(R^{6a})(R^{6b})(OR^7)$ (wherein nb, $R^{6a}$, $R^{6b}$ and $R^7$ have the same meanings as the above-described nd, $R^{13a}$, $R^{13b}$ and $R^{16}$, respectively), and

$-NR^{8a}R^{8b}$ [wherein $R^{8b}$ and $R^{8b}$ may be the same or different and each represent a hydrogen atom, substituted or unsubstituted lower alkyl, lower cycloalkyl, substituted or unsubstituted lower alkanoyl, substituted or unsubstituted aryl, a substituted or unsubstituted aromatic heterocyclic group, substituted or unsubstituted aroyl, lower alkoxycarbonyl, formyl, or formula ($B^1$)

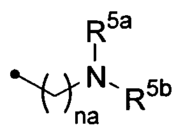

**($B^1$)**

(wherein na, $R^{5a}$ and $R^{5b}$ have the same meanings as defined above, respectively)]; or

11) an aromatic heterocyclic group substituted with a substituent selected from the group consisting of

$-CH_2NR^{4b}R^{4c}$ (wherein $R^{4b}$ and $R^{4c}$ have the same meanings as the above-described $R^{14a}$ and $R^{14b}$, respectively),

$-(CH_2)_{nb}-C(R^{6a})(R^{6b})(OR^7)$ (wherein nb, $R^{6a}$, $R^{6b}$ and $R^7$ have the same meanings as defined above, respectively), and

$-NR^{8a}R^{8b}$ (wherein $R^{8b}$ and $R^{8b}$ have the same meanings as defined above, respectively); and

Q represents a hydrogen atom or 3,4-dimethoxybenzyl},
or a pharmaceutically acceptable salt thereof.

**2.** The [1,2,4]triazolo[1,5-c]pyrimidine derivative according to claim 1, wherein $R^3$ is the following:

1) lower alkyl or hydroxy-substituted lower alkyl;
2) lower cycloalkyl;
3) formyl;
4) substituted or unsubstituted lower alkanoyl;
5) substituted or unsubstituted aroyl;
6) formula ($A^3$)

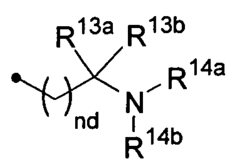

**($A^3$)**

(wherein nd, $R^{13a}$, $R^{13b}$, $R^{14a}$ and $R^{14b}$ have the same meanings as defined above, respectively);

7) formula ($C^3$)

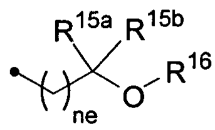

(C$^3$)

(wherein ne, $R^{15a}$, $R^{15b}$ and $R^{16}$ have the same meanings as defined above, respectively);
8) formula ($E^1$)

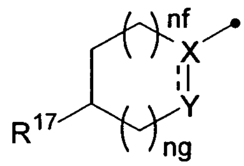

($E^1$)

(wherein nf, ng, X⎓Y and $R^{17}$ have the same meanings as defined above, respectively); or
9) formula ($F^1$)

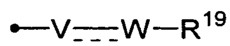

($F^1$)

(wherein V⎓W and $R^{19}$ have the same meanings as defined above, respectively),

or a pharmaceutically acceptable salt thereof.

**3.** The [1,2,4]triazolo[1,5-c]pyrimidine derivative according to claim 1, wherein $R^3$ is aryl substituted with a substituent selected from the group consisting of $-CH_2NHR^{4a}$ (wherein $R^{4a}$ has the same meaning as defined above), $-(CH_2)_{nb}-C(R^{6a})(R^{6b})(OR^7)$ (wherein nb, $R^{6a}$, $R^{6b}$ and $R^7$ have the same meanings as defined above, respectively), and $-NR^{8a}R^{8b}$ (wherein $R^{8b}$ and $R^{8b}$ have the same meanings as defined above, respectively), or a pharmaceutically acceptable salt thereof.

**4.** The [1,2,4]triazolo[1,5-c]pyrimidine derivative according to claim 1, wherein $R^3$ is aryl substituted with-$CH_2NHR^{4a}$ (wherein $R^{4a}$ has the same meaning as defined above), or a pharmaceutically acceptable salt thereof.

**5.** The [1,2,4]triazolo[1,5-c]pyrimidine derivative according to claim 3 or 4, wherein the aryl is phenyl, or a pharmaceutically acceptable salt thereof.

**6.** The [1,2,4]triazolo[1,5-c]pyrimidine derivative according to claim 1, wherein $R^3$ is an aromatic heterocyclic group substituted with a substituent selected from the group consisting of $-CH_2NR^{4b}R^{4c}$ (wherein $R^{4b}$ and $R^{4c}$ have the same meanings as defined above, respectively), $-(CH_2)_{nb}-C(R^{6a})(R^{6b})(OR^7)$ (wherein nb, $R^{6a}$, $R^{6b}$ and $R^7$ have the same meanings as defined above, respectively), and $-NR^{8a}R^{8b}$ (wherein $R^{8b}$ and $R^{8b}$ have the same meanings as defined above, respectively), or a pharmaceutically acceptable salt thereof.

**7.** The [1,2,4]triazolo[1,5-c]pyrimidine derivative according to claim 1, wherein $R^3$ is an aromatic heterocyclic group substituted with $-(CH_2)_{nb}-C(R^{6a})(R^{6b})(OR^7)$ (wherein nb, $R^{6a}$, $R^{6b}$ and $R^7$ have the same meanings as defined above, respectively), or a pharmaceutically acceptable salt thereof.

**8.** The [1,2,4]triazolo[1,5-c]pyrimidine derivative according to claim 1, wherein $R^3$ is an aromatic heterocyclic group substituted with $-NR^{8a}R^{8b}$ (wherein $R^{8b}$ and $R^{8b}$ have the same meanings as defined above, respectively), or a pharmaceutically acceptable salt thereof.

**9.** The [1,2,4]triazolo[1,5-c]pyrimidine derivative according to any one of claims 6 to 8, wherein the aromatic heterocyclic group is pyridyl or thiazolyl, or a pharmaceutically acceptable salt thereof.

**10.** The [1,2,4]triazolo[1,5-c]pyrimidine derivative according to claim 1, wherein $R^3$ is formula ($C^3$)

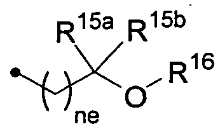

$(C^3)$

(wherein ne, $R^{15a}$, $R^{15b}$ and $R^{16}$ have the same meanings as defined above, respectively), or a pharmaceutically acceptable salt thereof.

**11.** The [1,2,4]triazolo[1,5-c]pyrimidine derivative according to claim 1, wherein $R^3$ is $-CH_2OR^{16}$ (wherein $R^{16}$ has the same meaning as defined above), or a pharmaceutically acceptable salt thereof.

**12.** The [1,2,4]triazolo[1,5-c]pyrimidine derivative according to claim 1, wherein $R^3$ is formula $(E^1)$

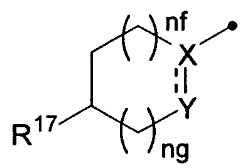

$(E^1)$

(wherein nf, ng, X---- Y and $R^{17}$ have the same meanings as defined above, respectively), or a pharmaceutically acceptable salt thereof.

**13.** The [1,2,4]triazolo[1,5-c]pyrimidine derivative according to claim 12, wherein nf is 1, ng is 1, and X---- Y is C=CH, or a pharmaceutically acceptable salt thereof.

**14.** The [1,2,4]triazolo[1,5-c]pyrimidine derivative according to claim 12 or 13, wherein $R^{17}$ is substituted or unsubstituted lower alkyl, or a pharmaceutically acceptable salt thereof.

**15.** The [1,2,4]triazolo[1,5-c]pyrimidine derivative according to claim 1, wherein $R^3$ is formula $(F^1)$

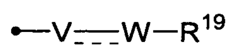

(wherein V---- W and $R^{19}$ have the same meanings as defined above, respectively), or a pharmaceutically acceptable salt thereof.

**16.** The [1,2,4]triazolo[1,5-c]pyrimidine derivative according to claim 1, wherein $R^3$ is formula $(A^3)$

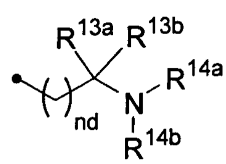

$(A^3)$

(wherein nd, $R^{13a}$, $R^{13b}$, $R^{14a}$ and $R^{14b}$ have the same meanings as defined above, respectively), or a pharmaceutically acceptable salt thereof.

**17.** The [1,2,4]triazolo[1,5-c]pyrimidine derivative according to claim 16, wherein nd is 0, and $R^{13a}$ and $R^{13b}$ are combined together to represent an oxygen atom, or a pharmaceutically acceptable salt thereof.

**18.** The [1,2,4]triazolo[1,5-c]pyrimidine derivative according to claim 16, wherein nd is 0, and $R^{13a}$ and $R^{13b}$ are each a hydrogen atom, or a pharmaceutically acceptable salt thereof.

**19.** The [1,2,4]triazolo[1,5-c]pyrimidine derivative according to any one of claims 16 to 18, wherein $R^{14a}$ and $R^{14b}$ may

be the same or different and are each a hydrogen atom or substituted or unsubstituted lower alkyl, or a pharmaceutically acceptable salt thereof.

**20.** The [1,2,4]triazolo[1,5-c]pyrimidine derivative according to any one of claims 16 to 18, wherein $R^{14a}$ and $R^{14b}$ form a substituted or unsubstituted heterocyclic group together with the adjacent nitrogen atom, or a pharmaceutically acceptable salt thereof.

**21.** The [1,2,4]triazolo[1,5-c]pyrimidine derivative according to claim 1, wherein $R^3$ is formyl, substituted or unsubstituted lower alkanoyl, or substituted or unsubstituted aroyl, or a pharmaceutically acceptable salt thereof.

**22.** The [1,2,4]triazolo[1,5-c]pyrimidine derivative according to any one of claims 1 to 21, wherein Q is a hydrogen atom, or a pharmaceutically acceptable salt thereof.

**23.** The [1,2,4]triazolo[1,5-c]pyrimidine derivative according to any one of claims 1 to 22, wherein $R^1$ is furyl, or a pharmaceutically acceptable salt thereof.

**24.** The [1,2,4]triazolo[1,5-c]pyrimidine derivative according to any one of claims 1 to 23, wherein $R^2$ is a hydrogen atom, or a pharmaceutically acceptable salt thereof.

**25.** A pharmaceutical composition comprising the [1,2,4]triazolo[1,5-c]pyrimidine derivative according to any one of claims 1 to 24 or a pharmaceutically acceptable salt thereof as an active ingredient.

**26.** A therapeutic agent for Parkinson's disease comprising the [1,2,4]triazolo[1,5-c]pyrimidine derivative according to any one of claims 1 to 24 or a pharmaceutically acceptable salt thereof as an active ingredient.

**27.** A therapeutic agent for depression comprising the [1,2,4]triazolo[1,5-c]pyrimidine derivative according to any one of claims 1 to 24 or a pharmaceutically acceptable salt thereof as an active ingredient.

**28.** A therapeutic and/or preventive agent for a disease induced by hyperactivity of an adenosine $A_{2A}$ receptor, comprising the [1,2,4]triazolo[1,5-c]pyrimidine derivative according to any one of claims 1 to 24 or a pharmaceutically acceptable salt thereof as an active ingredient.

**29.** Use of the [1,2,4]triazolo[1,5-c]pyrimidine derivative according to any one of claims 1 to 24 or a pharmaceutically acceptable salt thereof for the manufacture of a therapeutic agent for Parkinson's disease.

**30.** Use of the [1,2,4]triazolo[1,5-c]pyrimidine derivative according to any one of claims 1 to 24 or a pharmaceutically acceptable salt thereof for the manufacture of a therapeutic agent for depression.

**31.** Use of the [1,2,4]triazolo[1,5-c]pyrimidine derivative according to any one of claims 1 to 24 or a pharmaceutically acceptable salt thereof for the manufacture of a therapeutic and/or preventive agent for a disease induced by hyperactivity of an adenosine $A_{2A}$ receptor.

**32.** A therapeutic agent for a disease selected from the group consisting of Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease and intermittent claudications, comprising the [1,2,4]triazolo[1,5-c]pyrimidine derivative according to any one of claims 1 to 24 or a pharmaceutically acceptable salt thereof as an active ingredient.

**33.** Use of the [1,2,4]triazolo[1,5-c]pyrimidine derivative according to any one of claims 1 to 24 or a pharmaceutically acceptable salt thereof for the manufacture of a therapeutic agent for a disease selected from the group consisting of Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease and intermittent claudications.

**34.** A method for treating Parkinson's disease, comprising administering an effective amount of the [1,2,4]triazolo[1,5-c]pyrimidine derivative according to any one of claims 1 to 24 or a pharmaceutically acceptable salt thereof.

**35.** A method for treating depression, comprising administering an effective amount of the [1,2,4]triazolo[1,5-c]pyrimidine derivative according to any one of claims 1 to 24 or a pharmaceutically acceptable salt thereof.

**36.** A method for treating and/or preventing a disease induced by hyperactivity of an adenosine $A_{2A}$ receptor, comprising administering an effective amount of the [1,2,4]triazolo[1,5-c]pyrimidine derivative according to any one of claims 1 to 24 or a pharmaceutically acceptable salt thereof.

**37.** A method for treating a disease selected from the group consisting of Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, sleep disorders, ischemic heart disease and intermittent claudications, comprising administering an effective amount of the [1,2,4]triazolo[1,5-c] pyrimidine derivative according to any one of claims 1 to 24 or a pharmaceutically acceptable salt thereof.

**INTERNATIONAL SEARCH REPORT**

International application No.
PCT/JP03/12158

A. CLASSIFICATION OF SUBJECT MATTER
Int.Cl[7] C07D487/04, A61K31/519, 31/5377, 31/55, 31/553, A61P9/00, 9/10, 21/00, 25/00, 25/14, 25/16, 25/20, 25/24, 25/28, 43/00

According to International Patent Classification (IPC) or to both national classification and IPC

B. FIELDS SEARCHED

Minimum documentation searched (classification system followed by classification symbols)
Int.Cl[7] C07D487/04, A61K31/519, 31/5377, 31/55, 31/553, A61P9/00, 9/10, 21/00, 25/00, 25/14, 25/16, 25/20, 25/24, 25/28, 43/00

Documentation searched other than minimum documentation to the extent that such documents are included in the fields searched

Electronic data base consulted during the international search (name of data base and, where practicable, search terms used)
STN/CAS

C. DOCUMENTS CONSIDERED TO BE RELEVANT

| Category* | Citation of document, with indication, where appropriate, of the relevant passages | Relevant to claim No. |
|---|---|---|
| X<br>A | BROWN, D.J. et al., "Bis-s-triazolo[1, 5-a:1', 5-c] pyrimidine and some simple derivatives", Australian Journal of Chemistry, 33(5), pp1147-52 (1980), compound No. (7) compound | 1-24<br>25-33 |
| X | WO 95/03806 A1 (Kyowa Hakko Kogyo Co., Ltd.), 09 February, 1995 (09.02.95), Claims<br>& AU 7237294 A & CA 2144330 A<br>& EP 666079 A1 & US 5565460 A | 1-33 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 December, 2003 (01.12.03) | 16 December, 2003 (16.12.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/12158

C (Continuation). DOCUMENTS CONSIDERED TO BE RELEVANT

| Category* | Citation of document, with indication, where appropriate, of the relevant passages | Relevant to claim No. |
|---|---|---|
| X | EP 459702 A1 (IMPERIAL CHEMICAL INDUSTRIES PLC.), 04 December, 1991 (04.12.91), & GB 2244487 A & FI 912563 A & CA 2043424 A & NO 912051 A & IE 911818 A & CN 1056879 A & HU 911789 A & CS 9101595 A & ZA 9104094 A & PT 97776 A & OA 9358 A & JP 5-97855 A | 1-33 |
| X | EP 515107 A1 (IMPERIAL CHEMICAL INDUSTRIES PLC.), 25 November, 1992 (25.11.92), & MX 9202442 A & FI 922340 A & CA 2069455 A & NO 922028 A & AU 1709392 A & IE 921655 A & HU 62898 A & CS 91201533 A & ZA 9203767 A & US 5290776 A & NZ 242865 A & JP 5-155887 A | 1-33 |
| X | WO 98/42711 A1 (Kyowa Hakko Kogyo Co., Ltd.), 01 October, 1998 (01.10.98), Claims & EP 976753 A1 & US 6222035 B1 | 1-33 |
| X | WO 00/17201 A1 (Kyowa Hakko Kogyo Co., Ltd.), 30 March, 2000 (30.03.00), Claims & EP 1116722 A1 & CA 2344828 A & AU 5757999 A & NO 20011417 A & BR 9914040 A & HU 103921 A & NZ 510629 A | 1-33 |
| P,X | WO 03/068776 A (Kyowa Hakko Kogyo Co., Ltd.), 21 August, 2003 (21.08.03), Claims (Family: none) | 1-33 |

Form PCT/ISA/210 (continuation of second sheet) (July 1998)

## INTERNATIONAL SEARCH REPORT

International application No.
PCT/JP03/12158

**Box I Observations where certain claims were found unsearchable (Continuation of item 2 of first sheet)**

This international search report has not been established in respect of certain claims under Article 17(2)(a) for the following reasons:

1. [X] Claims Nos.: 34-37
because they relate to subject matter not required to be searched by this Authority, namely:
Claims 34-37 pertain to a method for treatment of the human body.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

In view of $R^3$ in the general formula (I), this international application is considered to involve the following seven inventions: <1> an invention relating to compounds in which $R^3$ in the general formula (I) is defined under "1)"; <2> an invention relating to compounds in which the $R^3$ is defined under "2)" and "8)"; <3> an invention relating to compounds in which the $R^3$ is defined under "3)," "4)," and "5)"; <4> an invention relating to compounds in which the $R^3$ is defined under "6)" and "7)"; <5> an invention relating to compounds in which the $R^3$ is defined under "9)"; <6> an invention relating to compounds in which the $R^3$ is defined under "10)"; and <7> an invention relating to compounds in which the $R^3$ is defined under "11)."

1. [X] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[X] No protest accompanied the payment of additional search fees.

Form PCT/ISA/210 (continuation of first sheet (1)) (July 1998)